# EUROPEAN PATENT SPECIFICATION

(11) **EP 2 630 254 B1**
(45) Date of publication and mention of the grant of the patent: **17.08.2016**
(21) Application number: 11771175.4
(22) Date of filing: 20.10.2011
(51) Int. Cl.: C12Q 1/68, G01N 33/68, G01N 33/574

(54) **USE OF SPECIFIC GENES OR THEIR ENCODED PROTEINS FOR A PROGNOSIS METHOD OF CLASSIFIED LUNG CANCER**
VERWENDUNG SPEZIFISCHER GENE ODER IHRER CODIERTEN PROTEINE FÜR EIN PROGNOSEVERFAHREN FÜR KLASSIFIZIERTEN LUNGENKREBS
UTILISATION DE GÈNES SPÉCIFIQUES OU DES PROTÉINES QU'ILS CODENT DANS UNE MÉTHODE PRONOSTIQUE D'UN CANCER DU POUMON CLASSÉ

(30) Priority: 20.10.2010 EP 10306143
(43) Date of publication of application: 28.08.2013
(73) Proprietor: Université Joseph Fourier, 38041 Grenoble Cedex 09 (FR)
(72) Inventor: PISON-ROUSSEAUX, Sophie, F-38410 Saint Martin D'Uriage (FR); KHOCHBIN, Saadi, F-38240 Meylan (FR)
(74) Representative: Grosset-Fournier, Chantal Catherine
(86) International application number: PCT/EP2011/068377
(87) International publication number: WO 2012/052526

(56) References cited:
- EP-A1- 2 107 127
- WO-A1-2010/065944
- WO-A2-2006/029176
- GURE ALI O ET AL: "Cancer-testis genes are coordinately expressed and are markers of poor outcome in non-small cell lung cancer.", CLINICAL CANCER RESEARCH : AN OFFICIAL JOURNAL OF THE AMERICAN ASSOCIATION FOR CANCER RESEARCH 15 NOV 2005 LNKD- PUBMED:16299236, vol. 11, no. 22, 15 November 2005 (2005-11-15), pages 8055-8062, XP002621272, ISSN: 1078-0432 cited in the application
- CARON C ET AL: "Functional characterization of ATAD2 as a new cancer/testis factor and a predictor of poor prognosis in breast and lung cancers.", ONCOGENE 16 SEP 2010 LNKD- PUBMED:20581866, vol. 29, no. 37, 16 September 2010 (2010-09-16), pages 5171-5181, XP002621273, ISSN: 1476-5594
- SURI ANIL: "CANCER TESTIS ANTIGENS--THEIR IMPORTANCE IN IMMUNOTHERAPY AND IN THE EARLY DETECTION OF CANCER", EXPERT OPINION ON BIOLOGICAL THERAPY, ASHLEY, LONDON, GB, vol. 6, no. 4, 1 April 2006 (2006-04-01), pages 379-389, XP009074181, ISSN: 1471-2598, DOI: DOI:10.1517/14712598.6.4.379
- CHEN YAO-TSENG ET AL: "Identification of cancer/testis-antigen genes by massively parallel signature sequencing", PROCEEDINGS OF THE NATIONAL ACADEMY OF SCIENCES OF THE UNITED STATES (PNAS), NATIONAL ACADEMY OF SCIENCE, US, vol. 102, no. 22, 1 May 2005 (2005-05-01), pages 7940-7945, XP002368175, ISSN: 0027-8424, DOI: DOI:10.1073/PNAS.0502583102

### SEQUENCE LISTING

<110> UNIVERSTITE JOSEPH FOURIER INSERM Pison-Rousseaux, Sophie Khochbin, Saadi
<120> USE OF SPECIFIC GENES OR THEIR ENCODED PROTEINS FOR A PROGNOSIS METHOD OF CLASSIFIED LUNG CANCER
<130> WOB 10 BF UJF GLUN
<150> EP 10306143.8
   <151> 2010-10-20
<160> 69
<170> PatentIn version 3.5
<210> 1
   <211> 240
   <212> DNA
   <213> Homo Sapiens
<400> 1
   atgtgttgca actactacgg caactcctgt ggctatggct ccggctgtgg ctgtggctat      60
   ggctctggct ctggctgtgg ctgtggctat ggaactggct atggctgtgg gtatggctgt     120
   gggtttggct cccattatgg ctgtggttat ggaactggct atggctgtgg atatggctct     180
   ggctctggct actgtggcta ccggccattt tgctttagaa gatgctattc ttcctgctaa     240
<210> 2
   <211> 1405
   <212> DNA
   <213> Homo Sapiens
<400> 2
   atcttaagag gcgttccttt ttgcatagtt cccatgagca tgagagaaga agcaatgcac      60
   gctccggcag attcctagga accaaatacc tctgaggagc accagatttc agcttatggg     120
   atgctttgat tgctctgtgg ctgcatttag gagaaggaag ctgcagtcat gcgtcatcac     180
   tgccagcctc acatctcttg acagttaaag ccttagggtg gagcaaggga aaatttaaaa     240
   taacaaatga agcaaaagca agaggtgatg ttccaaagca gaggaaggct aagtttatat     300
   atacaaatgt caagtgtgta tagtgcaaaa ctaggaccag ttggtggaat ctgtggtcaa     360
   aaacaaaagc cttccttttt ttttttcaag gcccagtccc aagacgcaag accacttgcg      420
   ccagcagcgt gcatcagcaa gatagcaaaa gcaggacgag agctgcccgg aagacatcta     480
   cctggccaga agacacctac cctggccgga agacatgtac ccctgaagat agagaaagag     540
   gccatcgtgt actacgtagc agtcatgtca gactgggaca cttcctgttt acagaggact     600
   ataaaacccc tgtcctgtcc tcacttgggg ctgacgccat cttaggcctc agcccgcctg     660
   cagccaggcg ttcgttaaaa cagcatgttg ctccacaccg ccttgtattg tttgttggtc     720
   ccactctctg ggctcgaacc aatacaagca cctttcaagc agtatattct tcagtgtctt     780
   gatcctccaa ataactctct tctaattcct cctgaccaca aaaagcactt atactctagg     840
   atgactgatt ccagcccagt ggcctggcaa gggtgaatta caccttgcat atcacactct     900
   tgacatttgt gtgcgctagc ataagaatta taattgaaac agggatttaa gtatctcctc     960
   tctaggtgcc taccctcctt ggactcaggt caaatttatt aaaggaagtt ttgtttctag    1020
   ataggttgtt tgaaataaaa taacagaatg ttcaagtaac acagtgtacc tacagctttt    1080
   aacaaaattg aggacttggg tctcgaaaca atttcctttg attttcaggt attttatcta    1140
   taaaaaggga gataaagcat tagttcatag gacagttata tgtttaaatg tgataatgta    1200
   tattaaccac cttgcatgta ttcaaatgtg ttttgaaatc taacgtctac attttgatag    1260
   tttaactctt ctacataagt gacttacaac aggcattaaa tattgtttgg cattttcata    1320
   tatctgtaac tgtatcttaa tctacaatga gcttaatttt aagtgtagca taaaacagaa    1380
   ccttcaataa agtggtaata ttagg                                          1405
<210> 3
   <211> 857
   <212> DNA
   <213> Homo Sapiens
<400> 3
   gcttctccca gctggagtag gtgggggagg ccagacatgg aggcccttcc tccagtcaga      60
   tccagccttt tggggatcct gttgcaggtt acgaggctct cagtgctgtt ggttcagaac     120
   cgagatcacc tctataattt cctgctcctc aagatcaacc tcttcaacca ctgggtgtca     180
   gggctggccc aggaggcccg ggggtcctgt aactggcagg cccacctacc cctgggagct      240
   gcagcctgcc ccctgggcca ggctctctgg gctgggctgg ctctgataca ggtccccgta     300
   tggctggtgc tacagggacc caggctgatg tgggctggca tgtggggcag caccaagggc     360
   ctgggcctgg ccttgctcag tgcctgggag cagctgggcc tgtctgtggc catctggaca     420
   gatctgtttt tgtcatgtct gcacggcctg atgttggtgg ccttgctctt ggtggtagtg     480
   acctggaggg tgtgtcagaa gtcccactgc ttccgactgg gcaggcagct cagtaaggcc     540
   ttgcaagtga actgcgtggt aaggaagctc ctggtacagc tgagacgtct gtattggtgg     600
   gtggagacta tgactgccct cacctcctgg cacctggcct atctcatcac ctggaccacc     660
   tgcctggcct cccacctgct gcaggctgcc tttgagcaca cgacccagct ggccgaggcc     720
   caggaggttg aaccccagga ggtctcaggg tcttccttgc tgccctcact gtctgcgtcc     780
   tcggactcag agtctggaac agttttgcca gagcaagaaa ctcccagaga ataaatgtat     840
   ccccatctgc ctctaaa                                                     857
<210> 4
   <211> 2521
   <212> DNA
   <213> Homo Sapiens
<400> 4
   aaacgagtgg agacacgagg accagcgcga gcggtcccgg tgggctaccc tccccctgcg 60
   acgacccccc ctcgctctga ccgactggtc ccctaaacgg tggcggcggt ttttggtcgt 120
   tgggccccgg gatttaggac caacatttga agacccgaag gggaactgca accatgaatg 180
   aagaaaatat agatggaaca aatggatgca gtaaagttcg aactggtatt cagaatgaag 240
   cagcattact tgctttgatg gaaaagactg gttacaacat ggttcaggaa aatggacaaa 300
   ggaaatttgg cggtcctcct ccaggttggg aaggtccacc tccacctaga ggctgtgaag 360
   tttttgtagg aaaaatacct cgtgatatgt atgaagatga gttagttcct gtatttgaaa 420
   gagctgggaa gatatatgaa tttcgactta tgatggaatt tagtggtgaa aatcgaggtt 480
   atgcttttgt gatgtacact acaaaagaag aagcccaatt agccatcaga attcttaata 540
   attatgaaat tcgaccaggg aagtttattg gtgtgtgtgt aagcctggat aattgtagat 600
   tatttattgg agctattccc aaggaaaaga agaaagaaga aattttagat gaaatgaaga 660
   aagttacaga aggagttgta gatgtcattg tttatccaag tgcaactgat aagaccaaaa 720
   atcgtggttt tgcatttgtg gaatatgaat ctcacagagc tgctgctatg gcaaggagga 780
   aactaattcc aggaacattc caactatggg gccacaccat tcaggtagat tgggctgacc 840
   cagagaaaga ggtggatgag gaaaccatgc agagagttaa agttctttat gtaagaaatt 900
   taatgatctc aactacagag gaaacaatta aagcagaatt caataaattt aagcctggtg 960
   cagttgaacg ggtaaagaaa cttagagatt atgcttttgt tcactttttc aaccgagaag 1020
   atgcagtggc tgccatgtct gttatgaatg gaaaatgcat tgatggagca agtattgagg 1080
   taacactagc taaaccagta aataaagaaa acacttggag acagcatctt aatggtcaga 1140
   ttagtccaaa ttctgaaaat ctgattgtgt ttgctaacaa agaagagagc cacccaaaaa 1200
   ctctaggcaa gctgccaact cttcctgctc gtctcaatgg tcagcatagc ccaagtccgc 1260
   ctgaagttga aagatgcact tacccttttt atcctggaac aaagcttact ccaattagta 1320
   tgtattcttt aaaatccaat cattttaatt ctgcagtaat gcatttggat tattactgca 1380
   acaaaaataa ctgggcacca ccagaatatt atttatattc aacaacaagt caagatggga 1440
   aagtactctt ggtgtataag atagttattc ctgctattgc aaatggatcc cagagttact 1500
   tcatgccaga caaactctgt actacgttag aagatgcaaa ggaactggca gcccagttta 1560
   cattacttca tttggactac aatttccatc gcagctcaat aaatagtctt tcccctgtta 1620
   gtgctaccct ctcttctggg actcccagcg tgcttcctta tacttcaagg ccttattctt 1680
   atccaggcta tcctttgtca ccaacaatat cacttgctaa tggcagccat gttggacagc 1740
   ggctatgtat ctccaatcag gcctccttct tctgaagaaa atactaacat tagtatgaaa 1800
   atttgtgtaa atttgtagta tgaaaacttg caaattaaaa tattgtttta ttttagaatc 1860
   gggtttgcat atttggtttt aaaaaggtat ttattccaaa gtactaaaca tcagctataa 1920
   ttcagaataa catggagttg tagaatttat aaaaatgcaa agtttaaaaa gttattcagt     1980
   ggtttctctt gataaaggta cagcaaacta ctattctttt taaacttcta ggattttctt     2040
   ctactttctg agtgggcaat agaacctagt catttatgtt tttttttttt tttgcataat     2100
   tttactaaat agtatttcac aaatattaaa gcacttgaag acaatggtta tagtagattt     2160
   gattaccaag gatcactatc tgtactggag attagaacaa ttatatgacc agaagcatct     2220
   aaccattatg taaaaagaaa tgatgagaca aaaagattaa gatacaaatt ttgtgcagta     2280
   ctaaagaaaa agcagtctac cattgtggtc cttgaaaata actatagata tttttgttat     2340
   ttgttagaca caaattataa ttttgttgtt aatgtattta agcattttat agttatgctt     2400
   tgtgtttttg atattctttg tattgttaat aacaagtgtt atgggttttt aatgttgaaa     2460
   tcatgtgtta atttttgtac ttgaattcaa attttttgac attaaatatg tgatgcttct     2520
   a                                                                     2521
<210> 5
   <211> 3305
   <212> DNA
   <213> Homo Sapiens
<400> 5
   cacaggcagg ccgtacctcg aaatcccagc ggggacacct gtcagggaga cgcaccctca      60
   caccgcccat gcatgcccga cgcatctttg ggatccttag ggatcacgtg gtgcttcctt     120
   gaatctccgc ttgaggtttc ttcaggcagg ttcggccttg cccgactcct aggatcgcag     180
   gaccatggcg acgatcccgc ggagagggga cgcactgcca cagacgcctg ggggccaagc     240
   cgctggggcc agtcccccgg caacggtggc ggttactgtg acgcgagtcc cccctcagcg     300
   ctagcgcctg gtgatcgcgc ctgggctctt cctgcgtctc caagctcagg ggctcctgct     360
   tctcagcact gctgtctgga aaaggcggga acgaggacta aggcaagccc agtgtggggc     420
   agagatggca acacctggaa ctagcttttc atgggggcag ataatgtgga gagtgtccca     480
   attacatctc cctttattct atcccatact actaaacccc accagctatt tgcctacaaa     540
   ctgtgtatca gtcaggacat caggacacca agataaagga ctagagtttg gtaatcaact     600
   actggctcct ccagcagctc agaatgtggc ctgtatgcat cacttcactt aattcattca     660
   agaaatgctg actgagttcc tgctgtgtgt tggacacttt cttagaagtg ggacacactg     720
   cagtgtgtgg gacaaaggct gggccgctca ttgcttatgt tctagttgaa gagagagtca     780
   atcaacaaca ttctactttc agacattgat aaatgctgtc atgaaaagac aaggaacatg     840
   aaaaggtaag aaaagggatg gagagagatg ggatgagggg tgcctgggca gacaatgcaa     900
   tgatctggca aaagagtagg acaatcatga gactgcatgt gttccagttt ctgctgctgt     960
   aaaacagcat ccacaggtta catctttact attctgttac atacatacta ttatttaaat    1020
   aaaaaatagt actagttaac tgtgaaaatt gcaattaata gggcagatta aacaaatcct    1080
   ttaaatttaa acaaatgttt tatggttttt tatcctctga tacattgttt caactttaaa 1140
   aaaccctctt tatttgatat ttaaactcta tattaatgta tattcttaag atacataaat 1200
   ttaagatttt tttcgagaca gggtctcatt ctgtcacaca gtctggagtg ctgtggtgca 1260
   attatggttc actgcagact ccacctctcc aggctcagat aatcctcctg cctcagactc 1320
   ctgagtatct gggaccacag gtgtgcatca ccacacccag ctaatttctt gtattttttt 1380
   gttgggatgg ggttttgcga tgttgcccag gctggtctca aactcctggg ctcaagtgat 1440
   ctgcctgcct tggtgtctca aagtgttggg atcacaggcg tgagccacca catccagcct 1500
   aatttaagat ttttaaaaag cagaatgtaa atatggtcat gcattgtgta attctgtgga 1560
   tactttctga gtaatgcatg gttaggtgac ttcatcatta tgtgaacatt atagagtgta 1620
   catacacaaa cctagatgat gtagcctgat ggtacagcct atcacttcga ggctacaaat 1680
   ccaaattgcc tggatactgt aaacaattat actcaatggt atttgtgtat ctaaacatgt 1740
   ctaaacatag aaagggtaca gtaaaaatat ggtataaaag ataagaaatc gtataccggt 1800
   atacagcact taccgtggat ggagcttgca agactggaag ttactccggg tgagtcagtg 1860
   actgagcggt gagtgaatgt gaaggctagg gcagtactgt atactactgt agactttata 1920
   tgtactgtat gcttaggcta cactaaattt atttgaattt ttttgtcggt aataaattaa 1980
   ccttagctta ctataatttc tttataaacc ttttaatttt taaaaatgtt ttgactctct 2040
   tgtaataaat tagcttcaaa cacattgtat agctgtacac aaatattttc tttatatcct 2100
   tattctataa tcttctactt aaaaattttt ttaatttttt tattttaatt taattttatt 2160
   ttattttgag acagagtctc actctctcac ccaggctgga gtgcagtggc gtgatcttaa 2220
   ctcactgcaa cctccgcttc ccaggtaata ttatttaaat aaaaatagta ctagttaact 2280
   gtgaaaattg caattaatag ggcagatcaa gcaattctct tgcctcagcc tccagacttt 2340
   tttgttaaaa actaagacac aaacacatta gcctaggtac acacagtgcc aggatcatca 2400
   aaatgtcatt aggcagtata aacttttcag ctccataaaa gttggaaaaa aaataataat 2460
   aataaataca attttataaa aagaaaattt tcagctctgc tataatctta tgggaccact 2520
   actgtatatg tggtccctca ttgaccaaaa catcattatg tggcacatga ttataacaaa 2580
   tgatggatgg cgaggttgtg aagaaactgg aacccttgtg cattactggt tgtaatataa 2640
   aaaggtacag ccactgtaaa aaaaaaaaca cagtttggtt atttcttcat aagttaatca 2700
   tagaattacc atataatcca gcaatttcac ttctaggtat atgcccaaaa taattgaata 2760
   aggtgtttaa acataaactt gtacaagaat gttcatagca gtacatttca caatactcag 2820
   ataatcctct cattggaagc aactcaaatg tccaccaact gatggtatta cccatgcaat 2880
   ggaatattat tcagtcataa aaaggaatga agtattgagg catgttacaa catgaaggaa 2940
   ctttgaggac agtttgttaa tacgctatat actgtgtaat tctatttata tgaaatatcc 3000
   agaataggta aatcattaga gactagagac agcagattag tggatgccag gagtttgggg 3060
   aaggaagggg tgggaagaat gggaagtgac tgcttaatgg atacagggtt tctttttggg 3120
   gtgatgaaaa atttctggaa ctagattgta gtgatggttg cacaacaccg taagtgtaat 3180
   aatgtcactg aattatacac tttaaagtga tttaaatggt aaattgtatt atttatgtat 3240
   tttaccacaa taaagaagtc acctatcaaa aaaaaaaaaa aaaaagaaaa aaaaaaaaaa 3300
   aaaaa 3305
<210> 6
   <211> 2499
   <212> DNA
   <213> Homo Sapiens
<400> 6
   cttctaattc tgttattgca actgcagacc gttacctggt acgctggctg ctacctccct      60
   cactcttgtc agagtcggag ctacaggcag tgccttcagc tctgagctca ggcatcccgg     120
   tccctgtttt tgcggttaag gactctaaag tgttgtgtcg tgttcatcaa ctttttctca     180
   acttccctgg ctctacctct tctgccacaa acgtcagcat ggtggtatct gccgaccctt     240
   tgtccagcga gagggcagag atgaacatcc tagaaatcaa ccaggaattg cgctcgcagc     300
   tggcagagag caatcagcag ttccgagacc tcaaagagaa attccttata actcaagcta     360
   ctgcctactc cctggccaac cagctgaaga aatacaagtg tgaagagtac aaagacatca     420
   tagactctgt gctgagggat gaactgcagt ccatggagaa gctggcagag aagctcaggc     480
   aagctgagga gctcaggcag tataaagccc tggttcactc tcaggcaaaa gagctgaccc     540
   agttacggga gaagttacgg gaagggagag atgcctcccg ctggctgaac aagcatctga     600
   aaaccctcct cactcctgat gaccctgaca agtcccaggg tcaggacctc cgagagcagc     660
   tggctgaggg gcacaggctg gcagagcacc ttgttcacaa gctgagccca gaaaatgatg     720
   aagatgaaga tgaggatgaa gacgacaaag acgaggaggt tgagaaagta caggaatcac     780
   ctgcccccag agaggtgcag aagactgaag aaaaggaagt ccctcaggac tcactggagg     840
   aatgtgctgt cacttgttca aatagtcaca acccttctaa ctccaaccag cctcacagga     900
   gcaccaaaat cacatttaag gaacacgaag tcgactctgc tctggttgta gagagtgaac     960
   accctcatga tgaagaggag gaagctctaa acattccccc agaaaatcaa aatgaccatg    1020
   aggaggagga ggggaaagcg ccagtgcccc ccagacacca tgacaagtcc aactcttacc    1080
   ggcatcgtga agtctctttc ttggcattgg atgaacagaa agtttgctcc gctcaggatg    1140
   ttgccaggga ttactccaat cccaaatggg atgaaacctc acttggcttc ctcgaaaagc    1200
   aaagtgatct tgaagaggtg aaaggacaag aaacagttgc tcccaggctc agcaggggac    1260
   cgctgagagt ggacaagcat gaaatccccc aggagtcact ggatggatgt tgcttgactc    1320
   cttccatcct tcctgacctg actccctcct accaccctta ttggagcact ttgtactctt    1380
   ttgaagacaa gcaagtcagc ttggctcttg tagacaaaat taaaaaggat caagaggaga 1440
   tagaagacca aagcccacca tgccccaggc tcagccagga gctgccagag gtgaaggagc 1500
   aggaagtccc agaggactct gtgaatgaag tttacttgac tccctcagtt caccatgacg 1560
   tgtctgactg ccaccagcct tatagcagca ccttgtcctc attggaggat cagcttgcct 1620
   gctctgctct ggatgtagcc tcccccaccg aggcggcctg tccccaaggg acttggagtg 1680
   gagacttgag ccaccaccag tcagaggtgc aagtttcaca ggcacagctg gaaccaagca 1740
   ccctggtgcc cagttgtctg cgactacagc tggatcaagg gttccactgt gggaacggct 1800
   tggcccagcg gggcctttcc tccaccacct gcagcttctc agccaatgct gattctggga 1860
   accaatggcc cttccaagag ctggttttag agccctctct ggggatgaag aaccctcccc 1920
   agctggaaga tgatgcactt gaaggctcag caagcaacac acaagggcgt caagtcactg 1980
   gccggattcg tgcctccctt gtcctgatac tgaagaccat cagaagaaga ctcccgttca 2040
   gcaagtggag actggcattc agattcgctg gcccgcatgc tgagagcgca gagataccaa 2100
   atactgctgg aaggacgcaa aggatggcag gatgaaagaa tgtcacaaaa agcagctttt 2160
   ccacttgata aaaacaacta aaacagcaaa gcaagtttaa gtccaaacac aatactgcag 2220
   gggtccttca ctgaggattg aatttcagac acagaatact cttgatgact tcaagccact 2280
   atgctccttt gatttgagaa gccacattcc atccccctcc aattgtgatc aatacctagg 2340
   gagaccaatg cccagatgga caaatagcat tgaccggcgt tagccctgtt tctcaattcc 2400
   catcgtgtag agaacaggag tccgcagctg ctggcaggag acagcatgtc agccgggact 2460
   ctgccagggc agagtatgag caatgccatg ttcttgctg 2499
<210> 7
   <211> 6093
   <212> DNA
   <213> Homo Sapiens
<400> 7
   acgccgccaa ctggcggggg tgcgggggag acaataattt gttccgcggt aataagaacg       60
   gtgactgctg gccgtggatc catttcacag gcctgccttc tctcactaac gctcttccta      120
   gtccccgggc caactcggac agtttgctca tttattgcaa cggtcaaggc tggcttgtgc      180
   cagaacggcg cgcgcgcgcg cacgcacgca cacacacggg gggaaacttt tttaaaaatg      240
   aaaggctaga agagctcagc ggcggcgcgg gcgctgcgcg agggctccgg agctgactcg      300
   ccgaggcagg aaatccctcc ggtcgcgacg cccggccccg gctcggcgcc cgcgtgggat      360
   ggtgcagcgc tcgccgccgg gcccgagagc tgctgcactg aaggccggcg acgatggcag      420
   cgcgcccgct gcccgtgtcc cccgcccgcg ccctcctgct cgccctggcc ggtgctctgc      480
   tcgcgccctg cgaggcccga ggggtgagct tatggaacca aggaagagct gatgaagttg      540
   tcagtgcctc tgttgggagt ggggacctct ggatcccagt gaagagcttc gactccaaga      600
   atcatccaga agtgctgaat attcgactac aacgggaaag caaagaactg atcataaatc 660
   tggaaagaaa tgaaggtctc attgccagca gtttcacgga aacccactat ctgcaagacg 720
   gtactgatgt ctccctcgct cgaaattaca cggtaattct gggtcactgt tactaccatg 780
   gacatgtacg gggatattct gattcagcag tcagtctcag cacgtgttct ggtctcaggg 840
   gacttattgt gtttgaaaat gaaagctatg tcttagaacc aatgaaaagt gcaaccaaca 900
   gatacaaact cttcccagcg aagaagctga aaagcgtccg gggatcatgt ggatcacatc 960
   acaacacacc aaacctcgct gcaaagaatg tgtttccacc accctctcag acatgggcaa 1020
   gaaggcataa aagagagacc ctcaaggcaa ctaagtatgt ggagctggtg atcgtggcag 1080
   acaaccgaga gtttcagagg caaggaaaag atctggaaaa agttaagcag cgattaatag 1140
   agattgctaa tcacgttgac aagttttaca gaccactgaa cattcggatc gtgttggtag 1200
   gcgtggaagt gtggaatgac atggacaaat gctctgtaag tcaggaccca ttcaccagcc 1260
   tccatgaatt tctggactgg aggaagatga agcttctacc tcgcaaatcc catgacaatg 1320
   cgcagcttgt cagtggggtt tatttccaag ggaccaccat cggcatggcc ccaatcatga 1380
   gcatgtgcac ggcagaccag tctgggggaa ttgtcatgga ccattcagac aatccccttg 1440
   gtgcagccgt gaccctggca catgagctgg gccacaattt cgggatgaat catgacacac 1500
   tggacagggg ctgtagctgt caaatggcgg ttgagaaagg aggctgcatc atgaacgctt 1560
   ccaccgggta cccatttccc atggtgttca gcagttgcag caggaaggac ttggagacca 1620
   gcctggagaa aggaatgggg gtgtgcctgt ttaacctgcc ggaagtcagg gagtctttcg 1680
   ggggccagaa gtgtgggaac agatttgtgg aagaaggaga ggagtgtgac tgtggggagc 1740
   cagaggaatg tatgaatcgc tgctgcaatg ccaccacctg taccctgaag ccggacgctg 1800
   tgtgcgcaca tgggctgtgc tgtgaagact gccagctgaa gcctgcagga acagcgtgca 1860
   gggactccag caactcctgt gacctcccag agttctgcac aggggccagc cctcactgcc 1920
   cagccaacgt gtacctgcac gatgggcact catgtcagga tgtggacggc tactgctaca 1980
   atggcatctg ccagactcac gagcagcagt gtgtcacgct ctggggacca ggtgctaaac 2040
   ctgcccctgg gatctgcttt gagagagtca attctgcagg tgatccttat ggcaactgtg 2100
   gcaaagtctc gaagagttcc tttgccaaat gcgagatgag agatgctaaa tgtggaaaaa 2160
   tccagtgtca aggaggtgcc agccggccag tcattggtac caatgccgtt tccatagaaa 2220
   caaacatccc cctgcagcaa ggaggccgga ttctgtgccg ggggacccac gtgtacttgg 2280
   gcgatgacat gccggaccca gggcttgtgc ttgcaggcac aaagtgtgca gatggaaaaa 2340
   tctgcctgaa tcgtcaatgt caaaatatta gtgtctttgg ggttcacgag tgtgcaatgc 2400
   agtgccacgg cagaggggtg tgcaacaaca ggaagaactg ccactgcgag gcccactggg 2460
   cacctccctt ctgtgacaag tttggctttg gaggaagcac agacagcggc cccatccggc 2520
   aagcagataa ccaaggttta accataggaa ttctggtgac catcctgtgt cttcttgctg 2580
   ccggatttgt ggtttatctc aaaaggaaga ccttgatacg actgctgttt acaaataaga 2640
   agaccaccat tgaaaaacta aggtgtgtgc gcccttcccg gccaccccgt ggcttccaac 2700
   cctgtcaggc tcacctcggc caccttggaa aaggcctgat gaggaagccg ccagattcct 2760
   acccaccgaa ggacaatccc aggagattgc tgcagtgtca gaatgttgac atcagcagac 2820
   ccctcaacgg cctgaatgtc cctcagcccc agtcaactca gcgagtgctt cctcccctcc 2880
   accgggctcc acgtgcacct agcgtccctg ccagacccct gccagccaag cctgcactta 2940
   ggcaggccca ggggacctgt aagccaaacc cccctcagaa gcctctgcct gcagatcctc 3000
   tggccagaac aactcggctc actcatgcct tggccaggac cccaggacaa tgggagactg 3060
   ggctccgcct ggcacccctc agacctgctc cacaatatcc acaccaagtg cccagatcca 3120
   cccacaccgc ctatattaag tgagaagccg acaccttttt tcaacagtga agacagaagt 3180
   ttgcactatc tttcagctcc agttggagtt ttttgtacca acttttagga ttttttttaa 3240
   tgtttaaaac atcattacta taagaacttt gagctactgc cgtcagtgct gtgctgtgct 3300
   atggtgctct gtctacttgc tcaggtactt gtaaattatt aatttatgca gaatgttgat 3360
   tacagtgcag tgcgctgtag taggcatttt taccatcact gagttttcca tggcaggaag 3420
   gcttgttgtg cttttagtat tttagtgaac ttgaaatatc ctgcttgatg ggattctgga 3480
   caggatgtgt ttgctttctg atcaaggcct tattggaaag cagtccccca actaccccca 3540
   gctgtgctta tggtaccaga tgcagctcaa gagatcccaa gtagaatctc agttgatttt 3600
   ctggattccc catctcaggc cagagccaag ggcttcaggg tccaggctgt gtttggcttt 3660
   cagggaggcc ctgtgcccct tgacaactgg caggcaggct cccagggaca cctgggagaa 3720
   atctggcttc tggccaggaa gctttggtga gaacctgggt tgcagacagg aatcttaagg 3780
   tgtagccaca ccaggataga gactggaaca ctagacaagc cagaacttga ccctgagctg 3840
   accagccgtg agcatgtttg gaaggggtct gtagtgtcac tcaaggcggt gcttgataga 3900
   aatgccaagc acttcttttt ctcgctgtcc tttctagagc actgccacca gtaggttatt 3960
   tagcttggga aaggtggtgt ttctgtaaga aacctactgc ccaggcactg caaaccgcca 4020
   cctccctata ctgcttggag ctgagcaaat caccacaaac tgtaatacaa tgatcctgta 4080
   ttcagacaga tgaggctttc catgggacca caactatttt cagatgtgaa ccattaacca 4140
   gatctagtca atcaagtctg tttactgcaa ggttcaactt attaacaatt aggcagactc 4200
   tttatgcttg caaaaactac aaccaatgga atgtgatgtt catgggtata gttcatgtct 4260
   gctatcatta ttcgtagata ttggacaaag aaccttctct atggggcatc ctctttttcc 4320
   aacttggctg caggaatctt taaaagatgc ttttaacaga gtctgaacct atttcttaaa 4380
   cacttgcaac ctacctgttg agcatcacag aatgtgataa ggaaatcaac ttgcttatca 4440
   acttcctaaa tattatgaga tgctggcttg ggcagcatcc ccttgaactc ttcactcttc 4500
   aaatgcctga ctagggagcc atgtttcaca aggtctttaa agtgactaat ggcatgagaa 4560
   atacaaaaat actcagataa ggtaaaatgc catgatgcct ctgtcttctg gactggtttt 4620
   cacattagaa gacaattgac aacagttaca taattcactc tgagtgtttt atgagaaagc 4680
   cttcttttgg gggtcaacag ttttcctatg ctttgaaaca gaaaaatatg taccaagaat 4740
   cttggtttgc cttccagaaa acaaaactgc atttcacttt cccggtgttc cccactgtat 4800
   ctaggcaaca tagtattcat gactatggat aaactaaaca cgtgacacaa acacacacaa 4860
   aagggaaccc agctctaata cattccaact cgtatagcat gcatctgttt attctatagt 4920
   tattaagttc tttaaaatgt aaagccatgc tggaaaataa tactgctgag atacatacag 4980
   aattactgta actgattaca cttggtaatt gtactaaagc caaacatata tatactatta 5040
   aaaaggttta cagaatttta tggtgcatta cgtgggcatt gtctttttag atgcccaaat 5100
   ccttagatct ggcatgttag cccttcctcc aattataaga ggatatgaac tgagtttttc 5160
   ttttgttgtt tgttcttagc tgtaattcct atgcttctat ttcagagagc caggagagtt 5220
   tgatattaaa ggaggttaaa actgtgatct tatgccatgt catcaatggc cacttagggg 5280
   ccatggctga tgacacattc ttatctctac agtactaatg tgttattata gagccatgca 5340
   ttttatttct gaataagaac atatttaaac taatattccc ttacaatatg gacagtatta 5400
   atccttccaa gatgcagtat ttatcaagtg aagcatattt agcagcaaat tccattttaa 5460
   cataacttag gaaccaataa ccagggtgtt ttgtggttgg gggaggcacg gggtggagta 5520
   ttctttttta tatcctcaaa acaaaaaaaa tcaatactta tatttcaatg gcaatctagt 5580
   atttttttaa aagactgtat aggcatgaat aatagaggtg gtttgagttt tgtagggcca 5640
   tcacctggaa agtcaatgtg actagacaca aagtagccca gaggctactt ttcttcctac 5700
   agcttattat agttgtaggt tctatgacct cacttcatgg gttccaggca attccgctga 5760
   aaggtttgtc tcctgaaatt ttttaagttt gttttcctga cacatgtaat cagatgtgta 5820
   gcaaccgagg gaaacgaagc ctaacattct ccattgtgga aatacacaca ggaggttaca 5880
   tttcacagcg tggatttttc cagcttacac atgtgggatg acatcacaga aaccacaaaa 5940
   gcagcaaatt aaactgtagg agagtcaata ctcctgacga gtctcggggg gggggcattt 6000
   ttatgccttc ttaactttat gagaattctc aggctgaact ataggccatt gttcccaggc 6060
   aaatcaatac atcaatgcat cctcaaaaaa aaa 6093
<210> 8
   <211> 2714
   <212> DNA
   <213> Homo Sapiens
<400> 8
   ggggctggca ttcagtgagg ctgggggaga agcttcctaa aagcattgcc attgaaaagc 60
   agggcatacc tatctgatac tgaggatgta gaatgcagtt tggagatgct tcctagctac 120
   ctaatcttcc aaggcaaacc atgcactctt catcctgata tttgtgatgc ctccctgggc      180
   tacatgacag agggtgcact gcaaaggatg tgaaggaaag tcaccacagg gcaaaggtgg      240
   aaacccaggg gcctttcccc agtgagactt tctggctgtt cctttggaac ttccacctgc      300
   tagttgcaga tgtttgccac tggatggggc cccatgctac gtgggaactg ttcaggactg      360
   tgggtactta gttccagcag agctggctcc accagggcac caggactgca ggcttctcaa      420
   ctcctggcct ggagaggcag cttcagcaac cagtgcctgg gaatccacta gccctttctc      480
   tgtgagtcac gtgagggcag aatctgctca aagtcagagg gctggagcaa cctggattgt      540
   gccagagtga gggcccagct cactggaggt cagagtaaga gagtcaagga tgggaagaaa      600
   cagggttttg ctcagtcttg cagatactga ggcactcagg aggctcagga tggcagcaac      660
   agggcatgtg ctaaacttac cctagcctct tccttttagg cagccaccac agaggacatc      720
   tgtttgcctc tgtaccctac cccatctcca tgctggtaga tatagtctgg ggataccttt      780
   aaaacaaaca aacaaacaaa caaaacagga tctgtctatc acctgtccaa atcctgaagc      840
   aaaatgagcc ttctttatgg ccaccttgga aaggcctgag tccccagcat cacctgggat      900
   acaaagtgtt ctgaggtggg ggcagagtgc cctcctgccc agatgccaca ctccacaata      960
   tctcttcctg gcagacaggt ttctcttaac ttcctttggc aaatttggtt tcacttcctt     1020
   ttgcaacttt agaacctttc tcaggtcagt cataaggtag tggggaagcc aggatttgaa     1080
   cccattctgt ctgattccag agctcatgca ttaaccacct gactgtggtg cctccccatc     1140
   atatgtgcat caagtatgat catttttgta gttctcagag tacttagcac catgcacatc     1200
   tgtaataagt acagccactg cctactggga ggtctaaagg ttcaactcat aagggaccca     1260
   caggccacac tgggctccag tctccttaaa agatgtggga cggagatcaa caattgtcag     1320
   cagaacccca tcaggcattt gtcttctcac tgagccttgc agcaaaccac acagctgtct     1380
   ataacctatc tgttcccaga ttcaagaaca caagctgctc atcagttggg tgcaggatcc     1440
   actctagtac aaaagctgtg tgtctcaagg cataattcct tatgattact tcatagctct     1500
   gcttttgtct tgagacaaaa gaaatttcct tcaattccta cttttgctga gatattgttt     1560
   tttgaaaagc aattaaatct tgtcaatcac ttttcccacc tctttgagat acttacatgg     1620
   cttttcttct ttattctctt agtgtggtaa attacattga tttattttaa atatgaaacc     1680
   agccttgaat tccaggaaca aactccattt ggacatgcta gaacaacctt ttatatttgt     1740
   ttagatttga tttcctgaag tggtgttaaa aggttttcat atctatgtct gtgagggata     1800
   ttaatctgta cattttttta ttactatttt ctggtgttat ggtaatgcta gtctcataga     1860
   atgaattagg aaaagttgcc tccttctgta ttttctgaga gtttgtgtgg cactggtaaa     1920
   atattttctg tatatgtttg acataattca ccaggaaaat tatttgtacc tacaagttcc     1980
   ctcctggaaa gatgttgata atcacttatt atcctttcat tgtctgccag atctgtggca     2040
   ttgctttttt ctctggtatt agtaatttaa attttccctt atttcttgat caattttggc     2100
   agagatttat caattttatt aatctctttt aaaaatctgt attgagcttt gttaattttc 2160
   tctattacat actttttatt tctactctta cgattgctat ttctttattt ctccttcgga 2220
   tttagcccat ttttctattt gtctaatttt aggatttaaa attcaatcat tatttttaga 2280
   tatttttatt tttaaatata aacatgaaag ttgcaatatt accttgggca ctgttttgac 2340
   tgcattccac aagtttttat ttgtcatgtt ttaatcacca ttcagttcaa aatactttct 2400
   aatgctcctt gttatttctt ctttgaccca ttggttatat aaaagtacgt tttgctttat 2460
   ttctaaatat gtgaagactt tctagatatc tcagttattt attactaatt taattttatt 2520
   gtagccagag aaaatacact gtatgtgttc aatctttgga aatttcttga gacttatttt 2580
   ttgacccagt atgtttgccc tcatggacat tccatattca ctagaaaaag aatgtgtgtt 2640
   caaataatgt tggatatagt gttatgcaaa tgccactaaa tcaagataaa tcttttgcaa 2700
   aaaaaaaaaa aaaa 2714
<210> 9
   <211> 3280
   <212> DNA
   <213> Homo Sapiens
<400> 9
   gggggaggga tgactaaaga caacggctgt aagagaactc cacaagagag tcagaacgaa      60
   aatgtgcaac aagtgcggcg gctcctacca tggcaggtga ttcgaggact cagcacgagg     120
   cgagagtagg gaccaggaag agccggaaaa cccgcctgtg attggccgtc cacgggtatc     180
   ggtcgttgtg attgggtgag gcccagacag acagctgcgt tttgaaccgc gtagggttct     240
   gggtagcaaa ggccttgcaa ggctcttaac cgaaaggggg agggggaagg tcgccaacaa     300
   acggctgagc tcacaatcct ggccggggcg tccccctccc ccatggagag agccagaccc     360
   gagccgccgc ctcagccgcg cccgttgcgt cccgctccgc ccccgctgcc ggtcgagggc     420
   acctcctttt gggcagcagc catggagccc cctccgtcgt ctcccacact gagcgcggca     480
   gccagtgcga ccttggcctc gtcgtgcggg gaggcagtgg cgtccggctt acagcccgcg     540
   gtgcggcggc tgctgcaggt gaagccagag caggtgttgc tgctaccaca gcctcaggcc     600
   cagaacgagg aagccgctgc ctcgtccgcg caggcgcggc tgttgcagtt caggcccgac     660
   ctgcggctcc tgcagccgcc gacagcgtca gacggcgcca cctccaggcc cgagttgcac     720
   ccggtgcagc ccctggcgct gcatgtcaag gccaagaagc agaagctggg gcccagcctg     780
   gatcagtcag tggggcctcg aggggccgtc gaaaccggtc ctagagcctc cagggtggtc     840
   aagttggaag gccccgggcc ggccctcggc tacttccgag gggacgagaa gggcaagctg     900
   gaggcggagg aggtcatgag agactcgatg caaggcgggg caggcaaaag cccggcagcc     960
   atccgagaag gtgtgatcaa aacggaggaa cccgagagac tcctcgagga ctgcaggctc    1020
   ggcgcggagc ccgcgtccaa tggcctggtt catggcagcg cggaggtcat cttggcccca    1080
   acgtccggtg cctttgggcc gcaccagcaa gaccttagga tccctttgac gctccacacg 1140
   gtcccccctg gggcccggat ccagtttcag ggagctccgc cttcagagct gataagattg 1200
   accaaggtcc ccctgacacc agtgcctact aaaatgcagt ccctactgga gccttctgta 1260
   aaaattgaaa ccaaagatgt cccgctcacc gtgttgccct cagatgcagg cataccagat 1320
   actcccttca gtaaggacag aaatggtcat gtgaagcgac ccatgaacgc atttatggtt 1380
   tgggcaagga tccaccgacc agcactagcc aaagctaacc cagcagccaa caatgcagaa 1440
   atcagtgtcc agcttgggtt agagtggaac aaacttagtg aagaacaaaa gaaaccctat 1500
   tacgatgaag cacaaaagat taaggaaaag cacagagagg aatttcctgg ttgggtttat 1560
   cagcctcgtc cagggaagcg aaaacgattc cctctaagtg tttccaatgt attttctggt 1620
   accacacaga atatcatctc tacaaatcct acaacagttt atccttaccg ctcacctacg 1680
   tactctgtgg taattcccag cctacagaat cccatcactc atccagttgg tgaaacctca 1740
   cctgctatcc agctgcccac acctgcagtc cagagcccaa gccctgtcac acttttccag 1800
   cccagcgtct ccagtgctgc tcaggtggct gtccaggatc caagtctacc tgtctatcca 1860
   gcactcccac cccaacgctt tactgggcct tcccaaacag acactcatca gctgcattct 1920
   gaagccactc acactgtgaa gcaacccact cctgtctctc tagagagcgc caacaggatt 1980
   tcaagtagtg caagtactgc ccatgccaga tttgcaactt cgaccatcca acctcctagg 2040
   gagtattcca gcgtttcccc ttgtcccaga agtgctccaa tcccccaggc ttctcccatt 2100
   ccacacccac atgtctacca gccccctccc cttggccatc cagccacact gttcgggaca 2160
   ccaccaagat tctcttttca tcacccttac ttcctacccg gacctcacta cttcccatca 2220
   agtacatgcc cttacagtcg gcctcccttt ggctatggaa attttccgag ttcaatgcca 2280
   gaatgcctta gttattatga agacaggtac ccaaaacatg agggtatctt ttcaacttta 2340
   aatagagact attcttttag agactactca agtgaatgca cacacagtga aaattctcgg 2400
   agttgtgaga acatgaatgg aacttcttac tataacagtc atagccacag tggggaagaa 2460
   aacttaaacc ctgtgcctca gctggacatt ggaaccttgg agaatgtctt cacagccccg 2520
   acatcaactc cttctagcat ccagcaagtc aatgtcaccg acagtgatga ggaggaagaa 2580
   gaaaaagtgc tcagggattt ataattttaa aacaaatatg cacagaaaat aaacatttct 2640
   taaaatatat tctgggtcag ttggtatgag aaaaaaaaaa gcctagaatt ctttgttgaa 2700
   agttttcagt cgtgatttga ggagttaaaa ccaaatgcaa tttatgtctt cataaaattt 2760
   tgattagtga aactagagtc tggatgtttc attgtaggaa tatttaagtt attaagtagt 2820
   ttaattttaa tggctgaaat ttgcatcaac atgtattatt attactttat cctggaacat 2880
   gcaaaatact gaagcctcac agttgtatgt gaggggaaag gggaaataaa tctagcatag 2940
   tgtgattttt attttatctc aggatacatt ttttaaatga ttttttgttt gctttttatg 3000
   taatacttat ggatgttgtc aatttttgat gtaacatttt gaaagtattt tgacaactcc     3060
   tagtgaactt ggacttggtt gctaaattta acttacacta ataaccaatt ataagttcca     3120
   aatgtgtttt aatggcacct gggtgattct tcagctaaat ttagtcattt ctgtttctaa     3180
   atatttttat cattttaaaa tatttttttt ccatttggca tacatcgttc tttgttgtaa     3240
   ttaaataaac atagataaaa ttgttaaaaa aaaaaaaaaa                            3280
<210> 10
   <211> 2919
   <212> DNA
   <213> Homo Sapiens
<400> 10
   agttgagatt cagaccttga cagttgtaac cgatagactg ggctggggca acgctgatga 60
   aaggcaaatt cctgagagaa aaatcaccca gactttctag aagttgttat gagtggacct 120
   gactacctag agagagtaat acagaagctc cgtgccgatc acagctacct cattaccatg 180
   ttcctccaag atggccgccc ctgaggagaa tcatcaactg ggctttacgg aagagagaaa 240
   tccaaacgac catcagcaac aaatgaaaga ctgaagttga aaggaaatcc ataatttttg 300
   aatgctggcc gaggtctgtg acctctgact gaatggtttc caagctccga attcctaggt 360
   gatgttttca tttgttcaca cttgctgaag aagggaaagg ctctctgatt tacgtgtaag 420
   caaaacaatc taaacagtag gataaaatca tgttggaatg gagttctgcg tggatgctcc 480
   tttgaaccaa atcagttcca caactgaccc tgtcagggac atataaattt agcattttgg 540
   tgtggataca gaagacgaga ttaacacaga tcaagtaaca tggaaataaa aaaggagaca 600
   atgctggatg atacaggggc agccaacaag aggagaaaac ccaagcattg gcaaatgcct 660
   cattatccaa agaacactga ggtgtggagc aaaactgtgt tgaatgaact gtagatggaa 720
   attctctcct tcccaccgag gagccgctgc aggttgcaga ctcccagaat gccacactga 780
   gaagaggagg gctaaaattc tagagcagaa tccctggggc agcccctgct gaagaagggt 840
   gcatctgttg agaatctcat caccgccccc accccgccca gccttcaatt tagaggaatt 900
   tctagttcac gcagaatgaa gacaagatca cataagtgag gacgacgccc catttacaag 960
   tgtgatttgg aagactgaag tggaaacctt gggaaggggc ctcagagtat gtcagctggg 1020
   gtcagacggg aacaggagtc tggctcaaca cacatttatt gcactcctgt ttcatgccaa 1080
   gaaatgtacc aaaatctggg actgtgtaac acagcaaagg aaaaggctca tctccaggaa 1140
   gcccccttgt ctaggaagag aaattgaata tcagcataag gtggtgaaaa gctgtgaagg 1200
   agatgagtgc caggagtcca tggaggaagg attctagcct ggacaggtga ggaatggctt 1260
   tctggaagaa gcagatgccc cagctgaact ttaaaatgcc catgtgaaat gaactgtctg 1320
   tatcttcaat cagcaaatgg agtaggtggc cactgtttaa ttctggaatg gggaagtgta 1380
   aaggtgtggt gtgtgtgttt acccctactt agggattcaa aacagacagt aaagaacaaa 1440
   agtctagccc tttgagcatt ctccatggga catttgaggt ctagctggcc ccagagaaag 1500
   agagacagaa gcaaggtcat atccatttga gaaaatcaaa gtgtctgctc caattgagtc 1560
   tggtgtcctt attcctggaa tagccacccc tactgctctg gtgctgattt acctccggca 1620
   gttcagtgct tcctggaagg tgattgtggt gaatagtcat gagaatctgg gaactggctc 1680
   ctgctctgtc tatcttggag atgcatcttt tgtgagaagc cagggcaaga atagggaggc 1740
   ttaggcaatg ggaagtttct gagtagataa agcagatggc agagcaaagg gacagagcct 1800
   ggcgagaggc tactccctgt agcaacctgc ctaagagtga gaggaaagca aggcatggct 1860
   atgagcacgt gtagtctcag ctacttggga agctgaggct gggggatcac ttgagcccag 1920
   gagttcaaga ccagcctagg caacatagtg agaccatgtc tctttaaaaa aaaaaaaaaa 1980
   agaaaaaggg agagagagaa aaaaaggtga caattccccc aacacacata aagtacataa 2040
   gagttaacaa ggacacagtg gcagaaaata gtcagaactg agcaagcatc agtgaggaaa 2100
   tgttttcctg aggagacttg agaagtctca aaaatgaaaa gaaagagtat ttggtgtaga 2160
   aagaatggca tgagatgtga aagctcattc atccatttat tccaggtata tctttttctt 2220
   cagtactttg ggaggcaata ggtgtaatag caagaacatt caggttagtg ggttagtgtg 2280
   agtctgccat atgtatgagg gttgaataca cattaggtgt ggtgactgtc atatgggagg 2340
   cactgcaagg tgaaggagac atgtcctcac atttggtctt tgggaagcga agtaggcaga 2400
   aacccaagtg caaagtggcc atttgttttc ctaatgctag ataagtacct gatgtgttgg 2460
   aagggtggag tagaactaga aactcctgga agttctttta agaacgaatg caggacctca 2520
   tcagcccgag tctttaaata agtcacacat ttttggtcac cacattctgg aagggggaca 2580
   aaaagatctg tcacagacaa aatttggaga tatttaaggc tttgttgact ttggcaaatg 2640
   cttgattata gttctgtggt ctgtttgttt tctagagtct ccatgaattg caagggaaaa 2700
   caagtggtac actggtattt ctatttgata acctgttctc tttggaattc cggttagaaa 2760
   tcaaaggctt acttgagttt taatgctttc gggacttcaa atgcttttct atcttcctaa 2820
   taatatatta acattttctg aagacaacat atatgtggaa aacaacatta tttactcaaa 2880
   taattttttt tgaaaaatgg accaaaaaaa aaaaaaaaa 2919
<210> 11
   <211> 1865
   <212> DNA
   <213> Homo Sapiens
<400> 11
   ggtgcgcatg cgcccggaga gctgaagggc gcgcggtgcc gacagggagc ttccttggct      60
   ggcatggcgg cgagcgcgct gcgaggcctg ccagtggctg gcggaggcga gagtagcgag     120
   agcgaggatg atggctggga gattgggtat ctcgaccgga cgtctcagaa attgaaaagg     180
   ctattaccca ttgaagaaaa gaaagaaaaa tttaagaaag caatgaccat cggagatgtt     240
   tcattggtcc aggagctcct agattctggc attagtgtag attccaactt tcagtatgga      300
   tggactcccc ttatgtatgc tgctagtgtt gccaatgcag agctggttcg ggtccttttg      360
   gacagaggtg ctaatgcaag ctttgagaag gataagcaaa gtattttgat aactgcatgt      420
   tctgctcatg gctcagagga acagatcttg aagtgtgtag aactactact ttcaagaaat      480
   gctgatccaa atgttgcttg taggagactt atgaccccaa tcatgtatgc tgctcgagat      540
   ggtcacaccc aggttgttgc tctccttgtt gctcatggag cagaagttaa tacccaggat      600
   gagaatggtt acactgcttt aacgtgggca gcacgtcagg gtcataaaaa tatagttttg      660
   aagttgcttg aacttggagc taataaaatg ctacaaacca aagatggaaa gatgccaagt      720
   gagattgcaa aaagaaacaa acatcatgag atcttcaact tactttcttt tactttaaat      780
   ccattggaag gaaaacttca acagctaact aaagaagaca ctatttgtaa aatattgaca      840
   acagattctg atagagaaaa agatcacatt tttagttcat atacagcatt tggagatctg      900
   gaagtatttt tacatggtct tggacttgaa catatgacag atttactaaa ggaaagggat      960
   ataacgttaa gacatctttt gaccatgagg gaagatgaat ttacaaagaa tggaattacc     1020
   agtaaagacc agcagaaaat tctggctgct cttaaagaac tacaggtaga agagatacaa     1080
   tttggagagc tatctgaaga gacaaagttg gaaatcagtg gtgatgagtt cctcaacttt     1140
   cttctcaaat taaataaaca gtgtggccat ttaataacag ctgtacagaa tgttattact     1200
   gagttacctg taaattctca aaagataaca ctggaatggg cttctcccca gaattttact     1260
   tcagtttgtg aagaattggt taataatgtt gaagatttga gtgaaaaggt ctgcaaacta     1320
   aaagacctaa ttcaaaagtt gcaaaatgaa cgggaaaatg atccaactca tatacaatta     1380
   agggaagaag tatctacatg gaatagtaga attttgaaga ggacagctat taccatatgc     1440
   ggattcggtt ttcttctttt catttgcaag ctaactttcc agaggaaata atccaaatat     1500
   ttgttttaag ttatatatac acatcttatt gaaccatcca aaaatcattg cttttaactg     1560
   tttgcagcac aatataactt tacattcttt gccaatattc cataggaaaa attttgacaa     1620
   tgtggagttt ttttttaaaa agtggtttgt tactttgact tataaataca atttgttatg     1680
   tttatgttaa ttgtgattag gtagtaaagt ataagtaatt ttttggtttg atcgtatcta     1740
   aatcacattc aaaattaaag ttatatctaa tatctttgac attttttaaa gtgtgttcat     1800
   ttgtcagtga atcgtacagg atttgtaaag tgttcataaa ttgagctttc ttttgtttct     1860
   ttttt                                                                  1865
<210> 12
   <211> 2195
   <212> DNA
   <213> Homo Sapiens
<400> 12
   ggctgcaggc ggggccggcg ggggcgcagc ggcccgggag gaagcagtac cttgaagaga 60
   aattggagag ggagtcaatt cctaggatag cagagagatg gacaacagac agaatagatg      120
   gagtttcaca atggtggcca tgtgtctgga attggtgggt tcttggtctc actgacttca      180
   agaatgaagc cgcacaccct cgcagtcacc ccagctctga tctttgccat cacagttgct      240
   acaatcggct ctttccagtt tggctacaac actggggtca tcaatgctcc tgagacgatc      300
   ataaaggaat ttatcaataa aactttgacg gacaaggcaa atgcccctcc ctctgaggtg      360
   ctgctcacga atctctggtc cttgtctgtg gccatatttt ccgtcggggg tatgatcggc      420
   tccttttccg tcggactctt tgttaaccgc tttggcaggc gcaattcaat gctgattgtc      480
   aacctgttgg ctgccactgg tggctgcctt atgggactgt gtaaaatagc tgagtcagtt      540
   gaaatgctga tcctgggccg cttggttatt ggcctcttct gcggactctg cacaggtttt      600
   gtgcccatgt acattggaga gatctcgcct actgccctga ggggtgcctt tggcactctc      660
   aaccagctgg gcatagttat tggaattctg gtggcccaga tctttggtct ggaactcatc      720
   cttgggtctg aagagctatg gccggtgcta ttaggcttta ccatccttcc agctatcctg      780
   caaagtgcag cccttccatg ttgccctgaa agtcccagat ttttgctcat taacagaaaa      840
   aaagaggaga atgctacgcg gatcctccag cggttgtggg gcacccagga tgtatcccaa      900
   gacatccagg agatgaaaga tgagagtgca aggatgtcac aagaaaagca agtcaccgtg      960
   ctggagctct ttagagtgtc cagctaccga cagcccatca tcatttccat tgtgctccag     1020
   ctctctcagc agctctctgg gatcaatgct gtgttctatt actcaacagg aatcttcaag     1080
   gatgcaggtg ttcaacagcc catctatgcc accatcagcg cgggtgtggt taatactatc     1140
   ttcactttac tttctctatt tctggtggaa agggcaggaa gaaggactct gcatatgata     1200
   ggccttggag ggatggcttt ttgttccacg ctcatgactg tttctttgtt attaaagaat     1260
   cactataatg ggatgagctt tgtctgtatt ggggctatct tggtctttgt ggcctgtttt     1320
   gaaattggac caggccccat tccctggttt attgtggccg aactcttcag ccagggcccc     1380
   cgcccagctg cgatggcagt ggccggctgc tccaactgga cctccaactt cctagtcgga     1440
   ttgctcttcc cctctgctgc ttactattta ggagcctacg tttttattat cttcaccggc     1500
   ttcctcatta ccttcttggc ctttaccttc ttcaaagtcc ctgagacccg tggcaggact     1560
   tttgaggata tcacacgggc ctttgaaggg caggcacacg gtgcagatag atctgggaag     1620
   gacggcgtca tggggatgaa cagcatcgag cctgctaagg agaccaccac caatgtctaa     1680
   gtcatgcctc cttccacctc cctcccggca tgggaaagcc acctctccct caacaaggga     1740
   gagactttat caggatgaac ccaggactgc ttctgaatgc tgctacttga tttctttctc     1800
   atcccacgca ctccatgagc accccaaggc tgcagtttgt tggatcttca atggcttttt     1860
   aaattttatt tcctggacat cctcttctgc ttaggagaga ccgagtgaac ctaccttcat     1920
   ttcaggaggg attggccgct tggcacatga caactttgcc agcttttcct cccttgggtt     1980
   ctgatattgc cgcactagag gatataggag aggaaaagta aggtgcagtt gccccaacct     2040
   cagacttacc aggaagcaga tacatatgag tgtggaagcc ggagggtgtt tatgtaagag     2100
   caccttcctc acttccatac agctctacgc ggcaaattaa cttgagtttt atttatctta     2160
   tcctctggtt taattacata aatatttatt tttta                                2195
<210> 13
   <211> 531
   <212> DNA
   <213> Homo Sapiens
<400> 13
   acgagcactg gagcttgcgt tacttggcct cacctcacct gtgctgtcca cgcctggctt       60
   tgtctcacct gacgcgatat gcctctcctg cgtgggcgct gtcctgcccg ccgccactac      120
   cgccgcttgg ccctgctcgg cctgcagccc gctccccgct tcgcccactc ggggcccccg      180
   cgccagcggc ccctgtctgc cgcggaaatg gctgttggac ttgtggtgtt ttttacgacc      240
   ttcttaacac cagctgcata tgtgctaggc aacctgaagc agttcagaag gaattagatg      300
   gaagatgatg ttgaacagct gttaacgtcc aaaaaacttt cagaaaaagc tgtgtttttg      360
   ttaacgagca aaattgccta gttgagttga tgcaaccatt gtggtattca ctttcctcat      420
   gtttatgatg aatattttgc acttttttag tactgtgcat tatatagatg tatagtcaaa      480
   aatgttctgc ttaagtgtta aataaaacgg aaacacttat tcgtgcttgg t               531
<210> 14
   <211> 2837
   <212> DNA
   <213> Homo Sapiens
<400> 14
   agtctcgcgg gaagctccgt tgtgggcgcc ccggctggtg gctgagctca ggccttcagg 60
   cagaggggag gcgagggcgg ggcggtcacg tgagagcact gccgcggtgg gttgtggggg 120
   tgctgcggcg ccgtttgctt tgccaaaccg acaaaagaga gatgatggcc aacgacgcca 180
   agcccgacgt gaagaccgtg caggtgctgc gggacacagc caaccgcctg cggatccatt 240
   ccatcagggc cacgtgtgcc tctggttctg gccagctcac gtcgtgctgc agtgcagcgg 300
   aggtcgtgtc tgtcctcttc ttccacacga tgaagtataa acagacagac ccagaacacc 360
   cggacaacga ccggttcatc ctctccaggg gacatgctgc tcctatcctc tatgctgctt 420
   gggtggaggt gggtgacatc agtgaatctg acttgctgaa cctgaggaaa cttcacagcg 480
   acttggagag acaccctacc ccccgattgc cgtttgttga cgtggcaaca gggtccctag 540
   gtcagggatt aggtactgca tgtggaatgg cttatactgg caagtacctt gacaaggcca 600
   gctaccgggt gttctgcctt atgggagatg gcgaatcctc agaaggctct gtgtgggagg 660
   cttttgcttt tgcctcccac tacaacttgg acaatctcgt ggcggtcttc gacgtgaacc 720
   gcttgggaca aagtggccct gcaccccttg agcatggcgc agacatctac cagaattgct 780
   gtgaagcctt tggatggaat acttacttag tggatggcca tgatgtggag gccttgtgcc 840
   aagcattttg gcaagcaagt caagtgaaga acaagcctac tgctatagtt gccaagacct 900
   tcaaaggtcg gggtattcca aatattgagg atgcagaaaa ttggcatgga aagccagtgc 960
   caaaagaaag agcagatgca attgtcaaat taattgagag tcagatacag accaatgaga 1020
   atctcatacc aaaatcgcct gtggaagact cacctcaaat aagcatcaca gatataaaaa 1080
   tgacctcccc acctgcttac aaagttggtg acaagatagc tactcagaaa acatatggtt 1140
   tggctctggc taaactgggc cgtgcaaatg aaagagttat tgttctgagt ggtgacacga 1200
   tgaactccac cttttctgag atattcagga aagaacaccc tgagcgtttc atagagtgta 1260
   ttattgctga acaaaacatg gtaagtgtgg cactaggctg tgctacacgt ggtcgaacca 1320
   ttgcttttgc tggtgctttt gctgcctttt ttactagagc attcgatcag ctccgaatgg 1380
   gagccatttc tcaagccaat atcaacctta ttggttccca ctgtggggta tccactggag 1440
   aagatggagt ctcccagatg gccctggagg atctagccat gttccgaagc attcccaatt 1500
   gtactgtttt ctatccaagt gatgccatct cgacagagca tgctatttat ctagccgcca 1560
   ataccaaggg aatgtgcttc attcgaacca gccaaccaga aactgcagtt atttataccc 1620
   cacaagaaaa ttttgagatt ggccaggcca aggtggtccg ccacggtgtc aatgataaag 1680
   tcacagtaat tggagctgga gttactctcc atgaagcctt agaagctgct gaccatcttt 1740
   ctcaacaagg tatttctgtc cgtgtcatcg acccatttac cattaaaccc ctggatgccg 1800
   ccaccatcat ctccagtgca aaagccacag gcggccgagt tatcacagtg gaggatcact 1860
   acagggaagg tggcattgga gaagctgttt gtgcagctgt ctccagggag cctgatatcc 1920
   ttgttcatca actggcagtg tcaggagtgc ctcaacgtgg gaaaactagt gaattgctgg 1980
   atatgtttgg aatcagtacc agacacatta tagcagccgt aacacttact ttaatgaagt 2040
   aaactaggct tatttctaaa aagtcaagtc tattggcttt ggcccaaaag cactggtatc 2100
   tttgtattaa attcatgttt attgtcacaa aaccattatt tatacctata cagttgtact 2160
   gtttctttta aagcaaagcc atttaacatc tttcttcatt cctaatttgg aaattaaagt 2220
   ttacctttct gttaatctat gtataaatgt tactctgagt tattaatgtg gattttaaaa 2280
   ttgtaagcaa tagaatagga aataaaacaa ctacctaata caaatatttc tgataagact 2340
   acaaatatct gactgagctg gggattaaag tagaggtaac tgtatcttaa atgagtatga 2400
   tttccttgta agttaaaaaa attgaaattt aattgtagac ttcaatagtc caagttttga 2460
   aggatgtttg agcttttgta taatgccatt tatacctgca gttttacaga taatgtttga 2520
   ctgcagttgc cttggaaatt cctccaaagt ttgccttcat ctctcctcta cagtttggag 2580
   gtgatggtgc agcagtggaa catctcttga tgcaccacac tacttgtgtt ctgtgaagtg 2640
   atgaaagtat aactggttct agtttgcaca ctacacacat agttttgtga agcttcagaa 2700
   atgttttttc ttttccttgt ggccaaacca gtttgttaat ctgattatat tcatctgcta 2760
   atgatactaa agttaatgta ataaagcatt taaaaatcag aaaaaaaaaa aaaaaaaaaa 2820
   aaaaaaaaaa aaaaaaa 2837
<210> 15
   <211> 3848
   <212> DNA
   <213> Homo Sapiens
<400> 15
   agcgttgctg ctgccttgca gtttgatctc agactgctgt gctagcaatc agcgagactc 60
   cgtgggcgta ggaccctcca agccaggtga aagagcttat gatcctaagc acttccataa 120
   tagggtcagt agaatcatga tcgatgatca taatgtcccc actctacggg agatggtagc 180
   attctccaag gaagtgttgg agtggatggc tcaagattct gaaaacatcg tagtgattca 240
   ctgtaaagga ggcaaagaat agatatgttg gatattttgc acaagtgaaa catagctaca 300
   actggaatct ccctccaaga aaaacactgt ttataaaaag attcgttatt tattcgattc 360
   atggtgttgg aacaggcgat ggatatgatc taaaagtcca aatagtaatg aagaaaaaga 420
   ttgtcttttc ctgtacttcc ttaaagaatt gtcgggtatt tcatgacact gaaacagaca 480
   gggtaataac tgatgtgttc aactgtccac ctctgtatga tgatgtgaaa gtgcaagctt 540
   cctcttcaag agaagagggc agcacacctc gcagggctaa ctggaagggg gagccatcca 600
   ggagacctgt gctcaactga tgggtgggga gcaatagcga gaacgaggga gggacctgag 660
   agtggaagcc tttattggga tgtaaggtgt tacctgagca ggtttcctac ggggaggtct 720
   aactggtgga tttaatgcaa gcagtcatga gttccatgga gtcatgctgt gactgagagg 780
   tggtcattga tatatccaca tggtccatgc agagtacggg ggtctgtagg gaggttatat 840
   ctagctgtcc cataatgaag tagtcaccaa cagaaggttg tataaggcag atactgggat 900
   cagtcacatt gagaaacctg gaggaggtga actggaaact gtcaagggtg actgaaccct 960
   gcttctgata tcagaaagtc caatttatat ttgaaaggga tgctgaggca caaaaaaatt 1020
   gtaagaattc actacaaaaa tacttggcta tatataagca taggtcctta gtagattctg 1080
   tttagcacta tctaaaccag attcaaattt cagcatttaa attaaatatc tatcatggaa 1140
   aataaactat tccttgaaaa ttttggtaga aacagcaaga gaaagcaata gcattttctt 1200
   aagcctcctc ctctgtgtct tgagtgtgtt attatagaat gcagagtgct acctattgaa 1260
   tggttataat tatttgataa atatataaag gaataaagga aggaactttg atttctttgg 1320
   aatgatagtt cttggcatca attttacttt taaaatattt tttttctttt ttaggatttt 1380
   cctaaatact atcacaacta cccttttttc ttctggttta acacatcttt aatacaaaat 1440
   aacgggtatg gatataatat caacccatag aaacaaccta atcttcaatg tctatgtata 1500
   agatgtaatg gcaagtcttt tgctggttgt cataagctta atttatagaa aacaaaaaat 1560
   ccttgagcca ccattgttca ttgccttact ccttttacgt tggctatttt aaaaatacag 1620
   ttgttcttga gacccccagt tgcagtatcc tcaaggtcca tgccatagga ctgtgttatg 1680
   agctcaaaag tattataatc agatcttaag tgtggaagta aattcctccc agagaagttc 1740
   aatatgaatc tgctcagtac cttcaacatg tcaggtcctc agtaggtgct gatttaccaa 1800
   tgacgaacca ccaccaaatt ttgtgctaaa gtaagggagg acctagggaa gcttcagcta 1860
   gctgaaaagc tgactgacac acttatatct aggagaagtt acaagacaca gtaagtatta 1920
   agaaatacag ctaaaaaatc attaaaattg gtagtctccc atttaaacat gggtttctaa 1980
   taactgaatt gggaaaactt tcttaaaaac tattaattgg aggctgggtg tggtggctca 2040
   tgcctgtaat cctagcactt tgggaggctg aggcgggcgg atcacctaag gttggaagtt 2100
   cgagactagc ctggccaaca tggtaaaact ccgtctctac taaaaataca aaaatcagcc 2160
   aggcgtggtg gcacatgcct gtaatcccag ctactcagga ggctgagcca gtagaatcgc 2220
   ttgaacccag gaggcagatt gcagtgagcc gagatcgcac cactacactc cagcctgggc 2280
   gacagagtga gactctgtct aaagaaaaaa gcaaaaaaac agaacaacta ttacttggat 2340
   ttggagatta ttgttcccag aaaaccttct gccatatttg gaaacttatt tctcagtcta 2400
   gaagttctcc actttaagta gcatttgttc tgtgctggtg aaaaactgag atttttttgt 2460
   attaaccata ctcttcaata caaaaggaga aaatattttt aaaatgcttc aggtcacagt 2520
   tgaggcagtt gctatgattg catgtggcat gaattggtag ttattgttac aaccagttct 2580
   agtcttttct tcaaatctga gctggatcta ataactcctt aagtccagca aggcaacagt 2640
   aaattaaacc tctggtctac acacttgcaa tacatacaca tttaatagat tttgatagag 2700
   tgaactttgg attggatgga aattttttaa aaatttgttt cttggatgca tacaaacaat 2760
   aagctttgac tcctaacatg agcaaagtcc ctcaattgtg agagctgggt ggagcttcat 2820
   ttgttgctgc tcctcaaatt gattcttggt aaaggataca gatttttcct ttgaaacacc 2880
   atgttcattt tggggaagca ataagttaga tcacctttat tttcactttt atataaattt 2940
   ctaaagattt ctgtaatatt taaatttata tactattggt aaagctgttt ttcttagttg 3000
   tgaaattgtt gtttagccaa aaatgccaac ttctgtcttt tagaacacta ggcataaatg 3060
   ggttaaccaa tttatgccta gtgttccatt attggaatgc taagcatgtg ggatttattt 3120
   atatcctact gctcaaggtc atcgccaagg gctgtttgca aaaattcaaa aaattgcaac 3180
   ctcaggcata aattaaaaga gatatagtat tttattattg ggttttgata catgtctaat 3240
   cagactgatt tctgtcacat atagaaattt agatactgta ttaaacctgg atgtcattaa 3300
   ttccataaaa agcaacgtta aaagaatcag tagcatgtgt tactgatgtg ttgctgaaga 3360
   ttaagatatt tttaagtctc accgaaaagg tagaaggagc caactgagac acaaaaaggg 3420
   gctgaggttc tattcatggt gagcaagtct ttttttttgt ttgtttcttc aagctctaac 3480
   aagggtgcct actacatggc ttttcagtta gccccaaaat aagatgtaac aatttttttt 3540
   tctattctta ggctttatct acaaagaaat gaattggata atcttcataa acaaaaaaca 3600
   tggaaaattt atcaaccaga atatgcagta gagatatatt ttaatgagaa atgacttaag 3660
   ttatgttgta actggtagct gattaagtat agttccctgc accccttctg ggaaagaatt 3720
   atgttctttc taaccctgcc acatagttat atgttctaaa tcttccttgc tggtacatct 3780
   atattgatat atgtatacac atgttcttta taaatctatt aaatatatac agaaaaaaaa 3840
   aaaaaaaa 3848
<210> 16
   <211> 1049
   <212> DNA
   <213> Homo Sapiens
<400> 16
   gttagacaca ggacctgctg ggccacagaa aggaggctct gggtagacgc actagattac 60
   tggataaatc acttcaattt cccaatgaat tttatattgt ttatttttat acctggagtt 120
   ttttccttaa aaagtagcac tttgaagcct actattgaag cattgcctaa tgtgctacct 180
   ttaaatgaag atgttaataa gcaggaagaa aagaatgaag atcatactcc caattatgct 240
   cctgctaatg agaaaaatgg caattattat aaagatataa aacaatatgt gttcacaaca 300
   caaaatccaa atggcactga gtctgaaata tctgtgagag ccacaactga cctgaatttt 360
   gctctaaaaa acgataaaac tgtcaatgca actacatatg aaaaatccac cattgaagaa 420
   gaaacaacta ctagcgaacc ctctcataaa aatattcaaa gatcaacccc aaacgtgcct 480
   gcattttgga caatgttagc taaagctata aatggaacag cagtggtcat ggatgataaa 540
   gatcaattat ttcacccaat tccagagtct gatgtgaatg ctacacaggg agaaaatcag 600
   ccagatctag aggatctgaa gatcaaaata atgctgggaa tctcgttgat gaccctcctc 660
   ctctttgtgg tcctcttggc attctgtagt gctacactgt acaaactgag gcatctgagt 720
   tataaaagtt gtgagagtca gtactctgtc aacccagagc tggccacgat gtcttacttt 780
   catccatcag aaggtgtttc agatacatcc ttttccaaga gtgcagagag cagcacattt 840
   ttgggtacca cttcttcaga tatgagaaga tcaggcacaa gaacatcaga atctaagata 900
   atgacggata tcatttccat aggctcagat aatgagatgc atgaaaacga tgagtcggtt 960
   acccggtgaa gaaatcaagg aacccggtga agaaatctta ttgatgaata aataacttta 1020
   attattttgt catcaaaaaa aaaaaaaaa 1049
<210> 17
   <211> 3591
   <212> DNA
   <213> Homo Sapiens
<400> 17
   ttactgggcg tatggcgtac agacacgagg ccggcgcccg ggaggcggtg ttcatccgcc      60
   cgggaaaaga gcgcctgttg ctcgctgccc gcgtgtccct ggctctctcg ggaacccagc     120
   gccgaaggcg aggtgggcgc gggccgaagg aggtcctggg aggtcggcgg cgcggaggga 180
   tctccgcggg agccgttggg gctgttggcc tcgggctgag gtgcaaggac caggactagg 240
   gcgagggcag cggtccaaga aatagaaaac aatgactggg agagcccgag ccagagccag 300
   aggaagggcc cgcggtcagg agacagcgca gctggtgggc tccactgcca gtcagcaacc 360
   tggttatatt cagcctaggc ctcagccgcc accagcagag ggggaattat ttggccgtgg 420
   acggcagaga ggaacagcag gaggaacagc caagtcacaa ggactccaga tatctgctgg 480
   atttcaggag ttatcgttag cagagagagg aggtcgtcgt agagattttc atgatcttgg 540
   tgtgaataca aggcagaacc tagaccatgt taaagaatca aaaacaggtt cttcaggcat 600
   tatagtaagg ttaagcacta accatttccg gctgacatcc cgtccccagt gggccttata 660
   tcagtatcac attgactata acccactgat ggaagccaga agactccgtt cagctcttct 720
   ttttcaacac gaagatctaa ttggaaagtg tcatgctttt gatggaacga tattattttt 780
   acctaaaaga ctacagcaaa aggttactga agtttttagt aagacccgga atggagagga 840
   tgtgaggata acgatcactt taacaaatga acttccacct acatcaccaa cttgtttgca 900
   gttctataat attattttca ggaggctttt gaaaatcatg aatttgcaac aaattggacg 960
   aaattattat aacccaaatg acccaattga tattccaagt cacaggttgg tgatttggcc 1020
   tggcttcact acttccatcc ttcagtatga aaacagcatc atgctctgca ctgacgttag 1080
   ccataaagtc cttcgaagtg agactgtttt ggatttcatg ttcaactttt atcatcagac 1140
   agaagaacat aaatttcaag aacaagtttc caaagaacta ataggtttag ttgttcttac 1200
   caagtataac aataagacat acagagtgga tgatattgac tgggaccaga atcccaagag 1260
   cacctttaag aaagccgacg gctctgaagt cagcttctta gaatactaca ggaagcaata 1320
   caaccaagag atcaccgact tgaagcagcc tgtcttggtc agccagccca agagaaggcg 1380
   gggccctggg gggacactgc cagggcctgc catgctcatt cctgagctct gctatcttac 1440
   aggtctaact gataaaatgc gtaatgattt taacgtgatg aaagacttag ccgttcatac 1500
   aagactaact ccagagcaaa ggcagcgtga agtgggacga ctcattgatt acattcataa 1560
   aaacgataat gttcaaaggg agcttcgaga ctggggtttg agctttgatt ccaacttact 1620
   gtccttctca ggaagaattt tgcaaacaga aaagattcac caaggtggaa aaacatttga 1680
   ttacaatcca caatttgcag attggtccaa agaaacaaga ggtgcaccat taattagtgt 1740
   taagccacta gataactggc tgttgatcta tacgcgaaga aattatgaag cagccaattc 1800
   attgatacaa aatctattta aagttacacc agccatgggc atgcaaatga gaaaagcaat 1860
   aatgattgaa gtggatgaca gaactgaagc ctacttaaga gtcttacagc aaaaggtcac 1920
   agcagacacc cagatagttg tctgtctgtt gtcaagtaat cggaaggaca aatacgatgc 1980
   tattaaaaaa tacctgtgta cagattgccc taccccaagt cagtgtgtgg tggcccgaac 2040
   cttaggcaaa cagcaaactg tcatggccat tgctacaaag attgccctac agatgaactg 2100
   caagatggga ggagagctct ggagggtgga catccccctg aagctcgtga tgatcgttgg 2160
   catcgattgt taccatgaca tgacagctgg gcggaggtca atcgcaggat ttgttgccag 2220
   catcaatgaa gggatgaccc gctggttctc acgctgcata tttcaggata gaggacagga 2280
   gctggtagat gggctcaaag tctgcctgca agcggctctg agggcttgga atagctgcaa 2340
   tgagtacatg cccagccgga tcatcgtgta ccgcgatggc gtaggagacg gccagctgaa 2400
   aacactggtg aactacgaag tgccacagtt tttggattgt ctaaaatcca ttggtagagg 2460
   ttacaaccct agactaacgg taattgtggt gaagaaaaga gtgaacacca gattttttgc 2520
   tcagtctgga ggaagacttc agaatccact tcctggaaca gttattgatg tagaggttac 2580
   cagaccagaa tggtatgact tttttatcgt gagccaggct gtgagaagtg gtagtgtttc 2640
   tcccacacat tacaatgtca tctatgacaa cagcggcctg aagccagacc acatacagcg 2700
   cttgacctac aagctgtgcc acatctatta caactggcca ggtgtcattc gtgttcctgc 2760
   tccttgccag tacgcccaca agctggcttt tcttgttggc cagagtattc acagagagcc 2820
   aaatctgtca ctgtcaaacc gcctttacta cctctaacct gcagaagacg atgcagccgc 2880
   ttttcttttt gaaatgactt tgggattttt ttaagctttt atttactttt tttttaactg 2940
   ttatctttct ggatgaaact tgggaagggg attaggagat ctagcatttt atttctagca 3000
   ttgctattca ccggcttcct tattttatac gtaaaaatta agattttata ttttatcttc 3060
   ttgtttctca tagatatttt gtgagcattt ttttgtttat tttgaagaaa tgtggataag 3120
   atacttggta gtataaaaca gactctctga gagtatttga aatgtgtttg gagatttact 3180
   taaacgtact ttcaggagtg agcaagtcct acttataaac ctatattaac tttatttttg 3240
   agatacctgt tttgaattta aaggagataa gaggcgtaaa gtaggatgct cactacaacc 3300
   ataggtgggg tttcagctca tatcttaaag ataaaaggta ctattatata acctatacac 3360
   aagatacagg agaaaatatg cttgattttt atttggcagg ggggctaggt tgtatgggag 3420
   taaaaaaaac attgaaaatt tttaaattgt ccaaagaaac attttaagac tctttaacaa 3480
   aaaaggccat gagtaaatct ctatattaac attactattt attttgtttt ggaactggga 3540
   catgattcta tttgttataa aataaaattg atgtgattgt caccttattt g 3591
<210> 18
   <211> 819
   <212> DNA
   <213> Homo Sapiens
<400> 18
   gtgatgctcc ctgggcctcc tgaccgcgcc ctcgcctggg aggcggggcg ggccgggttc        60
   tctctgtgac gtcacaaagg ccccgccatg cctctggctt tgacccttct gctgctctcg       120
   ggcttgggcg cccccggagg ctggggctgc ctgcagtgcg accccttggt gctggaggcc       180
   ctgggtcacc tgcgctccgc cctcatcccc agtcgcttcc agttggagca gctgcaggcg       240
   cgcgccgggg ccgtgctgat gggcatggag gggcctttct tccgggacta cgcgctgaac       300
   gtgtttgtgg ggaaagtgga gacaaatcaa ctggaccttg tggcgtcctt tgtcaagaac       360
   caaacgcagc acttaatggg taactctctg aaagatgagc ctctgctgga agagctggtg       420
   accctcaggg cgaatgtgat caaggaattc aagaaagttt taatttcata tgaattaaaa       480
   gcctgcaacc ccaaactttg ccgcttgcta aaagaagagg tgttggactg tttacattgc       540
   cagaggatca ctcccaagtg tatccacaaa aagtactgct ttgtcgaccg gcaaccccgc       600
   gtggccctgc agtaccagat ggacagcaaa tacccgagga accaggcgct gttgggcatc       660
   ctcatttctg tgtctctggc tgtctttgtc ttcgtggtca tcgtggtctc ggcttgtaca       720
   tacagacaaa accgaaaact cctgctgcag taggacggtg gtttgggggt aaggagaaag       780
   gaaaataaat ttaataaaat tggtgacaaa tccaaaaaa                              819
<210> 19
   <211> 5120
   <212> DNA
   <213> Homo Sapiens
<400> 19
   ggactcgcac tcggcggttg ttccagaaga aagagacagc gatggcggca gaggcttcga      60
   agactgggcc ttctaggtct tcctaccagc gaatggggag gaagagtcag ccctggggtg     120
   ccgctgaaat ccagtgcacc aggtgtggaa ggagggtatc cagatcatcc ggtcaccatt     180
   gtgaacttca atgtggacat gctttttgtg aactatgctt gttaatgact gaagaatgca     240
   ccacaattat atgccctgat tgtgaggttg ctacagctgt aaatactaga caacgctact     300
   acccaatggc tggatatatt aaggaagact ccataatgga aaaactgcag cctaagacga     360
   taaagaattg ttctcaggac tttaagaaga ctgctgatca gctaactact ggtttagaac     420
   gttcagcctc cacagacaag actcttttga actcatcagc tgtaatgttg gacactaata     480
   ctgcagaaga aattgatgaa gcattgaata cagcacacca tagtttcgaa cagttaagca     540
   ttgctggaaa agcacttgaa cacatgcaga agcaaacgat agaggaaaga gaaagagtta     600
   tagaagttgt ggagaaacag tttgaccaac ttttggcttt ttttgattcc aggaaaaaga     660
   acctgtgtga agaatttgca agaactactg atgattatct atcaaattta ataaaggcta     720
   aaagctacat tgaagagaaa aaaaataatt tgaatgcagc tatgaacata gcaagagcat     780
   tacaattatc gccttctcta agaacatact gtgacctgaa tcagattatc cggactttgc     840
   agttaacttc agatagtgaa ttagcacaag ttagttctcc acaactaagg aaccctccca     900
   ggttgagtgt gaattgcagt gagatcatct gtatgttcaa caatatggga aagattgaat     960
   ttagggactc aacaaaatgt tatccccaag aaaatgaaat tagacagaat gttcaaaaga    1020
   aatataataa caaaaaggaa ctttcttgtt acgatacata cccaccgcta gaaaagaaaa    1080
   aggttgacat gtctgtccta accagtgaag caccaccacc tcctttgcaa cctgagacaa 1140
   atgatgtaca tttagaagca aaaaacttcc agccacagaa agacgttgca acagcatccc 1200
   ctaaaaccat tgctgtgtta cctcagatgg gatctagccc tgatgtgata attgaagaaa 1260
   ttattgaaga caacgtggaa agttctgcag agctagtttt tgtaagccat gtaatagatc 1320
   cttgccattt ctacattcgg aagtattcac aaataaaaga cgccaaagta ctggagaaga 1380
   aggtgaatga attttgcaat aggagttcac accttgatcc ttcagacatt ttggaactag 1440
   gtgcaagaat atttgtcagc agtattaaaa atggaatgtg gtgtcgagga actatcacag 1500
   aattaattcc aatagagggt agaaatacca gaaaaccttg tagtccaacc agattatttg 1560
   tccatgaagt tgcactaata caaatattca tggtagattt tggaaattct gaagtcctga 1620
   ttgtcactgg agttgttgat acccatgtga gaccagaaca ctctgctaag caacatattg 1680
   cactaaatga tttatgtctg gttctaagga aatctgaacc atatactgaa gggctgctaa 1740
   aagacatcca gccattagca caaccatgct cattgaaaga cattgttcca cagaattcaa 1800
   atgaaggctg ggaagaggaa gctaaagtgg aatttttgaa aatggtaaat aacaaggctg 1860
   tttcaatgaa agtttttaga gaagaagatg gtgtgcttat tgtagatctg caaaaaccac 1920
   caccgaataa aataagcagt gatatgcctg tgtctcttag agatgcgcta gtttttatgg 1980
   aactagcaaa gtttaagtca caatcactaa gaagtcactt tgaaaaaaat actactttac 2040
   actatcatcc acctattttg cctaaagaaa tgacagatgt ttcagtaacg gtttgtcata 2100
   taaatagtcc tggagatttc tatcttcagt tgatagaggg cctggatatt ttatttctat 2160
   taaagacaat cgaggaattc tataaaagtg aagatggaga aaatctggaa atcctctgtc 2220
   cagttcaaga tcaagcctgt gtagctaaat ttgaagatgg aatttggtac cgagcaaaag 2280
   ttatcggatt gcctggacat caggaagttg aagttaaata tgtggacttt ggtaatactg 2340
   caaaaataac aatcaaagac gtgcgtaaaa taaaggatga gtttctgaat gccccagaga 2400
   aggcaattaa atgtaagttg gcctatattg aaccatataa aaggacaatg cagtggtcca 2460
   aagaagctaa agaaaaattt gaagaaaagg ctcaagataa atttatgaca tgttcagtta 2520
   tcaaaattct ggaagataat gtgctcttag ttgagctttt cgattctctt ggtgctcctg 2580
   aaatgactac tactagtatt aatgaccagc tagttaaaga gggcctagca tcttatgaaa 2640
   taggatacat cctcaaagat aattctcaaa agcatattga agtttgggat ccttctccag 2700
   aagaaattat ttcaaatgaa gtacacaact taaatcctgt gtctgcaaaa tctctaccta 2760
   atgagaattt tcagtcactt tataataagg aattgcctgt gcatatctgt aatgtaatat 2820
   ctcctgagaa gatttatgtt cagtggttgt taactgaaaa cttacttaat agtttagaag 2880
   aaaagatgat agctgcttat gaaaactcaa aatgggaacc tgttaaatgg gaaaatgata 2940
   tgcactgtgc tgttaagatc caagataaaa atcagtggcg aagaggccag atcatcagaa 3000
   tggttacaga cacattggta gaggtcttgc tgtatgatgt gggtgttgaa ctagtagtga 3060
   atgttgactg tttaagaaaa cttgaagaaa atctaaagac aatgggaaga ctctctttgg 3120
   aatgttctct ggttgacata agaccagctg gtgggagtga caagtggaca gcaacagctt 3180
   gtgactgtct ttcattgtac ctgactggag ctgtagcaac tataatctta caggtggata 3240
   gtgaggaaaa caacacaaca tggccattac ctgtgaaaat tttctgcaga gatgaaaaag 3300
   gagagcgtgt tgatgtttct aaatatttga ttaaaaaggg tttggctttg agagaaagga 3360
   gaattaataa cttagataac agccattcat tatctgagaa gtctctggaa gtccccctgg 3420
   aacaggaaga ttcagtagtt actaactgta ttaaaactaa ctttgaccct gacaagaaaa 3480
   ctgctgacat aatcagtgaa cagaaagtgt ctgaatttca ggagaaaatt ctagaaccaa 3540
   gaaccactag agggtataag ccaccagcta ttcctaacat gaacgtattt gaggcaacag 3600
   tcagctgtgt tggtgatgat ggaactatat ttgtagtacc taaactatca gaatttgagc 3660
   taataaaaat gacaaatgaa attcaaagta atttaaaatg ccttggtctt ttggagcctt 3720
   atttctggaa aaaaggagaa gcatgtgcag taagaggatc cgatactctg tggtatcgtg 3780
   gcaaggtgat ggaggttgta ggtggcgctg tcagagtaca atatttagat catggattca 3840
   ctgaaaagat tccgcagtgc catctttacc ctattttgct gtatcctgat ataccccagt 3900
   tttgtattcc ttgtcagctc cataatacca cacctgttgg gaatgtctgg caaccagatg 3960
   caatagaagt tcttcaacaa ctgctttcaa agagacaggt ggacattcac attatggagt 4020
   tacctaaaaa tccatgggag aaattgtcta ttcacctcta ttttgatgga atgtcacttt 4080
   cttattttat ggcatactat aaatactgta cttctgaaca tactgaggag atgttgaaag 4140
   aaaaaccaag atcagatcat gataaaaagt atgaagagga acaatgggaa ataaggtttg 4200
   aggaattgct ttcggctgaa acagacactc ctcttttacc accatatttg tcttcatctc 4260
   tgccttcccc aggagaactc tatgctgttc aagttaagca cgttgtctca cctaatgaag 4320
   tgtatatttg ccttgattct atagaaactt ctaaccagtc taaccagcat agtgacacag 4380
   atgatagtgg agtcagcggg gaatcagaat ccgagagcct tgatgaagca ctgcagaggg 4440
   ttaataagaa ggtagaggcg cttcctcctc tgacggattt tagaacagaa atgccttgcc 4500
   ttgcagaata tgatgatggc ttatggtata gagcgaagat tgttgccatt aaagaattta 4560
   atcctttatc tatcttagta caatttgttg attatggatc aactgcaaag ctgacattaa 4620
   acagactgtg ccaaattcct tctcatctta tgcggtatcc agctcgagcc ataaaggttc 4680
   tcttggcagg gtttaaacct cccttaaggg atctagggga gacaagaata ccatattgtc 4740
   ccaaatggag catggaggca ctgtgggcta tgatagactg tcttcaagga aaacaactct 4800
   atgctgtgtc catggctcca gcaccagaac agatagtgac attatatgac gatgaacagc 4860
   atccagttca tatgccgttg gtagaaatgg ggcttgcaga taaagatgaa taagtgccta 4920
   agtgtataca gtgagagcat ctatagaagc ctagaagaat tctgttatgt ttagactatg 4980
   tcttatcttt agactatttc aggcttaatt ttcctaactt gttcagcact agtgctttac 5040
   ctctcatttt taattgaact gttaggaatt gtgtggggaa aaaaagtaaa taaatgttcg 5100
   cttccaaaaa aaaaaaaaaa 5120
<210> 20
   <211> 1738
   <212> DNA
   <213> Homo Sapiens
<400> 20
   gatgtcatca ggctttgtga gggggactgt actgcccttt gagtactact gtgtggcccc 60
   gcaacccagc acaaccaggt atctgcttgg aacccagcca ccataaagcc tgctagctaa 120
   aaaaaatttt acatctctca gttcattcgg cacagacccc tgcctcattc agctgtgact 180
   ctgcttggaa aattcatcag ttacaaagca gccaatgcaa ttatctcaag ggaaattgaa 240
   aaatggacct ttgaaaatgc tagatttaca atgagaaatg ccataattca aggtttattc 300
   tatgggtcct tgacatttgg gatctggaca gctctgttat tcatatattt gcaccataat 360
   catgtgagca gctggcagaa gaaaagccag gagcctctgt cagcttggtc ccctggaaaa 420
   aaagtgcatc agcaaattat ctatggctca gagcaaatac caaaacctca tgtaatagtc 480
   aaaaggactg atgaagataa agcaaagtct atgttaggta cagattttaa ccatacaaac 540
   ccagaacttc ataaagaact tttaaaatat ggatttaatg tgattatcag tagaagcttg 600
   ggcatcgaaa gagaagtgcc agataccagg agtaaaatgt gtcttcaaaa acattaccca 660
   gcccgcctcc cgactgccag cattgtcatt tgcttctata atgaagaatg taatgccttg 720
   tttcagacca tgtccagtgt cacgaacctc acgccacact attttcttga agaaattatt 780
   ttggtagatg acatgagcaa agttgatgat ttgaaagaaa aactagacta tcacctggaa 840
   acttttcggg gaaaggttaa aataataaga aacaaaaaga gagaggggct gattcgagca 900
   aggctgattg gagcttctca tgcttcaggg gatgttctgg tgttcctgga cagccactgt 960
   gaggtgaaca gagtatggct ggagcccctg ctgcatgcca ttgccaagga ccccaaaatg 1020
   gtggtgtgcc ccctgataga tgtcattgat gatagaactc tggagtataa gccctctcct 1080
   cttgtaaggg gaacttttga ttggaaccta caatttaaat gggataatgt tttctcttat 1140
   gagatggatg gaccagaagg atctactaaa ccaatccggt cacctgcaat gtctggagga 1200
   atttttgcta tacgtcggca ttattttaat gaaattggac agtatgacaa ggatatggat 1260
   ttttggggaa gagaaaattt ggaactttca ctaaggatct ggatgtgtgg aggccaactc 1320
   tttataatcc cctgctctcg agtaggacat atcagtaaga aacaaactgg aaaaccttct 1380
   acaatcatca gtgctatgac acataactac ctaagactgg tgcacgtttg gctggatgaa 1440
   tataaggagc agtttttcct tcgaaagcct ggtctgaaat atgtcaccta cggaaatatt 1500
   cgcgagcgtg ttgagttaag gaaacgactg ggttgcaagt catttcagtg gtatttggat 1560
   aatgtcttcc cagagttgga ggcatctgtg aacagcctgt gaaaggaaaa caaatcactt 1620
   tcattaataa agggttaaaa gtctcctagt cattcaacat agtgtcacaa gagtgtaagt 1680
   ttggaacatc gtggaattac gtgaaatgca attaaaaaaa tatgaccaga cgtgaaaa 1738
<210> 21
   <211> 731
   <212> DNA
   <213> Homo Sapiens
<400> 21
   gagaactctt gagaccggga gcccagctgc ccaccctctg gacattcacc cagccaggtg 60
   gtctcgtcac ctcagaggct ccgccagact cctgcccagg ccaggactga ggcaagcctc 120
   aaggcacttc taggacctgc ctcttctcac caagatgaac tcactggttt cttggcagct 180
   actgcttttc ctctgtgcca cccactttgg ggagccatta gaaaaggtgg cctctgtggg 240
   gaattctaga cccacaggcc agcagctaga atccctgggc ctcctggccc ccggggagca 300
   gagcctgccg tgcaccgaga ggaagccagc tgctactgcc aggctgagcc gtcgggggac 360
   ctcgctgtcc ccgccccccg agagctccgg gagcccccag cagccgggcc tgtccgcccc 420
   ccacagccgc cagatccccg caccccaggg cgcggtgctg gtgcagcggg agaaggacct 480
   gccgaactac aactggaact ccttcggcct gcgcttcggc aagcgggagg cggcaccagg 540
   gaaccacggc agaagcgctg ggcggggctg agggcgcagg tgcggggcag tgaacttcag 600
   accccaaagg agtcagagca tgcggggcgg gggcgggggg cggggacgta gggctaaggg 660
   agggggcgct ggagcttcca acccgaggca ataaaagaaa tgttgcgtaa ctcaaaaaaa 720
   aaaaaaaaaa a 731
<210> 22
   <211> 1660
   <212> DNA
   <213> Homo Sapiens
<400> 22
   ggtgcactag caaaacaaac ttattttgaa cactcagctc ctagcgtgcg gcgctgccaa 60
   tcattaacct cctggtgcaa gtggcgcggc ctgtgccctt tataaggtgc gcgctgtgtc 120
   cagcgagcat cggccaccgc catcccatcc agcgagcatc tgccgccgcg ccgccgccac 180
   cctcccagag agcactggcc accgctccac catcacttgc ccagagtttg ggccaccgcc 240
   cgccgccacc agcccagaga gcatcggccc ctgtctgctg ctcgcgcctg gagatgtcag 300
   aggtccccgt tgctcgcgtc tggctggtac tgctcctgct gactgtccag gtcggcgtga 360
   cagccggcgc tccgtggcag tgcgcgccct gctccgccga gaagctcgcg ctctgcccgc 420
   cggtgtccgc ctcgtgctcg gaggtcaccc ggtccgccgg ctgcggctgt tgcccgatgt 480
   gcgccctgcc tctgggcgcc gcgtgcggcg tggcgactgc acgctgcgcc cggggactca 540
   gttgccgcgc gctgccgggg gagcagcaac ctctgcacgc cctcacccgc ggccaaggcg 600
   cctgcgtgca ggagtctgac gcctccgctc cccatgctgc agaggcaggg agccctgaaa 660
   gcccagagag cacggagata actgaggagg agctcctgga taatttccat ctgatggccc 720
   cttctgaaga ggatcattcc atcctttggg acgccatcag tacctatgat ggctcgaagg 780
   ctctccatgt caccaacatc aaaaaatgga aggagccctg ccgaatagaa ctctacagag 840
   tcgtagagag tttagccaag gcacaggaga catcaggaga agaaatttcc aaattttacc 900
   tgccaaactg caacaagaat ggattttatc acagcagaca gtgtgagaca tccatggatg 960
   gagaggcggg actctgctgg tgcgtctacc cttggaatgg gaagaggatc cctgggtctc 1020
   cagagatcag gggagacccc aactgccaga tatattttaa tgtacaaaac tgaaaccaga 1080
   tgaaataatg ttctgtcacg tgaaatattt aagtatatag tatatttata ctctagaaca 1140
   tgcacattta tatatatatg tatatgtata tatatatagt aactactttt tatactccat 1200
   acataacttg atatagaaag ctgtttattt attcactgta agtttatttt ttctacacag 1260
   taaaaacttg tactatgtta ataacttgtc ctatgtcaat ttgtatatca tgaaacactt 1320
   ctcatcatat tgtatgtaag taattgcatt tctgctcttc caaagctcct gcgtctgttt 1380
   ttaaagagca tggaaaaata ctgcctagaa aatgcaaaat gaaataagag agagtagttt 1440
   ttcagctagt ttgaaggagg acggttaact tgtatattcc accattcaca tttgatgtac 1500
   atgtgtaggg aaagttaaaa gtgttgatta cataatcaaa gctacctgtg gtgatgttgc 1560
   cacctgttaa aatgtacact ggatatgttg ttaaacacgt gtctataatg gaaacattta 1620
   caataaatat tctgcatgga aatactgtta aaaaaaaaaa 1660
<210> 23
   <211> 1949
   <212> DNA
   <213> Homo Sapiens
<400> 23
   aataaagggg tctgagccgg tcgcctgagc ctgaaaagtg ctgtcacgtc agcggaagga      60
   ggcgtcccag atcttctcag ctgtcttggt gccagccttc ctagtcttcc tacccacact     120
   cctacctgct gtcacaggcc acagccatca tgcctcgggg tcacaagagt aagctccgta     180
   cctgtgagaa acgccaagag accaatggtc agccacaggg tctcacgggt ccccaggcca     240
   ctgcagagaa gcaggaagag tcccactctt cctcatcctc ttctcgcgct tgtctgggtg     300
   attgtcgtag gtcttctgat gcctccattc ctcaggagtc tcagggagtg tcacccactg     360
   ggtctcctga tgcagttgtt tcatattcaa aatccgatgt ggctgccaac ggccaagatg     420
   agaaaagtcc aagcacctcc cgtgatgcct ccgttcctca ggagtctcag ggagcttcac     480
   ccactggctc tcctgatgca ggtgtttcag gctcaaaata tgatgtggct gccaacggcc     540
   aagatgagaa aagtccaagc acttcccatg atgtctccgt tcctcaggag tctcagggag     600
   cttcacccac tggctcgcct gatgcaggtg tttcaggctc aaaatatgat gtggctgccg     660
   agggtgaaga tgaggaaagt gtaagcgcct cacagaaagc catcattttt aagcgcttaa 720
   gcaaagatgc tgtaaagaag aaggcgtgca cgttggcgca attcctgcag aagaagtttg 780
   agaagaaaga gtccattttg aaggcagaca tgctgaagtg tgtccgcaga gagtacaagc 840
   cctacttccc tcagatcctc aacagaacct cccaacattt ggtggtggcc tttggcgttg 900
   aattgaaaga aatggattcc agcggcgagt cctacaccct tgtcagcaag ctaggcctcc 960
   ccagtgaagg aattctgagt ggtgataatg cgctgccgaa gtcgggtctc ctgatgtcgc 1020
   tcctggttgt gatcttcatg aacggcaact gtgccactga agaggaggtc tgggagttcc 1080
   tgggtctgtt ggggatatat gatgggatcc tgcattcaat ctatggggat gctcggaaga 1140
   tcattactga agatttggtg caagataagt acgtggttta ccggcaggtg tgcaacagtg 1200
   atcctccatg ctatgagttc ctgtggggtc cacgagccta tgctgaaacc accaagatga 1260
   gagtcctgcg tgttttggcc gacagcagta acaccagtcc cggtttatac ccacatctgt 1320
   atgaagacgc tttgatagat gaggtagaga gagcattgag actgagagct taaggcaggg 1380
   ctggcactat ttccttggcc agggtacctt atggggccat atcctacaga tcctcccatt 1440
   tctagggagg tctgaagtag aattttcact ttatgttaga agagagtagt gagctttcta 1500
   agtagtgcag tatagtagag gctggaggga acaagatatg tatctttctt ttgttacaca 1560
   tgagtaactt gcagatttat gttttatctc tgtcagttat caacattgtt cctgttaagt 1620
   gaaggtttat tttgcttcag attatacaat tatcaataac atagctctca cattcatggc 1680
   tgtttaacca atctgaaagt tacggtttgg gaattaataa aacaaagtca tacaacacat 1740
   tttctttgta attgagaact agataacatg gtaacagaga attgattttc atatgaatct 1800
   taactccaca gtaaaatagt tgacatcata atatgaagag aaagaaaagg aaaaacagaa 1860
   atgtaaaagt tgtttaattc ttggtttgcc taattcgttt tcctatttct tttcatacaa 1920
   ataaaggata cctggattta tttaggtta 1949
<210> 24
   <211> 79
   <212> PRT
   <213> Homo Sapiens
<400> 24
   Met Cys Cys Asn Tyr Tyr Gly Asn Ser Cys Gly Tyr Gly Ser Gly Cys
   1 5 10 15
   Gly Cys Gly Tyr Gly Ser Gly Ser Gly Cys Gly Cys Gly Tyr Gly Thr
   20 25 30
   Gly Tyr Gly Cys Gly Tyr Gly Cys Gly Phe Gly Ser His Tyr Gly Cys
   35 40 45
   Gly Tyr Gly Thr Gly Tyr Gly Cys Gly Tyr Gly Ser Gly Ser Gly Tyr
   50 55 60
   Cys Gly Tyr Arg Pro Phe Cys Phe Arg Arg Cys Tyr Ser Ser Cys
   65 70 75
<210> 25
   <211> 70
   <212> PRT
   <213> Homo Sapiens
<400> 25
   Met Lys Gln Lys Gln Glu Val Met Phe Gln Ser Arg Gly Arg Leu Ser
   1 5 10 15
   Leu Tyr Ile Gln Met Ser Ser Val Tyr Ser Ala Lys Leu Gly Pro Val
   20 25 30
   Gly Gly Ile Cys Gly Gln Lys Gln Lys Pro Ser Phe Phe Phe Phe Lys
   35 40 45
   Ala Gln Ser Gln Asp Ala Arg Pro Leu Ala Pro Ala Ala Cys Ile Ser
   50 55 60
   Lys Ile Ala Lys Ala Gly
   65 70
<210> 26
   <211> 265
   <212> PRT
   <213> Homo Sapiens
<400> 26
   Met Glu Ala Leu Pro Pro Val Arg Ser Ser Leu Leu Gly Ile Leu Leu
   1               5                   10                  15
   Gln Val Thr Arg Leu Ser Val Leu Leu Val Gln Asn Arg Asp His Leu
               20                  25                  30
   Tyr Asn Phe Leu Leu Leu Lys Ile Asn Leu Phe Asn His Trp Val Ser
           35                  40                  45
   Gly Leu Ala Gln Glu Ala Arg Gly Ser Cys Asn Trp Gln Ala His Leu
       50                  55                  60
   Pro Leu Gly Ala Ala Ala Cys Pro Leu Gly Gln Ala Leu Trp Ala Gly
   65                  70                  75                  80
   Leu Ala Leu Ile Gln Val Pro Val Trp Leu Val Leu Gln Gly Pro Arg
                   85                  90                  95
   Leu Met Trp Ala Gly Met Trp Gly Ser Thr Lys Gly Leu Gly Leu Ala
               100                 105                 110
   Leu Leu Ser Ala Trp Glu Gln Leu Gly Leu Ser Val Ala Ile Trp Thr
           115                 120                 125
   Asp Leu Phe Leu Ser Cys Leu His Gly Leu Met Leu Val Ala Leu Leu
       130                 135                 140
   Leu Val Val Val Thr Trp Arg Val Cys Gln Lys Ser His Cys Phe Arg
   145                 150                 155                 160
   Leu Gly Arg Gln Leu Ser Lys Ala Leu Gln Val Asn Cys Val Val Arg
                   165                 170                 175
   Lys Leu Leu Val Gln Leu Arg Arg Leu Tyr Trp Trp Val Glu Thr Met
               180                 185                 190
   Thr Ala Leu Thr Ser Trp His Leu Ala Tyr Leu Ile Thr Trp Thr Thr
           195                 200                 205
   Cys Leu Ala Ser His Leu Leu Gln Ala Ala Phe Glu His Thr Thr Gln
       210                 215                 220
   Leu Ala Glu Ala Gln Glu Val Glu Pro Gln Glu Val Ser Gly Ser Ser
   225                 230                 235                 240
   Leu Leu Pro Ser Leu Ser Ala Ser Ser Asp Ser Glu Ser Gly Thr Val
                   245                 250                 255
   Leu Pro Glu Gln Glu Thr Pro Arg Glu
               260                 265
<210> 27
   <211> 533
   <212> PRT
   <213> Homo Sapiens
<400> 27
   Met Asn Glu Glu Asn Ile Asp Gly Thr Asn Gly Cys Ser Lys Val Arg
   1               5                   10                  15
   Thr Gly Ile Gln Asn Glu Ala Ala Leu Leu Ala Leu Met Glu Lys Thr
               20                  25                  30
   Gly Tyr Asn Met Val Gln Glu Asn Gly Gln Arg Lys Phe Gly Gly Pro
           35                  40                  45
   Pro Pro Gly Trp Glu Gly Pro Pro Pro Pro Arg Gly Cys Glu Val Phe
       50                  55                  60
   Val Gly Lys Ile Pro Arg Asp Met Tyr Glu Asp Glu Leu Val Pro Val
   65                  70                  75                  80
   Phe Glu Arg Ala Gly Lys Ile Tyr Glu Phe Arg Leu Met Met Glu Phe
                   85                  90                  95
   Ser Gly Glu Asn Arg Gly Tyr Ala Phe Val Met Tyr Thr Thr Lys Glu
               100                 105                 110
   Glu Ala Gln Leu Ala Ile Arg Ile Leu Asn Asn Tyr Glu Ile Arg Pro
           115                 120                 125
   Gly Lys Phe Ile Gly Val Cys Val Ser Leu Asp Asn Cys Arg Leu Phe
       130                 135                 140
   Ile Gly Ala Ile Pro Lys Glu Lys Lys Lys Glu Glu Ile Leu Asp Glu
   145                 150                 155                 160
   Met Lys Lys Val Thr Glu Gly Val Val Asp Val Ile Val Tyr Pro Ser
                   165                 170                 175
   Ala Thr Asp Lys Thr Lys Asn Arg Gly Phe Ala Phe Val Glu Tyr Glu
               180                 185                 190
   Ser His Arg Ala Ala Ala Met Ala Arg Arg Lys Leu Ile Pro Gly Thr
           195                 200                 205
   Phe Gln Leu Trp Gly His Thr Ile Gln Val Asp Trp Ala Asp Pro Glu
       210                 215                 220
   Lys Glu Val Asp Glu Glu Thr Met Gln Arg Val Lys Val Leu Tyr Val
   225                 230                 235                 240
   Arg Asn Leu Met Ile Ser Thr Thr Glu Glu Thr Ile Lys Ala Glu Phe
                   245                 250                 255
   Asn Lys Phe Lys Pro Gly Ala Val Glu Arg Val Lys Lys Leu Arg Asp
               260                 265                 270
   Tyr Ala Phe Val His Phe Phe Asn Arg Glu Asp Ala Val Ala Ala Met
           275                 280                 285
   Ser Val Met Asn Gly Lys Cys Ile Asp Gly Ala Ser Ile Glu Val Thr
       290                 295                 300
   Leu Ala Lys Pro Val Asn Lys Glu Asn Thr Trp Arg Gln His Leu Asn
   305                 310                 315                 320
   Gly Gln Ile Ser Pro Asn Ser Glu Asn Leu Ile Val Phe Ala Asn Lys
   325 330 335
   Glu Glu Ser His Pro Lys Thr Leu Gly Lys Leu Pro Thr Leu Pro Ala
   340 345 350
   Arg Leu Asn Gly Gln His Ser Pro Ser Pro Pro Glu Val Glu Arg Cys
   355 360 365
   Thr Tyr Pro Phe Tyr Pro Gly Thr Lys Leu Thr Pro Ile Ser Met Tyr
   370 375 380
   Ser Leu Lys Ser Asn His Phe Asn Ser Ala Val Met His Leu Asp Tyr
   385 390 395 400
   Tyr Cys Asn Lys Asn Asn Trp Ala Pro Pro Glu Tyr Tyr Leu Tyr Ser
   405 410 415
   Thr Thr Ser Gln Asp Gly Lys Val Leu Leu Val Tyr Lys Ile Val Ile
   420 425 430
   Pro Ala Ile Ala Asn Gly Ser Gln Ser Tyr Phe Met Pro Asp Lys Leu
   435 440 445
   Cys Thr Thr Leu Glu Asp Ala Lys Glu Leu Ala Ala Gln Phe Thr Leu
   450 455 460
   Leu His Leu Asp Tyr Asn Phe His Arg Ser Ser Ile Asn Ser Leu Ser
   465 470 475 480
   Pro Val Ser Ala Thr Leu Ser Ser Gly Thr Pro Ser Val Leu Pro Tyr
   485 490 495
   Thr Ser Arg Pro Tyr Ser Tyr Pro Gly Tyr Pro Leu Ser Pro Thr Ile
   500 505 510
   Ser Leu Ala Asn Gly Ser His Val Gly Gln Arg Leu Cys Ile Ser Asn
   515 520 525
   Gln Ala Ser Phe Phe
   530
<210> 28
   <211> 123
   <212> PRT
   <213> Homo Sapiens
<400> 28
   Met Pro Asp Ala Ser Leu Gly Ser Leu Gly Ile Thr Trp Cys Phe Leu
   1               5                   10                  15
   Glu Ser Pro Leu Glu Val Ser Ser Gly Arg Phe Gly Leu Ala Arg Leu
               20                  25                  30
   Leu Gly Ser Gln Asp His Gly Asp Asp Pro Ala Glu Arg Gly Arg Thr
           35                  40                  45
   Ala Thr Asp Ala Trp Gly Pro Ser Arg Trp Gly Gln Ser Pro Gly Asn
       50                  55                  60
   Gly Gly Gly Tyr Cys Asp Ala Ser Pro Pro Ser Ala Leu Ala Pro Gly
   65                  70                  75                  80
   Asp Arg Ala Trp Ala Leu Pro Ala Ser Pro Ser Ser Gly Ala Pro Ala
                   85                  90                  95
   Ser Gln His Cys Cys Leu Glu Lys Ala Gly Thr Arg Thr Lys Ala Ser
               100                 105                 110
   Pro Val Trp Gly Arg Asp Gly Asn Thr Trp Asn
           115                 120
<210> 29
   <211> 638
   <212> PRT
   <213> Homo Sapiens
<400> 29
   Met Val Val Ser Ala Asp Pro Leu Ser Ser Glu Arg Ala Glu Met Asn
   1               5                   10                  15
   Ile Leu Glu Ile Asn Gln Glu Leu Arg Ser Gln Leu Ala Glu Ser Asn
               20                  25                  30
   Gln Gln Phe Arg Asp Leu Lys Glu Lys Phe Leu Ile Thr Gln Ala Thr
           35                  40                  45
   Ala Tyr Ser Leu Ala Asn Gln Leu Lys Lys Tyr Lys Cys Glu Glu Tyr
       50                  55                  60
   Lys Asp Ile Ile Asp Ser Val Leu Arg Asp Glu Leu Gln Ser Met Glu
   65                  70                  75                  80
   Lys Leu Ala Glu Lys Leu Arg Gln Ala Glu Glu Leu Arg Gln Tyr Lys
                   85                  90                  95
   Ala Leu Val His Ser Gln Ala Lys Glu Leu Thr Gln Leu Arg Glu Lys
               100                 105                 110
   Leu Arg Glu Gly Arg Asp Ala Ser Arg Trp Leu Asn Lys His Leu Lys
           115                 120                 125
   Thr Leu Leu Thr Pro Asp Asp Pro Asp Lys Ser Gln Gly Gln Asp Leu
       130                 135                 140
   Arg Glu Gln Leu Ala Glu Gly His Arg Leu Ala Glu His Leu Val His
   145                 150                 155                 160
   Lys Leu Ser Pro Glu Asn Asp Glu Asp Glu Asp Glu Asp Glu Asp Asp
                   165                 170                 175
   Lys Asp Glu Glu Val Glu Lys Val Gln Glu Ser Pro Ala Pro Arg Glu
               180                 185                 190
   Val Gln Lys Thr Glu Glu Lys Glu Val Pro Gln Asp Ser Leu Glu Glu
           195                 200                 205
   Cys Ala Val Thr Cys Ser Asn Ser His Asn Pro Ser Asn Ser Asn Gln
       210                 215                 220
   Pro His Arg Ser Thr Lys Ile Thr Phe Lys Glu His Glu Val Asp Ser
   225                 230                 235                 240
   Ala Leu Val Val Glu Ser Glu His Pro His Asp Glu Glu Glu Glu Ala
                   245                 250                 255
   Leu Asn Ile Pro Pro Glu Asn Gln Asn Asp His Glu Glu Glu Glu Gly
               260                 265                 270
   Lys Ala Pro Val Pro Pro Arg His His Asp Lys Ser Asn Ser Tyr Arg
           275                 280                 285
   His Arg Glu Val Ser Phe Leu Ala Leu Asp Glu Gln Lys Val Cys Ser
       290                 295                 300
   Ala Gln Asp Val Ala Arg Asp Tyr Ser Asn Pro Lys Trp Asp Glu Thr
   305                 310                 315                 320
   Ser Leu Gly Phe Leu Glu Lys Gln Ser Asp Leu Glu Glu Val Lys Gly
                   325                 330                 335
   Gln Glu Thr Val Ala Pro Arg Leu Ser Arg Gly Pro Leu Arg Val Asp
               340                 345                 350
   Lys His Glu Ile Pro Gln Glu Ser Leu Asp Gly Cys Cys Leu Thr Pro
           355                 360                 365
   Ser Ile Leu Pro Asp Leu Thr Pro Ser Tyr His Pro Tyr Trp Ser Thr
   370 375 380
   Leu Tyr Ser Phe Glu Asp Lys Gln Val Ser Leu Ala Leu Val Asp Lys
   385 390 395 400
   Ile Lys Lys Asp Gln Glu Glu Ile Glu Asp Gln Ser Pro Pro Cys Pro
   405 410 415
   Arg Leu Ser Gln Glu Leu Pro Glu Val Lys Glu Gln Glu Val Pro Glu
   420 425 430
   Asp Ser Val Asn Glu Val Tyr Leu Thr Pro Ser Val His His Asp Val
   435 440 445
   Ser Asp Cys His Gln Pro Tyr Ser Ser Thr Leu Ser Ser Leu Glu Asp
   450 455 460
   Gln Leu Ala Cys Ser Ala Leu Asp Val Ala Ser Pro Thr Glu Ala Ala
   465 470 475 480
   Cys Pro Gln Gly Thr Trp Ser Gly Asp Leu Ser His His Gln Ser Glu
   485 490 495
   Val Gln Val Ser Gln Ala Gln Leu Glu Pro Ser Thr Leu Val Pro Ser
   500 505 510
   Cys Leu Arg Leu Gln Leu Asp Gln Gly Phe His Cys Gly Asn Gly Leu
   515 520 525
   Ala Gln Arg Gly Leu Ser Ser Thr Thr Cys Ser Phe Ser Ala Asn Ala
   530 535 540
   Asp Ser Gly Asn Gln Trp Pro Phe Gln Glu Leu Val Leu Glu Pro Ser
   545 550 555 560
   Leu Gly Met Lys Asn Pro Pro Gln Leu Glu Asp Asp Ala Leu Glu Gly
   565 570 575
   Ser Ala Ser Asn Thr Gln Gly Arg Gln Val Thr Gly Arg Ile Arg Ala
   580 585 590
   Ser Leu Val Leu Ile Leu Lys Thr Ile Arg Arg Arg Leu Pro Phe Ser
   595 600 605
   Lys Trp Arg Leu Ala Phe Arg Phe Ala Gly Pro His Ala Glu Ser Ala
   610 615 620
   Glu Ile Pro Asn Thr Ala Gly Arg Thr Gln Arg Met Ala Gly
   625 630 635
<210> 30
   <211> 909
   <212> PRT
   <213> Homo Sapiens
<400> 30
   Met Ala Ala Arg Pro Leu Pro Val Ser Pro Ala Arg Ala Leu Leu Leu
   1               5                   10                  15
   Ala Leu Ala Gly Ala Leu Leu Ala Pro Cys Glu Ala Arg Gly Val Ser
               20                  25                  30
   Leu Trp Asn Gln Gly Arg Ala Asp Glu Val Val Ser Ala Ser Val Gly
           35                  40                  45
   Ser Gly Asp Leu Trp Ile Pro Val Lys Ser Phe Asp Ser Lys Asn His
       50                  55                  60
   Pro Glu Val Leu Asn Ile Arg Leu Gln Arg Glu Ser Lys Glu Leu Ile
   65                  70                  75                  80
   Ile Asn Leu Glu Arg Asn Glu Gly Leu Ile Ala Ser Ser Phe Thr Glu
                   85                  90                  95
   Thr His Tyr Leu Gln Asp Gly Thr Asp Val Ser Leu Ala Arg Asn Tyr
               100                 105                 110
   Thr Val Ile Leu Gly His Cys Tyr Tyr His Gly His Val Arg Gly Tyr
           115                 120                 125
   Ser Asp Ser Ala Val Ser Leu Ser Thr Cys Ser Gly Leu Arg Gly Leu
       130                 135                 140
   Ile Val Phe Glu Asn Glu Ser Tyr Val Leu Glu Pro Met Lys Ser Ala
   145                 150                 155                 160
   Thr Asn Arg Tyr Lys Leu Phe Pro Ala Lys Lys Leu Lys Ser Val Arg
                   165                 170                 175
   Gly Ser Cys Gly Ser His His Asn Thr Pro Asn Leu Ala Ala Lys Asn
               180                 185                 190
   Val Phe Pro Pro Pro Ser Gln Thr Trp Ala Arg Arg His Lys Arg Glu
           195                 200                 205
   Thr Leu Lys Ala Thr Lys Tyr Val Glu Leu Val Ile Val Ala Asp Asn
       210                 215                 220
   Arg Glu Phe Gln Arg Gln Gly Lys Asp Leu Glu Lys Val Lys Gln Arg
   225                 230                 235                 240
   Leu Ile Glu Ile Ala Asn His Val Asp Lys Phe Tyr Arg Pro Leu Asn
                   245                 250                 255
   Ile Arg Ile Val Leu Val Gly Val Glu Val Trp Asn Asp Met Asp Lys
               260                 265                 270
   Cys Ser Val Ser Gln Asp Pro Phe Thr Ser Leu His Glu Phe Leu Asp
           275                 280                 285
   Trp Arg Lys Met Lys Leu Leu Pro Arg Lys Ser His Asp Asn Ala Gln
       290                 295                 300
   Leu Val Ser Gly Val Tyr Phe Gln Gly Thr Thr Ile Gly Met Ala Pro
   305                 310                 315                 320
   Ile Met Ser Met Cys Thr Ala Asp Gln Ser Gly Gly Ile Val Met Asp
                   325                 330                 335
   His Ser Asp Asn Pro Leu Gly Ala Ala Val Thr Leu Ala His Glu Leu
               340                 345                 350
   Gly His Asn Phe Gly Met Asn His Asp Thr Leu Asp Arg Gly Cys Ser
           355                 360                 365
   Cys Gln Met Ala Val Glu Lys Gly Gly Cys Ile Met Asn Ala Ser Thr
       370                 375                 380
   Gly Tyr Pro Phe Pro Met Val Phe Ser Ser Cys Ser Arg Lys Asp Leu
   385                 390                 395                 400
   Glu Thr Ser Leu Glu Lys Gly Met Gly Val Cys Leu Phe Asn Leu Pro
                   405                 410                 415
   Glu Val Arg Glu Ser Phe Gly Gly Gln Lys Cys Gly Asn Arg Phe Val
               420                 425                 430
   Glu Glu Gly Glu Glu Cys Asp Cys Gly Glu Pro Glu Glu Cys Met Asn
           435                 440                 445
   Arg Cys Cys Asn Ala Thr Thr Cys Thr Leu Lys Pro Asp Ala Val Cys
       450                 455                 460
   Ala His Gly Leu Cys Cys Glu Asp Cys Gln Leu Lys Pro Ala Gly Thr
   465                 470                 475                 480
   Ala Cys Arg Asp Ser Ser Asn Ser Cys Asp Leu Pro Glu Phe Cys Thr
   485 490 495
   Gly Ala Ser Pro His Cys Pro Ala Asn Val Tyr Leu His Asp Gly His
   500 505 510
   Ser Cys Gln Asp Val Asp Gly Tyr Cys Tyr Asn Gly Ile Cys Gln Thr
   515 520 525
   His Glu Gln Gln Cys Val Thr Leu Trp Gly Pro Gly Ala Lys Pro Ala
   530 535 540
   Pro Gly Ile Cys Phe Glu Arg Val Asn Ser Ala Gly Asp Pro Tyr Gly
   545 550 555 560
   Asn Cys Gly Lys Val Ser Lys Ser Ser Phe Ala Lys Cys Glu Met Arg
   565 570 575
   Asp Ala Lys Cys Gly Lys Ile Gln Cys Gln Gly Gly Ala Ser Arg Pro
   580 585 590
   Val Ile Gly Thr Asn Ala Val Ser Ile Glu Thr Asn Ile Pro Leu Gln
   595 600 605
   Gln Gly Gly Arg Ile Leu Cys Arg Gly Thr His Val Tyr Leu Gly Asp
   610 615 620
   Asp Met Pro Asp Pro Gly Leu Val Leu Ala Gly Thr Lys Cys Ala Asp
   625 630 635 640
   Gly Lys Ile Cys Leu Asn Arg Gln Cys Gln Asn Ile Ser Val Phe Gly
   645 650 655
   Val His Glu Cys Ala Met Gln Cys His Gly Arg Gly Val Cys Asn Asn
   660 665 670
   Arg Lys Asn Cys His Cys Glu Ala His Trp Ala Pro Pro Phe Cys Asp
   675 680 685
   Lys Phe Gly Phe Gly Gly Ser Thr Asp Ser Gly Pro Ile Arg Gln Ala
   690 695 700
   Asp Asn Gln Gly Leu Thr Ile Gly Ile Leu Val Thr Ile Leu Cys Leu
   705 710 715 720
   Leu Ala Ala Gly Phe Val Val Tyr Leu Lys Arg Lys Thr Leu Ile Arg
   725 730 735
   Leu Leu Phe Thr Asn Lys Lys Thr Thr Ile Glu Lys Leu Arg Cys Val
   740 745 750
   Arg Pro Ser Arg Pro Pro Arg Gly Phe Gln Pro Cys Gln Ala His Leu
   755 760 765
   Gly His Leu Gly Lys Gly Leu Met Arg Lys Pro Pro Asp Ser Tyr Pro
   770 775 780
   Pro Lys Asp Asn Pro Arg Arg Leu Leu Gln Cys Gln Asn Val Asp Ile
   785 790 795 800
   Ser Arg Pro Leu Asn Gly Leu Asn Val Pro Gln Pro Gln Ser Thr Gln
   805 810 815
   Arg Val Leu Pro Pro Leu His Arg Ala Pro Arg Ala Pro Ser Val Pro
   820 825 830
   Ala Arg Pro Leu Pro Ala Lys Pro Ala Leu Arg Gln Ala Gln Gly Thr
   835 840 845
   Cys Lys Pro Asn Pro Pro Gln Lys Pro Leu Pro Ala Asp Pro Leu Ala
   850 855 860
   Arg Thr Thr Arg Leu Thr His Ala Leu Ala Arg Thr Pro Gly Gln Trp
   865 870 875 880
   Glu Thr Gly Leu Arg Leu Ala Pro Leu Arg Pro Ala Pro Gln Tyr Pro
   885 890 895
   His Gln Val Pro Arg Ser Thr His Thr Ala Tyr Ile Lys
   900 905
<210> 31
   <211> 123
   <212> PRT
   <213> Homo Sapiens
<400> 31
   Met Val Asp Pro Val Gly Glu Leu Ala Ala Lys Arg Ser Gly Leu Thr
   1               5                   10                  15
   Val Glu Asp Val Arg Ala Asn Val Pro Glu Glu Leu Ser Ile Trp Ile
               20                  25                  30
   Asp Pro Phe Gly Val Ser Tyr Gln Ile Gly Glu Lys Gly Ala Val Lys
           35                  40                  45
   Val Leu Tyr Leu Asp Asp Ser Asp Gly Cys Gly Ala Pro Glu Leu Asp
       50                  55                  60
   Met Lys Ile Lys Ser Ser Phe Thr Pro Asp Asp Gln Met Leu Phe Leu
   65                  70                  75                  80
   Leu Gly Ser Gln Asp Ser Ser Leu Ser Asn Ser Pro Ser Leu Cys Phe
                   85                  90                  95
   Ala His His Pro Ala Pro Pro Ser Ser Ser Ala Ser Pro Ser Pro Ser
               100                 105                 110
   Pro Ser Pro Trp Thr Pro Leu Leu Pro Pro Asn
           115                 120
<210> 32
   <211> 753
   <212> PRT
   <213> Homo Sapiens
<400> 32
<210> 33
   <211> 113
   <212> PRT
   <213> Homo Sapiens
<400> 33
   Met Glu Ile Lys Lys Glu Thr Met Leu Asp Asp Thr Gly Ala Asp Asn
   1 5 10 15
   Lys Arg Arg Glu Pro Lys His Trp Gln Met Arg His Tyr Pro Lys Asn
   20 25 30
   Thr Gly Pro Glu Tyr Val Cys Gln Leu Gly Ser Asp Arg Asn Arg Ser
   35 40 45
   Leu Thr Gln His Thr Phe Ile Ala Leu Leu Leu His Ala Lys Lys Cys
   50 55 60
   Thr Lys Ile Trp Asp Cys Val Thr Gln Arg Arg Lys Arg Leu Ile Ser
   65 70 75 80
   Arg Arg Pro Pro Cys Leu Gly Arg Glu Thr Glu His Gln His Lys Val
   85 90 95
   Val Lys Ser Tyr Glu Gly Gly Glu Cys His Glu Ser Thr Glu Glu Gly
   100 105 110
   Phe
<210> 34
   <211> 475
   <212> PRT
   <213> Homo Sapiens
<400> 34
   Met Ala Ala Ser Ala Leu Arg Gly Leu Pro Val Ala Gly Gly Gly Glu
   1               5                   10                  15
   Ser Ser Glu Ser Glu Asp Asp Gly Trp Glu Ile Gly Tyr Leu Asp Arg
               20                  25                  30
   Thr Ser Gln Lys Leu Lys Arg Leu Leu Pro Ile Glu Glu Lys Lys Glu
           35                  40                  45
   Lys Phe Lys Lys Ala Met Thr Ile Gly Asp Val Ser Leu Val Gln Glu
       50                  55                  60
   Leu Leu Asp Ser Gly Ile Ser Val Asp Ser Asn Phe Gln Tyr Gly Trp
   65                  70                  75                  80
   Thr Pro Leu Met Tyr Ala Ala Ser Val Ala Asn Ala Glu Leu Val Arg
                   85                  90                  95
   Val Leu Leu Asp Arg Gly Ala Asn Ala Ser Phe Glu Lys Asp Lys Gln
               100                 105                 110
   Ser Ile Leu Ile Thr Ala Cys Ser Ala His Gly Ser Glu Glu Gln Ile
           115                 120                 125
   Leu Lys Cys Val Glu Leu Leu Leu Ser Arg Asn Ala Asp Pro Asn Val
       130                 135                 140
   Ala Cys Arg Arg Leu Met Thr Pro Ile Met Tyr Ala Ala Arg Asp Gly
   145                 150                 155                 160
   His Thr Gln Val Val Ala Leu Leu Val Ala His Gly Ala Glu Val Asn
                   165                 170                 175
   Thr Gln Asp Glu Asn Gly Tyr Thr Ala Leu Thr Trp Ala Ala Arg Gln
               180                 185                 190
   Gly His Lys Asn Ile Val Leu Lys Leu Leu Glu Leu Gly Ala Asn Lys
           195                 200                 205
   Met Leu Gln Thr Lys Asp Gly Lys Met Pro Ser Glu Ile Ala Lys Arg
       210                 215                 220
   Asn Lys His His Glu Ile Phe Asn Leu Leu Ser Phe Thr Leu Asn Pro
   225                 230                 235                 240
   Leu Glu Gly Lys Leu Gln Gln Leu Thr Lys Glu Asp Thr Ile Cys Lys
                   245                 250                 255
   Ile Leu Thr Thr Asp Ser Asp Arg Glu Lys Asp His Ile Phe Ser Ser
               260                 265                 270
   Tyr Thr Ala Phe Gly Asp Leu Glu Val Phe Leu His Gly Leu Gly Leu
           275                 280                 285
   Glu His Met Thr Asp Leu Leu Lys Glu Arg Asp Ile Thr Leu Arg His
       290                 295                 300
   Leu Leu Thr Met Arg Glu Asp Glu Phe Thr Lys Asn Gly Ile Thr Ser
   305                 310                 315                 320
   Lys Asp Gln Gln Lys Ile Leu Ala Ala Leu Lys Glu Leu Gln Val Glu
                   325                 330                 335
   Glu Ile Gln Phe Gly Glu Leu Ser Glu Glu Thr Lys Leu Glu Ile Ser
               340                 345                 350
   Gly Asp Glu Phe Leu Asn Phe Leu Leu Lys Leu Asn Lys Gln Cys Gly
           355                 360                 365
   His Leu Ile Thr Ala Val Gln Asn Val Ile Thr Glu Leu Pro Val Asn
       370                 375                 380
   Ser Gln Lys Ile Thr Leu Glu Trp Ala Ser Pro Gln Asn Phe Thr Ser
   385                 390                 395                 400
   Val Cys Glu Glu Leu Val Asn Asn Val Glu Asp Leu Ser Glu Lys Val
                   405                 410                 415
   Cys Lys Leu Lys Asp Leu Ile Gln Lys Leu Gln Asn Glu Arg Glu Asn
               420                 425                 430
   Asp Pro Thr His Ile Gln Leu Arg Glu Glu Val Ser Thr Trp Asn Ser
           435                 440                 445
   Arg Ile Leu Lys Arg Thr Ala Ile Thr Ile Cys Gly Phe Gly Phe Leu
       450                 455                 460
   Leu Phe Ile Cys Lys Leu Thr Phe Gln Arg Lys
   465                 470                 475
<210> 35
   <211> 520
   <212> PRT
   <213> Homo Sapiens
<400> 35
   Met Glu Phe His Asn Gly Gly His Val Ser Gly Ile Gly Gly Phe Leu
   1 5 10 15
   Val Ser Leu Thr Ser Arg Met Lys Pro His Thr Leu Ala Val Thr Pro
   20 25 30
   Ala Leu Ile Phe Ala Ile Thr Val Ala Thr Ile Gly Ser Phe Gln Phe
   35 40 45
   Gly Tyr Asn Thr Gly Val Ile Asn Ala Pro Glu Thr Ile Ile Lys Glu
   50 55 60
   Phe Ile Asn Lys Thr Leu Thr Asp Lys Ala Asn Ala Pro Pro Ser Glu
   65 70 75 80
   Val Leu Leu Thr Asn Leu Trp Ser Leu Ser Val Ala Ile Phe Ser Val
   85 90 95
   Gly Gly Met Ile Gly Ser Phe Ser Val Gly Leu Phe Val Asn Arg Phe
   100 105 110
   Gly Arg Arg Asn Ser Met Leu Ile Val Asn Leu Leu Ala Ala Thr Gly
   115 120 125
   Gly Cys Leu Met Gly Leu Cys Lys Ile Ala Glu Ser Val Glu Met Leu
   130 135 140
   Ile Leu Gly Arg Leu Val Ile Gly Leu Phe Cys Gly Leu Cys Thr Gly
   145 150 155 160
   Phe Val Pro Met Tyr Ile Gly Glu Ile Ser Pro Thr Ala Leu Arg Gly
   165 170 175
   Ala Phe Gly Thr Leu Asn Gln Leu Gly Ile Val Ile Gly Ile Leu Val
   180 185 190
   Ala Gln Ile Phe Gly Leu Glu Leu Ile Leu Gly Ser Glu Glu Leu Trp
   195 200 205
   Pro Val Leu Leu Gly Phe Thr Ile Leu Pro Ala Ile Leu Gln Ser Ala
   210 215 220
   Ala Leu Pro Cys Cys Pro Glu Ser Pro Arg Phe Leu Leu Ile Asn Arg
   225 230 235 240
   Lys Lys Glu Glu Asn Ala Thr Arg Ile Leu Gln Arg Leu Trp Gly Thr
   245 250 255
   Gln Asp Val Ser Gln Asp Ile Gln Glu Met Lys Asp Glu Ser Ala Arg
               260                 265                 270
   Met Ser Gln Glu Lys Gln Val Thr Val Leu Glu Leu Phe Arg Val Ser
           275                 280                 285
   Ser Tyr Arg Gln Pro Ile Ile Ile Ser Ile Val Leu Gln Leu Ser Gln
       290                 295                 300
   Gln Leu Ser Gly Ile Asn Ala Val Phe Tyr Tyr Ser Thr Gly Ile Phe
   305                 310                 315                 320
   Lys Asp Ala Gly Val Gln Gln Pro Ile Tyr Ala Thr Ile Ser Ala Gly
                   325                 330                 335
   Val Val Asn Thr Ile Phe Thr Leu Leu Ser Leu Phe Leu Val Glu Arg
               340                 345                 350
   Ala Gly Arg Arg Thr Leu His Met Ile Gly Leu Gly Gly Met Ala Phe
           355                 360                 365
   Cys Ser Thr Leu Met Thr Val Ser Leu Leu Leu Lys Asn His Tyr Asn
       370                 375                 380
   Gly Met Ser Phe Val Cys Ile Gly Ala Ile Leu Val Phe Val Ala Cys
   385                 390                 395                 400
   Phe Glu Ile Gly Pro Gly Pro Ile Pro Trp Phe Ile Val Ala Glu Leu
                   405                 410                 415
   Phe Ser Gln Gly Pro Arg Pro Ala Ala Met Ala Val Ala Gly Cys Ser
               420                 425                 430
   Asn Trp Thr Ser Asn Phe Leu Val Gly Leu Leu Phe Pro Ser Ala Ala
           435                 440                 445
   Tyr Tyr Leu Gly Ala Tyr Val Phe Ile Ile Phe Thr Gly Phe Leu Ile
       450                 455                 460
   Thr Phe Leu Ala Phe Thr Phe Phe Lys Val Pro Glu Thr Arg Gly Arg
   465                 470                 475                 480
   Thr Phe Glu Asp Ile Thr Arg Ala Phe Glu Gly Gln Ala His Gly Ala
                   485                 490                 495
   Asp Arg Ser Gly Lys Asp Gly Val Met Gly Met Asn Ser Ile Glu Pro
               500                 505                 510
   Ala Lys Glu Thr Thr Thr Asn Val
           515                 520
<210> 36
   <211> 72
   <212> PRT
   <213> Homo Sapiens
<400> 36
   Met Pro Leu Leu Arg Gly Arg Cys Pro Ala Arg Arg His Tyr Arg Arg
   1               5                   10                  15
   Leu Ala Leu Leu Gly Leu Gln Pro Ala Pro Arg Phe Ala His Ser Gly
               20                  25                  30
   Pro Pro Arg Gln Arg Pro Leu Ser Ala Ala Glu Met Ala Val Gly Leu
           35                  40                  45
   Val Val Phe Phe Thr Thr Phe Leu Thr Pro Ala Ala Tyr Val Leu Gly
       50                  55                  60
   Asn Leu Lys Gln Phe Arg Arg Asn
   65                  70
<210> 37
   <211> 626
   <212> PRT
   <213> Homo Sapiens
<400> 37
   Met Met Ala Asn Asp Ala Lys Pro Asp Val Lys Thr Val Gln Val Leu
   1               5                   10                  15
   Arg Asp Thr Ala Asn Arg Leu Arg Ile His Ser Ile Arg Ala Thr Cys
               20                  25                  30
   Ala Ser Gly Ser Gly Gln Leu Thr Ser Cys Cys Ser Ala Ala Glu Val
           35                  40                  45
   Val Ser Val Leu Phe Phe His Thr Met Lys Tyr Lys Gln Thr Asp Pro
       50                  55                  60
   Glu His Pro Asp Asn Asp Arg Phe Ile Leu Ser Arg Gly His Ala Ala
   65                  70                  75                  80
   Pro Ile Leu Tyr Ala Ala Trp Val Glu Val Gly Asp Ile Ser Glu Ser
                   85                  90                  95
   Asp Leu Leu Asn Leu Arg Lys Leu His Ser Asp Leu Glu Arg His Pro
               100                 105                 110
   Thr Pro Arg Leu Pro Phe Val Asp Val Ala Thr Gly Ser Leu Gly Gln
           115                 120                 125
   Gly Leu Gly Thr Ala Cys Gly Met Ala Tyr Thr Gly Lys Tyr Leu Asp
       130                 135                 140
   Lys Ala Ser Tyr Arg Val Phe Cys Leu Met Gly Asp Gly Glu Ser Ser
   145                 150                 155                 160
   Glu Gly Ser Val Trp Glu Ala Phe Ala Phe Ala Ser His Tyr Asn Leu
                   165                 170                 175
   Asp Asn Leu Val Ala Val Phe Asp Val Asn Arg Leu Gly Gln Ser Gly
               180                 185                 190
   Pro Ala Pro Leu Glu His Gly Ala Asp Ile Tyr Gln Asn Cys Cys Glu
           195                 200                 205
   Ala Phe Gly Trp Asn Thr Tyr Leu Val Asp Gly His Asp Val Glu Ala
       210                 215                 220
   Leu Cys Gln Ala Phe Trp Gln Ala Ser Gln Val Lys Asn Lys Pro Thr
   225                 230                 235                 240
   Ala Ile Val Ala Lys Thr Phe Lys Gly Arg Gly Ile Pro Asn Ile Glu
                   245                 250                 255
   Asp Ala Glu Asn Trp His Gly Lys Pro Val Pro Lys Glu Arg Ala Asp
               260                 265                 270
   Ala Ile Val Lys Leu Ile Glu Ser Gln Ile Gln Thr Asn Glu Asn Leu
           275                 280                 285
   Ile Pro Lys Ser Pro Val Glu Asp Ser Pro Gln Ile Ser Ile Thr Asp
       290                 295                 300
   Ile Lys Met Thr Ser Pro Pro Ala Tyr Lys Val Gly Asp Lys Ile Ala
   305                 310                 315                 320
   Thr Gln Lys Thr Tyr Gly Leu Ala Leu Ala Lys Leu Gly Arg Ala Asn
                   325                 330                 335
   Glu Arg Val Ile Val Leu Ser Gly Asp Thr Met Asn Ser Thr Phe Ser
               340                 345                 350
   Glu Ile Phe Arg Lys Glu His Pro Glu Arg Phe Ile Glu Cys Ile Ile
           355                 360                 365
   Ala Glu Gln Asn Met Val Ser Val Ala Leu Gly Cys Ala Thr Arg Gly
       370                 375                 380
   Arg Thr Ile Ala Phe Ala Gly Ala Phe Ala Ala Phe Phe Thr Arg Ala
   385 390 395 400
   Phe Asp Gln Leu Arg Met Gly Ala Ile Ser Gln Ala Asn Ile Asn Leu
   405 410 415
   Ile Gly Ser His Cys Gly Val Ser Thr Gly Glu Asp Gly Val Ser Gln
   420 425 430
   Met Ala Leu Glu Asp Leu Ala Met Phe Arg Ser Ile Pro Asn Cys Thr
   435 440 445
   Val Phe Tyr Pro Ser Asp Ala Ile Ser Thr Glu His Ala Ile Tyr Leu
   450 455 460
   Ala Ala Asn Thr Lys Gly Met Cys Phe Ile Arg Thr Ser Gln Pro Glu
   465 470 475 480
   Thr Ala Val Ile Tyr Thr Pro Gln Glu Asn Phe Glu Ile Gly Gln Ala
   485 490 495
   Lys Val Val Arg His Gly Val Asn Asp Lys Val Thr Val Ile Gly Ala
   500 505 510
   Gly Val Thr Leu His Glu Ala Leu Glu Ala Ala Asp His Leu Ser Gln
   515 520 525
   Gln Gly Ile Ser Val Arg Val Ile Asp Pro Phe Thr Ile Lys Pro Leu
   530 535 540
   Asp Ala Ala Thr Ile Ile Ser Ser Ala Lys Ala Thr Gly Gly Arg Val
   545 550 555 560
   Ile Thr Val Glu Asp His Tyr Arg Glu Gly Gly Ile Gly Glu Ala Val
   565 570 575
   Cys Ala Ala Val Ser Arg Glu Pro Asp Ile Leu Val His Gln Leu Ala
   580 585 590
   Val Ser Gly Val Pro Gln Arg Gly Lys Thr Ser Glu Leu Leu Asp Met
   595 600 605
   Phe Gly Ile Ser Thr Arg His Ile Ile Ala Ala Val Thr Leu Thr Leu
   610 615 620
   Met Lys
   625
<210> 38
   <211> 522
   <212> PRT
   <213> Homo Sapiens
<400> 38
<210> 39
   <211> 294
   <212> PRT
   <213> Homo Sapiens
<400> 39
   Met Asn Phe Ile Leu Phe Ile Phe Ile Pro Gly Val Phe Ser Leu Lys
   1 5 10 15
   Ser Ser Thr Leu Lys Pro Thr Ile Glu Ala Leu Pro Asn Val Leu Pro
   20 25 30
   Leu Asn Glu Asp Val Asn Lys Gln Glu Glu Lys Asn Glu Asp His Thr
   35 40 45
   Pro Asn Tyr Ala Pro Ala Asn Glu Lys Asn Gly Asn Tyr Tyr Lys Asp
   50 55 60
   Ile Lys Gln Tyr Val Phe Thr Thr Gln Asn Pro Asn Gly Thr Glu Ser
   65 70 75 80
   Glu Ile Ser Val Arg Ala Thr Thr Asp Leu Asn Phe Ala Leu Lys Asn
   85 90 95
   Asp Lys Thr Val Asn Ala Thr Thr Tyr Glu Lys Ser Thr Ile Glu Glu
   100 105 110
   Glu Thr Thr Thr Ser Glu Pro Ser His Lys Asn Ile Gln Arg Ser Thr
   115 120 125
   Pro Asn Val Pro Ala Phe Trp Thr Met Leu Ala Lys Ala Ile Asn Gly
   130 135 140
   Thr Ala Val Val Met Asp Asp Lys Asp Gln Leu Phe His Pro Ile Pro
   145 150 155 160
   Glu Ser Asp Val Asn Ala Thr Gln Gly Glu Asn Gln Pro Asp Leu Glu
   165 170 175
   Asp Leu Lys Ile Lys Ile Met Leu Gly Ile Ser Leu Met Thr Leu Leu
   180 185 190
   Leu Phe Val Val Leu Leu Ala Phe Cys Ser Ala Thr Leu Tyr Lys Leu
           195                 200                 205
   Arg His Leu Ser Tyr Lys Ser Cys Glu Ser Gln Tyr Ser Val Asn Pro
       210                 215                 220
   Glu Leu Ala Thr Met Ser Tyr Phe His Pro Ser Glu Gly Val Ser Asp
   225                 230                 235                 240
   Thr Ser Phe Ser Lys Ser Ala Glu Ser Ser Thr Phe Leu Gly Thr Thr
                   245                 250                 255
   Ser Ser Asp Met Arg Arg Ser Gly Thr Arg Thr Ser Glu Ser Lys Ile
               260                 265                 270
   Met Thr Asp Ile Ile Ser Ile Gly Ser Asp Asn Glu Met His Glu Asn
           275                 280                 285
   Asp Glu Ser Val Thr Arg
       290
<210> 40
   <211> 861
   <212> PRT
   <213> Homo Sapiens
<400> 40
   Met Thr Gly Arg Ala Arg Ala Arg Ala Arg Gly Arg Ala Arg Gly Gln
   1               5                   10                  15
   Glu Thr Ala Gln Leu Val Gly Ser Thr Ala Ser Gln Gln Pro Gly Tyr
               20                  25                  30
   Ile Gln Pro Arg Pro Gln Pro Pro Pro Ala Glu Gly Glu Leu Phe Gly
           35                  40                  45
   Arg Gly Arg Gln Arg Gly Thr Ala Gly Gly Thr Ala Lys Ser Gln Gly
       50                  55                  60
   Leu Gln Ile Ser Ala Gly Phe Gln Glu Leu Ser Leu Ala Glu Arg Gly
   65                  70                  75                  80
   Gly Arg Arg Arg Asp Phe His Asp Leu Gly Val Asn Thr Arg Gln Asn
                   85                  90                  95
   Leu Asp His Val Lys Glu Ser Lys Thr Gly Ser Ser Gly Ile Ile Val
               100                 105                 110
   Arg Leu Ser Thr Asn His Phe Arg Leu Thr Ser Arg Pro Gln Trp Ala
           115                 120                 125
   Leu Tyr Gln Tyr His Ile Asp Tyr Asn Pro Leu Met Glu Ala Arg Arg
       130                 135                 140
   Leu Arg Ser Ala Leu Leu Phe Gln His Glu Asp Leu Ile Gly Lys Cys
   145                 150                 155                 160
   His Ala Phe Asp Gly Thr Ile Leu Phe Leu Pro Lys Arg Leu Gln Gln
                   165                 170                 175
   Lys Val Thr Glu Val Phe Ser Lys Thr Arg Asn Gly Glu Asp Val Arg
               180                 185                 190
   Ile Thr Ile Thr Leu Thr Asn Glu Leu Pro Pro Thr Ser Pro Thr Cys
           195                 200                 205
   Leu Gln Phe Tyr Asn Ile Ile Phe Arg Arg Leu Leu Lys Ile Met Asn
       210                 215                 220
   Leu Gln Gln Ile Gly Arg Asn Tyr Tyr Asn Pro Asn Asp Pro Ile Asp
   225                 230                 235                 240
   Ile Pro Ser His Arg Leu Val Ile Trp Pro Gly Phe Thr Thr Ser Ile
                   245                 250                 255
   Leu Gln Tyr Glu Asn Ser Ile Met Leu Cys Thr Asp Val Ser His Lys
               260                 265                 270
   Val Leu Arg Ser Glu Thr Val Leu Asp Phe Met Phe Asn Phe Tyr His
           275                 280                 285
   Gln Thr Glu Glu His Lys Phe Gln Glu Gln Val Ser Lys Glu Leu Ile
       290                 295                 300
   Gly Leu Val Val Leu Thr Lys Tyr Asn Asn Lys Thr Tyr Arg Val Asp
   305                 310                 315                 320
   Asp Ile Asp Trp Asp Gln Asn Pro Lys Ser Thr Phe Lys Lys Ala Asp
                   325                 330                 335
   Gly Ser Glu Val Ser Phe Leu Glu Tyr Tyr Arg Lys Gln Tyr Asn Gln
               340                 345                 350
   Glu Ile Thr Asp Leu Lys Gln Pro Val Leu Val Ser Gln Pro Lys Arg
           355                 360                 365
   Arg Arg Gly Pro Gly Gly Thr Leu Pro Gly Pro Ala Met Leu Ile Pro
       370                 375                 380
   Glu Leu Cys Tyr Leu Thr Gly Leu Thr Asp Lys Met Arg Asn Asp Phe
   385 390 395 400
   Asn Val Met Lys Asp Leu Ala Val His Thr Arg Leu Thr Pro Glu Gln
   405 410 415
   Arg Gln Arg Glu Val Gly Arg Leu Ile Asp Tyr Ile His Lys Asn Asp
   420 425 430
   Asn Val Gln Arg Glu Leu Arg Asp Trp Gly Leu Ser Phe Asp Ser Asn
   435 440 445
   Leu Leu Ser Phe Ser Gly Arg Ile Leu Gln Thr Glu Lys Ile His Gln
   450 455 460
   Gly Gly Lys Thr Phe Asp Tyr Asn Pro Gln Phe Ala Asp Trp Ser Lys
   465 470 475 480
   Glu Thr Arg Gly Ala Pro Leu Ile Ser Val Lys Pro Leu Asp Asn Trp
   485 490 495
   Leu Leu Ile Tyr Thr Arg Arg Asn Tyr Glu Ala Ala Asn Ser Leu Ile
   500 505 510
   Gln Asn Leu Phe Lys Val Thr Pro Ala Met Gly Met Gln Met Arg Lys
   515 520 525
   Ala Ile Met Ile Glu Val Asp Asp Arg Thr Glu Ala Tyr Leu Arg Val
   530 535 540
   Leu Gln Gln Lys Val Thr Ala Asp Thr Gln Ile Val Val Cys Leu Leu
   545 550 555 560
   Ser Ser Asn Arg Lys Asp Lys Tyr Asp Ala Ile Lys Lys Tyr Leu Cys
   565 570 575
   Thr Asp Cys Pro Thr Pro Ser Gln Cys Val Val Ala Arg Thr Leu Gly
   580 585 590
   Lys Gln Gln Thr Val Met Ala Ile Ala Thr Lys Ile Ala Leu Gln Met
   595 600 605
   Asn Cys Lys Met Gly Gly Glu Leu Trp Arg Val Asp Ile Pro Leu Lys
   610 615 620
   Leu Val Met Ile Val Gly Ile Asp Cys Tyr His Asp Met Thr Ala Gly
   625 630 635 640
   Arg Arg Ser Ile Ala Gly Phe Val Ala Ser Ile Asn Glu Gly Met Thr
   645 650 655
   Arg Trp Phe Ser Arg Cys Ile Phe Gln Asp Arg Gly Gln Glu Leu Val
   660 665 670
   Asp Gly Leu Lys Val Cys Leu Gln Ala Ala Leu Arg Ala Trp Asn Ser
   675 680 685
   Cys Asn Glu Tyr Met Pro Ser Arg Ile Ile Val Tyr Arg Asp Gly Val
   690 695 700
   Gly Asp Gly Gln Leu Lys Thr Leu Val Asn Tyr Glu Val Pro Gln Phe
   705 710 715 720
   Leu Asp Cys Leu Lys Ser Ile Gly Arg Gly Tyr Asn Pro Arg Leu Thr
   725 730 735
   Val Ile Val Val Lys Lys Arg Val Asn Thr Arg Phe Phe Ala Gln Ser
   740 745 750
   Gly Gly Arg Leu Gln Asn Pro Leu Pro Gly Thr Val Ile Asp Val Glu
   755 760 765
   Val Thr Arg Pro Glu Trp Tyr Asp Phe Phe Ile Val Ser Gln Ala Val
   770 775 780
   Arg Ser Gly Ser Val Ser Pro Thr His Tyr Asn Val Ile Tyr Asp Asn
   785 790 795 800
   Ser Gly Leu Lys Pro Asp His Ile Gln Arg Leu Thr Tyr Lys Leu Cys
   805 810 815
   His Ile Tyr Tyr Asn Trp Pro Gly Val Ile Arg Val Pro Ala Pro Cys
   820 825 830
   Gln Tyr Ala His Lys Leu Ala Phe Leu Val Gly Gln Ser Ile His Arg
   835 840 845
   Glu Pro Asn Leu Ser Leu Ser Asn Arg Leu Tyr Tyr Leu
   850 855 860
<210> 41
   <211> 221
   <212> PRT
   <213> Homo Sapiens
<400> 41
   Met Pro Leu Ala Leu Thr Leu Leu Leu Leu Ser Gly Leu Gly Ala Pro
   1               5                   10                  15
   Gly Gly Trp Gly Cys Leu Gln Cys Asp Pro Leu Val Leu Glu Ala Leu
               20                  25                  30
   Gly His Leu Arg Ser Ala Leu Ile Pro Ser Arg Phe Gln Leu Glu Gln
           35                  40                  45
   Leu Gln Ala Arg Ala Gly Ala Val Leu Met Gly Met Glu Gly Pro Phe
       50                  55                  60
   Phe Arg Asp Tyr Ala Leu Asn Val Phe Val Gly Lys Val Glu Thr Asn
   65                  70                  75                  80
   Gln Leu Asp Leu Val Ala Ser Phe Val Lys Asn Gln Thr Gln His Leu
                   85                  90                  95
   Met Gly Asn Ser Leu Lys Asp Glu Pro Leu Leu Glu Glu Leu Val Thr
               100                 105                 110
   Leu Arg Ala Asn Val Ile Lys Glu Phe Lys Lys Val Leu Ile Ser Tyr
           115                 120                 125
   Glu Leu Lys Ala Cys Asn Pro Lys Leu Cys Arg Leu Leu Lys Glu Glu
       130                 135                 140
   Val Leu Asp Cys Leu His Cys Gln Arg Ile Thr Pro Lys Cys Ile His
   145                 150                 155                 160
   Lys Lys Tyr Cys Phe Val Asp Arg Gln Pro Arg Val Ala Leu Gln Tyr
                   165                 170                 175
   Gln Met Asp Ser Lys Tyr Pro Arg Asn Gln Ala Leu Leu Gly Ile Leu
               180                 185                 190
   Ile Ser Val Ser Leu Ala Val Phe Val Phe Val Val Ile Val Val Ser
           195                 200                 205
   Ala Cys Thr Tyr Arg Gln Asn Arg Lys Leu Leu Leu Gln
       210                 215                 220
<210> 42
   <211> 1623
   <212> PRT
   <213> Homo Sapiens
<400> 42
   Met Ala Ala Glu Ala Ser Lys Thr Gly Pro Ser Arg Ser Ser Tyr Gln
   1               5                   10                  15
   Arg Met Gly Arg Lys Ser Gln Pro Trp Gly Ala Ala Glu Ile Gln Cys
               20                  25                  30
   Thr Arg Cys Gly Arg Arg Val Ser Arg Ser Ser Gly His His Cys Glu
           35                  40                  45
   Leu Gln Cys Gly His Ala Phe Cys Glu Leu Cys Leu Leu Met Thr Glu
       50                  55                  60
   Glu Cys Thr Thr Ile Ile Cys Pro Asp Cys Glu Val Ala Thr Ala Val
   65                  70                  75                  80
   Asn Thr Arg Gln Arg Tyr Tyr Pro Met Ala Gly Tyr Ile Lys Glu Asp
                   85                  90                  95
   Ser Ile Met Glu Lys Leu Gln Pro Lys Thr Ile Lys Asn Cys Ser Gln
               100                 105                 110
   Asp Phe Lys Lys Thr Ala Asp Gln Leu Thr Thr Gly Leu Glu Arg Ser
           115                 120                 125
   Ala Ser Thr Asp Lys Thr Leu Leu Asn Ser Ser Ala Val Met Leu Asp
       130                 135                 140
   Thr Asn Thr Ala Glu Glu Ile Asp Glu Ala Leu Asn Thr Ala His His
   145                 150                 155                 160
   Ser Phe Glu Gln Leu Ser Ile Ala Gly Lys Ala Leu Glu His Met Gln
                   165                 170                 175
   Lys Gln Thr Ile Glu Glu Arg Glu Arg Val Ile Glu Val Val Glu Lys
               180                 185                 190
   Gln Phe Asp Gln Leu Leu Ala Phe Phe Asp Ser Arg Lys Lys Asn Leu
           195                 200                 205
   Cys Glu Glu Phe Ala Arg Thr Thr Asp Asp Tyr Leu Ser Asn Leu Ile
       210                 215                 220
   Lys Ala Lys Ser Tyr Ile Glu Glu Lys Lys Asn Asn Leu Asn Ala Ala
   225                 230                 235                 240
   Met Asn Ile Ala Arg Ala Leu Gln Leu Ser Pro Ser Leu Arg Thr Tyr
                   245                 250                 255
   Cys Asp Leu Asn Gln Ile Ile Arg Thr Leu Gln Leu Thr Ser Asp Ser
               260                 265                 270
   Glu Leu Ala Gln Val Ser Ser Pro Gln Leu Arg Asn Pro Pro Arg Leu
           275                 280                 285
   Ser Val Asn Cys Ser Glu Ile Ile Cys Met Phe Asn Asn Met Gly Lys
       290                 295                 300
   Ile Glu Phe Arg Asp Ser Thr Lys Cys Tyr Pro Gln Glu Asn Glu Ile
   305                 310                 315                 320
   Arg Gln Asn Val Gln Lys Lys Tyr Asn Asn Lys Lys Glu Leu Ser Cys
                   325                 330                 335
   Tyr Asp Thr Tyr Pro Pro Leu Glu Lys Lys Lys Val Asp Met Ser Val
               340                 345                 350
   Leu Thr Ser Glu Ala Pro Pro Pro Pro Leu Gln Pro Glu Thr Asn Asp
           355                 360                 365
   Val His Leu Glu Ala Lys Asn Phe Gln Pro Gln Lys Asp Val Ala Thr
       370                 375                 380
   Ala Ser Pro Lys Thr Ile Ala Val Leu Pro Gln Met Gly Ser Ser Pro
   385                 390                 395                 400
   Asp Val Ile Ile Glu Glu Ile Ile Glu Asp Asn Val Glu Ser Ser Ala
                   405                 410                 415
   Glu Leu Val Phe Val Ser His Val Ile Asp Pro Cys His Phe Tyr Ile
               420                 425                 430
   Arg Lys Tyr Ser Gln Ile Lys Asp Ala Lys Val Leu Glu Lys Lys Val
           435                 440                 445
   Asn Glu Phe Cys Asn Arg Ser Ser His Leu Asp Pro Ser Asp Ile Leu
       450                 455                 460
   Glu Leu Gly Ala Arg Ile Phe Val Ser Ser Ile Lys Asn Gly Met Trp
   465                 470                 475                 480
   Cys Arg Gly Thr Ile Thr Glu Leu Ile Pro Ile Glu Gly Arg Asn Thr
                   485                 490                 495
   Arg Lys Pro Cys Ser Pro Thr Arg Leu Phe Val His Glu Val Ala Leu
               500                 505                 510
   Ile Gln Ile Phe Met Val Asp Phe Gly Asn Ser Glu Val Leu Ile Val
           515                 520                 525
   Thr Gly Val Val Asp Thr His Val Arg Pro Glu His Ser Ala Lys Gln
       530                 535                 540
   His Ile Ala Leu Asn Asp Leu Cys Leu Val Leu Arg Lys Ser Glu Pro
   545 550 555 560
   Tyr Thr Glu Gly Leu Leu Lys Asp Ile Gln Pro Leu Ala Gln Pro Cys
   565 570 575
   Ser Leu Lys Asp Ile Val Pro Gln Asn Ser Asn Glu Gly Trp Glu Glu
   580 585 590
   Glu Ala Lys Val Glu Phe Leu Lys Met Val Asn Asn Lys Ala Val Ser
   595 600 605
   Met Lys Val Phe Arg Glu Glu Asp Gly Val Leu Ile Val Asp Leu Gln
   610 615 620
   Lys Pro Pro Pro Asn Lys Ile Ser Ser Asp Met Pro Val Ser Leu Arg
   625 630 635 640
   Asp Ala Leu Val Phe Met Glu Leu Ala Lys Phe Lys Ser Gln Ser Leu
   645 650 655
   Arg Ser His Phe Glu Lys Asn Thr Thr Leu His Tyr His Pro Pro Ile
   660 665 670
   Leu Pro Lys Glu Met Thr Asp Val Ser Val Thr Val Cys His Ile Asn
   675 680 685
   Ser Pro Gly Asp Phe Tyr Leu Gln Leu Ile Glu Gly Leu Asp Ile Leu
   690 695 700
   Phe Leu Leu Lys Thr Ile Glu Glu Phe Tyr Lys Ser Glu Asp Gly Glu
   705 710 715 720
   Asn Leu Glu Ile Leu Cys Pro Val Gln Asp Gln Ala Cys Val Ala Lys
   725 730 735
   Phe Glu Asp Gly Ile Trp Tyr Arg Ala Lys Val Ile Gly Leu Pro Gly
   740 745 750
   His Gln Glu Val Glu Val Lys Tyr Val Asp Phe Gly Asn Thr Ala Lys
   755 760 765
   Ile Thr Ile Lys Asp Val Arg Lys Ile Lys Asp Glu Phe Leu Asn Ala
   770 775 780
   Pro Glu Lys Ala Ile Lys Cys Lys Leu Ala Tyr Ile Glu Pro Tyr Lys
   785 790 795 800
   Arg Thr Met Gln Trp Ser Lys Glu Ala Lys Glu Lys Phe Glu Glu Lys
                   805                 810                 815
   Ala Gln Asp Lys Phe Met Thr Cys Ser Val Ile Lys Ile Leu Glu Asp
               820                 825                 830
   Asn Val Leu Leu Val Glu Leu Phe Asp Ser Leu Gly Ala Pro Glu Met
           835                 840                 845
   Thr Thr Thr Ser Ile Asn Asp Gln Leu Val Lys Glu Gly Leu Ala Ser
       850                 855                 860
   Tyr Glu Ile Gly Tyr Ile Leu Lys Asp Asn Ser Gln Lys His Ile Glu
   865                 870                 875                 880
   Val Trp Asp Pro Ser Pro Glu Glu Ile Ile Ser Asn Glu Val His Asn
                   885                 890                 895
   Leu Asn Pro Val Ser Ala Lys Ser Leu Pro Asn Glu Asn Phe Gln Ser
               900                 905                 910
   Leu Tyr Asn Lys Glu Leu Pro Val His Ile Cys Asn Val Ile Ser Pro
           915                 920                 925
   Glu Lys Ile Tyr Val Gln Trp Leu Leu Thr Glu Asn Leu Leu Asn Ser
       930                 935                 940
   Leu Glu Glu Lys Met Ile Ala Ala Tyr Glu Asn Ser Lys Trp Glu Pro
   945                 950                 955                 960
   Val Lys Trp Glu Asn Asp Met His Cys Ala Val Lys Ile Gln Asp Lys
                   965                 970                 975
   Asn Gln Trp Arg Arg Gly Gln Ile Ile Arg Met Val Thr Asp Thr Leu
               980                 985                 990
   Val Glu Val Leu Leu Tyr Asp Val  Gly Val Glu Leu Val  Val Asn Val
           995                 1000                1005
   Asp Cys  Leu Arg Lys Leu Glu  Glu Asn Leu Lys Thr  Met Gly Arg
       1010                1015                1020
   Leu Ser  Leu Glu Cys Ser Leu  Val Asp Ile Arg Pro  Ala Gly Gly
       1025                1030                1035
   Ser Asp  Lys Trp Thr Ala Thr  Ala Cys Asp Cys Leu  Ser Leu Tyr
       1040                1045                1050
   Leu Thr Gly Ala Val Ala Thr Ile Ile Leu Gln Val Asp Ser Glu
   1055 1060 1065
   Glu Asn Asn Thr Thr Trp Pro Leu Pro Val Lys Ile Phe Cys Arg
   1070 1075 1080
   Asp Glu Lys Gly Glu Arg Val Asp Val Ser Lys Tyr Leu Ile Lys
   1085 1090 1095
   Lys Gly Leu Ala Leu Arg Glu Arg Arg Ile Asn Asn Leu Asp Asn
   1100 1105 1110
   Ser His Ser Leu Ser Glu Lys Ser Leu Glu Val Pro Leu Glu Gln
   1115 1120 1125
   Glu Asp Ser Val Val Thr Asn Cys Ile Lys Thr Asn Phe Asp Pro
   1130 1135 1140
   Asp Lys Lys Thr Ala Asp Ile Ile Ser Glu Gln Lys Val Ser Glu
   1145 1150 1155
   Phe Gln Glu Lys Ile Leu Glu Pro Arg Thr Thr Arg Gly Tyr Lys
   1160 1165 1170
   Pro Pro Ala Ile Pro Asn Met Asn Val Phe Glu Ala Thr Val Ser
   1175 1180 1185
   Cys Val Gly Asp Asp Gly Thr Ile Phe Val Val Pro Lys Leu Ser
   1190 1195 1200
   Glu Phe Glu Leu Ile Lys Met Thr Asn Glu Ile Gln Ser Asn Leu
   1205 1210 1215
   Lys Cys Leu Gly Leu Leu Glu Pro Tyr Phe Trp Lys Lys Gly Glu
   1220 1225 1230
   Ala Cys Ala Val Arg Gly Ser Asp Thr Leu Trp Tyr Arg Gly Lys
   1235 1240 1245
   Val Met Glu Val Val Gly Gly Ala Val Arg Val Gln Tyr Leu Asp
   1250 1255 1260
   His Gly Phe Thr Glu Lys Ile Pro Gln Cys His Leu Tyr Pro Ile
   1265 1270 1275
   Leu Leu Tyr Pro Asp Ile Pro Gln Phe Cys Ile Pro Cys Gln Leu
   1280 1285 1290
   His Asn Thr Thr Pro Val Gly Asn Val Trp Gln Pro Asp Ala Ile
   1295 1300 1305
   Glu Val Leu Gln Gln Leu Leu Ser Lys Arg Gln Val Asp Ile His
   1310 1315 1320
   Ile Met Glu Leu Pro Lys Asn Pro Trp Glu Lys Leu Ser Ile His
   1325 1330 1335
   Leu Tyr Phe Asp Gly Met Ser Leu Ser Tyr Phe Met Ala Tyr Tyr
   1340 1345 1350
   Lys Tyr Cys Thr Ser Glu His Thr Glu Glu Met Leu Lys Glu Lys
   1355 1360 1365
   Pro Arg Ser Asp His Asp Lys Lys Tyr Glu Glu Glu Gln Trp Glu
   1370 1375 1380
   Ile Arg Phe Glu Glu Leu Leu Ser Ala Glu Thr Asp Thr Pro Leu
   1385 1390 1395
   Leu Pro Pro Tyr Leu Ser Ser Ser Leu Pro Ser Pro Gly Glu Leu
   1400 1405 1410
   Tyr Ala Val Gln Val Lys His Val Val Ser Pro Asn Glu Val Tyr
   1415 1420 1425
   Ile Cys Leu Asp Ser Ile Glu Thr Ser Asn Gln Ser Asn Gln His
   1430 1435 1440
   Ser Asp Thr Asp Asp Ser Gly Val Ser Gly Glu Ser Glu Ser Glu
   1445 1450 1455
   Ser Leu Asp Glu Ala Leu Gln Arg Val Asn Lys Lys Val Glu Ala
   1460 1465 1470
   Leu Pro Pro Leu Thr Asp Phe Arg Thr Glu Met Pro Cys Leu Ala
   1475 1480 1485
   Glu Tyr Asp Asp Gly Leu Trp Tyr Arg Ala Lys Ile Val Ala Ile
   1490 1495 1500
   Lys Glu Phe Asn Pro Leu Ser Ile Leu Val Gln Phe Val Asp Tyr
   1505 1510 1515
   Gly Ser Thr Ala Lys Leu Thr Leu Asn Arg Leu Cys Gln Ile Pro
   1520 1525 1530
   Ser His Leu Met Arg Tyr Pro Ala Arg Ala Ile Lys Val Leu Leu
   1535 1540 1545
   Ala Gly Phe Lys Pro Pro Leu Arg Asp Leu Gly Glu Thr Arg Ile
   1550 1555 1560
   Pro Tyr Cys Pro Lys Trp Ser Met Glu Ala Leu Trp Ala Met Ile
   1565 1570 1575
   Asp Cys Leu Gln Gly Lys Gln Leu Tyr Ala Val Ser Met Ala Pro
   1580 1585 1590
   Ala Pro Glu Gln Ile Val Thr Leu Tyr Asp Asp Glu Gln His Pro
   1595 1600 1605
   Val His Met Pro Leu Val Glu Met Gly Leu Ala Asp Lys Asp Glu
   1610 1615 1620
<210> 43
   <211> 443
   <212> PRT
   <213> Homo Sapiens
<400> 43
   Met Arg Asn Ala Ile Ile Gln Gly Leu Phe Tyr Gly Ser Leu Thr Phe
   1 5 10 15
   Gly Ile Trp Thr Ala Leu Leu Phe Ile Tyr Leu His His Asn His Val
   20 25 30
   Ser Ser Trp Gln Lys Lys Ser Gln Glu Pro Leu Ser Ala Trp Ser Pro
   35 40 45
   Gly Lys Lys Val His Gln Gln Ile Ile Tyr Gly Ser Glu Gln Ile Pro
   50 55 60
   Lys Pro His Val Ile Val Lys Arg Thr Asp Glu Asp Lys Ala Lys Ser
   65 70 75 80
   Met Leu Gly Thr Asp Phe Asn His Thr Asn Pro Glu Leu His Lys Glu
   85 90 95
   Leu Leu Lys Tyr Gly Phe Asn Val Ile Ile Ser Arg Ser Leu Gly Ile
   100 105 110
   Glu Arg Glu Val Pro Asp Thr Arg Ser Lys Met Cys Leu Gln Lys His
   115 120 125
   Tyr Pro Ala Arg Leu Pro Thr Ala Ser Ile Val Ile Cys Phe Tyr Asn
   130 135 140
   Glu Glu Cys Asn Ala Leu Phe Gln Thr Met Ser Ser Val Thr Asn Leu
   145 150 155 160
   Thr Pro His Tyr Phe Leu Glu Glu Ile Ile Leu Val Asp Asp Met Ser
   165 170 175
   Lys Val Asp Asp Leu Lys Glu Lys Leu Asp Tyr His Leu Glu Thr Phe
   180 185 190
   Arg Gly Lys Val Lys Ile Ile Arg Asn Lys Lys Arg Glu Gly Leu Ile
   195 200 205
   Arg Ala Arg Leu Ile Gly Ala Ser His Ala Ser Gly Asp Val Leu Val
   210 215 220
   Phe Leu Asp Ser His Cys Glu Val Asn Arg Val Trp Leu Glu Pro Leu
   225 230 235 240
   Leu His Ala Ile Ala Lys Asp Pro Lys Met Val Val Cys Pro Leu Ile
   245 250 255
   Asp Val Ile Asp Asp Arg Thr Leu Glu Tyr Lys Pro Ser Pro Leu Val
   260 265 270
   Arg Gly Thr Phe Asp Trp Asn Leu Gln Phe Lys Trp Asp Asn Val Phe
   275 280 285
   Ser Tyr Glu Met Asp Gly Pro Glu Gly Ser Thr Lys Pro Ile Arg Ser
   290 295 300
   Pro Ala Met Ser Gly Gly Ile Phe Ala Ile Arg Arg His Tyr Phe Asn
   305 310 315 320
   Glu Ile Gly Gln Tyr Asp Lys Asp Met Asp Phe Trp Gly Arg Glu Asn
   325 330 335
   Leu Glu Leu Ser Leu Arg Ile Trp Met Cys Gly Gly Gln Leu Phe Ile
   340 345 350
   Ile Pro Cys Ser Arg Val Gly His Ile Ser Lys Lys Gln Thr Gly Lys
   355 360 365
   Pro Ser Thr Ile Ile Ser Ala Met Thr His Asn Tyr Leu Arg Leu Val
   370 375 380
   His Val Trp Leu Asp Glu Tyr Lys Glu Gln Phe Phe Leu Arg Lys Pro
   385 390 395 400
   Gly Leu Lys Tyr Val Thr Tyr Gly Asn Ile Arg Glu Arg Val Glu Leu
   405 410 415
   Arg Lys Arg Leu Gly Cys Lys Ser Phe Gln Trp Tyr Leu Asp Asn Val
   420 425 430
   Phe Pro Glu Leu Glu Ala Ser Val Asn Ser Leu
   435 440
<210> 44
   <211> 138
   <212> PRT
   <213> Homo Sapiens
<400> 44
   Met Asn Ser Leu Val Ser Trp Gln Leu Leu Leu Phe Leu Cys Ala Thr
   1 5 10 15
   His Phe Gly Glu Pro Leu Glu Lys Val Ala Ser Val Gly Asn Ser Arg
   20 25 30
   Pro Thr Gly Gln Gln Leu Glu Ser Leu Gly Leu Leu Ala Pro Gly Glu
   35 40 45
   Gln Ser Leu Pro Cys Thr Glu Arg Lys Pro Ala Ala Thr Ala Arg Leu
   50 55 60
   Ser Arg Arg Gly Thr Ser Leu Ser Pro Pro Pro Glu Ser Ser Gly Ser
   65 70 75 80
   Pro Gln Gln Pro Gly Leu Ser Ala Pro His Ser Arg Gln Ile Pro Ala
   85 90 95
   Pro Gln Gly Ala Val Leu Val Gln Arg Glu Lys Asp Leu Pro Asn Tyr
   100 105 110
   Asn Trp Asn Ser Phe Gly Leu Arg Phe Gly Lys Arg Glu Ala Ala Pro
   115 120 125
   Gly Asn His Gly Arg Ser Ala Gly Arg Gly
   130 135
<210> 45
   <211> 259
   <212> PRT
   <213> Homo Sapiens
<400> 45
   Met Ser Glu Val Pro Val Ala Arg Val Trp Leu Val Leu Leu Leu Leu
   1               5                   10                  15
   Thr Val Gln Val Gly Val Thr Ala Gly Ala Pro Trp Gln Cys Ala Pro
   20 25 30
   Cys Ser Ala Glu Lys Leu Ala Leu Cys Pro Pro Val Ser Ala Ser Cys
   35 40 45
   Ser Glu Val Thr Arg Ser Ala Gly Cys Gly Cys Cys Pro Met Cys Ala
   50 55 60
   Leu Pro Leu Gly Ala Ala Cys Gly Val Ala Thr Ala Arg Cys Ala Arg
   65 70 75 80
   Gly Leu Ser Cys Arg Ala Leu Pro Gly Glu Gln Gln Pro Leu His Ala
   85 90 95
   Leu Thr Arg Gly Gln Gly Ala Cys Val Gln Glu Ser Asp Ala Ser Ala
   100 105 110
   Pro His Ala Ala Glu Ala Gly Ser Pro Glu Ser Pro Glu Ser Thr Glu
   115 120 125
   Ile Thr Glu Glu Glu Leu Leu Asp Asn Phe His Leu Met Ala Pro Ser
   130 135 140
   Glu Glu Asp His Ser Ile Leu Trp Asp Ala Ile Ser Thr Tyr Asp Gly
   145 150 155 160
   Ser Lys Ala Leu His Val Thr Asn Ile Lys Lys Trp Lys Glu Pro Cys
   165 170 175
   Arg Ile Glu Leu Tyr Arg Val Val Glu Ser Leu Ala Lys Ala Gln Glu
   180 185 190
   Thr Ser Gly Glu Glu Ile Ser Lys Phe Tyr Leu Pro Asn Cys Asn Lys
   195 200 205
   Asn Gly Phe Tyr His Ser Arg Gln Cys Glu Thr Ser Met Asp Gly Glu
   210 215 220
   Ala Gly Leu Cys Trp Cys Val Tyr Pro Trp Asn Gly Lys Arg Ile Pro
   225 230 235 240
   Gly Ser Pro Glu Ile Arg Gly Asp Pro Asn Cys Gln Ile Tyr Phe Asn
   245 250 255
   Val Gln Asn
<210> 46
   <211> 407
   <212> PRT
   <213> Homo Sapiens
<400> 46
   Met Pro Arg Gly His Lys Ser Lys Leu Arg Thr Cys Glu Lys Arg Gln
   1               5                   10                  15
   Glu Thr Asn Gly Gln Pro Gln Gly Leu Thr Gly Pro Gln Ala Thr Ala
               20                  25                  30
   Glu Lys Gln Glu Glu Ser His Ser Ser Ser Ser Ser Ser Arg Ala Cys
           35                  40                  45
   Leu Gly Asp Cys Arg Arg Ser Ser Asp Ala Ser Ile Pro Gln Glu Ser
       50                  55                  60
   Gln Gly Val Ser Pro Thr Gly Ser Pro Asp Ala Val Val Ser Tyr Ser
   65                  70                  75                  80
   Lys Ser Asp Val Ala Ala Asn Gly Gln Asp Glu Lys Ser Pro Ser Thr
                   85                  90                  95
   Ser Arg Asp Ala Ser Val Pro Gln Glu Ser Gln Gly Ala Ser Pro Thr
               100                 105                 110
   Gly Ser Pro Asp Ala Gly Val Ser Gly Ser Lys Tyr Asp Val Ala Ala
           115                 120                 125
   Asn Gly Gln Asp Glu Lys Ser Pro Ser Thr Ser His Asp Val Ser Val
       130                 135                 140
   Pro Gln Glu Ser Gln Gly Ala Ser Pro Thr Gly Ser Pro Asp Ala Gly
   145                 150                 155                 160
   Val Ser Gly Ser Lys Tyr Asp Val Ala Ala Glu Gly Glu Asp Glu Glu
                   165                 170                 175
   Ser Val Ser Ala Ser Gln Lys Ala Ile Ile Phe Lys Arg Leu Ser Lys
               180                 185                 190
   Asp Ala Val Lys Lys Lys Ala Cys Thr Leu Ala Gln Phe Leu Gln Lys
           195                 200                 205
   Lys Phe Glu Lys Lys Glu Ser Ile Leu Lys Ala Asp Met Leu Lys Cys
       210                 215                 220
   Val Arg Arg Glu Tyr Lys Pro Tyr Phe Pro Gln Ile Leu Asn Arg Thr
   225                 230                 235                 240
   Ser Gln His Leu Val Val Ala Phe Gly Val Glu Leu Lys Glu Met Asp
   245 250 255
   Ser Ser Gly Glu Ser Tyr Thr Leu Val Ser Lys Leu Gly Leu Pro Ser
   260 265 270
   Glu Gly Ile Leu Ser Gly Asp Asn Ala Leu Pro Lys Ser Gly Leu Leu
   275 280 285
   Met Ser Leu Leu Val Val Ile Phe Met Asn Gly Asn Cys Ala Thr Glu
   290 295 300
   Glu Glu Val Trp Glu Phe Leu Gly Leu Leu Gly Ile Tyr Asp Gly Ile
   305 310 315 320
   Leu His Ser Ile Tyr Gly Asp Ala Arg Lys Ile Ile Thr Glu Asp Leu
   325 330 335
   Val Gln Asp Lys Tyr Val Val Tyr Arg Gln Val Cys Asn Ser Asp Pro
   340 345 350
   Pro Cys Tyr Glu Phe Leu Trp Gly Pro Arg Ala Tyr Ala Glu Thr Thr
   355 360 365
   Lys Met Arg Val Leu Arg Val Leu Ala Asp Ser Ser Asn Thr Ser Pro
   370 375 380
   Gly Leu Tyr Pro His Leu Tyr Glu Asp Ala Leu Ile Asp Glu Val Glu
   385 390 395 400
   Arg Ala Leu Arg Leu Arg Ala
   405
<210> 47
   <211> 568
   <212> DNA
   <213> Artificial Sequence
<220>
   <223> probe derived from KRTAP21-1
<220>
   <221> misc_feature
   <222> (537)..(538)
   <223> n is a, c, g, or t
<220>
   <221> misc_feature
   <222> (541)..(543)
   <223> n is a, c, g, or t
<400> 47
   ctaacttagt gctttgagtt taaattcttt tttcagccac tgtggtatgt tgattactat      60
   attaataatg tgtctccttt atgtgttttt aaatagttac ttggtcatga actatttata     120
   agctttggaa acatgatttc atttattctg caggataact tcaggaagtg ggtattaatt     180
   ctcactgtat aagtgaagac attaagcacc agagtttaag tagcttgatc cactgaaaga     240
   gattcaactg tgtggaacct atgccgcgga gaattgcttt ctgccattgt tattgtccag     300
   cagttgccga atagctctga cctgagcaaa gcagtgaaac gcggtgctgt tgagtgggtt     360
   gccactgcac ctttttcaac tactctagtc ttgtggagaa aacacccctt ctaaattttt     420
   tcttcattga atactctccc tcagctccag ggtaccatat agagttttct tgtattttag     480
   agttactgtt ttatcaaagt gtaccagtct tttgtattac atttctcctg tctaaannaa     540
   nnntgtggtt tctactcatt atggatat                                        568
<210> 48
   <211> 417
   <212> DNA
   <213> Artificial Sequence
<220>
   <223> probe derived from C12orf37
<400> 48
   ttcctcctga ccacaaaaag cacttatact ctaggatgac tgattccagc ccagtggcct      60
   ggcaagggtg aattacacct tgcatatcac actcttgaca tttgtgtgcg ctagcataag     120
   aattataatt gaaacaggga tttaagtatc tcctctctag gtgcctaccc tccttggact     180
   caggtcaaat ttattaaagg aagttttgtt tctagatagg ttgtttgaaa taaaataaca     240
   gaatgttcaa gtaacacagt gtacctacag cttttaacaa aattgaggac ttgggtctcg     300
   aaacaatttc ctttgatttt caggtatttt atctataaaa agggagataa agcattagtt     360
   cataggacag ttatatgttt aaatgtgata atgtatatta accaccttgc atgtatt        417
<210> 49
   <211> 287
   <212> DNA
   <213> Artificial Sequence
<220>
   <223> probe derived from WBSCR28
<220>
   <221> misc_feature
   <222> (118)..(118)
   <223> n is a, c, g, or t
<220>
   <221> misc_feature
   <222> (177)..(178)
   <223> n is a, c, g, or t
<220>
   <221> misc_feature
   <222> (240)..(240)
   <223> n is a, c, g, or t
<400> 49
   taaggccttg caagtgaact gcgtggtaag gaagctcctg gtacagctga gacgtctgta      60
   ttggtgggtg gagactatga ctgccctcac ctcctggcac ctggcctatc tcatcacntg     120
   gaccacctgc ctggcctccc acctgctgca ggctgccttt gagcacacga cccagcnngc     180
   cgaggcccag gaggttgaac cccaggaggt ctcagggtct tccttgctgc cctcactgtn     240
   tgcgtcctcg gactcagagt ctggaacagt tttgccagag caagaaa                  287
<210> 50
   <211> 355
   <212> DNA
   <213> Artificial Sequence
<220>
   <223> probe derived from RBM46
<220>
   <221> misc_feature
   <222> (41)..(41)
   <223> n is a, c, g, or t
<220>
   <221> misc_feature
   <222> (56)..(56)
   <223> n is a, c, g, or t
<400> 50
   agcacttgaa gacaatggtt atagtagatt tgattaccaa ngatcactat ctgtanctgg      60
   agattagaac aattatatga ccagaagcat ctaaccatta tgtaaaaaga aatgatgaga     120
   caaaaagatt aagatacaaa ttttgtgcag tactaaagaa aaagcagtct accattgtgg     180
   tccttgaaaa taactataga tatttttgtt atttgttaga cacaaattat aattttgttg     240
   ttaatgtatt taagcatttt atagttatgc tttgtgtttt tgatattctt tgtattgtta     300
   ataacaagtg ttatgggttt ttaatgttga aatcatgtgt taatttttgt acttg          355
<210> 51
   <211> 381
   <212> DNA
   <213> Artificial Sequence
<220>
   <223> probe derived from DKFZp761D1
<400> 51
   gcagtacatt tcacaatact cagataatcc tctcattgga agcaactcaa atgtccacca     60
   actgatggta ttacccatgc aatggaatat tattcagtca taaaaaggaa tgaagtattg    120
   aggcatgtta caacatgaag gaactttgag gacagtttgt taatacgcta tatactgtgt    180
   aattctattt atatgaaata tccagaatag gtaaatcatt agagactaga gacagcagat    240
   tagtggatgc caggagtttg gggaaggaag gggtgggaag aatgggaagt gactgcttaa    300
   tggatacagg gtttcttttt ggggtgatga aaaatttctg gaactagatt gtagtgatgg    360
   ttgcacaaca ccgtaagtgt a                                              381
<210> 52
   <211> 497
   <212> DNA
   <213> Artificial Sequence
<220>
   <223> probe derived from NBPF4
<220>
   <221> misc_feature
   <222> (284)..(284)
   <223> n is a, c, g, or t
<220>
   <221> misc_feature
   <222> (393)..(393)
   <223> n is a, c, g, or t
<220>
   <221> misc_feature
   <222> (438)..(438)
   <223> n is a, c, g, or t
<400> 52
   gcagagaagg cccagtgtgt ccatccccag tgcggtgata ctaggatggt cacttggtta 60
   aggaggggtc taggagctct gtcccttgta aagacatctt atttgtaagt aatttggaaa 120
   gtggtttgaa atagtataaa tatcctgtat tctagtgatc ttcttcagaa cattttatca 180
   ccaattaatc accccgtctg tgtcagttat tatatttaag tttgtacatt gaaaattgtc 240
   tatctcaaaa tcttacctta tacttgcttt tgctggcatt cttngtaaaa aagatcattc 300
   cctgcccaaa ttttaacttt catccaaaat taattttaat ttctttttgc tggcattctg 360
   ttgtgaaaaa gaatattctc tgccccaatt atnactttca tccaaaatta attttagtcc 420
   atcagttaaa attttaantt ttaaatctgt ttaattaaaa catttcttgc ctctcactct 480
   ggactattgg atttttt 497
<210> 53
   <211> 500
   <212> DNA
   <213> Artificial Sequence
<220>
   <223> probe derived from ADAM12
<400> 53
   gtcttctgga ctggttttca cattagaaga caattgacaa cagttacata attcactctg 60
   agtgttttat gagaaagcct tcttttgggg tcaacagttt tcctatgctt tgaaacagaa 120
   aaatatgtac caagaatctt ggtttgcctt ccagaaaaca aaactgcatt tcactttccc 180
   ggtgttcccc actgtatcta ggcaacatag tattcatgac tatggataaa ctaaacacgt 240
   gacacaaaca cacacaaaag ggaacccagc tctaatacat tccaactcgt atagcatgca 300
   tctgtttatt ctatagttat taagttcttt aaaatgtaaa gccatgctgg aaaataatac 360
   tgctgagata catacagaat tactgtaact gattacactt ggtaattgta ctaaagccaa 420
   acatatatat actattaaaa aggtttacag aattttatgg tgcattacgt gggcattgtc 480
   tttttagatg cccaaatcct 500
<210> 54
   <211> 420
   <212> DNA
   <213> Artificial Sequence
<220>
   <223> probe derived from CDNA clone IMAGE:5299000
<400> 54
   gttgcaatat taccttgggc actgttttga ctgcattcca caagttttta tttgtcatgt        60
   tttaatcacc attcagttca aaatactttc taatgctcct tgttatttct tctttgaccc       120
   attggttata taaaagtacg ttttgcttta tttctaaata tgtgaagact ttctagatat       180
   ctcagttatt tattactaat ttaattttat tgtagccaga gaaaatacac tgtatgtgtt       240
   caatctttgg aaatttcttg agacttattt tttgacccag tatgtttgcc ctcatgaaca       300
   ttccatattc actagaaaaa gaatgtgtgt tcaaataatg ttggatatag tgttatgcaa       360
   atgccactaa atcaagataa atcttttgca ctcttgtttt ttgcctgctt gttctaacaa       420
<210> 55
   <211> 544
   <212> DNA
   <213> Artificial Sequence
<220>
   <223> probe direved from SOX30
<400> 55
   gaaagttttc agtcgtgatt tgaggagtta aaaccaaatg caatttatgt cttcataaaa        60
   ttttgattag tgaaactaga gtctggatgt ttcattgtag gaatatttaa gttattaagt       120
   agtttaattt taatggctga aatttgcatc aacatgtatt attattactt tatcctggaa       180
   catgcaaaat actgaagcct cacagttgta tgtgagggga aaggggaaat aaatctagca       240
   tagtgtgatt tttattttat ctcaggatac attttttaaa tgattttttg tttgcttttt       300
   atgtaatact tatggatgtt gtcaattttt gatgtaacat tttgaaagta ttttgacaac       360
   tcctagtgaa cttggacttg gttgctaaat ttaacttaca ctaataacca attataagtt       420
   ccaaatgtgt tttaatggca cctgggtgat tcttcagcta aatttagtca tttctgtttc       480
   taaatatttt tatcatttta aaatattttt tttccatttg gcatacatcg ttctttgttg       540
   taat                                                                    544
<210> 56
   <211> 493
   <212> DNA
   <213> Artificial Sequence
<220>
   <223> probe direved from CDNA clone IMAGE:5296886
<400> 56
   tgactgtcat atgggaggca ctgcaaggtg aaggagacat gtcctcacat ttggtctttg 60
   ggaagcgaag taggcagaaa cccaagtgca aagtggccat ttgttttcct aatgctagat 120
   aagtacctga tgtgttggaa gggtggagta gaactagaaa ctcctggaag ttcttttaag 180
   aacgaatgca ggacctcatc agcccgagtc tttaaataag tcacacattt ttggtcacca 240
   cattctggaa gggggacaaa aagatctgtc acagacaaaa tttggagata tttaaggctt 300
   tgttgacttt ggcaaatgct tgattatagt tctgtggtct gtttgttttc tagagtctcc 360
   atgaattgca agggaaaaca agtggtacac tggtatttct atttgataac ctgttctctt 420
   tggaattccg gttagaaatc aaaggcttac ttgagtttta atgctttcgg gacttcaaat 480
   gcttttctat ctt 493
<210> 57
   <211> 375
   <212> DNA
   <213> Artificial Sequence
<220>
   <223> probe derived from ASZ1
<400> 57
   gataacactg gaatgggctt ctccccagaa ttttacttca gtttgtgaag aattggttaa 60
   taatgttgaa gatttgagtg aaaaggtctg caaactaaaa gacctaattc aaaagttgca 120
   aaatgaacgg gaaaatgatc caactcatat acaattaagg gaagaagtat ctacatggaa 180
   tagtagaatt ttgaagagga cagctattac catatgcgga ttcggttttc ttcttttcat 240
   ttgcaagcta actttccaga ggaaataatc caaatatttg ttttaagtta tatatacaca 300
   tcttattgaa ccatccaaaa atcattgctt ttaactgttt gcagcacaat ataactttac 360
   attctttgcc aatat 375
<210> 58
   <211> 504
   <212> DNA
   <213> Artificial Sequence
<220>
   <223> probe derived from SLC2A14
<220>
   <221> misc_feature
   <222> (171)..(171)
   <223> n is a, c, g, or t
<220>
   <221> misc_feature
   <222> (184)..(184)
   <223> n is a, c, g, or t
<220>
   <221> misc_feature
   <222> (186)..(186)
   <223> n is a, c, g, or t
<220>
   <221> misc_feature
   <222> (192)..(192)
   <223> n is a, c, g, or t
<220>
   <221> misc_feature
   <222> (264)..(264)
   <223> n is a, c, g, or t
<220>
   <221> misc_feature
   <222> (324)..(324)
   <223> n is a, c, g, or t
<220>
   <221> misc_feature
   <222> (326)..(326)
   <223> n is a, c, g, or t
<220>
   <221> misc_feature
   <222> (330)..(330)
   <223> n is a, c, g, or t
<220>
   <221> misc_feature
   <222> (342)..(342)
   <223> n is a, c, g, or t
<220>
   <221> misc_feature
   <222> (344)..(344)
   <223> n is a, c, g, or t
<400> 58
   agagaccgag tgaacctacc ttcatttcag gagggattgg ccgcttggca catgacaact      60
   ttgccagctt ttcctccctt gggttctgat attgccgcac tagaggatat aggagaggaa     120
   aagtaaggtg cagttgcccc aacctcagac ttaccaggaa gcagatacat ntgagtgtgg     180
   aagncngagg gngtttatgt aagagcacct tcctcacttc catacagctc tacgcggcaa     240
   attaacttga gttttattta tctnatcctc tggtttaatt acataaatat ttatttttta      300
   agtgtaattt tgccaaataa taanancagn aaggaaattg anantagagg gaggtgttta     360
   aagagaggtt atagagtaaa agatttgatg ctggagaggt taaggtgcaa taagaattca     420
   gggagaaatg ttgttcatta ttggagggta aatgatgtgg tgcctgaggt ctgtacatta 480
   cctcttaaca atttctgtcc ttca 504
<210> 59
   <211> 313
   <212> DNA
   <213> Artificial Sequence
<220>
   <223> probe derived from COX8C
<220>
   <221> misc_feature
   <222> (47)..(47)
   <223> n is a, c, g, or t
<400> 59
   ccctgtctgc cgcggaaatg gctgttggac ttgtggtgtt ttttacnacc ttcttaacac 60
   cagctgcata tgtgctaggc aacctgaagc agttcagaag gaattagatg gaagatgatg 120
   ttgaacagct gttaacgtcc aaaaaacttt cagaaaaagc tgtgtttttg ttaacgagca 180
   aaattgccta gttgagttga tgcaaccatt gtggtattca ctttcctcat gtttatgatg 240
   aatattttgc acttttttag tactgtgcat tatatagatg tatagtcaaa aatgttctgc 300
   ttaagtgtta aat 313
<210> 60
   <211> 353
   <212> DNA
   <213> Artificial Sequence
<220>
   <223> probe derived from TKTL2
<400> 60
   gactacaaat atctgactga gctggggatt aaagtagagg taactgtatc ttaaatgagt 60
   atgatttcct tgtaagttaa aaaaattgaa atttaattgt agacttcaat agtccaagtt 120
   ttgaaggatg tttgagcttt tgtataatgc catttatacc tgcagtttta cagataatgt 180
   ttgactgcag ttgccttgga aattcctcca aagtttgcct tcatctctcc tctacagttt 240
   ggaggtgatg gtgcagcagt ggaacatctc ttgatgcacc acactacttg tgttctgtga 300
   agtgatgaaa gtataactgg ttctagtttg cacactacac acatagtttt gtg 353
<210> 61
   <211> 364
   <212> DNA
   <213> Artificial Sequence
<220>
   <223> probe derived from TPTE2P2
<220>
   <221> misc_feature
   <222> (37)..(37)
   <223> n is a, c, g, or t
<220>
   <221> misc_feature
   <222> (90)..(90)
   <223> n is a, c, g, or t
<220>
   <221> misc_feature
   <222> (92)..(92)
   <223> n is a, c, g, or t
<220>
   <221> misc_feature
   <222> (102)..(102)
   <223> n is a, c, g, or t
<220>
   <221> misc_feature
   <222> (107)..(107)
   <223> n is a, c, g, or t
<220>
   <221> misc_feature
   <222> (120)..(120)
   <223> n is a, c, g, or t
<220>
   <221> misc_feature
   <222> (179)..(179)
   <223> n is a, c, g, or t
<220>
   <221> misc_feature
   <222> (214)..(214)
   <223> n is a, c, g, or t
<220>
   <221> misc_feature
   <222> (222)..(222)
   <223> n is a, c, g, or t
<220>
   <221> misc_feature
   <222> (277)..(277)
   <223> n is a, c, g, or t
<400> 61
   ctgaggttct attcatggtg agcaagtctt ttttttngtt tgtttcttca agctctaaca      60
   agggtgccta ctacatggct tttcagttan cnccaaaata anatgtnaca attttttttn     120
   ctattcttag gctttatcta caaagaaatg aattggataa tcttcataaa caaaaaacnt     180
   ggaaaattta tcaaccagaa tatgcagtag aganatattt tnatgagaaa tgacttaagt     240
   tatgttgtaa ctggtagctg attaagtata gttcccngca ccccttctgg gaaagaatta     300
   tgttctttct aaccctgcca catagttata tgttctaaat cttccttgct ggtacatcta     360
   tatt                                                                  364
<210> 62
   <211> 217
   <212> DNA
   <213> Artificial Sequence
<220>
   <223> probe derived from C9orf11
<220>
   <221> misc_feature
   <222> (55)..(55)
   <223> n is a, c, g, or t
<220>
   <221> misc_feature
   <222> (59)..(60)
   <223> n is a, c, g, or t
<220>
   <221> misc_feature
   <222> (62)..(64)
   <223> n is a, c, g, or t
<220>
   <221> misc_feature
   <222> (66)..(70)
   <223> n is a, c, g, or t
<220>
   <221> misc_feature
   <222> (72)..(72)
   <223> n is a, c, g, or t
<220>
   <221> misc_feature
   <222> (74)..(74)
   <223> n is a, c, g, or t
<220>
   <221> misc_feature
   <222> (77)..(79)
   <223> n is a, c, g, or t
<220>
   <221> misc_feature
   <222> (81)..(84)
   <223> n is a, c, g, or t
<220>
   <221> misc_feature
   <222> (88)..(88)
   <223> n is a, c, g, or t
<220>
   <221> misc_feature
   <222> (90)..(93)
   <223> n is a, c, g, or t
<220>
   <221> misc_feature
   <222> (95)..(95)
   <223> n is a, c, g, or t
<220>
   <221> misc_feature
   <222> (99)..(104)
   <223> n is a, c, g, or t
<220>
   <221> misc_feature
   <222> (106)..(110)
   <223> n is a, c, g, or t
<400> 62
   gtatttaaat tactacactg tattcatacc tggagaatat cattatgtga cttanaaann 60
   tnnntnnnnn tncnctnnnc nnnncatnan nnnanagcnn nnnnannnnn tttatactgc 120
   taccttttaa agtcactaaa atcttttaaa tggtgaatgt ctaagagtca gtcttcaggt 180
   atacctgaca aaggatgaga aatgagcttt tgtagaa 217
<210> 63
   <211> 546
   <212> DNA
   <213> Artificial Sequence
<220>
   <223> probe derived from PIWIL1
<220>
   <221> misc_feature
   <222> (148)..(148)
   <223> n is a, c, g, or t
<400> 63
   attcaccggc ttccttattt tatatgtaaa aattaagatt ttatatttta tcttcttgtt        60
   tctcatagat attttgtgag catttttttg tttattttga agaaatgtgg ataagatact      120
   tggtagtata aaacagactc tctgagangt atttgaaatg tgtttggaga tttacttaaa       180
   cgtactttca ggagtgagca agtcctactt ataaacctat attaacttta tttttgagat       240
   acctgttttg aatttaaagg agataagagg cgtaaagtag gatgctcact acaaccatag       300
   gtggggtttc agctcatatc ttaaagataa aaggtactat tatataacct atacacaaga       360
   tacaggagaa aatatgcttg atttttattt ggcagggggg ctaggttgta tgggagtaaa       420
   aaaaacattg aaaattttta aattgtccaa agaaacattt taagactctt taacaaaaaa       480
   ggccatgagt aaatctctat attaacatta ctatttattt tgttttggaa ctgggacatg       540
   attcta                                                                    546
<210> 64
   <211> 521
   <212> DNA
   <213> Artificial Sequence
<220>
   <223> probe derived from C19orf41
<400> 64
   ggccgtgcgt gatgggcatg gaggggcctt tcttccggga ctacgcgctg aacgtgtttg        60
   tggggaaagt ggagacaaat caactggacc ttgtggcgtc ctttgtcaag aaccaaacgc       120
   agcacttaat gggtaactct ctgaaagatg agcctctgct ggaagagctg gtgaccctca       180
   gggcgaatgt gatcaaggaa ttcaagaaag ttttaatttc atatgaatta aaagcctgca       240
   accccaaact ttgccgcttg ctaaaagaag aggtgttgga ctgtttacat tgccagagga       300
   tcactcccaa gtgtatccac aaaaagtact gctttgtcga ccggcaaccc cgcgtggccc       360
   tgcagtacca gatggacagc aaatacccga ggaaccaggc gctgttgggc atcctcattt       420
   ctgtgtctct ggctgtcttt gtcttcgtgg tcatcgtggt ctcggcttgt acatacagac       480
   aaaaccgaaa actcctgctg cagtaggacg gtggtttggg g                            521
<210> 65
   <211> 486
   <212> DNA
   <213> Artificial Sequence
<220>
   <223> probe derived from RNF17
<400> 65
   gaatttaatc ctttatctat cttagtacaa tttgttgatt atggatcaac tgcaaagctg 60
   acattaaaca gactgtgcca aattccttct catcttatgc ggtatccagc tcgagccata 120
   aaggttctct tggcagggtt taaacctccc ttaagggatc taggggagac aagaatacca 180
   tattgtccca aatggagcat ggaggcactg tgggctatga tagactgtct tcaaggaaaa 240
   caactctatg ctgtgtccat ggctccagca ccagaacaga tagtgacatt atatgacgat 300
   gaacagcatc cagttcatat gccgttggta gaaatggggc ttgcagataa agatgaataa 360
   gtgcctaagt gtatacagtg agagcatcta tagaagccta gaagaattct gttatgttta 420
   gactatgtct tatctttaga ctatttcagg cttaattttc ctaacttgtt cagccctagt 480
   gcttta 486
<210> 66
   <211> 306
   <212> DNA
   <213> Artificial Sequence
<220>
   <223> probe derived from GALNTL5
<220>
   <221> misc_feature
   <222> (44)..(44)
   <223> n is a, c, g, or t
<400> 66
   ggaactttca ctaaggatct ggatgtgtgg aggccaactc tttnataatc ccctgctctc 60
   gagtaggaca tatcagtaag aaacaaactg gaaaaccttc tacaatcatc agtgctatga 120
   cacataacta cctaagactg gtgcacgttt ggctggatga atataaggag cagttttttc 180
   ttcgaaagcc tggtctgaaa tatgtcacct acggaaatat tcgcgagcgt gttgagttaa 240
   ggaaacgact gggttgcaag tcatttcagt ggtatttgga taatgtcttc ccagagttgg 300
   aggcat 306
<210> 67
   <211> 511
   <212> DNA
   <213> Artificial Sequence
<220>
   <223> probe derived from KISS1
<400> 67
   aagcctcaag gcacttctag gacctggctc ttctcaccaa gatgaactca ctggtttctt 60
   ggcagctact gcttttcctc tgtgccaccc actttgggga gccattagaa aaggtggcct 120
   ctgtggggaa ttctagaccc acaggccagc agctagaatc cctgggcctc ctggcccccg 180
   gggagcagag cctgccgtgc accgagagga agccagctgc tactgccagg ctgagccgtc 240
   gggggacctc gctgtccccg ccccccgaga gctccgggag ccgccagcag ccgggcctgt 300
   ccgcccccca cagccgccag atccccgcac cccagggcgc ggtgctggtg cagcgggaga 360
   aggacctgcc gaactacaac tggaactcct tcggcctgcg cttcggcaag cgggaggcgg 420
   caccagggaa ccacggcaga agcgctgggc ggggctgggg cgcaggtgcg gggcagtgaa 480
   cttcagaccc caaaggagtc agagcatgcg g 511
<210> 68
   <211> 526
   <212> DNA
   <213> Artificial Sequence
<220>
   <223> probe derived from IGFBP1
<400> 68
   aataatgttc tgtcacgtga aatatttaag tatatagtat atttatactc tagaacatgc 60
   acatttatat atatatgtat atgtatatat atatagtaac tactttttat actccataca 120
   taacttgata tagaaagctg tttatttatt cactgtaagt ttattttttc tacacagtaa 180
   aaacttgtac tatgttaata acttgtccta tgtcaatttg tatatcatga aacacttctc 240
   atcatattgt atgtaagtaa ttgcatttct gctcttccaa agctcctgcg tctgttttta 300
   aagagcatgg aaaaatactg cctagaaaat gcaaaatgaa ataagagaga gtagtttttc 360
   agctagtttg aaggaggacg gttaacttgt atattccacc attcacattt gatgtacatg 420
   tgtagggaaa gttaaaagtg ttgattacat aatcaaagct acctgtggtg atgttgccac 480
   ctgttaaaat gtacactgga tatgttgtta aacacgtgtc gataat 526
<210> 69
   <211> 279
   <212> DNA
   <213> Artificial Sequence
<220>
   <223> probe derived from MAGEB6
<220>
   <221> misc_feature
   <222> (138)..(138)
   <223> n is a, c, g, or t
<220>
   <221> misc_feature
   <222> (167)..(167)
   <223> n is a, c, g, or t
<400> 69
   tcacattcat ggctgtttaa ccaatctgaa agttacggtt tgggaattaa taaaacaaag      60
   tcatacaaca cattttcttt gtaattgaga actagataac atggtaacag agaattgatt     120
   ttcatatgaa tcttaacncc acagtaaaat agttgacatc ataatangaa gagaaagaaa     180
   aggaaaaaca gaaatgtaaa agttgtttaa ttcttggttt gcctaattcg ttttcctatt     240
   tcttttcata caaataaagg atacctggat ttatttagg                            279

## Description

The present invention relates to a prognosis method of lung cancer by using specific genes.

Lung cancer is a disease of uncontrolled cell growth in tissues of the lung. This growth may lead to metastasis, which is the invasion of adjacent tissues and infiltration beyond the lungs. The vast majority of primary lung cancers are carcinomas of the lung, derived from epithelial cells. Lung cancer, the most common cause of cancer-related death in men and women, is responsible for 1.3 million deaths worldwide annually, as of 2004. The most common symptoms are shortness of breath, coughing (including coughing up blood), and weight loss.

Due to the high prevalence of this type of tumors, there is a need to efficiently diagnose lung cancer. Moreover, it is important to propose a prognosis method that allows the pathologist to determine, when a patient is afflicted by lung tumors, the survival rate during a short and a long period, and consequently to propose an adapted therapy. Presently, several clinical and pathological parameters help defining the prognosis, including histological subtypes, TNM stages (tumour size, presence of tumour cells in lymph nodes, presence of distant metastasis).

Cancer Testis (CT) genes are genes that are expressed in testis cells, but not expressed in somatic non pathologic cells. In cancers, CT genes are deregulated and are expressed ectopically in somatic cells. They appear as good candidate for cancer diagnosis. Some works have intended to identify a "general" strategy for diagnosing lung cancer, by detecting cancer testis gene expression.

For instance, the international application WO 2009/121878 discloses the use of a minimal group of CT genes for identifying any somatic or ovarian cancer. However, even if specific genes, or combinations of genes, can be used for diagnosing cancer, there is no indication that these genes can be used to establish a reliable prognosis during a short or a long period.

The international application WO 2006/029176 discloses cancer testis genes (CT genes) as indicative markers for the cancer progression. A liste of 20 prefered genes is cited. Therefore, there is a need to provide prognosis marker that can give specific evolution of tumoral progression, and patient survival, for instance by using CT genes.

Upon pathological diagnosis, the pathologist is able to define the histological subtype of lung cancer. Of all lung tumours, Small cell lung cancers (SCLC) represent the least frequent tumours with the worse outcome. Among Non Small Cell Lung cancers (NSCLC), the most frequent histological subtypes are squamous cell carcinoma (SQC), adenocarcinoma (ADK), large cell neuro-endocrine tumours (LCNE) and basaloid tumours (BAS), the former two being of better prognosis than the latter two. However, although the histological subtype of lung cancer represents one of the parameters influencing prognosis after surgery in operable lung cancer, patients with the same histological type of tumours have variable outcome, some die rapidly with signs of tumour aggressivity (local relapse after surgery, metastasis...) whereas others survive longer and/or die of other causes. Hence tumour aggressivity is not specifically defined by its histological subtype, and there is a need for molecular markers defining particularly aggressive tumours within each given histological subtype, in order to adapt therapeutic approaches accordingly.

Gure Ali O. et al., discloses that cancer-testis genes (CT genes) may find use as markers of poor outcome in Non-Small Cell Lung cancer (NSCLC). They evaluated the association of CT-X expression with variables of disease and outcome. CT-X expression was found to be occasionally associated with advanced disease and other variables that indicate worse prognosis in all major histologic types of NSCLC *(*Clinical Cancer Research, 2005).

One aim of the invention is to provide a simple, rapid, easy-to-use and effective method for giving a prognosis of lung cancer.

Another aim of the invention is to provide a specific prognosis method for lung tumors that have been histologically classified.

The present disclosure describes the use of at least one element chosen among:
- at least 4 genes chosen among a group of 23 genes comprising or consisting of the nucleic acid sequences SEQ ID NO 1 to 23,
- or fragments of said genes
- or complementary sequences of said genes
- or sequences having at least 80% homology with said genes or fragment thereof,
- or protein coded by said genes, said proteins comprising or consisting of the amino acid sequences SEQ ID NO 24 to 46
- or fragments of said proteins,
- or antibodies directed against said proteins,
said at least 4 genes being such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13 , and
d. at least one gene belongs to a fourth set D of 9 genes comprising or consisting of the nucleic acid sequences SEQ ID NO: 14-23,
for the implementation of a prognosis method, preferably *in vitro,* of the survival rate of a patient afflicted by a lung cancer, said lung cancer being previously classified as a lung tumour belonging from the group consisting of adenocarcinma (ADK), Squamous cell carcinoma (SQC), Basaloid tumours (BAS) and Large Cell Neuroendocrine (LCNE), wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
▪ if none of the genes of said set A, B, C and D is expressed, the patient survival rate during a period of time from 30 to 120 months after the diagnosis of said lung cancer is from about 19% to about 88% , and
▪ if at least one gene of one of said sets A, B, C and D is expressed, the patient survival rate during a period of time from 30 to 120 months after the diagnosis of said lung cancer is from about 0% to about 73%.

The present invention relates to the use of at least one element chosen among:
- at least 4 genes chosen among a group of 23 genes comprising or consisting of the nucleic acid sequences SEQ ID NO 1 to 23,
- or complementary sequences of said genes
- or sequences having at least 80% homology with said genes or fragment thereof,
- or protein coded by said genes, said proteins comprising or consisting of the amino acid sequences SEQ ID NO 24 to 46
said at least 4 genes being such that
e. at least one gene belongs to a first set A of 4 genes comprising or consisting of the nucleic acid sequences SEQ ID NO: 1 to 4,
f. at least one gene belongs to a second set B of 3 genes comprising or consisting of the nucleic acid sequences SEQ ID NO: 5 to 7
g. at least one gene belongs to a third set C of 6 genes comprising or consisting of the nucleic acid sequences SEQ ID NO: 8 to 13 , and
h. at least one gene belongs to a fourth set D of 9 genes comprising or consisting of the nucleic acid sequences SEQ ID NO: 14-23,
for the implementation of a prognosis method, preferably *in vitro,* of the survival rate of a patient afflicted by a lung cancer, said lung cancer being previously classified as a lung tumour belonging from the group consisting of adenocarcinma (ADK), Squamous cell carcinoma (SQC), Basaloid tumours (BAS) and Large Cell Neuroendocrine (LCNE), wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
▪ if none of the genes of said set A, B, C and D is expressed, the patient survival rate during a period of time from 30 to 120 months after the diagnosis of said lung cancer is from about 19% to about 88% , and
▪ if at least one gene of one of said sets A, B, C and D is expressed, the patient survival rate during a period of time from 30 to 120 months after the diagnosis of said lung cancer is from about 0% to about 73%.

The present invention is based on the unexpected observation made by the Inventors that the expression of at least 4 genes of a group of 23 determined genes is sufficient to determine the survival rate of a patient afflicted by a lung cancer, said lung cancer being classified among the following histological tumors: (ADK adenocarcinoma), Squamous cell carcinoma (SQC), Basaloid tumours (BAS) and Large Cell Neuroendocrine (LCNE).

In other words, and as explained and exemplified hereafter, according to the invention the determination of the gene expression status on a ON/OFF basis of at least 4 genes chosen among a set of 23 genes allow to estimate at 30 months, or 60 months or 120 months after the diagnosis of a lung cancer, the probability of survival of an individual afflicted by one of the category of lung cancer belonging to the group of ADK, SQC, BAS and LCNE.

The prognosis method proposed by the Inventors can also be carried out by detecting the expression of proteins expressed by the above mentioned at least 4 genes chosen among proteins coded by said 23 genes, also on a absence/presence basis.

Another aspect of the disclosure is that the diagnosis can also be carried out by determining the presence, of at least 4 specific antibodies specifically recognising at least 4 proteins coded by said at least 4 genes mentioned above. The above antibodies are specific of one protein, each protein being coded by one gene of the set of 23 genes.

A key aspect of the disclosure is the concept of ON/OFF for gene expression and presence/absence for the encoded proteins and antibodies detecting these proteins. This specific approach has the advantage of simplifying the analyses and making them independent of complex statistical tests to measure variations in expression levels applied to the majority of the existing tests.

The ON/OFF status of gene expression is established by determination of a threshold of gene expression allowing them to decide on the ON/OFF status of a gene such that:
a. if a gene is expressed at a level lower than the threshold, the gene is considered as not expressed or weakly expressed (defined as OFF), and
b. if a gene is expressed at a level upper to the threshold, the gene is considered as being expressed (defined as ON).

The Inventors have identified 23 genes comprising or being constituted by the nucleic acid sequences SEQ ID NO 1 to 23, as being cancer testis genes (CT genes) that can be used to carry out the prognosis method according to the invention.

The above 23 genes have been identified as being liable to be "expressed" (form here by, expressed refers to the ON status and not expressed to the OFF status) in lung cancer cells, but not in healthy samples. In other words, the above 23 genes are such as
a. they are not expressed, or weakly expressed in lung cells, and
b. they maybe expressed in lung tumor cells.

By "not expressed" it is defined in the invention the fact that the transcription of a gene is either not carried out, or is not detectable by common techniques known in the art, such as Quantitative RT-PCR, Northern blot or when microarrays data are considered.

By "weakly expressed", it is defined in the invention that a gene is expressed at a low level, meaning that the values are within the range of those measured for healthy tissue samples by Q-RT-PCR and by Northern blots or below the threshold when microarray data are considered. These values are considered as false-positive expressions, due to probe cross hybridization for instance. All the expression falling in these categories are considered as "OFF".

The difference between the absence of expression, or weak expression, and the expression determines its ON/OFF status, which is a key step of the invention. Indeed, the Inventors have identified that the ON status of the above 23 genes is a key step to determine the prognosis of lung cancer.

On microarrays, the expression level of the above mentioned genes is determined by the fact that a threshold of expression has been identified by the Inventors allowing to determine expression (ON) and non-expression (OFF) of said genes. The threshold determination is detailed hereafter, in the Example section.

For the microarrays, the threshold enabling to determine the expression status of a gene (ON versus OFF) is calculated by using the signal mean value and distribution obtained from transcriptomic data (in the same technology) with the corresponding probes in a large number of somatic tissues (which do not express the genes).

A similar strategy enables determining a threshold for the presence/absence of the encoded proteins or antibodies. For each protein or antibody, the mean value and distribution of the signal intensities obtained in an appropriate number of control somatic tissues serves as a basis for calculating the threshold.

According to the invention, the prognosis is carried out as described hereafter:
By "expressed", the invention defined that the transcript of a gene is detectable by the above known techniques while it is not detectable in healthy tissues or determined as being above the threshold when microarrays data are considered.

In the invention, the terms "for the implementation of a prognosis method" means "for carrying out a prognosis method". The skilled person understands that he can obtain a prognosis according to the invention when using one of the element mentioned above. The 23 genes according to the invention have been classified by the Inventors in four sets:
a. a first set of 4 genes,
b. a second set of 3 genes
c. a third set of 6 genes, and
d. a fourth set of 9 genes.

The first set, also called in the invention set A, of 4 genes consists of the genes comprising or constituted by the nucleic acid sequences SEQ ID NO: 1, SEQ ID NO: 2, SEQ ID NO: 3, SEQ ID NO: 4.

The second set, also called in the invention set B, of 3 genes consists of the genes comprising or constituted by the nucleic acid sequences SEQ ID NO: 5, SEQ ID NO: 6 and SEQ ID NO: 7.

The third set, also called in the invention set C, of 6 genes consists of the genes comprising or constituted by the nucleic acid sequences SEQ ID NO: 8, SEQ ID NO: 9, SEQ ID NO: 10, SEQ ID NO: 11, SEQ ID NO: 12 and SEQ ID NO: 13.

The fourth set also called in the invention set D, of 9 genes consists of the genes comprising or constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, and SEQ ID NO: 23.

Set A is specific of BAS tumors, set B is specific of ADK tumors, set C is specific of SQC tumors and set D is specific of LCNE tumors.

The inventors have also defined subsets of each of the above sets A and B as follows:
Set A is divided into 4 subsets A1, A4 and A5, said subset being such that:
   a. subset A1 consists of the genes comprising or being constituted by the nucleic acid sequences SEQ ID NO: 1 and SEQ ID NO: 2,
   b. subset A4 consists of the gene comprising or being constituted by the nucleic acid sequence SEQ ID NO: 3, and
   c. subset A5 consists of the genes comprising or being constituted by the nucleic acid sequence SEQ ID NO: 4,
Set A can also be divided into the following subsets:
   a. subset A1 consists of the genes comprising or being constituted by the nucleic acid sequences SEQ ID NO: 1 and SEQ ID NO: 2,
   b. subset A2 consists of the genes comprising or being constituted by the nucleic acid sequences SEQ ID NO: 1, SEQ ID NO: 2 and SEQ ID NO: 3, and
   c. subset A3 consists of the genes comprising or being constituted by the nucleic acid sequences SEQ ID NO: 1, SEQ ID NO: 2, SEQ ID NO: 3 and SEQ ID NO: 4.
Set B is divided into 2 subsets B1 and B3, said subset being such that:
   a. subset B1 consists of the genes comprising or being constituted by the nucleic acid sequences SEQ ID NO: 5 and SEQ ID NO: 6, and
   b. subset B3 consists of the gene comprising or being constituted by the nucleic acid sequence SEQ ID NO: 7.
Set B can also be divided into the following subsets:
   c. subset B1 consists of the genes comprising or being constituted by the nucleic acid sequences SEQ ID NO: 5, and SEQ ID NO: 6,
   d. subset B2 consists of the genes comprising or being constituted by the nucleic acid sequences SEQ ID NO: 5, SEQ ID NO: 6 and SEQ ID NO: 7.
Set C is divided into 3 subsets C1, C4 and C5, said subset being such that:
   a. subset C1 consists of the genes comprising or being constituted by the nucleic acid sequences SEQ ID NO: 8 and SEQ ID NO: 9,
   b. subset C4 consists of the genes comprising or being constituted by the nucleic acid sequences SEQ ID NO: 10 and SEQ ID NO: 11, and
   c. subset C5 consists of the genes comprising or being constituted by the nucleic acid sequences SEQ ID NO: 12 and SEQ ID NO: 13.
Set C can also be divided into the following subsets:
   a. subset C1 consists of the genes comprising or being constituted by the nucleic acid sequences SEQ ID NO: 8, and SEQ ID NO: 9,
   b. subset C2 consists of the genes comprising or being constituted by the nucleic acid sequences SEQ ID NO: 8, SEQ ID NO: 9, SEQ ID NO: 10 and SEQ ID NO: 11, and
   c. subset C3 consists of the genes comprising or being constituted by the nucleic acid sequences SEQ ID NO: 8, SEQ ID NO: 9, SEQ ID NO: 10, SEQ ID NO: 11, SEQ ID NO: 12 and SEQ ID NO: 13.
Set D is divided into 3 subsets D1, D5, D6 and D7, said subset being such that:
   a. subset D1 consists of the genes comprising or being constituted by the nucleic acid sequences SEQ ID NO: 14 and SEQ ID NO: 15,
   b. subset D5 consists of the genes comprising or being constituted by the nucleic acid sequences SEQ ID NO: 16, SEQ ID NO: 17, SEQ ID NO: 18, SEQ ID NO: 19 and SEQ ID NO: 20,
   c. subset D6 consists of the gene comprising or being constituted by the nucleic acid sequence SEQ ID NO: 21 and
   d. subset D7 consists of the genes comprising or being constituted by the nucleic acid sequences SEQ ID NO: 22 and SEQ ID NO: 23.
Set D can also be divided into the following subsets:
   a. subset D1 consists of the genes comprising or being constituted by the nucleic acid sequences SEQ ID NO: 14 and SEQ ID NO: 15,
   b. subset D2 consists of the genes comprising or being constituted by the nucleic acid sequences SEQ ID NO: 14 and SEQ ID NO: 15, SEQ ID NO: 16, SEQ ID NO: 17, SEQ ID NO: 18, SEQ ID NO: 19 and SEQ ID NO: 20,
   c. subset D3 consists of the genes comprising or being constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, and
   d. subset D4 consists of the genes comprising or being constituted by the nucleic acid sequences SEQ ID NO: 14, SEQ ID NO: 15, SEQ ID NO: 16, SEQ ID NO: 17, SEQ ID NO: 18, SEQ ID NO: 19, SEQ ID NO: 20 , SEQ ID NO: 21, SEQ ID NO: 22 and SEQ ID NO: 23.

Thus, the prognosis method according to the invention is such that, when determining the expression status of 4 genes on a ON/OFF basis, belonging to the set of 23 genes comprising or consisting of nucleic acid sequences SEQ ID NO: 1 to 23,
a. if none of said at least 4 genes are expressed , the patient survival rate at 30 months, 60 months or 120 months following the diagnosis is from about 19% to about 88% or more, and
b. if at least one gene of the sets A, B, C or D is expressed, according to the defined expression threshold, the patient survival rate at 30 months, 60 months or 120 months following the diagnosis is from about 0% to about 73%.

Therefore, the prognosis method according to the invention can be carried out as by measuring at least the expression of the following 720 combinations of genes:
SEQ ID NO: 1+5+8+14, SEQ ID NO: 1+5+9+14, SEQ ID NO: 1+5+10+14, SEQ ID NO: 1+5+11+14, SEQ ID NO: 1+5+12+14, SEQ ID NO: 1+5+12+14, SEQ ID NO: 1+5+8+15, SEQ ID NO: 1+5+9+15, SEQ ID NO: 1+5+10+15, SEQ ID NO: 1+5+11+15, SEQ ID NO: 1+5+12+15, SEQ ID NO: 1+5+12+15, SEQ ID NO: 1+5+8+16, SEQ ID NO: 1+5+9+16, SEQ ID NO: 1+5+10+16, SEQ ID NO: 1+5+11+16, SEQ ID NO: 1+5+12+16, SEQ ID NO: 1+5+12+16, SEQ ID NO: 1+5+8+17, SEQ ID NO: 1+5+9+17, SEQ ID NO: 1+5+10+17, SEQ ID NO: 1+5+11+17, SEQ ID NO: 1+5+12+17, SEQ ID NO: 1+5+12+17, SEQ ID NO: 1+5+8+18, SEQ ID NO: 1+5+9+18, SEQ ID NO: 1+5+10+18, SEQ ID NO: 1+5+11+18, SEQ ID NO: 1+5+12+18, SEQ ID NO: 1+5+12+18, SEQ ID NO: 1+5+8+19, SEQ ID NO: 1+5+9+19, SEQ ID NO: 1+5+10+19, SEQ ID NO: 1+5+11+19, SEQ ID NO: 1+5+12+19, SEQ ID NO: 1+5+12+19, SEQ ID NO: 1+5+8+20, SEQ ID NO: 1+5+9+20, SEQ ID NO: 1+5+10+20, SEQ ID NO: 1+5+11+20, SEQ ID NO: 1+5+12+20, SEQ ID NO: 1+5+12+20, SEQ ID NO: 1+5+8+21, SEQ ID NO: 1+5+9+21, SEQ ID NO: 1+5+10+21, SEQ ID NO: 1+5+11+21, SEQ ID NO: 1+5+12+21, SEQ ID NO: 1+5+12+21, SEQ ID NO: 1+5+8+22, SEQ ID NO: 1+5+9+22, SEQ ID NO: 1+5+10+22, SEQ ID NO: 1+5+11+22, SEQ ID NO: 1+5+12+22, SEQ ID NO: 1+5+12+22, SEQ ID NO: 1+5+8+23, SEQ ID NO: 1+5+9+23, SEQ ID NO: 1+5+10+23, SEQ ID NO: 1+5+11+23, SEQ ID NO: 1+5+12+23, SEQ ID NO: 1+5+12+23, SEQ ID NO: 1+6+8+14, SEQ ID NO: 1+6+9+14, SEQ ID NO: 1+6+10+14, SEQ ID NO: 1+6+11+14, SEQ ID NO: 1+6+12+14, SEQ ID NO: 1+6+12+14, SEQ ID NO: 1+6+8+15, SEQ ID NO: 1+6+9+15, SEQ ID NO: 1+6+10+15, SEQ ID NO: 1+6+11+15, SEQ ID NO: 1+6+12+15, SEQ ID NO: 1+6+12+15, SEQ ID NO: 1+6+8+16, SEQ ID NO: 1+6+9+16, SEQ ID NO: 1+6+10+16, SEQ ID NO: 1+6+11+16, SEQ ID NO: 1+6+12+16, SEQ ID NO: 1+6+12+16, SEQ ID NO: 1+6+8+17, SEQ ID NO: 1+6+9+17, SEQ ID NO: 1+6+10+17, SEQ ID NO: 1+6+11+17, SEQ ID NO: 1+6+12+17, SEQ ID NO: 1+6+12+17, SEQ ID NO: 1+6+8+18, SEQ ID NO: 1+6+9+18, SEQ ID NO: 1+6+10+18, SEQ ID NO: 1+6+11+18, SEQ ID NO: 1+6+12+18, SEQ ID NO: 1+6+12+18, SEQ ID NO: 1+6+8+19, SEQ ID NO: 1+6+9+19, SEQ ID NO: 1+6+10+19, SEQ ID NO: 1+6+11+19, SEQ ID NO: 1+6+12+19, SEQ ID NO: 1+6+12+19, SEQ ID NO: 1+6+8+20, SEQ ID NO: 1+6+9+20, SEQ ID NO: 1+6+10+20, SEQ ID NO: 1+6+11+20, SEQ ID NO: 1+6+12+20, SEQ ID NO: 1+6+12+20, SEQ ID NO: 1+6+8+21, SEQ ID NO: 1+6+9+21, SEQ ID NO: 1+6+10+21, SEQ ID NO: 1+6+11+21, SEQ ID NO: 1+6+12+21, SEQ ID NO: 1+6+12+21, SEQ ID NO: 1+6+8+22, SEQ ID NO: 1+6+9+22, SEQ ID NO: 1+6+10+22, SEQ ID NO: 1+6+11+22, SEQ ID NO: 1+6+12+22, SEQ ID NO: 1+6+12+22, SEQ ID NO: 1+6+8+23, SEQ ID NO: 1+6+9+23, SEQ ID NO: 1+6+10+23, SEQ ID NO: 1+6+11+23, SEQ ID NO: 1+6+12+23, SEQ ID NO: 1+6+12+23, SEQ ID NO: 1+7+8+14, SEQ ID NO: 1+7+9+14, SEQ ID NO: 1+7+10+14, SEQ ID NO: 1+7+11+14, SEQ ID NO: 1+7+12+14, SEQ ID NO: 1+7+12+14, SEQ ID NO: 1+7+8+15, SEQ ID NO: 1+7+9+15, SEQ ID NO: 1+7+10+15, SEQ ID NO: 1+7+11+15, SEQ ID NO: 1+7+12+15, SEQ ID NO: 1+7+12+15, SEQ ID NO: 1+7+8+16, SEQ ID NO: 1+7+9+16, SEQ ID NO: 1+7+10+16, SEQ ID NO: 1+7+11+16, SEQ ID NO: 1+7+12+16, SEQ ID NO: 1+7+12+16, SEQ ID NO: 1+7+8+17, SEQ ID NO: 1+7+9+17, SEQ ID NO: 1+7+10+17, SEQ ID NO: 1+7+11+17, SEQ ID NO: 1+7+12+17, SEQ ID NO: 1+7+12+17, SEQ ID NO: 1+7+8+18, SEQ ID NO: 1+7+9+18, SEQ ID NO: 1+7+10+18, SEQ ID NO: 1+7+11+18, SEQ ID NO: 1+7+12+18, SEQ ID NO: 1+7+12+18, SEQ ID NO: 1+7+8+19, SEQ ID NO: 1+7+9+19, SEQ ID NO: 1+7+10+19, SEQ ID NO: 1+7+11+19, SEQ ID NO: 1+7+12+19, SEQ ID NO: 1+7+12+19, SEQ ID NO: 1+7+8+20, SEQ ID NO: 1+7+9+20, SEQ ID NO: 1+7+10+20, SEQ ID NO: 1+7+11+20, SEQ ID NO: 1+7+12+20, SEQ ID NO: 1+7+12+20, SEQ ID NO: 1+7+8+21, SEQ ID NO: 1+7+9+21, SEQ ID NO: 1+7+10+21, SEQ ID NO: 1+7+11+21, SEQ ID NO: 1+7+12+21, SEQ ID NO: 1+7+12+21, SEQ ID NO: 1+7+8+22, SEQ ID NO: 1+7+9+22, SEQ ID NO: 1+7+10+22, SEQ ID NO: 1+7+11+22, SEQ ID NO: 1+7+12+22, SEQ ID NO: 1+7+12+22, SEQ ID NO: 1+7+8+23, SEQ ID NO: 1+7+9+23, SEQ ID NO: 1+7+10+23, SEQ ID NO: 1+7+11+23, SEQ ID NO: 1+7+12+23, SEQ ID NO: 1+7+12+23, SEQ ID NO: 2+5+8+14, SEQ ID NO: 2+5+9+14, SEQ ID NO: 1+5+10+14, SEQ ID NO: 2+5+11+14, SEQ ID NO: 2+5+12+14, SEQ ID NO: 2+5+12+14, SEQ ID NO: 2+5+8+15, SEQ ID NO: 2+5+9+15, SEQ ID NO: 1+5+10+15, SEQ ID NO: 2+5+11+15, SEQ ID NO: 2+5+12+15, SEQ ID NO: 2+5+12+15, SEQ ID NO: 2+5+8+16, SEQ ID NO: 2+5+9+16, SEQ ID NO: 1+5+10+16, SEQ ID NO: 2+5+11+16, SEQ ID NO: 2+5+12+16, SEQ ID NO: 2+5+12+16, SEQ ID NO: 2+5+8+17, SEQ ID NO: 2+5+9+17, SEQ ID NO: 1+5+10+17, SEQ ID NO: 2+5+11+17, SEQ ID NO: 2+5+12+17, SEQ ID NO: 2+5+12+17, SEQ ID NO: 2+5+8+18, SEQ ID NO: 2+5+9+18, SEQ ID NO: 1+5+10+18, SEQ ID NO: 2+5+11+18, SEQ ID NO: 2+5+12+18, SEQ ID NO: 2+5+12+18, SEQ ID NO: 2+5+8+19, SEQ ID NO: 2+5+9+19, SEQ ID NO: 1+5+10+19, SEQ ID NO: 2+5+11+19, SEQ ID NO: 2+5+12+19, SEQ ID NO: 2+5+12+19, SEQ ID NO: 2+5+8+20, SEQ ID NO: 2+5+9+20, SEQ ID NO: 1+5+10+20, SEQ ID NO: 2+5+11+20, SEQ ID NO: 2+5+12+20, SEQ ID NO: 2+5+12+20, SEQ ID NO: 2+5+8+21, SEQ ID NO: 2+5+9+21, SEQ ID NO: 1+5+10+21, SEQ ID NO: 2+5+11+21, SEQ ID NO: 2+5+12+21, SEQ ID NO: 2+5+12+21, SEQ ID NO: 2+5+8+22, SEQ ID NO: 2+5+9+22, SEQ ID NO: 1+5+10+22, SEQ ID NO: 2+5+11+22, SEQ ID NO: 2+5+12+22, SEQ ID NO: 2+5+12+22, SEQ ID NO: 2+5+8+23, SEQ ID NO: 2+5+9+23, SEQ ID NO: 1+5+10+23, SEQ ID NO: 2+5+11+23, SEQ ID NO: 2+5+12+23, SEQ ID NO: 2+5+12+23, SEQ ID NO: 2+6+8+14, SEQ ID NO: 2+6+9+14, SEQ ID NO: 2+6+10+14, SEQ ID NO: 2+6+11+14, SEQ ID NO: 2+6+12+14, SEQ ID NO: 2+6+12+14, SEQ ID NO: 2+6+8+15, SEQ ID NO: 2+6+9+15, SEQ ID NO: 2+6+10+15, SEQ ID NO: 2+6+11+15, SEQ ID NO: 2+6+12+15, SEQ ID NO: 2+6+12+15, SEQ ID NO: 2+6+8+16, SEQ ID NO: 2+6+9+16, SEQ ID NO: 2+6+10+16, SEQ ID NO: 2+6+11+16, SEQ ID NO: 2+6+12+16, SEQ ID NO: 2+6+12+16, SEQ ID NO: 2+6+8+17, SEQ ID NO: 2+6+9+17, SEQ ID NO: 2+6+10+17, SEQ ID NO: 2+6+11+17, SEQ ID NO: 2+6+12+17, SEQ ID NO: 2+6+12+17, SEQ ID NO: 2+6+8+18, SEQ ID NO: 2+6+9+18, SEQ ID NO: 2+6+10+18, SEQ ID NO: 2+6+11+18, SEQ ID NO: 2+6+12+18, SEQ ID NO: 2+6+12+18, SEQ ID NO: 2+6+8+19, SEQ ID NO: 2+6+9+19, SEQ ID NO: 2+6+10+19, SEQ ID NO: 2+6+11+19, SEQ ID NO: 2+6+12+19, SEQ ID NO: 2+6+12+19, SEQ ID NO: 2+6+8+20, SEQ ID NO: 2+6+9+20, SEQ ID NO: 2+6+10+20, SEQ ID NO: 2+6+11+20, SEQ ID NO: 2+6+12+20, SEQ ID NO: 2+6+12+20, SEQ ID NO: 2+6+8+21, SEQ ID NO: 2+6+9+21, SEQ ID NO: 2+6+10+21, SEQ ID NO: 2+6+11+21, SEQ ID NO: 2+6+12+21, SEQ ID NO: 2+6+12+21, SEQ ID NO: 2+6+8+22, SEQ ID NO: 2+6+9+22, SEQ ID NO: 2+6+10+22, SEQ ID NO: 2+6+11+22, SEQ ID NO: 2+6+12+22, SEQ ID NO: 2+6+12+22, SEQ ID NO: 2+6+8+23, SEQ ID NO: 2+6+9+23, SEQ ID NO: 2+6+10+23, SEQ ID NO: 2+6+11+23, SEQ ID NO: 2+6+12+23, SEQ ID NO: 2+6+12+23, SEQ ID NO: 2+7+8+14, SEQ ID NO: 2+7+9+14, SEQ ID NO: 2+7+10+14, SEQ ID NO: 2+7+11+14, SEQ ID NO: 2+7+12+14, SEQ ID NO: 2+7+12+14, SEQ ID NO: 2+7+8+15, SEQ ID NO: 2+7+9+15, SEQ ID NO: 2+7+10+15, SEQ ID NO: 2+7+11+15, SEQ ID NO: 2+7+12+15, SEQ ID NO: 2+7+12+15, SEQ ID NO: 2+7+8+16, SEQ ID NO: 2+7+9+16, SEQ ID NO: 2+7+10+16, SEQ ID NO: 2+7+11+16, SEQ ID NO: 2+7+12+16, SEQ ID NO: 2+7+12+16, SEQ ID NO: 2+7+8+17, SEQ ID NO: 2+7+9+17, SEQ ID NO: 2+7+10+17, SEQ ID NO: 2+7+11+17, SEQ ID NO: 2+7+12+17, SEQ ID NO: 2+7+12+17, SEQ ID NO: 2+7+8+18, SEQ ID NO: 2+7+9+18, SEQ ID NO: 2+7+10+18, SEQ ID NO: 2+7+11+18, SEQ ID NO: 2+7+12+18, SEQ ID NO: 2+7+12+18, SEQ ID NO: 2+7+8+19, SEQ ID NO: 2+7+9+19, SEQ ID NO: 2+7+10+19, SEQ ID NO: 2+7+11+19, SEQ ID NO: 2+7+12+19, SEQ ID NO: 2+7+12+19, SEQ ID NO: 2+7+8+20, SEQ ID NO: 2+7+9+20, SEQ ID NO: 2+7+10+20, SEQ ID NO: 2+7+11+20, SEQ ID NO: 2+7+12+20, SEQ ID NO: 2+7+12+20, SEQ ID NO: 2+7+8+21, SEQ ID NO: 2+7+9+21, SEQ ID NO: 2+7+10+21, SEQ ID NO: 2+7+11+21, SEQ ID NO: 2+7+12+21, SEQ ID NO: 2+7+12+21, SEQ ID NO: 2+7+8+22, SEQ ID NO: 2+7+9+22, SEQ ID NO: 2+7+10+22, SEQ ID NO: 2+7+11+22, SEQ ID NO: 2+7+12+22, SEQ ID NO: 2+7+12+22, SEQ ID NO: 2+7+8+23, SEQ ID NO: 2+7+9+23, SEQ ID NO: 2+7+10+23, SEQ ID NO: 2+7+11+23, SEQ ID NO: 2+7+12+23, SEQ ID NO: 2+7+12+23, SEQ ID NO: 3+5+8+14, SEQ ID NO: 3+5+9+14, SEQ ID NO: 3+5+10+14, SEQ ID NO: 3+5+11+14, SEQ ID NO: 3+5+12+14, SEQ ID NO: 3+5+12+14, SEQ ID NO: 3+5+8+15, SEQ ID NO: 3+5+9+15, SEQ ID NO: 3+5+10+15, SEQ ID NO: 3+5+11+15, SEQ ID NO: 3+5+12+15, SEQ ID NO: 3+5+12+15, SEQ ID NO: 3+5+8+16, SEQ ID NO: 3+5+9+16, SEQ ID NO: 3+5+10+16, SEQ ID NO: 3+5+11+16, SEQ ID NO: 3+5+12+16, SEQ ID NO: 3+5+12+16, SEQ ID NO: 3+5+8+17, SEQ ID NO: 3+5+9+17, SEQ ID NO: 3+5+10+17, SEQ ID NO: 3+5+11+17, SEQ ID NO: 3+5+12+17, SEQ ID NO: 3+5+12+17, SEQ ID NO: 3+5+8+18, SEQ ID NO: 3+5+9+18, SEQ ID NO: 3+5+10+18, SEQ ID NO: 3+5+11+18, SEQ ID NO: 3+5+12+18, SEQ ID NO: 3+5+12+18, SEQ ID NO: 3+5+8+19, SEQ ID NO: 3+5+9+19, SEQ ID NO: 3+5+10+19, SEQ ID NO: 3+5+11+19, SEQ ID NO: 3+5+12+19, SEQ ID NO: 3+5+12+19, SEQ ID NO: 3+5+8+20, SEQ ID NO: 3+5+9+20, SEQ ID NO: 3+5+10+20, SEQ ID NO: 3+5+11+20, SEQ ID NO: 3+5+12+20, SEQ ID NO: 3+5+12+20, SEQ ID NO: 3+5+8+21, SEQ ID NO: 3+5+9+21, SEQ ID NO: 3+5+10+21, SEQ ID NO: 3+5+11+21, SEQ ID NO: 3+5+12+21, SEQ ID NO: 3+5+12+21, SEQ ID NO: 3+5+8+22, SEQ ID NO: 3+5+9+22, SEQ ID NO: 3+5+10+22, SEQ ID NO: 3+5+11+22, SEQ ID NO: 3+5+12+22, SEQ ID NO: 3+5+12+22, SEQ ID NO: 3+5+8+23, SEQ ID NO: 3+5+9+23, SEQ ID NO: 3+5+10+23, SEQ ID NO: 3+5+11+23, SEQ ID NO: 3+5+12+23, SEQ ID NO: 3+5+12+23, SEQ ID NO: 3+6+8+14, SEQ ID NO: 3+6+9+14, SEQ ID NO: 3+6+10+14, SEQ ID NO: 3+6+11+14, SEQ ID NO: 3+6+12+14, SEQ ID NO: 3+6+12+14, SEQ ID NO: 3+6+8+15, SEQ ID NO: 3+6+9+15, SEQ ID NO: 3+6+10+15, SEQ ID NO: 3+6+11+15, SEQ ID NO: 3+6+12+15, SEQ ID NO: 3+6+12+15, SEQ ID NO: 3+6+8+16, SEQ ID NO: 3+6+9+16, SEQ ID NO: 3+6+10+16, SEQ ID NO: 3+6+11+16, SEQ ID NO: 3+6+12+16, SEQ ID NO: 3+6+12+16, SEQ ID NO: 3+6+8+17, SEQ ID NO: 3+6+9+17, SEQ ID NO: 3+6+10+17, SEQ ID NO: 3+6+11+17, SEQ ID NO: 3+6+12+17, SEQ ID NO: 3+6+12+17, SEQ ID NO: 3+6+8+18, SEQ ID NO: 3+6+9+18, SEQ ID NO: 3+6+10+18, SEQ ID NO: 3+6+11+18, SEQ ID NO: 3+6+12+18, SEQ ID NO: 3+6+12+18, SEQ ID NO: 3+6+8+19, SEQ ID NO: 3+6+9+19, SEQ ID NO: 3+6+10+19, SEQ ID NO: 3+6+11+19, SEQ ID NO: 3+6+12+19, SEQ ID NO: 3+6+12+19, SEQ ID NO: 3+6+8+20, SEQ ID NO: 3+6+9+20, SEQ ID NO: 3+6+10+20, SEQ ID NO: 3+6+11+20, SEQ ID NO: 3+6+12+20, SEQ ID NO: 3+6+12+20, SEQ ID NO: 3+6+8+21, SEQ ID NO: 3+6+9+21, SEQ ID NO: 3+6+10+21, SEQ ID NO: 3+6+11+21, SEQ ID NO: 3+6+12+21, SEQ ID NO: 3+6+12+21, SEQ ID NO: 3+6+8+22, SEQ ID NO: 3+6+9+22, SEQ ID NO: 3+6+10+22, SEQ ID NO: 3+6+11+22, SEQ ID NO: 3+6+12+22, SEQ ID NO: 3+6+12+22, SEQ ID NO: 3+6+8+23, SEQ ID NO: 3+6+9+23, SEQ ID NO: 3+6+10+23, SEQ ID NO: 3+6+11+23, SEQ ID NO: 3+6+12+23, SEQ ID NO: 3+6+12+23, SEQ ID NO: 3+7+8+14, SEQ ID NO: 3+7+9+14, SEQ ID NO: 3+7+10+14, SEQ ID NO: 3+7+11+14, SEQ ID NO: 3+7+12+14, SEQ ID NO: 3+7+12+14, SEQ ID NO: 3+7+8+15, SEQ ID NO: 3+7+9+15, SEQ ID NO: 3+7+10+15, SEQ ID NO: 3+7+11+15, SEQ ID NO: 3+7+12+15, SEQ ID NO: 3+7+12+15, SEQ ID NO: 3+7+8+16, SEQ ID NO: 3+7+9+16, SEQ ID NO: 3+7+10+16, SEQ ID NO: 3+7+11+16, SEQ ID NO: 3+7+12+16, SEQ ID NO: 3+7+12+16, SEQ ID NO: 3+7+8+17, SEQ ID NO: 3+7+9+17, SEQ ID NO: 3+7+10+17, SEQ ID NO: 3+7+11+17, SEQ ID NO: 3+7+12+17, SEQ ID NO: 3+7+12+17, SEQ ID NO: 3+7+8+18, SEQ ID NO: 3+7+9+18, SEQ ID NO: 3+7+10+18, SEQ ID NO: 3+7+11+18, SEQ ID NO: 3+7+12+18, SEQ ID NO: 3+7+12+18, SEQ ID NO: 3+7+8+19, SEQ ID NO: 3+7+9+19, SEQ ID NO: 3+7+10+19, SEQ ID NO: 3+7+11+19, SEQ ID NO: 3+7+12+19, SEQ ID NO: 3+7+12+19, SEQ ID NO: 3+7+8+20, SEQ ID NO: 3+7+9+20, SEQ ID NO: 3+7+10+20, SEQ ID NO: 3+7+11+20, SEQ ID NO: 3+7+12+20, SEQ ID NO: 3+7+12+20, SEQ ID NO: 3+7+8+21, SEQ ID NO: 3+7+9+21, SEQ ID NO: 3+7+10+21, SEQ ID NO: 3+7+11+21, SEQ ID NO: 3+7+12+21, SEQ ID NO: 3+7+12+21, SEQ ID NO: 3+7+8+22, SEQ ID NO: 3+7+9+22, SEQ ID NO: 3+7+10+22, SEQ ID NO: 3+7+11+22, SEQ ID NO: 3+7+12+22, SEQ ID NO: 3+7+12+22, SEQ ID NO: 3+7+8+23, SEQ ID NO: 3+7+9+23, SEQ ID NO: 3+7+10+23, SEQ ID NO: 3+7+11+23, SEQ ID NO: 3+7+12+23, SEQ ID NO: 3+7+12+23, SEQ ID NO: 4+5+8+14, SEQ ID NO: 4+5+9+14, SEQ ID NO: 4+5+10+14, SEQ ID NO: 4+5+11+14, SEQ ID NO: 4+5+12+14, SEQ ID NO: 4+5+12+14, SEQ ID NO: 4+5+8+15, SEQ ID NO: 4+5+9+15, SEQ ID NO: 4+5+10+15, SEQ ID NO: 4+5+11+15, SEQ ID NO: 4+5+12+15, SEQ ID NO: 4+5+12+15, SEQ ID NO: 4+5+8+16, SEQ ID NO: 4+5+9+16, SEQ ID NO: 4+5+10+16, SEQ ID NO: 4+5+11+16, SEQ ID NO: 4+5+12+16, SEQ ID NO: 4+5+12+16, SEQ ID NO: 4+5+8+17, SEQ ID NO: 4+5+9+17, SEQ ID NO: 4+5+10+17, SEQ ID NO: 4+5+11+17, SEQ ID NO: 4+5+12+17, SEQ ID NO: 4+5+12+17, SEQ ID NO: 4+5+8+18, SEQ ID NO: 4+5+9+18, SEQ ID NO: 4+5+10+18, SEQ ID NO: 4+5+11+18, SEQ ID NO: 4+5+12+18, SEQ ID NO: 4+5+12+18, SEQ ID NO: 4+5+8+19, SEQ ID NO: 4+5+9+19, SEQ ID NO: 4+5+10+19, SEQ ID NO: 4+5+11+19, SEQ ID NO: 4+5+12+19, SEQ ID NO: 4+5+12+19, SEQ ID NO: 4+5+8+20, SEQ ID NO: 4+5+9+20, SEQ ID NO: 4+5+10+20, SEQ ID NO: 4+5+11+20, SEQ ID NO: 4+5+12+20, SEQ ID NO: 4+5+12+20, SEQ ID NO: 4+5+8+21, SEQ ID NO: 4+5+9+21, SEQ ID NO: 4+5+10+21, SEQ ID NO: 4+5+11+21, SEQ ID NO: 4+5+12+21, SEQ ID NO: 4+5+12+21, SEQ ID NO: 4+5+8+22, SEQ ID NO: 4+5+9+22, SEQ ID NO: 4+5+10+22, SEQ ID NO: 4+5+11+22, SEQ ID NO: 4+5+12+22, SEQ ID NO: 4+5+12+22, SEQ ID NO: 4+5+8+23, SEQ ID NO: 4+5+9+23, SEQ ID NO: 4+5+10+23, SEQ ID NO: 4+5+11+23, SEQ ID NO: 4+5+12+23, SEQ ID NO: 4+5+12+23, SEQ ID NO: 4+6+8+14, SEQ ID NO: 4+6+9+14, SEQ ID NO: 4+6+10+14, SEQ ID NO: 4+6+11+14, SEQ ID NO: 4+6+12+14, SEQ ID NO: 4+6+12+14, SEQ ID NO: 4+6+8+15, SEQ ID NO: 4+6+9+15, SEQ ID NO: 4+6+10+15, SEQ ID NO: 4+6+11+15, SEQ ID NO: 4+6+12+15, SEQ ID NO: 4+6+12+15, SEQ ID NO: 4+6+8+16, SEQ ID NO: 4+6+9+16, SEQ ID NO: 4+6+10+16, SEQ ID NO: 4+6+11+16, SEQ ID NO: 4+6+12+16, SEQ ID NO: 4+6+12+16, SEQ ID NO: 4+6+8+17, SEQ ID NO: 4+6+9+17, SEQ ID NO: 4+6+10+17, SEQ ID NO: 4+6+11+17, SEQ ID NO: 4+6+12+17, SEQ ID NO: 4+6+12+17, SEQ ID NO: 4+6+8+18, SEQ ID NO: 4+6+9+18, SEQ ID NO: 4+6+10+18, SEQ ID NO: 4+6+11+18, SEQ ID NO: 4+6+12+18, SEQ ID NO: 4+6+12+18, SEQ ID NO: 4+6+8+19, SEQ ID NO: 4+6+9+19, SEQ ID NO: 4+6+10+19, SEQ ID NO: 4+6+11+19, SEQ ID NO: 4+6+12+19, SEQ ID NO: 4+6+12+19, SEQ ID NO: 4+6+8+20, SEQ ID NO: 4+6+9+20, SEQ ID NO: 4+6+10+20, SEQ ID NO: 4+6+11+20, SEQ ID NO: 4+6+12+20, SEQ ID NO: 4+6+12+20, SEQ ID NO: 4+6+8+21, SEQ ID NO: 4+6+9+21, SEQ ID NO: 4+6+10+21, SEQ ID NO: 4+6+11+21, SEQ ID NO: 4+6+12+21, SEQ ID NO: 4+6+12+21, SEQ ID NO: 4+6+8+22, SEQ ID NO: 4+6+9+22, SEQ ID NO: 4+6+10+22, SEQ ID NO: 4+6+11+22, SEQ ID NO: 4+6+12+22, SEQ ID NO: 4+6+12+22, SEQ ID NO: 4+6+8+23, SEQ ID NO: 4+6+9+23, SEQ ID NO: 4+6+10+23, SEQ ID NO: 4+6+11+23, SEQ ID NO: 4+6+12+23, SEQ ID NO: 4+6+12+23, SEQ ID NO: 4+7+8+14, SEQ ID NO: 4+7+9+14, SEQ ID NO: 4+7+10+14, SEQ ID NO: 4+7+11+14, SEQ ID NO: 4+7+12+14, SEQ ID NO: 4+7+12+14, SEQ ID NO: 4+7+8+15, SEQ ID NO: 4+7+9+15, SEQ ID NO: 4+7+10+15, SEQ ID NO: 4+7+11+15, SEQ ID NO: 4+7+12+15, SEQ ID NO: 4+7+12+15, SEQ ID NO: 4+7+8+16, SEQ ID NO: 4+7+9+16, SEQ ID NO: 4+7+10+16, SEQ ID NO: 4+7+11+16, SEQ ID NO: 4+7+12+16, SEQ ID NO: 4+7+12+16, SEQ ID NO: 4+7+8+17, SEQ ID NO: 4+7+9+17, SEQ ID NO: 4+7+10+17, SEQ ID NO: 4+7+11+17, SEQ ID NO: 4+7+12+17, SEQ ID NO: 4+7+12+17, SEQ ID NO: 4+7+8+18, SEQ ID NO: 4+7+9+18, SEQ ID NO: 4+7+10+18, SEQ ID NO: 4+7+11+18, SEQ ID NO: 4+7+12+18, SEQ ID NO: 4+7+12+18, SEQ ID NO: 4+7+8+19, SEQ ID NO: 4+7+9+19, SEQ ID NO: 4+7+10+19, SEQ ID NO: 4+7+11+19, SEQ ID NO: 4+7+12+19, SEQ ID NO: 4+7+12+19, SEQ ID NO: 4+7+8+20, SEQ ID NO: 4+7+9+20, SEQ ID NO: 4+7+10+20, SEQ ID NO: 4+7+11+20, SEQ ID NO: 4+7+12+20, SEQ ID NO: 4+7+12+20, SEQ ID NO: 4+7+8+21, SEQ ID NO: 4+7+9+21, SEQ ID NO: 4+7+10+21, SEQ ID NO: 4+7+11+21, SEQ ID NO: 4+7+12+21, SEQ ID NO: 4+7+12+21, SEQ ID NO: 4+7+8+22, SEQ ID NO: 4+7+9+22, SEQ ID NO: 4+7+10+22, SEQ ID NO: 4+7+11+22, SEQ ID NO: 4+7+12+22, SEQ ID NO: 4+7+12+22, SEQ ID NO: 4+7+8+23, SEQ ID NO: 4+7+9+23, SEQ ID NO: 4+7+10+23, SEQ ID NO: 4+7+11+23, SEQ ID NO: 4+7+12+23 and SEQ ID NO: 4+7+12+23.

For instance, the prognosis method according to the invention can be carried out by determining the expression status of the above combinations SEQ ID NO: 1 + SEQ ID NO: 5 + SEQ ID NO: 13 + SEQ ID NO: 23, wherein
a. if neither SEQ ID NO: 1 nor SEQ ID NO : 5, nor SEQ ID NO: 13 nor SEQ ID NO: 23 is expressed , the patient survival rate at 30 months, 60 months or 120 months following the diagnosis is from about 19% to about 88% or more, and
b. if either SEQ ID NO: 1 or SEQ ID NO : 5, or SEQ ID NO: 13 or SEQ ID NO: 23 is expressed , the patient survival rate at 30 months, 60 months or 120 months following the diagnosis is from about 0% to about 73%.

According to the invention, the terms "about X%" means that the percentage of survival proposed for the prognosis method have to be considered with a standard deviation corresponding to individual variability. This standard deviation is 5%.

To summarise, the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 genes being such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13, and
   d. at least one gene belongs to a fourth set D of 9 genes comprising or consisting of the nucleic acid sequences SEQ ID NO: 14-23,
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30, and
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46,
for the implementation of a prognosis method, *preferably in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
   ▪ if none of the 23 genes of said set is expressed, the patient survival rate during a period of time from 30 to 120 months after the diagnosis of said lung cancer is from about 59% to about 78% or more, and
   ▪ if at least one gene of at least one set A, B, C or D is expressed , the patient survival rate during a period of time from 30 to 120 months after the diagnosis of said lung cancer is about from about 3% to about 70%,
   or
   ▪ if none of the 23 proteins of said set is expressed, the patient survival rate during a period of time from 30 to 120 months after the diagnosis of said lung cancer is from about 59% to about 78% or more, and
   ▪ if at least one protein of at least one set AP, BP, CP or DP is expressed, the patient survival rate during a period of time from 30 to 120 months after the diagnosis of said lung cancer is about from about 3% to about 70%,
or
   wherein said gene, protein is determined as being expressed when:
   - either it is expressed in a sample of a patient afflicted by a lung cancer but not in a control sample of an healthy individual,
   - or it is expressed at a level above a threshold corresponding to a background signal observed in a series of reference healthy tissues for each detection method, and
   said gene, protein is determined as being not expressed when:
   - it is neither expressed in a sample of a patient afflicted by a lung cancer nor in a control sample of an healthy individual,
   - or it is expressed in a sample of a patient afflicted by a lung cancer at a level substancially equal or inferior to the level in a control sample of an healthy individual.

According to the invention, "a control sample of an healthy individual" corresponds to a somatic tissue in which the CT gene is not expressed or weakly expressed as defined above.

In the invention "is expressed at a level above a threshold corresponding to a background signal observed in a series of reference healthy tissues for each detection method" means that the threshold, which corresponds to the key step of the invention, is determined by measuring the background signal in negative controle samples of healthy tissues, in which there is no expression of CT genes.

This background signal depends upon the method used to carry out the invention. However, it is easy for a skilled person to measure such threshold whatever the method used, i.e.:
- if the number of control samples is significantly statistically representative, e.g. at least 30 independent control samples, and the background signal of these control sample follows a normal distributation (Gaussian distribution), the threshold is determined by the mean + 2 standard deviations of the background signal measured in the control samples,
- If the number of control sample is not statically representative, e.g. less than 30 independent control samples, and the background signal of these control sample does not follow a normal distributation, the threshold is determined as being the maximal value of the background signal measured in the control samples.

According to the invention, the gene comprising or consisting of the nucleic acid sequence SEQ ID NO : 1 codes for the protein comprising or consisting of the amino acid sequence SEQ ID NO : 24, the gene comprising or consisting of the nucleic acid sequence SEQ ID NO : 2 codes for the protein comprising or consisting of the amino acid sequence SEQ ID NO : 25, the gene comprising or consisting of the nucleic acid sequence SEQ ID NO : 3 codes for the protein comprising or consisting of the amino acid sequence SEQ ID NO : 26, the gene comprising or consisting of the nucleic acid sequence SEQ ID NO : 4 codes for the protein comprising or consisting of the amino acid sequence SEQ ID NO : 27, the gene comprising or consisting of the nucleic acid sequence SEQ ID NO : 5 codes for the protein comprising or consisting of the amino acid sequence SEQ ID NO : 28, the gene comprising or consisting of the nucleic acid sequence SEQ ID NO : 6 codes for the protein comprising or consisting of the amino acid sequence SEQ ID NO : 29, the gene comprising or consisting of the nucleic acid sequence SEQ ID NO : 7 codes for the protein comprising or consisting of the amino acid sequence SEQ ID NO : 30, the gene comprising or consisting of the nucleic acid sequence SEQ ID NO : 8 codes for the protein comprising or consisting of the amino acid sequence SEQ ID NO : 31, the gene comprising or consisting of the nucleic acid sequence SEQ ID NO : 9 codes for the protein comprising or consisting of the amino acid sequence SEQ ID NO : 32, the gene comprising or consisting of the nucleic acid sequence SEQ ID NO : 10 codes for the protein comprising or consisting of the amino acid sequence SEQ ID NO : 33, the gene comprising or consisting of the nucleic acid sequence SEQ ID NO : 11 codes for the protein comprising or consisting of the amino acid sequence SEQ ID NO : 34, the gene comprising or consisting of the nucleic acid sequence SEQ ID NO : 12 codes for the protein comprising or consisting of the amino acid sequence SEQ ID NO : 35, the gene comprising or consisting of the nucleic acid sequence SEQ ID NO : 13 codes for the protein comprising or consisting of the amino acid sequence SEQ ID NO : 36, the gene comprising or consisting of the nucleic acid sequence SEQ ID NO : 14 codes for the protein comprising or consisting of the amino acid sequence SEQ ID NO : 37, the gene comprising or consisting of the nucleic acid sequence SEQ ID NO : 15 codes for the protein comprising or consisting of the amino acid sequence SEQ ID NO : 38, the gene comprising or consisting of the nucleic acid sequence SEQ ID NO : 16 codes for the protein comprising or consisting of the amino acid sequence SEQ ID NO : 39, the gene comprising or consisting of the nucleic acid sequence SEQ ID NO : 17 codes for the protein comprising or consisting of the amino acid sequence SEQ ID NO : 40, the gene comprising or consisting of the nucleic acid sequence SEQ ID NO : 18 codes for the protein comprising or consisting of the amino acid sequence SEQ ID NO : 41, the gene comprising or consisting of the nucleic acid sequence SEQ ID NO : 19 codes for the protein comprising or consisting of the amino acid sequence SEQ ID NO : 42, the gene comprising or consisting of the nucleic acid sequence SEQ ID NO : 20 codes for the protein comprising or consisting of the amino acid sequence SEQ ID NO : 43, the gene comprising or consisting of the nucleic acid sequence SEQ ID NO : 21 codes for the protein comprising or consisting of the amino acid sequence SEQ ID NO : 44, the gene comprising or consisting of the nucleic acid sequence SEQ ID NO : 22 codes for the protein comprising or consisting of the amino acid sequence SEQ ID NO : 45 and the gene comprising or consisting of the nucleic acid sequence SEQ ID NO : 23 codes for the protein comprising or consisting of the amino acid sequence SEQ ID NO : 46.

The 23 genes according to the invention code for 23 proteins. The 23 proteins have been classified by the Inventors in two sets:
a. a first set of 4 proteins, expressed or present in expressed in BAS,
b. a second set of 3 proteins, expressed in ADK,
c. a third set of 6 proteins, expressed in SQC, and
d. a fourth set of 10 protéins expressed in LCNE.

The first set, also called in the invention set AP, of 4 proteins consists of the proteins comprising or constituted by the amino acid sequences SEQ ID NO: 24, SEQ ID NO: 25, SEQ ID NO: 26 and SEQ ID NO: 27.

The second set, also called in the invention set BP, of 3 proteins consists of the proteins comprising or constituted by the amino acid sequences SEQ ID NO: 28, SEQ ID NO: 29 and SEQ ID NO: 30.

The third set, also called in the invention set BP, of 6 proteins consists of the proteins comprising or constituted by the amino acid sequences SEQ ID NO: 31, SEQ ID NO: 32, SEQ ID NO: 33, SEQ ID NO: 34, SEQ ID NO: 35 and SEQ ID NO: 36.

The fourth set, also called in the invention set BP, of 10 proteins consists of the proteins comprising or constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The inventors have also defined subsets of each of the above sets AP and BP as follows: Set AP is divided into 4 subsets AP1, AP5, and AP6, said subset being such that:
a. subset AP1 consists of the proteins comprising or being constituted by the amino acid sequences SEQ ID NO: 24 and SEQ ID NO: 25,
b. subset AP5 consists of the protein comprising or being constituted by the amino acid sequence SEQ ID NO: 26, and
c. subset AP6 consists of the proteins comprising or being constituted by the amino acid sequences SEQ ID NO: 27.

Set AP can also be divided into the following subsets:
a. subset AP1 consists of the proteins comprising or being constituted by the amino acid sequences SEQ ID NO: 24 and SEQ ID NO: 25,
b. subset AP2 consists of the proteins comprising or being constituted by the amino acid sequences SEQ ID NO: 24, SEQ ID NO: 25 and SEQ ID NO: 26,
c. subset AP3 consists of the proteins comprising or being constituted by the amino acid sequences SEQ ID NO: 24, SEQ ID NO: 25, SEQ ID NO: 26 and SEQ ID NO: 27.

Set BP is divided into 3 subsets BP1 and BP3, said subset being such that:
a. subset BP1 consists of the proteins comprising or being constituted by the amino acid sequences SEQ ID NO: 28 and SEQ ID NO: 29, and
b. subset BP3 consists of the proteins comprising or being constituted by the amino acid sequences SEQ ID NO: 30.

Set BP can also be divided into the following subsets:
a. subset BP1 consists of the proteins comprising or being constituted by the amino acid sequences SEQ ID NO: 28 and SEQ ID NO: 29, and
b. subset BP2 consists of the proteins comprising or being constituted by the amino acid sequences SEQ ID NO: 28, SEQ ID NO: 29SEQ ID NO: 30.

Set CP is divided into 3 subsets CP1, CP4 and CP5, said subset being such that:
a. subset CP1 consists of the proteins comprising or being constituted by the amino acid sequences SEQ ID NO: 3 land SEQ ID NO: 32,
b. subset CP4 consists of the proteins comprising or being constituted by the amino acid sequences SEQ ID NO: 33 and SEQ ID NO: 34,
c. subset CP5 consists of the proteins comprising or being constituted by the amino acid sequences SEQ ID NO: 35 and SEQ ID NO: 36.

Set CP can also be divided into the following subsets:
a. subset CP1 consists of the proteins comprising or being constituted by the amino acid sequences SEQ ID NO: 3 land SEQ ID NO: 32,
b. subset CP2 consists of the proteins comprising or being constituted by the amino acid sequences SEQ ID NO: 31, SEQ ID NO: 32, SEQ ID NO: 33 and SEQ ID NO: 34, and
c. subset CP3 consists of the proteins comprising or being constituted by the amino acid sequences SEQ ID NO: 31, SEQ ID NO: 32, SEQ ID NO: 33, SEQ ID NO: 34, SEQ ID NO: 35 and SEQ ID NO: 36.

Set DP is divided into 4 subsets DP1, DP5, DP6, DP7, said subset being such that:
a. subset DP1 consists of the proteins comprising or being constituted by the amino acid sequences SEQ ID NO: 37 and SEQ ID NO: 38,
b. subset DP5 consists of the proteins comprising or being constituted by the amino acid sequences SEQ ID NO: 39, SEQ ID NO: 40, SEQ ID NO: 41, SEQ ID NO: 42 and SEQ ID NO: 43,
c. subset DP6 consists of the proteins comprising or being constituted by the amino acid sequences SEQ ID NO: 44, and
d. subset DP7 consists of the proteins comprising or being constituted by the amino acid sequences SEQ ID NO: 45 and SEQ ID NO: 46

Set DP can also be divided into the following subsets:
a. subset DP1 consists of the proteins comprising or being constituted by the amino acid sequences SEQ ID NO: 37 and SEQ ID NO: 38,
b. subset DP2 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42 and SEQ ID NO: 43,
c. subset DP3 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43 and SEQ ID NO: 44.
d. subset DP4 consists of the proteins comprising or being constituted by the amino acid sequences SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

In one advantageous embodiment, the invention relates to the use as defined above of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46 said
   ◆ at least 4 genes being such that
      a. at least one gene belongs to a first subset A1 of 2 genes comprising or consisting of the nucleic acid sequences SEQ ID NO: 1 to 2
      b. at least one gene belongs to a second subset B1 of 2 genes comprising or consisting of the nucleic acid sequences SEQ ID NO: 5 to 6
      c. at least one gene belongs to a third subset C1 of 2 genes comprising or consisting of the nucleic acid sequences SEQ ID NO: 8 to 9, and
      d. at least one gene belongs to a fourth subset D1 of 2 genes comprising or consisting of the nucleic acid sequences SEQ ID NO: 14 to 15
   ◆ at least 4 proteins being such that
      a. at least one protein belongs to a first subset AP1 of 2proteins comprising or consisting of the amino acid sequences SEQ ID NO: 24 to 25,
      b. at least one protein belongs to a second subset BP1 of 2 proteins comprising or consisting of the amino acid sequences SEQ ID NO: 28 to 29
      c. at least one protein belongs to a second subset CP1 of 2 proteins comprising or consisting of the amino acid sequences SEQ ID NO: 31 to 32,
      d. at least one protein belongs to a second set DP1 of 2 proteins comprising or consisting of the amino acid sequences SEQ ID NO: 37 to 38,
for the implementation of a prognosis method, preferably *in vitro,* of the survival rate of a patient afflicted by a lung cancer, said prognosis being such that:
either
   ▪ if none of the genes of said subset A1, B1, C1 and D1 is expressed, the patient survival rate during a period of time from 30 to 120 months after the diagnosis of said lung cancer is from about 59% to about 78% or more, and
   ▪ if at least one gene of at least one subset A1, B, C1 or D1 is expressed , the patient survival rate during a period of time from 30 to 120 months after the diagnosis of said lung cancer is about from about 3% to about 70%,
or
   ▪ if none of the proteins of said subset AP1, BP1, CP1 and DP1 is expressed, the patient survival rate during a period of time from 30 to 120 months after the diagnosis of said lung cancer is from about 59% to about 78% or more, and
   ▪ if at least one protein of at least one subset AP1, BP1, CP1 and DP1 is expressed, the patient survival rate during a period of time from 30 to 120 months after the diagnosis of said lung cancer is about from about 3% to about 70%.

In this advantageous embodiment, the prognosis method according to the invention can be carried out as by measuring at least the expression of the following 6 combinations of proteins: SEQ ID NO : 24 + 28 + 31 + 37, SEQ ID NO : 24 + 28 + 31 + 38, SEQ ID NO : 24+28+32+37, SEQ ID NO : 24 + 28 + 32 + 38, SEQ ID NO :24 + 29 + 31 +37, SEQ ID NO : 24 + 29 + 31 + 38, SEQ ID NO : 24 + 29 + 32 + 37, SEQ ID NO : 24 + 29 + 32 + 38, SEQ ID NO : 25 + 28 + 31 + 37, SEQ ID NO : 25 + 28 + 31 + 38, SEQ ID NO : 25 + 28 + 32 + 37, SEQ ID NO : 25 + 28 + 32 + 38, SEQ ID NO : 25 + 29 + 31 + 37, SEQ ID NO : 25 + 29 + 31 + 38, SEQ ID NO : 25 + 29 + 32 + 37 and SEQ ID NO : 25 + 29+32+38.

For instance, the prognosis method according to the invention can be carried out by determining the expression status of the above combination SEQ ID NO: 24 + SEQ ID NO: 28 + SEQ ID NO: 32 + SEQ ID NO: 37, wherein:
▪ if none of the genes SEQ ID NO: 1, SEQ ID NO: 5, SEQ ID NO: 8 and SEQ ID NO: 15 is expressed, the patient survival rate during a period of time from 30 to 120 months after the diagnosis of said lung cancer is from about 59% to about 78% or more, and
▪ if at least one gene SEQ ID NO: 1, SEQ ID NO: 5, SEQ ID NO: 8 or SEQ ID NO: 15 is expressed , the patient survival rate during a period of time from 30 to 120 months after the diagnosis of said lung cancer is about from about 3% to about 70%.

Moreover, the prognosis method according to the invention can be carried out as by measuring at least the expression of the following 16 combinations of proteins: SEQ ID NO : 24 + 25 + 31 + 37, SEQ ID NO : 24 + 25 + 31 + 38, SEQ ID NO : 24 + 25 + 32 + 37, SEQ ID NO : 24 + 25 + 32 + 38, SEQ ID NO : 24 + 29 + 31 + 37, SEQ ID NO : 24 + 29 + 31 + 38, SEQ ID NO : 24 + 29 + 32 + 37, SEQ ID NO : 24 + 29 + 32 + 38, SEQ ID NO : 25 + 25 + 31 + 37, SEQ ID NO : 25 + 25 + 31 + 38, SEQ ID NO : 25 + 25 + 32 + 37, SEQ ID NO : 25 + 25 + 32 + 38, SEQ ID NO : 25 + 29 + 31 + 37, SEQ ID NO : 25 + 29 + 31 +38, SEQ ID NO : 25 + 29 + 32 + 37 and SEQ ID NO : 25 + 29 + 32 + 38.

For instance, the prognosis method according to the invention can be carried out by determining the expression status of the above combination SEQ ID NO: 24 + SEQ ID NO: 5 + SEQ ID NO: 8 + SEQ ID NO: 15, wherein:
▪ if none of the proteins SEQ ID NO: 24, SEQ ID NO: 28, SEQ ID NO: 32 and SEQ ID NO: 37 is expressed, the patient survival rate during a period of time from 30 to 120 months after the diagnosis of said lung cancer is from about 59% to about 78% or more, and
▪ if at least one protein SEQ ID NO: 24, SEQ ID NO: 28, SEQ ID NO: 32 or SEQ ID NO: 37 is expressed , the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A1 of 2 genes comprising or consisting of the nucleic acid sequences SEQ ID NO: 1 to 2
   b. at least one gene belongs to a second subset B1 of 2 genes comprising or consisting of the nucleic acid sequences SEQ ID NO: 5 to 6
   c. at least one gene belongs to a third subset C1 of 2 genes comprising or consisting of the nucleic acid sequences SEQ ID NO: 8 to 9, and
   d. at least one gene belongs to a fourth subset D1 of 2 genes comprising or consisting of the nucleic acid sequences SEQ ID NO: 14 to 15
for the implementation of a prognosis method, preferably *in vitro,* of the survival rate of a patient afflicted by a lung cancer, said prognosis being such that:
▪ if none of the genes of said subset A1, B1, C1 and D1 is expressed, the patient survival rate during a period of time from 30 to 120 months after the diagnosis of said lung cancer is from about 59% to about 78% or more, and
▪ if at least one gene of at least one subset A1, B, C1 or D1 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 proteins being such that
   a. at least one protein belongs to a first subset AP1 of 2proteins comprising or consisting of the amino acid sequences SEQ ID NO: 24 to 25,
   b. at least one protein belongs to a second subset BP1 of 2 proteins comprising or consisting of the amino acid sequences SEQ ID NO: 28 to 29
   c. at least one protein belongs to a second subset CP1 of 2 proteins comprising or consisting of the amino acid sequences SEQ ID NO: 31 to 32,
   d. at least one protein belongs to a second set DP1 of 2 proteins comprising or consisting of the amino acid sequences SEQ ID NO: 37 to 38,
   for the implementation of a prognosis method, preferably *in vitro,* of the survival rate of a patient afflicted by a lung cancer, said prognosis being such that:
   ▪ if none of the proteins of said subset AP1, BP1, CP1 and DP1 is expressed, the patient survival rate during a period of time from 30 to 120 months after the diagnosis of said lung cancer is from about 59% to about 78% or more, and
   ◆ if at least one protein of at least one subset AP1, BP1, CP1 and DP1 is expressed, the patient survival rate during a period of time from 30 to 120 months after the diagnosis of said lung cancer is about from about 3% to about 70%.

In one advantageous embodiment, the invention relates to the use as defined above of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 genes being such that
   a. at least one gene belongs to a first subset A4 comprising or consisting of the nucleic acid sequences SEQ ID NO: 3
   b. at least one gene belongs to a second subset B1 of 2 genes comprising or consisting of the nucleic acid sequences SEQ ID NO: 5 to 6
   c. at least one gene belongs to a third subset C4 of 2 genes comprising or consisting of the nucleic acid sequences SEQ ID NO: 10 to 11, and
   d. at least one gene belongs to a fourth subset D5 of 2 genes comprising or consisting of the nucleic acid sequences SEQ ID NO: 16 to 19
◆ at least 4 proteins being such that
   a. at least one protein belongs to a first subset AP4 comprising or consisting of the amino acid sequences SEQ ID NO: 26,
   b. at least one protein belongs to a second subset BP1 of 2 proteins comprising or consisting of the amino acid sequences SEQ ID NO: 33 to 34
   c. at least one protein belongs to a second subset CP4 of 5 proteins comprising or consisting of the amino acid sequences SEQ ID NO: 37 to 38,
   d. at least one protein belongs to a second set DP5 of 2 proteins comprising or consisting of the amino acid sequences SEQ ID NO: 39 to 43,
for the implementation of a prognosis method, preferably *in vitro,* of the survival rate of a patient afflicted by a lung cancer, said prognosis being such that: either
▪ if none of the genes of said subset A4, B1, C4 and D5 is expressed, the patient survival rate during a period of time from 30 to 120 months after the diagnosis of said lung cancer is from about 59% to about 78% or more, and
▪ if at least one gene of at least one subset A4, B, C4 or D5 is expressed , the patient survival rate during a period of time from 30 to 120 months after the diagnosis of said lung cancer is about from about 3% to about 70%,
or
▪ if none of the proteins of said subset AP4, BP1, CP4 and DP5 is expressed, the patient survival rate during a period of time from 30 to 120 months after the diagnosis of said lung cancer is from about 59% to about 78% or more, and
▪ if at least one protein of at least one subset AP4, BP1, CP4 and DP5 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A4 comprising or consisting of the nucleic acid sequences SEQ ID NO: 3
   b. at least one gene belongs to a second subset B1 of 2 genes comprising or consisting of the nucleic acid sequences SEQ ID NO: 5 to 6
   c. at least one gene belongs to a third subset C4 of 2 genes comprising or consisting of the nucleic acid sequences SEQ ID NO: 10 to 11, and
   d. at least one gene belongs to a fourth subset D5 of 2 genes comprising or consisting of the nucleic acid sequences SEQ ID NO: 16 to 19
for the implementation of a prognosis method, preferably *in vitro,* of the survival rate of a patient afflicted by a lung cancer, said prognosis being such that:
▪ if none of the genes of said subset A4, B1, C4 and D5 is expressed, the patient survival rate during a period of time from 30 to 120 months after the diagnosis of said lung cancer is from about 59% to about 78% or more, and
▪ if at least one gene of at least one subset A4, B1, C4 or D5 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 proteins being such that
◆ at least 4 proteins being such that
   e. at least one protein belongs to a first subset AP4 comprising or consisting of the amino acid sequences SEQ ID NO: 26,
   f. at least one protein belongs to a second subset BP1 of 2 proteins comprising or consisting of the amino acid sequences SEQ ID NO: 33 to 34
   g. at least one protein belongs to a second subset CP4 of 5 proteins comprising or consisting of the amino acid sequences SEQ ID NO: 37 to 38,
   h. at least one protein belongs to a second set DP5 of 2 proteins comprising or consisting of the amino acid sequences SEQ ID NO: 39 to 43,
for the implementation of a prognosis method, preferably *in vitro,* of the survival rate of a patient afflicted by a lung cancer, said prognosis being such that:
▪ if none of the proteins of said subset AP4, BP1, CP4 and DP5 is expressed, the patient survival rate during a period of time from 30 to 120 months after the diagnosis of said lung cancer is from about 59% to about 78% or more, and
▪ if at least one protein of at least one subset AP4, BP1, CP4 and DP5 is expressed, the patient survival rate during a period of time from 30 to 120 months after the diagnosis of said lung cancer is about from about 3% to about 70%.

In one advantageous embodiment, the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C2 of 4 genes comprising or consisting of the nucleic acid sequences SEQ ID NO: 8 to 11, and
   d. at least one gene belongs to a fourth subset D2 of 7 genes comprising or consisting of the nucleic acid sequences SEQ ID NO: 14 to 20
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP2 of 4 proteins comprising or consisting of the amino acid sequences SEQ ID NO: 31 to 34,
   d. at least one protein belongs to a second set DP2 of 7 proteins comprising or consisting of the amino acid sequences SEQ ID NO: 37 to 43,
for the implementation of a prognosis method, preferably *in vitro,* of the survival rate of a patient afflicted by a lung cancer, said prognosis being such that:
either
   ▪ if none of the genes of said subset A2, B1, C2 and D2 is expressed, the patient survival rate during a period of time from 30 to 120 months after the diagnosis of said lung cancer is from about 59% to about 78% or more, and
   ▪ if at least one gene of at least one subset A2, B 1, C2 or D2 is expressed , the patient survival rate during a period of time from 30 to 120 months after the diagnosis of said lung cancer is about from about 3% to about 70%,
   or
   ▪ if none of the proteins of said subset AP2, BP1, CP2 and DP2 is expressed, the patient survival rate during a period of time from 30 to 120 months after the diagnosis of said lung cancer is from about 59% to about 78% or more, and
   ▪ if at least one protein of at least one subset AP2, BP1, CP2 and DP2 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C2 of 4 genes comprising or consisting of the nucleic acid sequences SEQ ID NO: 8 to 11, and
   d. at least one gene belongs to a fourth subset D2 of 7 genes comprising or consisting of the nucleic acid sequences SEQ ID NO: 14 to 20
for the implementation of a prognosis method, preferably *in vitro,* of the survival rate of a patient afflicted by a lung cancer, said prognosis being such that:
▪ if none of the genes of said subset A2, B1, C2 and D21 is expressed, the patient survival rate during a period of time from 30 to 120 months after the diagnosis of said lung cancer is from about 59% to about 78% or more, and
▪ if at least one gene of at least one subset A2, B1, C2 or D2 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP2 of 4 proteins comprising or consisting of the amino acid sequences SEQ ID NO: 31 to 34,
   d. at least one protein belongs to a second set DP2 of 7 proteins comprising or consisting of the amino acid sequences SEQ ID NO: 37 to 43,
for the implementation of a prognosis method, preferably *in vitro,* of the survival rate of a patient afflicted by a lung cancer, said prognosis being such that:
▪ if none of the proteins of said subset AP2, BP1, CP2 and DP2 is expressed, the patient survival rate during a period of time from 30 to 120 months after the diagnosis of said lung cancer is from about 59% to about 78% or more, and
if at least one protein of at least one subset AP2, BP1, CP2 and DP2 is expressed, the patient survival rate during a period of time from 30 to 120 months after the diagnosis of said lung cancer is about from about 3% to about 70%.

In one advantageous embodiment, the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C3 of 6 genes comprising or consisting of the nucleic acid sequences SEQ ID NO: 8 to 13, and
   d. at least one gene belongs to a fourth subset D3 of 8 genes comprising or consisting of the nucleic acid sequences SEQ ID NO: 14 to 21,
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP3 of 6 proteins comprising or consisting of the amino acid sequences SEQ ID NO: 31 to 36,
   d. at least one protein belongs to a second set DP3 of 8 proteins comprising or consisting of the amino acid sequences SEQ ID NO: 37 to 44,
for the implementation of a prognosis method, preferably *in vitro,* of the survival rate of a patient afflicted by a lung cancer, said prognosis being such that:
either
   ▪ if none of the genes of said subset A2, B1, C3 and D3 is expressed, the patient survival rate during a period of time from 30 to 120 months after the diagnosis of said lung cancer is from about 59% to about 78% or more, and
   ▪ if at least one gene of at least one subset A2, B1, C3 or D3 is expressed , the patient survival rate during a period of time from 30 to 120 months after the diagnosis of said lung cancer is about from about 3% to about 70%,
or
   ▪ if none of the proteins of said subset AP2, BP1, CP3 and DP3 is expressed, the patient survival rate during a period of time from 30 to 120 months after the diagnosis of said lung cancer is from about 59% to about 78% or more, and
   ▪ if at least one protein of at least one subset AP2, BP1, CP3 and DP3 is expressed, the patient survival rate during a period of time from 30 to 120 months after the diagnosis of said lung cancer is about from about 3% to about 70%.

Another advantageous embodiment of the invention relates to the use of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first subset A2 of 3 genes comprising or consisting of the nucleic acid sequences SEQ ID NO: 1 to 3
   b. at least one gene belongs to a second subset B1 of 2 genes comprising or consisting of the nucleic acid sequences SEQ ID NO: 5 to 6
   c. at least one gene belongs to a third subset C3 of 6 genes comprising or consisting of the nucleic acid sequences SEQ ID NO: 8 to 13, and
   d. at least one gene belongs to a fourth subset D3 of 8 genes comprising or consisting of the nucleic acid sequences SEQ ID NO: 14 to 21,
for the implementation of a prognosis method, preferably *in vitro,* of the survival rate of a patient afflicted by a lung cancer, said prognosis being such that:
▪ if none of the genes of said subset A2, B1, C3 and D3 is expressed, the patient survival rate during a period of time from 30 to 120 months after the diagnosis of said lung cancer is from about 59% to about 78% or more, and
▪ if at least one gene of at least one subset A2, B1, C3 or D3 is expressed , the patient survival rate during a period of time from 30 to 120 months after the diagnosis of said lung cancer is about from about 3% to about 70%.

Another advantageous embodiment of the invention relates to the use of
- proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 proteins being such that
   a. at least one protein belongs to a first subset AP2 of 3 proteins comprising or consisting of the amino acid sequences SEQ ID NO: 24 to 26,
   b. at least one protein belongs to a second subset BP1 of 2 proteins comprising or consisting of the amino acid sequences SEQ ID NO: 28 to 29
   c. at least one protein belongs to a second subset CP3 of 6 proteins comprising or consisting of the amino acid sequences SEQ ID NO: 31 to 36,
   d. at least one protein belongs to a second set DP3 of 8 proteins comprising or consisting of the amino acid sequences SEQ ID NO: 37 to 44,
for the implementation of a prognosis method, preferably *in vitro,* of the survival rate of a patient afflicted by a lung cancer, said prognosis being such that:
▪ if none of the proteins of said subset AP2, BP1, CP3 and DP3 is expressed, the patient survival rate during a period of time from 30 to 120 months after the diagnosis of said lung cancer is from about 59% to about 78% or more, and
▪ if at least one protein of at least one subset AP2, BP1, CP3 and DP3 is expressed, the patient survival rate during a period of time from 30 to 120 months after the diagnosis of said lung cancer is about from about 3% to about 70%.

In one other embodiment, the invention relates to the use according as defined above, wherein said prognosis is such that
▪ when said tumor is ADK :
   o if none of the genes of said set B or of the subset B1 is expressed, the patient survival rate is from about 76% to about 86%
   o if at least one of the genes of the set B or of the subset B1 is expressed, the patient survival rate is from about 39% to about 63%,
      or
   o if none of the proteins of said set BP or of the subset BP1 is expressed, the patient survival rate is from about 76% to about 86%
   o if at least one of the proteins of the set BP1 or of the subset BP1 is expressed, the patient survival rate is from about 39% to about 63%,
      or
▪ when said tumor is BAS :
   o if none of the genes of the set A or of subsets A1 or A2 is expressed, the patient survival rate is about 19% to about 65%
   o if at least one of the genes of the set A or of subsets A1 or A2 is expressed, the patient survival rate is about 0% to about 33%,
      or
   o if none of the proteins of the set AP or of subsets AP1 or AP2 is expressed, the patient survival rate is about 19% to about 65%
   o if at least one of the proteins of the set AP or of subsets AP1 or AP2 is expressed, the patient survival rate is about 0% to about 33%,
      or
▪ when said tumor is SQC :
   o if none of the genes of the set C or of the subsets C1 or C2 is expressed, the patient survival rate is about 58% to about 88%
   o if at least one of the genes of the set C or of the subsets C1 or C2 is expressed, the patient survival rate is from about 33% to about 64%,
      or
   o if none of the proteins of the set CP or of the subsets CP1 or CP2 is expressed, the patient survival rate is about 58% to about 88%
   o if at least one of the proteins of the set CP or of the subsets CP1 or CP2 is expressed, the patient survival rate is from about 33% to about 64%,
      or
▪ when said tumor is LCNE :
   o if none of the genes of the set D or of the subsets D1, D2 or D3 is expressed, the patient survival rate is from about 40% to about 65%
   o if at least one of the genes of the set D or of the subsets D1, D2 or D3 is expressed, the patient survival rate is from about 0% to 7%,
      or
   o if none of the proteins of the set DP or of the subsets DP1, DP2 or DP3 is expressed, the patient survival rate is from about 40% to about 65%
   o if at least one of the proteins of the set DP or of the subsets DP1, DP2 or DP3 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
▪ when said tumor is BAS :
   o if none of the genes of subset A1 is expressed, the patient survival rate is about 19% to about 65%
   o if at least one of the genes of subsets A1 is expressed, the patient survival rate is about 0% to about 33%,
      or
   o if none of the proteins of subsets AP1 is expressed, the patient survival rate is about 19% to about 65%
   o if at least one of the proteins of the subsets AP1 or is expressed, the patient survival rate is about 0% to about 33%,
      or
▪ when said tumor is SQC :
   o if none of the genes of the subset C1 is expressed, the patient survival rate is about 58% to about 88%
   o if at least one of the genes of the subset C1 is expressed, the patient survival rate is from about 33% to about 64%,
      or
   o if none of the proteins of the subset CP 1 is expressed, the patient survival rate is about 58% to about 88%
   o if at least one of the proteins of the subset CP1 is expressed, the patient survival rate is from about 33% to about 64%,
      or
▪ when said tumor is LCNE :
   o if none of the genes of the subset D1 is expressed, the patient survival rate is from about 40% to about 65%
   o if at least one of the genes of the subset D1 is expressed, the patient survival rate is from about 0% to 7%,
      or
   o if none of the proteins of the subset DP1 is expressed, the patient survival rate is from about 40% to about 65%
   o if at least one of the proteins of the subset DP1 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
▪ when said tumor is BAS :
   o if none of the genes of subset A1 is expressed, the patient survival rate is about 19% to about 65%
   o if at least one of the genes of subsets A1 is expressed, the patient survival rate is about 0% to about 33%,
▪ when said tumor is SQC :
   o if none of the genes of the subset C1 is expressed, the patient survival rate is about 58% to about 88%
   o if at least one of the genes of the subset C1 is expressed, the patient survival rate is from about 33% to about 64%,
▪ when said tumor is LCNE :
   o if none of the genes of the subset D1 is expressed, the patient survival rate is from about 40% to about 65%
   o if at least one of the genes of the subset D1 is expressed, the patient survival rate is from about 0% to 7%.

In one other embodiment, the invention relates to the use according as defined above, wherein said prognosis is such that
▪ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
▪ when said tumor is BAS :
   o if none of the proteins of subsets AP1 is expressed, the patient survival rate is about 19% to about 65%
   ∘ if at least one of the proteins of the subsets AP1 or is expressed, the patient survival rate is about 0% to about 33%,
▪ when said tumor is SQC :
   o if none of the proteins of the subset CP1 is expressed, the patient survival rate is about 58% to about 88%
   o if at least one of the proteins of the subset CP1 is expressed, the patient survival rate is from about 33% to about 64%,
▪ when said tumor is LCNE :
   o if none of the proteins of the subset DP1 is expressed, the patient survival rate is from about 40% to about 65%
   o if at least one of the proteins of the subset DP1 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
▪ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
      or
   o if none of the proteins of subsets AP2 is expressed, the patient survival rate is about 19% to about 65%
   o if at least one of the proteins of the subsets AP2 or is expressed, the patient survival rate is about 0% to about 33%,
      or
▪ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
      or
   o if none of the proteins of the subset CP2 is expressed, the patient survival rate is about 58% to about 88%
   o if at least one of the proteins of the subset CP2 is expressed, the patient survival rate is from about 33% to about 64%,
      or
▪ when said tumor is LCNE :
   o if none of the genes of the subset D2 is expressed, the patient survival rate is from about 40% to about 65%
   o if at least one of the genes of the subset D2 is expressed, the patient survival rate is from about 0% to 7%,
      or
   o if none of the proteins of the subset DP2 is expressed, the patient survival rate is from about 40% to about 65%
   o if at least one of the proteins of the subset DP2 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
▪ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
▪ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
▪ when said tumor is LCNE :
   o if none of the genes of the subset D2 is expressed, the patient survival rate is from about 40% to about 65%
   o if at least one of the genes of the subset D2 is expressed, the patient survival rate is from about 0% to 7%.

In one other embodiment, the invention relates to the use according as defined above, wherein said prognosis is such that
▪ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
▪ when said tumor is BAS :
   o if none of the proteins of subsets AP2 is expressed, the patient survival rate is about 19% to about 65%
   o if at least one of the proteins of the subsets AP2 or is expressed, the patient survival rate is about 0% to about 33%,
▪ when said tumor is SQC :
   o if none of the proteins of the subset CP2 is expressed, the patient survival rate is about 58% to about 88%
   o if at least one of the proteins of the subset CP2 is expressed, the patient survival rate is from about 33% to about 64%,
▪ when said tumor is LCNE :
   o if none of the proteins of the subset DP2 is expressed, the patient survival rate is from about 40% to about 65%
   o if at least one of the proteins of the subset DP2 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
      or
   o if none of the proteins of the subset BP1 is expressed, the patient survival rate is from about 76% to about 86%
   o if at least one of the subset BP1 is expressed, the patient survival rate is from about 39% to about 63%,
      or
▪ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
      or
   o if none of the proteins of subsets AP2 is expressed, the patient survival rate is about 19% to about 65%
   o if at least one of the proteins of the subsets AP2 or is expressed, the patient survival rate is about 0% to about 33%,
      or
▪ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
      or
   o if none of the proteins of the subset CP2 is expressed, the patient survival rate is about 58% to about 88%
   o if at least one of the proteins of the subset CP2 is expressed, the patient survival rate is from about 33% to about 64%,
      or
▪ when said tumor is LCNE :
   o if none of the genes of the subset D3 is expressed, the patient survival rate is from about 40% to about 65%
   o if at least one of the genes of the subset D3 is expressed, the patient survival rate is from about 0% to 7%,
      or
   o if none of the proteins of the subset DP3 is expressed, the patient survival rate is from about 40% to about 65%
   o if at least one of the proteins of the subset DP3 is expressed, the patient survival rate is from about 0% to 7%.

In one other embodiment, the invention relates to the use according as defined above, wherein said prognosis is such that
▪ when said tumor is ADK :
   o if none of the genes of the B1 is expressed, the patient survival rate is from about 76% to about 86%
   o if at least one of the genes of the subset B1 is expressed, the patient survival rate is from about 39% to about 63%,
▪ when said tumor is BAS :
   o if none of the genes of subset A2 is expressed, the patient survival rate is about 19% to about 65%
   o if at least one of the genes of subsets A2 is expressed, the patient survival rate is about 0% to about 33%,
▪ when said tumor is SQC :
   o if none of the genes of the subset C2 is expressed, the patient survival rate is about 58% to about 88%
   o if at least one of the genes of the subset C2 is expressed, the patient survival rate is from about 33% to about 64%,
▪ when said tumor is LCNE :
   o if none of the genes of the subset D3 is expressed, the patient survival rate is from about 40% to about 65%
   o if at least one of the genes of the subset D3 is expressed, the patient survival rate is from about 0% to 7%.

In one other embodiment, the invention relates to the use according as defined above, wherein said prognosis is such that
▪ when said tumor is ADK :
   o if none of the proteins of the subset BP1 is expressed, the patient survival rate is from about 76% to about 86%
   o if at least one of the subset BP1 is expressed, the patient survival rate is from about 39% to about 63%,
▪ when said tumor is BAS :
   o if none of the proteins of subsets AP2 is expressed, the patient survival rate is about 19% to about 65%
   o if at least one of the proteins of the subsets AP2 or is expressed, the patient survival rate is about 0% to about 33%,
▪ when said tumor is SQC :
   o if none of the proteins of the subset CP2 is expressed, the patient survival rate is about 58% to about 88%
   o if at least one of the proteins of the subset CP2 is expressed, the patient survival rate is from about 33% to about 64%,
▪ when said tumor is LCNE :
   o if none of the proteins of the subset DP3 is expressed, the patient survival rate is from about 40% to about 65%
   o if at least one of the proteins of the subset DP3 is expressed, the patient survival rate is from about 0% to 7%.

In one particular embodiment, the invention relates to the use as defined above, wherein
▪ when said tumor is SQC :
   o if none of the genes of said set C or of subsets C1 and C2 is expressed, the patient survival rate is about 58% to about 88%
   o if one of the genes of the set C or of subsets C1 and C2 is expressed, the patient survival rate is from about 45% to about 73%,
   o if at least two of the genes of the set C or of subsets C1 and C2 are expressed, the patient survival rate is from about 14%, to about 50%,
      or
   o if none of the proteins of said set CP or of subsets CP1 and CP2 is expressed, the patient survival rate is about 58% to about 88%
   o if one of the proteins of the set CP or of subsets CP1 and CP2 is expressed, the patient survival rate is from about 45% to about 73%,
   o if at least two of the proteins of the set CP or of subsets CP1 and CP2 are expressed, the patient survival rate is from about 14%, to about 50%.

In one particular embodiment, the invention relates to the use as defined above, wherein
▪ when said tumor is SQC :
   o if none of the genes of said set C or of subsets C1 and C2 is expressed, the patient survival rate is about 58% to about 88%
   o if one of the genes of the set C or of subsets C1 and C2 is expressed, the patient survival rate is from about 45% to about 73%,
   o if at least two of the genes of the set C or of subsets C1 and C2 are expressed, the patient survival rate is from about 14%, to about 50%.

In one particular embodiment, the invention relates to the use as defined above, wherein
▪ when said tumor is SQC :
   o if none of the genes of said subset is expressed, the patient survival rate is about 58% to about 88%
   o if one of the genes of the subset C1 is expressed, the patient survival rate is from about 45% to about 73%,
   o if at least two of the genes of the subset C1 are expressed, the patient survival rate is from about 14%, to about 50%.

In one particular embodiment, the invention relates to the use as defined above, wherein
▪ when said tumor is SQC :
   o if none of the genes of said subset is expressed, the patient survival rate is about 58% to about 88%
   o if one of the genes of the subset C2 is expressed, the patient survival rate is from about 45% to about 73%,
   o if at least two of the genes of the subset C2 are expressed, the patient survival rate is from about 14%, to about 50%.

In one particular embodiment, the invention relates to the use as defined above, wherein
▪ when said tumor is SQC :
   o if none of the proteins of said set CP or of subsets CP1 and CP2 is expressed, the patient survival rate is about 58% to about 88%
   o if one of the proteins of the set CP or of subsets CP1 and CP2 is expressed, the patient survival rate is from about 45% to about 73%,
   o if at least two of the proteins of the set CP or of subsets CP1 and CP2 are expressed, the patient survival rate is from about 14%, to about 50%.

In one particular embodiment, the invention relates to the use as defined above, wherein
▪ when said tumor is SQC :
   o if none of the proteins of said subset CP1 is expressed, the patient survival rate is about 58% to about 88%
   o if one of the proteins of the subset CP1 is expressed, the patient survival rate is from about 45% to about 73%,
   o if at least two of the proteins of the subset CP1 are expressed, the patient survival rate is from about 14%, to about 50%.

In one particular embodiment, the invention relates to the use as defined above, wherein
▪ when said tumor is SQC :
   o if none of the proteins of said subset CP2 is expressed, the patient survival rate is about 58% to about 88%
   o if one of the proteins of the subset CP2 is expressed, the patient survival rate is from about 45% to about 73%,
   o if at least two of the proteins of the subset CP2 are expressed, the patient survival rate is from about 14%, to about 50%.

In another advantageaous embodiment, the invention relates to present disclosure describes the use as defined above, wherein during a period of time of 120 months from the diagnosis of said lung cancer, wherein
▪ when said tumor is ADK :
   o if none of the genes of said set B or of the subset B1 is expressed, the patient survival rate is about 76%
   o if at least one of the genes of the set B or of the subset B1 is expressed, the patient survival rate is about 39%,
      or
   o if none of the proteins of said set BP or of the subset BP1 is expressed, the patient survival rate is about 76% ,
   o if at least one of the proteins of the set BP or of the subset BP1 is expressed, the patient survival rate is about 39%,
      or
▪ when said tumor is BAS :
   o if none of the genes of the set A or of subsets A1 or A2 is expressed, the patient survival rate is about 19%
   o if at least one of the genes of the set A or of subsets A1 or A2 is expressed, the patient survival rate is about 0%,
      or
   o if none of the proteins of the set AP or of subsets AP1 or AP2 is expressed, the patient survival rate is about 19%
   o if at least one of the proteins of the set AP or of subsets AP1 or AP2 is expressed, the patient survival rate is about 0%,
      or
▪ when said tumor is SQC :
   o if none of the genes of the set C or of the subsets C1 or C2 is expressed, the patient survival rate is about 58%
   o if at least one of the genes of the set C or of the subsets C1 or C2 is expressed, the patient survival rate is about 33%,
      or
   o if none of the proteins of the set CP or of the subsets CP1 or CP2 is expressed, the patient survival rate is about 58%
   o if at least one of the proteins of the set CP or of the subsets CP1 or CP2 is expressed, the patient survival rate is about 33%, or
▪ when said tumor is LCNE :
   o if none of the genes of the set D or of the subsets D1, D2 or D3 is expressed, the patient survival rate is about 40%
   o if at least one of the genes of the set D or of the subsets D1, D2 or D3 is expressed, the patient survival rate is about 0%,
      or
   o if none of the proteins of the set DP or of the subsets DP1, DP2 or DP3 is expressed, the patient survival rate is about 40%
   o if at least one of the proteins of the set DP or of the subsets DP1, DP2 or DP3 is expressed, the patient survival rate is about 0%.

In one particular embodiment, the invention relates to the use as defined above, wherein
▪ when said tumor is SQC :
   o if none of the genes of the set C or of the subsets C1 or C2 is expressed, the patient survival rate is about 58%
   o if one of the genes the set C or of the subsets C1 or C2 is expressed, the patient survival rate is about 45%,
   o if at least two of the genes of the set C or of the subsets C1 or C2 is expressed, the patient survival rate is about 14%,
      or
   o if none of the protein of the set CP or of the subsets CP 1 or CP2 is expressed, the patient survival rate is about 58%
   o if one of the genes the set C or of the subsets CP1 or CP2 is expressed, the patient survival rate is about 45%,
   o if at least two of the genes of the set C or of the subsets CP1 or CP2 is expressed, the patient survival rate is about 14%.

In another advantageous embodiment, the invention relates to the use as defined above, wherein during a period of time of 60 months from the diagnosis of said lung cancer, wherein
▪ when said tumor is ADK :
   o if none of the genes of said set B or of the subset B1 is expressed, the patient survival rate is about 82%
   o if at least one of the genes of the set B or of the subset B1 is expressed, the patient survival rate is about 46%,
      or
   o if none of the proteins of said set BP or of the subset BP1 is expressed, the patient survival rate is about 82% ,
   o if at least one of the proteins of the set BP or of the subset BP1 is expressed, the patient survival rate is about 46%,
      or
▪ when said tumor is BAS :
   o if none of the genes of the set A or of subsets A1 or A2 is expressed, the patient survival rate is about 35%
   o if at least one of the genes of the set A or of subsets A1 or A2 is expressed, the patient survival rate is about 7%,
      or
   o if none of the proteins of the set AP or of subsets AP1 or AP2 is expressed, the patient survival rate is about 35%
   o if at least one of the proteins of the set AP or of subsets AP1 or AP2 is expressed, the patient survival rate is about 7%,
      or
▪ when said tumor is SQC :
   o if none of the genes of the set C or of the subsets C1 or C2 is expressed, the patient survival rate is about 77%
   o if at least one of the genes of the set C or of the subsets C1 or C2 is expressed, the patient survival rate is about 42%,
      or
   o if none of the proteins of the set CP or of the subsets CP1 or CP2 is expressed, the patient survival rate is about 77%
   o if at least one of the proteins of the set CP or of the subsets CP1 or CP2 is expressed, the patient survival rate is about 42%, or
▪ when said tumor is LCNE :
   o if none of the genes of the set D or of the subsets D1, D2 or D3 is expressed, the patient survival rate is about 55%
   o if at least one of the genes of the set D or of the subsets D1, D2 or D3 is expressed, the patient survival rate is about 4%,
      or
   o if none of the proteins of the set DP or of the subsets DP1, DP2 or DP3 is expressed, the patient survival rate is about 55%
   o if at least one of the proteins of the set DP or of the subsets DP1, DP2 or DP3 is expressed, the patient survival rate is about 4%.

In one particular embodiment, the invention relates to the use as defined above, wherein
▪ when said tumor is SQC :
   o if none of the genes of the set C or of the subsets C1 or C2 is expressed, the patient survival rate is about 77%
   o if one of the genes the set C or of the subsets C1 or C2 is expressed, the patient survival rate is about 59%,
   o if at least two of the genes of the set C or of the subsets C1 or C2 is expressed, the patient survival rate is about 14%,
      or
   o if none of the protein of the set CP or of the subsets CP1 or CP2 is expressed, the patient survival rate is about 77%
   o if one of the genes the set C or of the subsets CP1 or CP2 is expressed, the patient survival rate is about 59%,
   o if at least two of the genes of the set C or of the subsets CP1 or CP2 is expressed, the patient survival rate is about 14%.

In another advantageous embodiment, the invention relates to the use as defined above, wherein during a period of time of 30 months from the diagnosis of said lung cancer, wherein
▪ when said tumor is ADK :
   o if none of the genes of said set B or of the subset B1 is expressed, the patient survival rate is about 86%
   o if at least one of the genes of the set B or of the subset B1 is expressed, the patient survival rate is about 63%,
      or
   o if none of the proteins of said set BP or of the subset BP1 is expressed, the patient survival rate is about 86% ,
   o if at least one of the proteins of the set BP or of the subset BP1 is expressed, the patient survival rate is about 63%,
      or
▪ when said tumor is BAS :
   o if none of the genes of the set A or of subsets A1 or A2 is expressed, the patient survival rate is about 65%
   o if at least one of the genes of the set A or of subsets A1 or A2 is expressed, the patient survival rate is about 33%,
      or
   o if none of the proteins of the set AP or of subsets AP1 or AP2 is expressed, the patient survival rate is about 63%
   o if at least one of the proteins of the set AP or of subsets AP1 or AP2 is expressed, the patient survival rate is about 33%,
      or
▪ when said tumor is SQC :
   o if none of the genes of the set C or of the subsets C1 or C2 is expressed, the patient survival rate is about 88%
   o if at least one of the genes of the set C or of the subsets C 1 or C2 is expressed, the patient survival rate is about 64%,
      or
   o if none of the proteins of the set CP or of the subsets CP 1 or CP2 is expressed, the patient survival rate is about 88%
   o if at least one of the proteins of the set CP or of the subsets CP1 or CP2 is expressed, the patient survival rate is about 64%,
      or
▪ when said tumor is LCNE :
   o if none of the genes of the set D or of the subsets D1, D2 or D3 is expressed, the patient survival rate is about 65%
   o if at least one of the genes of the set D or of the subsets D1, D2 or D3 is expressed, the patient survival rate is about 7%,
      or
   o if none of the proteins of the set DP or of the subsets DP1, DP2 or DP3 is expressed, the patient survival rate is about 65%
   o if at least one of the proteins of the set DP or of the subsets DP1, DP2 or DP3 is expressed, the patient survival rate is about 7%.

In one particular embodiment, the invention relates to the use as defined above, wherein
▪ when said tumor is SQC :
   o if none of the genes of the set C or of the subsets C1 or C2 is expressed, the patient survival rate is about 88%
   o if one of the genes the set C or of the subsets C1 or C2 is expressed, the patient survival rate is about 73%,
   o if at least two of the genes of the set C or of the subsets C1 or C2 is expressed, the patient survival rate is about 50%,
      or
   o if none of the protein of the set CP or of the subsets CP 1 or CP2 is expressed, the patient survival rate is about 88%
   o if one of the genes the set C or of the subsets CP1 or CP2 is expressed, the patient survival rate is about 73%,
   o if at least two of the genes of the set C or of the subsets CP1 or CP2 is expressed, the patient survival rate is about 50%.

Advantageously, the disclosure describes the use of at least one element chosen among:
- at least 4 genes chosen among a set of 23 genes comprising or consisting of the nucleic acid sequences SEQ ID NO 1 to 23,
- or fragments of said least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
- or fragments of said proteins comprising or consisting in amino acid sequences SEQ ID NO 24 to 46,
- or antibodies directed against said proteins comprising or consisting in amino acid sequences SEQ ID NO 24 to 46,
said
◆ at least 4 genes being such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13, and
   d. at least one gene belongs to a fourth set D of 9 genes comprising or consisting of the nucleic acid sequences SEQ ID NO: 14-23,
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30, and
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46,
◆ at least 4 antibodies directed against said 4 proteins being such that
   a. at least one antibody that interact with at least one protein that belongs to a first set AP of 4 proteins comprising or consisting of the amino acid sequences SEQ ID NO: 24-27,
   b. at least one antibody that interact with at least one protein that belongs a second set BP of 3 proteins comprising or consisting of the amino acid sequences SEQ ID NO: 28-30,
   c. at least one antibody that interact with at least one protein that belongs a third set CP of 6 proteins comprising or consisting of the amino acid sequences SEQ ID NO: 31-36, and
   d. at least one antibody that interact with at least one protein that belongs a fourth set DP of 9 proteins comprising or consisting of the amino acid sequences SEQ ID NO: 37-46,
for the implementation of a prognosis method, *preferably in vitro,* of the survival rate of a patient afflicted by a lung cancer, said lung cancer being previously classified as a lung tumour belonging from the group consisting of squamous cell carcinoma (SQC), adenocarcinoma (ADK), large cell neuro-endocrine tumours (LCNE) and basaloid tumours (BAS),
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
   + during a period of time of 30 months after the diagnosis of said lung cancer
   ▪ when said tumor is ADK :
      o if none of the 23 genes, or of the 23 proteins, or of the 23 antibodies of said set is expressed, the patient survival rate is about 86%, or more
      o if at least one of the genes, or of the proteins, or of the antibodies of the set B is expressed, the patient survival rate is about 63%,
   ▪ when said tumor is BAS :
      o if none of the 23 genes, or of the 23 proteins, or of the 23 antibodies of said set is expressed, the patient survival rate is about 65% , or more
      o if at least one of the genes, or of the proteins, or of the antibodies of the set A is expressed, the patient survival rate is about 33%,
   ▪ when said tumor is SQC :
      o if none of the 23 genes, or of the 23 proteins, or of the 23 antibodies of said set is expressed, the patient survival rate is about 88% , or more
      o if at least one of the genes, or of the proteins, or of the antibodies of the set C is expressed, the patient survival rate is about 64%
   ▪ when said tumor is LCNE :
      o if none of the 23 genes, or of the 23 proteins, or of the 23 antibodies of said set is expressed, the patient survival rate is about 65% , or more
      o if at least one of the genes, or of the proteins, or of the antibodies of the set D is expressed, the patient survival rate is about 7%,
or,
   + during a period of time of 60 months after the diagnosis of said lung cancer
   ▪ when said tumor is ADK :
      o if none of the 23 genes, or of the 23 proteins, or of the 23 antibodies of said set is expressed, the patient survival rate is about 82%, or more
      o if at least one of the genes, or of the proteins, or of the antibodies of the set B is expressed, the patient survival rate is about 46%,
   ▪ when said tumor is BAS :
      o if none of the 23 genes, or of the 23 proteins, or of the 23 antibodies of said set is expressed, the patient survival rate is about 35%, or more
      o if at least one of the genes, or of the proteins, or of the antibodies of the set A is expressed, the patient survival rate is about 7%,
   ▪ when said tumor is SQC :
      o if none of the 23 genes, or of the 23 proteins, or of the 23 antibodies of said set is expressed, the patient survival rate is about 77%, or more
      o if at least one of the genes, or of the proteins, or of the antibodies of the set C is expressed, the patient survival rate is about 42%
   ▪ when said tumor is LCNE :
      o if none of the 23 genes, or of the 23 proteins, or of the 23 antibodies of said set is expressed, the patient survival rate is about 55%, or more
      o if at least one of the genes, or of the proteins, or of the antibodies of the set D is expressed, the patient survival rate is about 4%,
or,
   + during a period of time of 120 months after the diagnosis of said lung cancer
   ▪ when said tumor is ADK :
      o if none of the 23 genes, or of the 23 proteins, or of the 23 antibodies of said set is expressed, the patient survival rate is about 76%, or more
      o if at least one of the genes, or of the proteins, or of the antibodies of the set B is expressed, the patient survival rate is about 39%,
   ▪ when said tumor is BAS :
      o if none of the 23 genes, or of the 23 proteins, or of the 23 antibodies of said set is expressed, the patient survival rate is about 19%, or more
      o if at least one of the genes, or of the proteins, or of the antibodies of the set A is expressed, the patient survival rate is about 0%,
   ▪ when said tumor is SQC :
      o if none of the 23 genes, or of the 23 proteins, or of the 23 antibodies of said set is expressed, the patient survival rate is about 58%, or more
      o if at least one of the genes, or of the proteins, or of the antibodies of the set C is expressed, the patient survival rate is about 33%,
   ▪ when said tumor is LCNE :
      o if none of the 23 genes, or of the 23 proteins, or of the 23 antibodies of said set is expressed, the patient survival rate is about 40%, or more
      o if at least one of the genes, or of the proteins, or of the antibodies of the set D is expressed, the patient survival rate is about 0%,
wherein said gene, protein or antibody is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method , and said gene, protein or antibody is determined as being not expressed when:
- it is neither expressed in a sample of a patient afflicted by a lung cancer nor in a control sample of an healthy individual,
- or it is expressed in a sample of a patient afflicted by a lung cancer at a level substancially equal or inferior to the level in a control sample of an healthy individual.

The invention relates to the use of at least one element chosen among:
- at least 4 genes chosen among a set of 23 genes comprising or consisting of the nucleic acid sequences SEQ ID NO 1 to 23,
- or complementary sequences of said at least 4 genes chosen among a set of 23 genes comprising or consisting of the nucleic acid sequences SEQ ID NO 1 to 23,
- or sequences having at least 80% homology with said genes,
- or proteins coded by said at least 4 genes chosen among a set of 23 genes comprising or consisting of the nucleic acid sequences SEQ ID NO 1 to 23, said proteins comprising or consisting in amino acid sequences SEQ ID NO 24 to 46,
said
◆ at least 4 genes being such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13, and
   d. at least one gene belongs to a fourth set D of 9 genes comprising or consisting of the nucleic acid sequences SEQ ID NO: 14-23,
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30, and
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46,
for the implementation of an *in vitro* prognosis method of the survival rate of a patient afflicted by a lung cancer, said lung cancer being previously classified as a lung tumour belonging from the group consisting of squamous cell carcinoma, adenocarcinoma, large cell neuro-endocrine tumours and basaloid tumours,
wherein
- the nucleic acid sequences SEQ ID NO: 1 to 4 and/or the amino acid sequences SEQ ID NO: 24 to 27 are expressed in basaloid tumours,
- the nucleic acid sequences SEQ ID NO: 5 to 7 and/or the amino acid sequences SEQ ID NO: 28 to 30 are expressed in adenocarcinoma,
- the nucleic acid sequences SEQ ID NO: 8 to 13 and/or the amino acid sequences SEQ ID NO: 31 to 36 are expressed in squamous cell carcinoma,
- the nucleic acid sequences SEQ ID NO: 14 to 23 and/or the amino acid sequences SEQ ID NO: 37 to 46 are expressed in large cell neuro-endocrine tumours,
said prognosis being such that:
either
   + during a period of time of 30 months after the diagnosis of said lung cancer
   ▪ when said tumor is adenocarcinoma:
      o if none of the 23 genes, or of the 23 proteins of said set is expressed, the patient survival rate is about 86%,
      o if at least one of the genes, or of the proteins of the set B is expressed, the patient survival rate is about 63%,
   ▪ when said tumor is basaloid tumours:
      o if none of the 23 genes, or of the 23 proteins of said set is expressed, the patient survival rate is about 65% ,
      o if at least one of the genes, or of the proteins of the set A is expressed, the patient survival rate is about 33%,
   ▪ when said tumor is squamous cell carcinoma :
      o if none of the 23 genes, or of the 23 proteins of said set is expressed, the patient survival rate is about 88% ,
      o if at least one of the genes, or of the proteins of the set C is expressed, the patient survival rate is about 64%
   ▪ when said tumor is large cell neuro-endocrine tumours:
      o if none of the 23 genes, or of the 23 proteins of said set is expressed, the patient survival rate is about 65% ,
      o if at least one of the genes, or of the proteins of the set D is expressed, the patient survival rate is about 7%,
or,
   + during a period of time of 60 months after the diagnosis of said lung cancer
   ▪ when said tumor is adenocarcinoma :
      o if none of the 23 genes, or of the 23 proteins of said set is expressed, the patient survival rate is about 82%,
      o if at least one of the genes, or of the proteins of the set B is expressed, the patient survival rate is about 46%,
   ▪ when said tumor is basaloid tumours:
      o if none of the 23 genes, or of the 23 proteins of said set is expressed, the patient survival rate is about 35%,
      o if at least one of the genes, or of the proteins of the set A is expressed, the patient survival rate is about 7%,
   ▪ when said tumor is squamous cell carcinoma :
      o if none of the 23 genes, or of the 23 proteins of said set is expressed, the patient survival rate is about 77%,
      o if at least one of the genes, or of the proteins of the set C is expressed, the patient survival rate is about 42%
   ▪ when said tumor is large cell neuro-endocrine tumours:
      o if none of the 23 genes, or of the 23 proteins of said set is expressed, the patient survival rate is about 55%,
      o if at least one of the genes, or of the proteins of the set D is expressed, the patient survival rate is about 4%,
or,
   + during a period of time of 120 months after the diagnosis of said lung cancer
   ▪ when said tumor is adenocarcinoma:
      o if none of the 23 genes, or of the 23 proteins of said set is expressed, the patient survival rate is about 76%,
      o if at least one of the genes, or of the proteins of the set B is expressed, the patient survival rate is about 39%,
   ▪ when said tumor is basaloid tumours:
      o if none of the 23 genes, or of the 23 proteins of said set is expressed, the patient survival rate is about 19%,
      o if at least one of the genes, or of the proteins of the set A is expressed, the patient survival rate is about 0%,
   ▪ when said tumor is squamous cell carcinoma:
      o if none of the 23 genes, or of the 23 proteins of said set is expressed, the patient survival rate is about 58%,
      o if at least one of the genes, or of the proteins of the set C is expressed, the patient survival rate is about 33%,
   ▪ when said tumor is large cell neuro-endocrine tumours:
      o if none of the 23 genes, or of the 23 proteins of said set is expressed, the patient survival rate is about 40%,
      o if at least one of the genes, or of the proteins of the set D is expressed, the patient survival rate is about 0%,
wherein said gene or protein is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method , and
said gene or protein is determined as being not expressed when:
- it is neither expressed in a sample of a patient afflicted by a lung cancer nor in a control sample of an healthy individual,
- or it is expressed in a sample of a patient afflicted by a lung cancer at a level substancially equal or inferior to the level in a control sample of an healthy individual.

According to the invention "if none of the 23 genes, or of the 23 proteins of said set A, B, C, D, AP, BP, CP, DP, or subranges as defined above, is expressed, the patient survival rate is about X%, or more" means that the surviaval rate of sid patient is at the worst X% at the determined period of time (30, 60 or 120 months) but can be more favourable. Thus, when none of 23 genes, or of the 23 proteins of said set A, B, C, D, AP, BP, CP, DP, or subranges as defined above, is expressed, the survival rate belong to the range [X%-100%], wherein X is the percentage as mentioned above.
However, the probability is most closer to the X% than the 100% of survival.

In another advantageous embodiment, the disclosure describes the use as defined above, of:
- at least 4 genes chosen among a group of 23 genes comprising or consisting of the nucleic acid sequences SEQ ID NO 1 to 23,
- or fragments of said genes
- or complementary sequences of said genes
- or sequences having at least 80% homology with said genes or fragment thereof,
- or protein coded by said genes, said proteins comprising or consisting of the nucleic acid sequences SEQ ID NO 24 to 46,
said at least 4 genes being such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13 , and
d. at least one gene belongs to a fourth set D of 9 genes comprising or consisting of the nucleic acid sequences SEQ ID NO: 14-23,
for the implementation of a prognosis method, preferably *in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
+ during a period of time of 30 months after the diagnosis of said lung cancer
   ▪ when said tumor is ADK :
      o if none of the 23 genes of said set is expressed, the patient survival rate is about 86%, or more
      o if at least one of the genes of the set B is expressed, the patient survival rate is about 63%,
   ▪ when said tumor is BAS :
      o if none of the 23 genes of said set is expressed, the patient survival rate is about 65% , or more
      o if at least one of the genes of the set A is expressed, the patient survival rate is about 33%,
   ▪ when said tumor is SQC :
      o if none of the 23 genes of said set is expressed, the patient survival rate is about 88% , or more
      o if at least one of the genes of the set C is expressed, the patient survival rate is about 64%
   ▪ when said tumor is LCNE :
      o if none of the 23 genes of said set is expressed, the patient survival rate is about 65% , or more
      o if at least one of the genes of the set D is expressed, the patient survival rate is about 7%,
or,
   + during a period of time of 60 months after the diagnosis of said lung cancer
   ▪ when said tumor is ADK :
      o if none of the 23 genes of said set is expressed, the patient survival rate is about 82%, or more
      o if at least one of the genes of the set B is expressed, the patient survival rate is about 46%,
   ▪ when said tumor is BAS :
      o if none of the 23 genes of said set is expressed, the patient survival rate is about 35%, or more
      o if at least one of the genes of the set A is expressed, the patient survival rate is about 7%,
   ▪ when said tumor is SQC :
      o if none of the 23 genes of said set is expressed, the patient survival rate is about 77%, or more
      o if at least one of the genes of the set C is expressed, the patient survival rate is about 42%
   ▪ when said tumor is LCNE :
      o if none of the 23 genes of said set is expressed, the patient survival rate is about 55%, or more
      o if at least one of the genes of the set D is expressed, the patient survival rate is about 4%,
or,
   + during a period of time of 120 months after the diagnosis of said lung cancer
   ▪ when said tumor is ADK :
      o if none of the 23 genes of said set is expressed, the patient survival rate is about 76%, or more
      o if at least one of the genes of the set B is expressed, the patient survival rate is about 39%,
   ▪ when said tumor is BAS :
      o if none of the 23 genes of said set is expressed, the patient survival rate is about 19%, or more
      o if at least one of the genes of the set A is expressed, the patient survival rate is about 0%,
   ▪ when said tumor is SQC :
      o if none of the 23 genes of said set is expressed, the patient survival rate is about 58%, or more
      o if at least one of the genes of the set C is expressed, the patient survival rate is about 33%,
   ▪ when said tumor is LCNE :
      o if none of the 23 genes of said set is expressed, the patient survival rate is about 40%, or more
      o if at least one of the genes of the set D is expressed, the patient survival rate is about 0%.

In another advantageous embodiment, the invention relates to the use as defined above, of:
- at least 4 genes chosen among a group of 23 genes comprising or consisting of the nucleic acid sequences SEQ ID NO 1 to 23,
- or complementary sequences of said genes
- or sequences having at least 80% homology with said genes or fragment thereof,
- or protein coded by said genes, said proteins comprising or consisting of the nucleic acid sequences SEQ ID NO 24 to 46,
said at least 4 genes being such that
e. at least one gene belongs to a first set A of 4 genes comprising or consisting of the nucleic acid sequences SEQ ID NO: 1 to 4,
f. at least one gene belongs to a second set B of 3 genes comprising or consisting of the nucleic acid sequences SEQ ID NO: 5 to 7
g. at least one gene belongs to a third set C of 6 genes comprising or consisting of the nucleic acid sequences SEQ ID NO: 8 to 13 , and
h. at least one gene belongs to a fourth set D of 9 genes comprising or consisting of the nucleic acid sequences SEQ ID NO: 14-23,
for the implementation of a prognosis method, preferably *in vitro,* of the survival rate of a patient afflicted by a lung cancer, said lung cancer being previously classified as a lung tumour belonging from the group consisting of ADK, SQC, BAS et LCNE,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
+ during a period of time of 30 months after the diagnosis of said lung cancer
   ▪ when said tumor is ADK :
      o if none of the 23 genes of said set is expressed, the patient survival rate is about 86%, or more
      o if at least one of the genes of the set B is expressed, the patient survival rate is about 63%,
   ▪ when said tumor is BAS :
      o if none of the 23 genes of said set is expressed, the patient survival rate is about 65% , or more
      o if at least one of the genes of the set A is expressed, the patient survival rate is about 33%,
   ▪ when said tumor is SQC :
      o if none of the 23 genes of said set is expressed, the patient survival rate is about 88% , or more
      o if at least one of the genes of the set C is expressed, the patient survival rate is about 64%
   ▪ when said tumor is LCNE :
      o if none of the 23 genes of said set is expressed, the patient survival rate is about 65% , or more
      o if at least one of the genes of the set D is expressed, the patient survival rate is about 7%,
or,
   + during a period of time of 60 months after the diagnosis of said lung cancer
   ▪ when said tumor is ADK :
      o if none of the 23 genes of said set is expressed, the patient survival rate is about 82%, or more
      o if at least one of the genes of the set B is expressed, the patient survival rate is about 46%,
   ▪ when said tumor is BAS :
      o if none of the 23 genes of said set is expressed, the patient survival rate is about 35%, or more
      o if at least one of the genes of the set A is expressed, the patient survival rate is about 7%,
   ▪ when said tumor is SQC :
      o if none of the 23 genes of said set is expressed, the patient survival rate is about 77%, or more
      o if at least one of the genes of the set C is expressed, the patient survival rate is about 42%
   ▪ when said tumor is LCNE :
      o if none of the 23 genes of said set is expressed, the patient survival rate is about 55%, or more
      o if at least one of the genes of the set D is expressed, the patient survival rate is about 4%,
or,
   + during a period of time of 120 months after the diagnosis of said lung cancer
   ▪ when said tumor is ADK :
      o if none of the 23 genes of said set is expressed, the patient survival rate is about 76%, or more
      o if at least one of the genes of the set B is expressed, the patient survival rate is about 39%,
   ▪ when said tumor is BAS :
      o if none of the 23 genes of said set is expressed, the patient survival rate is about 19%, or more
      o if at least one of the genes of the set A is expressed, the patient survival rate is about 0%,
   ▪ when said tumor is SQC :
      o if none of the 23 genes of said set is expressed, the patient survival rate is about 58%, or more
      o if at least one of the genes of the set C is expressed, the patient survival rate is about 33%,
   ▪ when said tumor is LCNE :
      o if none of the 23 genes of said set is expressed, the patient survival rate is about 40%, or more
      o if at least one of the genes of the set D is expressed, the patient survival rate is about 0%.

The present invention relates to the use as defined above, wherein
◆ said at least 4 genes are such that
   a. at least one gene belongs to a first set A of 4 genes comprising or consisting of the nucleic acid sequences SEQ ID NO: 1 to 4,
   b. at least one gene belongs to a second set B of 3 genes comprising or consisting of the nucleic acid sequences SEQ ID NO: 5 to 7,
   c. at least one gene belongs to a third set C of 6 genes comprising or consisting of the nucleic acid sequences SEQ ID NO: 8 to 13,
   d. at least one gene belongs to a fourth set D of 9 genes comprising or consisting of the nucleic acid sequences SEQ ID NO: 14-23, is chosen among the nucleic acid sequences SEQ ID NO 14 or SEQ ID NO: 16 to 22,
◆ said at least 4 proteins are such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30, and
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46, is chosen among the amino acid sequences SEQ ID NO 37 or SEQ ID NO 39-45.

In another advantageous embodiment, the invention relates to the use as defined above, wherein
a. said at least one gene belongs to a first set A of 4 genes comprising or consisting of the nucleic acid sequences SEQ ID NO: 1 to 4, is chosen among the nucleic acid sequence SEQ ID NO: 1 to 3, i.e belongs to a first subset A2 as defined above,
b. at least one gene belongs to a second set B of 3 genes comprising or consisting of the nucleic acid sequences SEQ ID NO: 5 to 7, is chosen among the nucleic acid sequence SEQ ID NO: 5 or 6, i.e belongs to a second subset B1as defined above,
c. at least one gene belongs to a third set C of 6 genes comprising or consisting of the nucleic acid sequences SEQ ID NO: 8 to 13, is chosen among the nucleic acid sequence SEQ ID NO: 8 to 11, i.e belongs to a third subset C2 as defined above and
d. at least one gene belongs to a fourth set D of 9 genes comprising or consisting of the nucleic acid sequences SEQ ID NO: 14-23, is chosen among the nucleic acid sequence SEQ ID NO: 16 to 20 i.e belongs to a fourth subset D5 as defined above.

In another advantageous embodiment, the invention relates to the use as defined above, wherein
+ during a period of time of 30 months after the diagnosis of said lung cancer
   ▪ when said tumor is SQC :
      o if none of the 23 genes of said set is expressed, the patient survival rate is about 88%
      o if one of the genes of the set C is expressed, the patient survival rate is about 73%,
      o if at least two of the genes of the set C are expressed, the patient survival rate is about 50%.
+ during a period of time of 60 months after the diagnosis of said lung cancer
   ▪ when said tumor is SQC :
      o if none of the 23 genes of said set is expressed, the patient survival rate is about 77%
      o if one of the genes of the set C is expressed, the patient survival rate is about 59%,
      o if at least two of the genes of the set C are expressed, the patient survival rate is about 14%,
+ during a period of time of 120 months after the diagnosis of said lung cancer
   ▪ when said tumor is SQC :
      o if none of the 23 genes of said set is expressed, the patient survival rate is about 58%
      o if one of the genes of the set C is expressed, the patient survival rate is about 45%,
      o if at least two of the genes of the set C are expressed, the patient survival rate is about 14%.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, as defined above, said lung cancer being previously classified as a lung tumour belonging from the group consisting of ADK, SQC, BAS et LCNE,
said method comprising a step of measuring, in said biological sample, the expression of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
- or proteins coded by said least 4 genes chosen among a set of 23 genes comprising or consisting of the nucleic acid sequences SEQ ID NO 1 to 23, said proteins comprising or consisting in amino acid sequences SEQ ID NO 24 to 46
said
◆ at least 4 genes being such that
   a. at least one gene belongs to a first set A of 4 genes comprising or consisting of the nucleic acid sequences SEQ ID NO: 1 to 4, or a subset A1 as defined above,
   b. at least one gene belongs to a second set B of 3 genes comprising or consisting of the nucleic acid sequences SEQ ID NO: 5 to 7, or a subset B 1 or B2 as defined above
   c. at least one gene belongs to a third set C of 6 genes comprising or consisting of the nucleic acid sequences SEQ ID NO: 8 to 13 , or a subset C 1 or C2 as defined above, and
   d. at least one gene belongs to a fourth set D of 9 genes comprising or consisting of the nucleic acid sequences SEQ ID NO: 14-23, or a subset D1, D2 or D3 as defined above,
◆ at least 4 proteins being such that
   a. at least one protein belongs to a first set AP of 4 proteins comprising or consisting of the amino acid sequences SEQ ID NO: 24-27, or a subset AP1 as defined above
   b. at least one protein belongs to a second set BP of 3 proteins comprising or consisting of the amino acid sequences SEQ ID NO: 28-30, or a subset BP1 or BP2 as defined above
   c. at least one protein belongs to a second set CP of 6 proteins comprising or consisting of the amino acid sequences SEQ ID NO: 31-36, or a subset CP1 or CP2 as defined above,
   d. at least one protein belongs to a second set DP of 9 proteins comprising or consisting of the amino acid sequences SEQ ID NO: 37-46, or a subset DP1, DP2 or DP3 as defined above,
wherein
- the nucleic acid sequences SEQ ID NO: 1 to 4 and/or the amino acid sequences SEQ ID NO: 24 to 27 are expressed in BAS,
- the nucleic acid sequences SEQ ID NO: 5 to 7 and/or the amino acid sequences SEQ ID NO: 28 to 30 are expressed in ADK,
- the nucleic acid sequences SEQ ID NO: 8 to 13 and/or the amino acid sequences SEQ ID NO: 31 to 36 are expressed in SQC,
- the nucleic acid sequences SEQ ID NO: 14 to 23 and/or the amino acid sequences SEQ ID NO: 37 to 46 are expressed in LCNE,
said prognosis being such that:
either
   ▪ if none of the genes of said set A, B, C and D is expressed, the patient survival rate during a period of time from 30 to 120 months after the diagnosis of said lung cancer is from about 59% to about 78% or more, and
   ▪ if at least one gene of at least one set A, B, C or D is expressed , the patient survival rate during a period of time from 30 to 120 months after the diagnosis of said lung cancer is about from about 3% to about 70%,
or
   ▪ if none of the proteins of said set AP, BP, CP and DP is expressed, the patient survival rate during a period of time from 30 to 120 months after the diagnosis of said lung cancer is from about 59% to about 78% or more, and
   ▪ if at least one protein of at least one set AP, BP, CP and DP is expressed, the patient survival rate during a period of time from 30 to 120 months after the diagnosis of said lung cancer is about from about 3% to about 70%.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, as defined above,
said method comprising a step of measuring, in said biological sample, the expression of
- at least 4 genes chosen among a set of 23 genes comprising or consisting of the nucleic acid sequences SEQ ID NO 1 to 23,
- or complementary sequences of said least 4 genes chosen among a set of 23 genes comprising or consisting of the nucleic acid sequences SEQ ID NO 1 to 23,
- or sequences having at least 80% homology with said genes or fragment thereof,
said
◆ at least 4 genes being such that
   a. at least one gene belongs to a first set A of 4 genes comprising or consisting of the nucleic acid sequences SEQ ID NO: 1 to 4, or a subset A1 as defined above,
   b. at least one gene belongs to a second set B of 3 genes comprising or consisting of the nucleic acid sequences SEQ ID NO: 5 to 7, or a subset B 1 or B2 as defined above
   c. at least one gene belongs to a third set C of 6 genes comprising or consisting of the nucleic acid sequences SEQ ID NO: 8 to 13 , or a subset C1 or C2 as defined above, and
   d. at least one gene belongs to a fourth set D of 9 genes comprising or consisting of the nucleic acid sequences SEQ ID NO: 14-23, or a subset D1, D2 or D3 as defined above,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
▪ if none of the genes of said set A, B, C and D is expressed, the patient survival rate during a period of time from 30 to 120 months after the diagnosis of said lung cancer is from about 59% to about 78% or more, and
▪ if at least one gene of at least one set A, B, C or D is expressed , the patient survival rate during a period of time from 30 to 120 months after the diagnosis of said lung cancer is about from about 3% to about 70%.

Advantageously, the disclosure describes a prognosis method, preferably in vitro, of the survival rate of a patient afflicted by a lung tumour, from a biological sample containing said lung tumor belonging from the group consisting of ADK, SQC, BAS et LCNE, at a time from 30 to 120 months after the diagnosis of said lung cancer,
said method comprising a step of measuring, in said biological sample, the expression of
- at least 4 genes chosen among a group of 23 genes comprising or consisting of the nucleic acid sequences SEQ ID NO 1 to 23,
- or fragments of said genes
- or complementary sequences of said genes said at least 4 genes being such that
   a. at least one gene belongs to a first set A of 4 genes comprising or consisting of the nucleic acid sequences SEQ ID NO: 1 to 4
   b. at least one gene belongs to a second set B of 3 genes comprising or consisting of the nucleic acid sequences SEQ ID NO: 5 to 7
   c. at least one gene belongs to a third set C of 6 genes comprising or consisting of the nucleic acid sequences SEQ ID NO: 8 to 13, and
   d. at least one gene belongs to a fourth set D of 9 genes comprising or consisting of the nucleic acid sequences SEQ ID NO: 14-23,
wherein
- the nucleic acid sequences SEQ ID NO: 1 to 4 are expressed in BAS,
- the nucleic acid sequences SEQ ID NO: 5 to 7 are expressed in ADK,
- the nucleic acid sequences SEQ ID NO: 8 to 13 are expressed in SQC,
- the nucleic acid sequences SEQ ID NO: 14 to 23 are expressed in LCNE,
said prognosis being such that:
+ during a period of time of 30 months after the diagnosis of said lung cancer
   ▪ when said tumor is ADK :
      o if none of the 23 genes of said set is expressed, the patient survival rate is about 86%, or more
      o if at least one of the genes of the set B is expressed, the patient survival rate is about 63%,
   ▪ when said tumor is BAS :
      o if none of the 23 genes of said set is expressed, the patient survival rate is about 65% , or more
      o if at least one of the genes of the set A is expressed, the patient survival rate is about 33%,
   ▪ when said tumor is SQC :
      o if none of the 23 genes of said set is expressed, the patient survival rate is about 88% , or more
      o if at least one of the genes of the set C is expressed, the patient survival rate is about 64%
   ▪ when said tumor is LCNE :
      o if none of the 23 genes of said set is expressed, the patient survival rate is about 65% , or more
      o if at least one of the genes of the set D is expressed, the patient survival rate is about 7%,
or,
   + during a period of time of 60 months after the diagnosis of said lung cancer
   ▪ when said tumor is ADK :
      o if none of the 23 genes of said set is expressed, the patient survival rate is about 82%, or more
      o if at least one of the genes of the set B is expressed, the patient survival rate is about 46%,
   ▪ when said tumor is BAS :
      o if none of the 23 genes of said set is expressed, the patient survival rate is about 35%, or more
      o if at least one of the genes of the set A is expressed, the patient survival rate is about 7%,
   ▪ when said tumor is SQC :
      o if none of the 23 genes of said set is expressed, the patient survival rate is about 77%, or more
      o if at least one of the genes of the set C is expressed, the patient survival rate is about 42%
   ▪ when said tumor is LCNE :
      o if none of the 23 genes of said set is expressed, the patient survival rate is about 55%, or more
      o if at least one of the genes of the set D is expressed, the patient survival rate is about 4%,
or,
   + during a period of time of 120 months after the diagnosis of said lung cancer
   ▪ when said tumor is ADK :
      o if none of the 23 genes of said set is expressed, the patient survival rate is about 76%, or more
      o if at least one of the genes of the set B is expressed, the patient survival rate is about 39%,
   ▪ when said tumor is BAS :
      o if none of the 23 genes of said set is expressed, the patient survival rate is about 19%, or more
      o if at least one of the genes of the set A is expressed, the patient survival rate is about 0%,
   ▪ when said tumor is SQC :
      o if none of the 23 genes of said set is expressed, the patient survival rate is about 58%, or more
      o if at least one of the genes of the set C is expressed, the patient survival rate is about 33%,
   ▪ when said tumor is LCNE :
      o if none of the 23 genes of said set is expressed, the patient survival rate is about 40%, or more
      o if at least one of the genes of the set D is expressed, the patient survival rate is about 0%.

The invention relates to an *In vitro* prognosis method of the survival rate of a patient afflicted by a lung tumour, from a biological sample containing said lung tumor belonging from the group consisting of adenocarcinoma, squamous cell carcinoma, basaloid tumours and large cell neuro-endocrine tumours, at a time from 30 to 120 months after the diagnosis of said lung cancer,
said method comprising a step of measuring, in said biological sample, the expression of
- at least 4 genes chosen among a group of 23 genes comprising or consisting of the nucleic acid sequences SEQ ID NO 1 to 23,
- or complementary sequences of said genes
said at least 4 genes being such that
a. at least one gene belongs to a first set A of 4 genes comprising or consisting of the nucleic acid sequences SEQ ID NO: 1 to 4
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13, and
d. at least one gene belongs to a fourth set D of 9 genes comprising or consisting of the nucleic acid sequences SEQ ID NO: 14-23,
wherein
- the nucleic acid sequences SEQ ID NO: 1 to 4 are expressed in basaloid tumours,
- the nucleic acid sequences SEQ ID NO: 5 to 7 are expressed in adenocarcinoma,
- the nucleic acid sequences SEQ ID NO: 8 to 13 are expressed in squamous cell carcinoma,
- the nucleic acid sequences SEQ ID NO: 14 to 23 are expressed in large cell neuro-endocrine tumours,
said prognosis being such that:
+ during a period of time of 30 months after the diagnosis of said lung cancer
   ▪ when said tumor is adenocarcinoma:
      o if none of the 23 genes of said set is expressed, the patient survival rate is about 86%,
      o if at least one of the genes of the set B is expressed, the patient survival rate is about 63%,
   ▪ when said tumor is basaloid tumours:
      o if none of the 23 genes of said set is expressed, the patient survival rate is about 65% ,
      o if at least one of the genes of the set A is expressed, the patient survival rate is about 33%,
   ▪ when said tumor is squamous cell carcinoma:
      o if none of the 23 genes of said set is expressed, the patient survival rate is about 88% ,
      o if at least one of the genes of the set C is expressed, the patient survival rate is about 64%
   ▪ when said tumor is large cell neuro-endocrine tumours:
      o if none of the 23 genes of said set is expressed, the patient survival rate is about 65% ,
      o if at least one of the genes of the set D is expressed, the patient survival rate is about 7%,
or,
   + during a period of time of 60 months after the diagnosis of said lung cancer
   ▪ when said tumor is adenocarcinoma:
      o if none of the 23 genes of said set is expressed, the patient survival rate is about 82%,
      o if at least one of the genes of the set B is expressed, the patient survival rate is about 46%,
   ▪ when said tumor is basaloid tumours:
      o if none of the 23 genes of said set is expressed, the patient survival rate is about 35%,
      o if at least one of the genes of the set A is expressed, the patient survival rate is about 7%,
   ▪ when said tumor is squamous cell carcinoma:
      o if none of the 23 genes of said set is expressed, the patient survival rate is about 77%,
      o if at least one of the genes of the set C is expressed, the patient survival rate is about 42%
   ▪ when said tumor is large cell neuro-endocrine tumours:
      o if none of the 23 genes of said set is expressed, the patient survival rate is about 55%,
      o if at least one of the genes of the set D is expressed, the patient survival rate is about 4%,
or,
   + during a period of time of 120 months after the diagnosis of said lung cancer
   ▪ when said tumor is adenocarcinoma:
      o if none of the 23 genes of said set is expressed, the patient survival rate is about 76%,
      o if at least one of the genes of the set B is expressed, the patient survival rate is about 39%,
   ▪ when said tumor is basaloid tumours:
      o if none of the 23 genes of said set is expressed, the patient survival rate is about 19%,
      o if at least one of the genes of the set A is expressed, the patient survival rate is about 0%,
   ▪ when said tumor is squamous cell carcinoma:
      o if none of the 23 genes of said set is expressed, the patient survival rate is about 58%,
      o if at least one of the genes of the set C is expressed, the patient survival rate is about 33%,
   ▪ when said tumor is large cell neuro-endocrine tumours:
      o if none of the 23 genes of said set is expressed, the patient survival rate is about 40%,
      o if at least one of the genes of the set D is expressed, the patient survival rate is about 0%.

In one advantageous embodiment, the invention relates to the prognosis method as defined above, wherein
a. said at least one gene belongs to a first set A of 4 genes comprising or consisting of the nucleic acid sequences SEQ ID NO: 1 to 4, is chosen among the nucleic acid sequence SEQ ID NO: 1 to 3,
b. at least one gene belongs to a second set B of 3 genes comprising or consisting of the nucleic acid sequences SEQ ID NO: 5 to 7, is chosen among the nucleic acid sequence SEQ ID NO: 5 or 6,
c. at least one gene belongs to a third set C of 6 genes comprising or consisting of the nucleic acid sequences SEQ ID NO: 8 to 13, is chosen among the nucleic acid sequence SEQ ID NO: 8 to 11, and
d. at least one gene belongs to a fourth set D of 9 genes comprising or consisting of the nucleic acid sequences SEQ ID NO: 14-23, is chosen among the nucleic acid sequence SEQ ID NO: 16 to 20.

In one another advantageous embodiment, the invention relates to the prognosis method previously defined, wherein
+ during a period of time of 30 months after the diagnosis of said lung cancer
   ▪ when said tumor is SQC :
      o if none of the 23 genes of said set is expressed, the patient survival rate is about 88%
      o if one of the genes of the set C is expressed, the patient survival rate is about 73%,
      o if at least two of the genes of the set C are expressed, the patient survival rate is about 50%.
+ during a period of time of 60 months after the diagnosis of said lung cancer
   o when said tumor is SQC :
      o if none of the 23 genes of said set is expressed, the patient survival rate is about 77%
      o if one of the genes of the set C is expressed, the patient survival rate is about 59%,
      o if at least two of the genes of the set C are expressed, the patient survival rate is about 14%,
+ during a period of time of 120 months after the diagnosis of said lung cancer
   ▪ when said tumor is SQC :
      o if none of the 23 genes of said set is expressed, the patient survival rate is about 58%
      o if one of the genes of the set C is expressed, the patient survival rate is about 45%,
      o if at least two of the genes of the set C are expressed, the patient survival rate is about 14%.

In another advantageous embodiment, the invention relates to a prognosis method previously defined, wherein the step of measuring is carried out by using a technique chosen among the set consisting of:
a. Quantitative PCR,
b. DNA CHIP, and
c. Northern blot.

In another advantageous embodiment, the invention relates to a prognosis method as previously defined, wherein the step of measuring is carried out by using nucleic acid molecules consisting of from 15 to 100 nucleotides molecules being complementary to said at least 4 genes.

In one advantageous embodiment, the invention relates to a prognosis method as defined above, wherein the step of measuring is carried out by DNA CHIP using
- at least one nucleic acid probe comprising or being constituted by the nucleic acid sequence SEQ ID NO: 47 to SEQ ID NO: 50,
- at least one nucleic acid probe comprising or being constituted by the nucleic acid sequence SEQ ID NO: 51 to SEQ ID NO: 53,
- at least one nucleic acid probe comprising or being constituted by the nucleic acid sequence SEQ ID NO: 54 to SEQ ID NO: 59, and
- at least one nucleic acid probe comprising or being constituted by the nucleic acid sequence SEQ ID NO: 60 to SEQ ID NO: 69.

In another advantageous embodiment, the invention relates to the method as defined above, wherein the step of measuring is carried out by DNA CHIP using at least 2, preferably at least 3 nucleic acid probe as defined above.

An advantageous embodiment of the invention relates to the above method wherein the nucleic acid probes comprising or being constituted by the nucleic acid sequences SEQ ID NO: 47 to 69 are used, together.

In the invention the correspondence between the genes and the nucleic acid probes are as follows: SEQ ID NO:1 is able to be detected by the nucleic acid probe comprising or being constituted by the nucleic acid sequence SEQ ID NO: 47, SEQ ID NO:2 is able to be detected by the nucleic acid probe comprising or being constituted by the nucleic acid sequence SEQ ID NO: 48, SEQ ID NO:3 is able to be detected by the nucleic acid probe comprising or being constituted by the nucleic acid sequence SEQ ID NO: 49, SEQ ID NO:4 is able to be detected by the nucleic acid probe comprising or being constituted by the nucleic acid sequence SEQ ID NO: 50, SEQ ID NO:5 is able to be detected by the nucleic acid probe comprising or being constituted by the nucleic acid sequence SEQ ID NO: 51, SEQ ID NO:6 is able to be detected by the nucleic acid probe comprising or being constituted by the nucleic acid sequence SEQ ID NO: 52, SEQ ID NO:7 is able to be detected by the nucleic acid probe comprising or being constituted by the nucleic acid sequence SEQ ID NO: 53, SEQ ID NO:8 is able to be detected by the nucleic acid probe comprising or being constituted by the nucleic acid sequence SEQ ID NO: 54, SEQ ID NO:9 is able to be detected by the nucleic acid probe comprising or being constituted by the nucleic acid sequence SEQ ID NO: 55, SEQ ID NO:10 is able to be detected by the nucleic acid probe comprising or being constituted by the nucleic acid sequence SEQ ID NO: 56, SEQ ID NO: 11 is able to be detected by the nucleic acid probe comprising or being constituted by the nucleic acid sequence SEQ ID NO: 57, SEQ ID NO:12 is able to be detected by the nucleic acid probe comprising or being constituted by the nucleic acid sequence SEQ ID NO: 58, SEQ ID NO: 13 is able to be detected by the nucleic acid probe comprising or being constituted by the nucleic acid sequence SEQ ID NO: 59, SEQ ID NO:14 is able to be detected by the nucleic acid probe comprising or being constituted by the nucleic acid sequence SEQ ID NO: 60, SEQ ID NO:15 is able to be detected by the nucleic acid probe comprising or being constituted by the nucleic acid sequence SEQ ID NO: 61, SEQ ID NO:16 is able to be detected by the nucleic acid probe comprising or being constituted by the nucleic acid sequence SEQ ID NO: 62, SEQ ID NO: 17 is able to be detected by the nucleic acid probe comprising or being constituted by the nucleic acid sequence SEQ ID NO: 63, SEQ ID NO:18 is able to be detected by the nucleic acid probe comprising or being constituted by the nucleic acid sequence SEQ ID NO: 64, SEQ ID NO:19 is able to be detected by the nucleic acid probe comprising or being constituted by the nucleic acid sequence SEQ ID NO: 65, SEQ ID NO:20 is able to be detected by the nucleic acid probe comprising or being constituted by the nucleic acid sequence SEQ ID NO: 66, SEQ ID NO:21 is able to be detected by the nucleic acid probe comprising or being constituted by the nucleic acid sequence SEQ ID NO: 67, SEQ ID NO:22 is able to be detected by the nucleic acid probe comprising or being constituted by the nucleic acid sequence SEQ ID NO: 68, SEQ ID NO:23 is able to be detected by the nucleic acid probe comprising or being constituted by the nucleic acid sequence SEQ ID NO: 69.

The invention also relates to a prognosis method, preferably *in vitro,* of the survival rate of a patient afflicted by a lung tumour, from a biological sample containing said lung tumor, at a time from 30 to 120 months after the diagnosis of said lung cancer, said method comprising a step of measuring, in said biological sample, the expression of
- at least 4 proteins chosen among a set of 23 proteins coded by 23 genes comprising or consisting of the nucleic acid sequences SEQ ID NO 1 to 23,
said at least 4 proteins being such that
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30,
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46,
wherein
- the amino acid sequences SEQ ID NO: 24 to 27 are expressed in BAS,
- the amino acid sequences SEQ ID NO: 28 to 30 are expressed in ADK,
- the amino acid sequences SEQ ID NO: 31 to 36 are expressed in SQC,
- the amino acid sequences SEQ ID NO: 37 to 46 are expressed in LCNE,
said prognosis method being such that
▪ if none of the proteins is expressed, the patient survival rate is from about 59% to about 78%, or more, and
▪ if at least one protein of said set AP, BP, CP and DP is expressed, the patient survival rate from about 3% to about 70%.

In an advantageous embodiment the disclosure describes a prognosis method, preferably *in vitro,* of the survival rate of a patient afflicted by a lung tumour, from a biological sample containing said lung tumor, at a time from 30 to 120 months after the diagnosis of said lung cancer,
said method comprising a step of measuring, in said biological sample, the expression of
- at least 4 proteins chosen among a set of 23 proteins coded by 23 genes comprising or consisting of the nucleic acid sequences SEQ ID NO 1 to 23,
- or fragments of said proteins,
said at least 4 proteins being such that
◆ at least 4 proteins being such that
   a. at least one protein belongs to a first set AP of 4 proteins comprising or consisting of the amino acid sequences SEQ ID NO: 24-27,
   b. at least one protein belongs to a second set BP of 3 proteins comprising or consisting of the amino acid sequences SEQ ID NO: 28-30,
   c. at least one protein belongs to a third set CP of 6 proteins comprising or consisting of the amino acid sequences SEQ ID NO: 31-36,
   d. at least one protein belongs to a fourth set DP of 9 proteins comprising or consisting of the amino acid sequences SEQ ID NO: 37-46,
wherein
- the amino acid sequences SEQ ID NO: 24 to 27 are expressed in BAS,
- the amino acid sequences SEQ ID NO: 28 to 30 are expressed in ADK,
- the amino acid sequences SEQ ID NO: 31 to 36 are expressed in SQC,
- the amino acid sequences SEQ ID NO: 37 to 46 are expressed in LCNE,
said prognosis method being such that
+ during a period of time of 30 months after the diagnosis of said lung cancer
▪ when said tumor is ADK :
   o if none of the 23 proteins of said set is expressed, the patient survival rate is about 86%, or more
   o if at least one of the proteins of the set BP is expressed, the patient survival rate is about 63%,
▪ when said tumor is BAS :
   o if none of the 23 proteins of said set is expressed, the patient survival rate is about 65% , or more
   o if at least one of the proteins of the set AP is expressed, the patient survival rate is about 33%,
▪ when said tumor is SQC :
   o if none of the 23 proteins of said set is expressed, the patient survival rate is about 88% , or more
   o if at least one of the proteins of the set CP is expressed, the patient survival rate is about 64%
▪ when said tumor is LCNE :
   o if none of the 23 proteins of said set is expressed, the patient survival rate is about 65% , or more
   o if at least one of the proteins of the set DP is expressed, the patient survival rate is about 7%,
or,
+ during a period of time of 60 months after the diagnosis of said lung cancer
▪ when said tumor is ADK :
   o if none of the 23 proteins of said set is expressed, the patient survival rate is about 82%, or more
   o if at least one of the proteins of the set BP is expressed, the patient survival rate is about 46%,
▪ when said tumor is BAS :
   o if none of the 23 proteins of said set is expressed, the patient survival rate is about 35%, or more
   o if at least one of the proteins of the set AP is expressed, the patient survival rate is about 7%,
▪ when said tumor is SQC :
   o if none of the 23 proteins of said set is expressed, the patient survival rate is about 77%, or more
   o if at least one of the proteins of the set CP is expressed, the patient survival rate is about 42%
▪ when said tumor is LCNE :
   o if none of the 23 proteins of said set is expressed, the patient survival rate is about 55%, or more
   o if at least one of the proteins of the set DP is expressed, the patient survival rate is about 4%,
or,
+ during a period of time of 120 months after the diagnosis of said lung cancer
▪ when said tumor is ADK :
   o if none of the 23 proteins of said set is expressed, the patient survival rate is about 76%, or more
   o if at least one of the proteins of the set BP is expressed, the patient survival rate is about 39%,
▪ when said tumor is BAS :
   o if none of the 23 proteins of said set is expressed, the patient survival rate is about 19%, or more
   o if at least one of the proteins of the set AP is expressed, the patient survival rate is about 0%,
▪ when said tumor is SQC :
   o if none of the 23 proteins of said set is expressed, the patient survival rate is about 58%, or more
   o if at least one of the proteins of the set CP is expressed, the patient survival rate is about 33%,
▪ when said tumor is LCNE :
   o if none of the 23 proteins of said set is expressed, the patient survival rate is about 40%, or more
   o if at least one of the proteins of the set DP is expressed, the patient survival rate is about 0%.

The invention relates to an *In vitro* prognosis method of the survival rate of a patient afflicted by a lung tumour, from a biological sample containing said lung tumor, at a time from 30 to 120 months after the diagnosis of said lung cancer,
said method comprising a step of measuring, in said biological sample, the expression of
- at least 4 proteins chosen among a set of 23 proteins coded by 23 genes comprising or consisting of the nucleic acid sequences SEQ ID NO 1 to 23,
said at least 4 proteins being such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30,
c. at least one protein belongs to a third set CP of 6 proteins comprising or consisting of the amino acid sequences SEQ ID NO: 31-36,
d. at least one protein belongs to a fourth set DP of 9 proteins comprising or consisting of the amino acid sequences SEQ ID NO: 37-46,
wherein
- the amino acid sequences SEQ ID NO: 24 to 27 are expressed in basaloid tumours,
- the amino acid sequences SEQ ID NO: 28 to 30 are expressed in adenocarcinoma,
- the amino acid sequences SEQ ID NO: 31 to 36 are expressed in squamous cell carcinoma,
- the amino acid sequences SEQ ID NO: 37 to 46 are expressed in large cell neuro-endocrine tumours,
said prognosis method being such that
+ during a period of time of 30 months after the diagnosis of said lung cancer
▪ when said tumor is adenocarcinoma:
   o if none of the 23 proteins of said set is expressed, the patient survival rate is about 86%,
   o if at least one of the proteins of the set BP is expressed, the patient survival rate is about 63%,
▪ when said tumor is basaloid tumours:
   o if none of the 23 proteins of said set is expressed, the patient survival rate is about 65% ,
   o if at least one of the proteins of the set AP is expressed, the patient survival rate is about 33%,
▪ when said tumor is squamous cell carcinoma:
   o if none of the 23 proteins of said set is expressed, the patient survival rate is about 88% ,
   o if at least one of the proteins of the set CP is expressed, the patient survival rate is about 64%
▪ when said tumor is large cell neuro-endocrine tumours:
   o if none of the 23 proteins of said set is expressed, the patient survival rate is about 65% ,
   o if at least one of the proteins of the set DP is expressed, the patient survival rate is about 7%,
or,
+ during a period of time of 60 months after the diagnosis of said lung cancer
▪ when said tumor is adenocarcinoma:
   o if none of the 23 proteins of said set is expressed, the patient survival rate is about 82%,
   o if at least one of the proteins of the set BP is expressed, the patient survival rate is about 46%,
▪ when said tumor is basaloid tumours:
   o if none of the 23 proteins of said set is expressed, the patient survival rate is about 35%,
   o if at least one of the proteins of the set AP is expressed, the patient survival rate is about 7%,
▪ when said tumor is squamous cell carcinoma:
   o if none of the 23 proteins of said set is expressed, the patient survival rate is about 77%,
   o if at least one of the proteins of the set CP is expressed, the patient survival rate is about 42%
▪ when said tumor is large cell neuro-endocrine tumours:
   o if none of the 23 proteins of said set is expressed, the patient survival rate is about 55%,
   o if at least one of the proteins of the set DP is expressed, the patient survival rate is about 4%,
or,
+ during a period of time of 120 months after the diagnosis of said lung cancer
▪ when said tumor is adenocarcinoma:
   o if none of the 23 proteins of said set is expressed, the patient survival rate is about 76%,
   o if at least one of the proteins of the set BP is expressed, the patient survival rate is about 39%,
▪ when said tumor is basaloid tumours:
   o if none of the 23 proteins of said set is expressed, the patient survival rate is about 19%,
   o if at least one of the proteins of the set AP is expressed, the patient survival rate is about 0%,
▪ when said tumor is squamous cell carcinoma:
   o if none of the 23 proteins of said set is expressed, the patient survival rate is about 58%,
   o if at least one of the proteins of the set CP is expressed, the patient survival rate is about 33%,
▪ when said tumor is large cell neuro-endocrine tumours:
   o if none of the 23 proteins of said set is expressed, the patient survival rate is about 40%,
   o if at least one of the proteins of the set DP is expressed, the patient survival rate is about 0%.

In one advantageous embodiment, the inevention relates to the prognosis method, as defined above, wherein
a. said at least one gene belongs to a first set AP of 4 genes comprising or consisting of the nucleic acid sequences SEQ ID NO: 24 to 27, is chosen among the nucleic acid sequence SEQ ID NO: 24 to 27,
b. at least one gene belongs to a second set BP of 3 genes comprising or consisting of the nucleic acid sequences SEQ ID NO: 28 to 30, is chosen among the nucleic acid sequence SEQ ID NO: 28 or 29,
c. at least one gene belongs to a third set CP of 6 genes comprising or consisting of the nucleic acid sequences SEQ ID NO: 31 to 36, is chosen among the nucleic acid sequence SEQ ID NO: 31 to 34, and
d. at least one gene belongs to a fourth set DP of 9 genes comprising or consisting of the nucleic acid sequences SEQ ID NO: 37 to 46, is chosen among the nucleic acid sequence SEQ ID NO: 37 to 43.

In another adavantageous embodiment, the invention relates to the prognosis method as defined above, wherein
+ during a period of time of 30 months after the diagnosis of said lung cancer
   ▪ when said tumor is SQC :
      o if none of the 23 proteins of said set is expressed, the patient survival rate is about 88%
      o if one of the proteins of the set CP is expressed, the patient survival rate is about 73%,
      o if at least two of the proteins of the set CP are expressed, the patient survival rate is about 50%.
+ during a period of time of 60 months after the diagnosis of said lung cancer
   o when said tumor is SQC :
      o if none of the 23 proteins of said set is expressed, the patient survival rate is about 77%
      o if one of the proteins of the set CP is expressed, the patient survival rate is about 59%,
      o if at least two of the proteins of the set CP are expressed, the patient survival rate is about 14%,
+ during a period of time of 120 months after the diagnosis of said lung cancer
   ▪ when said tumor is SQC :
      o if none of the 23 proteins of said set is expressed, the patient survival rate is about 58%
      o if one of the proteins of the set CP is expressed, the patient survival rate is about 45%,
      o if at least two of the proteins of the set CP are expressed, the patient survival rate is about 14%.

In one advantageous embodiment, the invention relates to the prognosis methodas defined above wherein the step of measuring is carried out by using a technique chosen among the set consisting of:
a. western Blot,
b. ELISA,
c. Immunofluorescence, and
d. Immunohistochemistry.

In one other advantageous embodiment, the invention relates to a prognosis method, as defined above, wherein the step of measuring is carried out by using antibodies directed against said at least 4 proteins coded by said at least two genes.

Another advantageaous embodiment of the invention relates to a prognosis method, as defined above, further comprising a step of comparison of said measured expression to the expression in at least one control sample.

The invention also concern a kit comprising a DNA CHIP comprising at least the nucleic acid probes comprising or being constituted by the nucleic acid sequences SEQ ID NO : 47 to 69.

### FIGURES

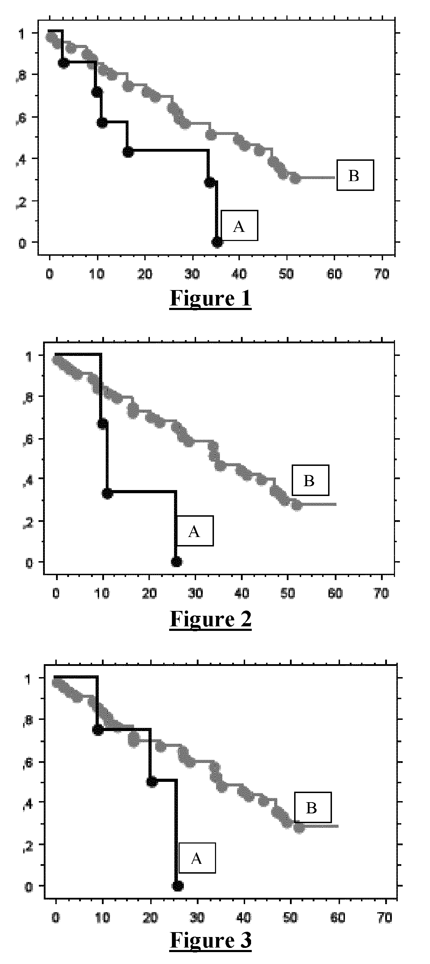

**Figure 1** represents the cumulative survival rate (Kaplan Mayer curve) over 60 months of patients expressing (A) or not (B) the gene SEQ ID NO: 4 (gene 391). Y- axis represents cumulative survival in %, X-axis represents time in months.
**Figure 2** represents the cumulative survival rate (Kaplan Mayer curve) over 60 months of patients expressing (A) or not (B) the gene SEQ ID NO: 3 (gene 417). Y- axis represents cumulative survival in %, X-axis represents time in months.
**Figure 3** represents the cumulative survival rate (Kaplan Mayer curve) over 60 months of patients expressing (A) or not (B) the gene SEQ ID NO: 2 (gene 1261). Y- axis represents cumulative survival in %, X-axis represents time in months.

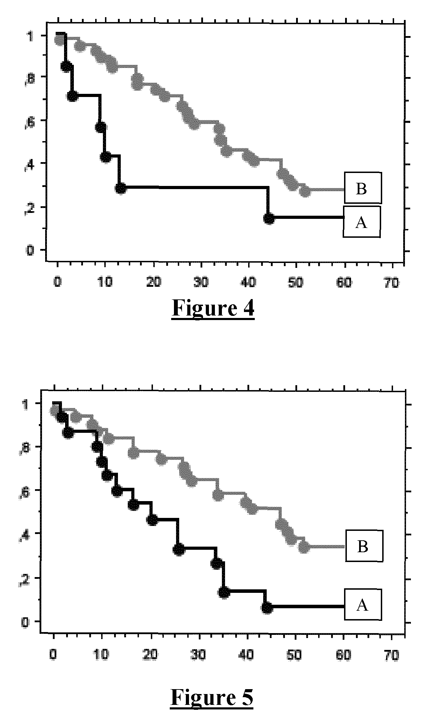

**Figure 4** represents the cumulative survival rate (Kaplan Mayer curve) over 60 months of patients expressing (A) or not (B) the following genes: SEQ ID NO: 1 (gene 1292). Y-axis represents cumulative survival in %, X-axis represents time in months.
**Figure 5** represents the cumulative survival rate (Kaplan Mayer curve) over 60 months of patients expressing (A) or not (B) at least one of the following genes: SEQ ID NO: 1 (gene 1292), SEQ ID NO: 2 (gene 1261), SEQ ID NO: 3 (gene 417) and SEQ ID NO: 4 (gene 391). Y- axis represents cumulative survival in %, X-axis represents time in months.

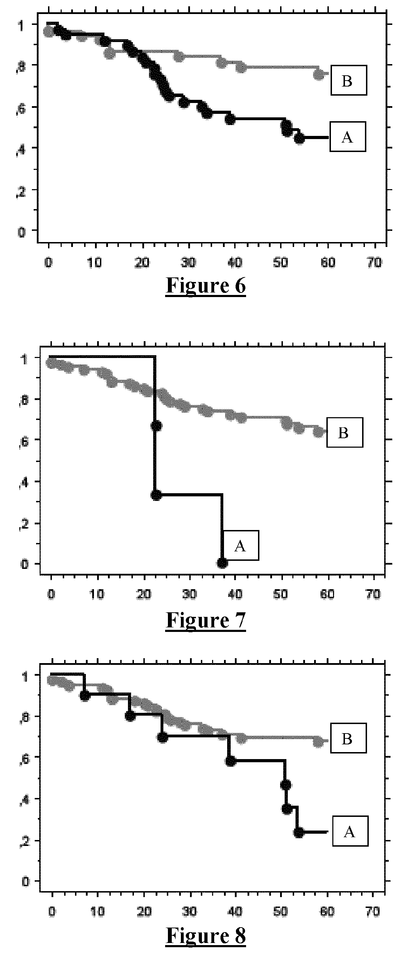

**Figure 6** represents the cumulative survival rate (Kaplan Mayer curve) over 60 months of patients expressing (A) or not (B) the following genes: SEQ ID NO: 7 (gene 59). Y- axis represents cumulative survival in %, X-axis represents time in months.
**Figure 7** represents the cumulative survival rate (Kaplan Mayer curve) over 60 months of patients expressing (A) or not (B) the following genes: SEQ ID NO: 6 (gene 222). Y-axis represents cumulative survival in %, X-axis represents time in months.
**Figure 8** represents the cumulative survival rate (Kaplan Mayer curve) over 60 months of patients expressing (A) or not (B) the following genes: SEQ ID NO: 5 (gene 1371). Y-axis represents cumulative survival in %, X-axis represents time in months.

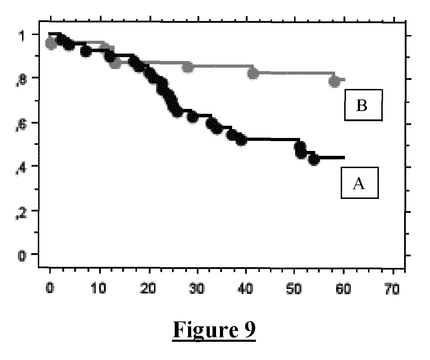

**Figure 9** represents the cumulative survival rate (Kaplan Mayer curve) over 60 months of patients expressing (A) or not (B) at least one of the following genes: SEQ ID NO: 5 (gene 1371), SEQ ID NO: 6 (gene 222) and SEQ ID NO: 7 (gene 59). Y- axis represents cumulative survival in %, X-axis represents time in months.

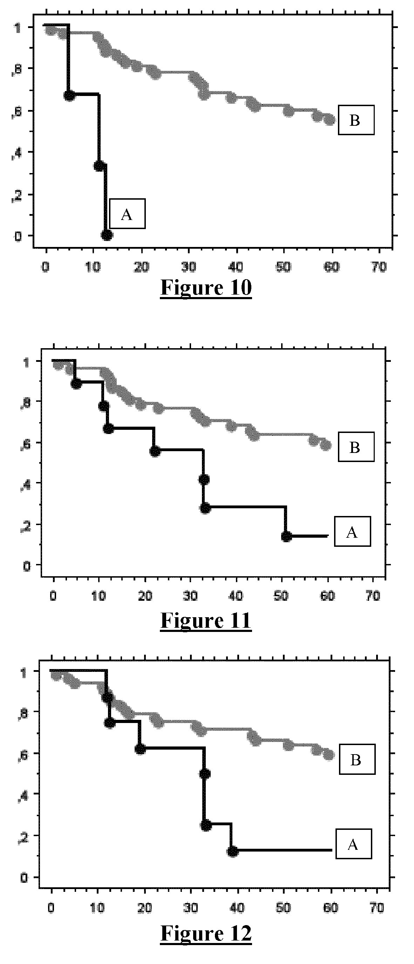

**Figure 10** represents the cumulative survival rate (Kaplan Mayer curve) over 60 months of patients expressing (A) or not (B) the gene SEQ ID NO: 12 (gene 440). Y- axis represents cumulative survival in %, X-axis represents time in months.
**Figure 11** represents the cumulative survival rate (Kaplan Mayer curve) over 60 months of patients expressing (A) or not (B) the gene SEQ ID NO: 9 (gene 442). Y- axis represents cumulative survival in %, X-axis represents time in months.
**Figure 12** represents the cumulative survival rate (Kaplan Mayer curve) over 60 months of patients expressing (A) or not (B) the gene SEQ ID NO: 8 (gene 266). Y- axis represents cumulative survival in %, X-axis represents time in months.

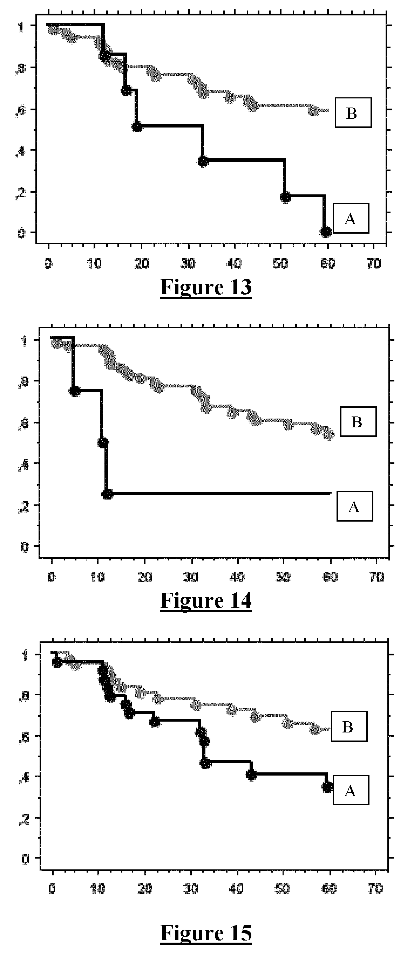

**Figure 13** represents the cumulative survival rate (Kaplan Mayer curve) over 60 months of patients expressing (A) or not (B) the gene SEQ ID NO: 13 (gene 1161). Y- axis represents cumulative survival in %, X-axis represents time in months.
**Figure 14** represents the cumulative survival rate (Kaplan Mayer curve) over 60 months of patients expressing (A) or not (B) the gene SEQ ID NO: 11 (gene 356). Y- axis represents cumulative survival in %, X-axis represents time in months.
**Figure 15** represents the cumulative survival rate (Kaplan Mayer curve) over 60 months of patients expressing (A) or not (B) the gene SEQ ID NO: 10 (gene 160). Y- axis represents cumulative survival in %, X-axis represents time in months.

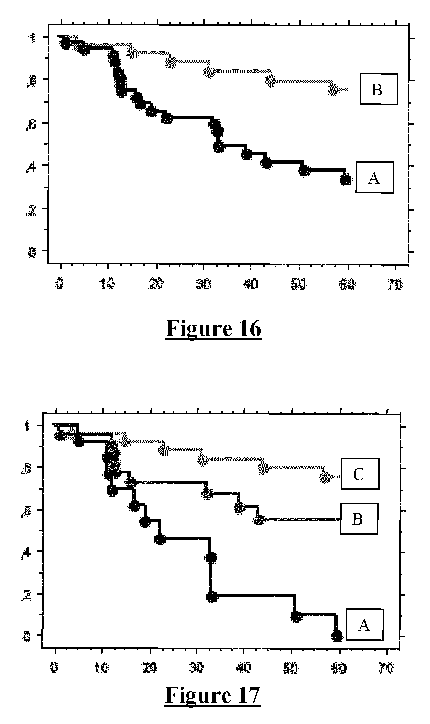

**Figure 16** represents the cumulative survival rate (Kaplan Mayer curve) over 60 months of patients expressing (A) or not (B) at least one of the following genes: SEQ ID NO: 8 (gene 266), SEQ ID NO: 9 (gene 442), SEQ ID NO: 10 (gene 160), SEQ ID NO: 11 (gene 356), SEQ ID NO: 12 (gene 440) and SEQ ID NO : 13 (gene 1161). Y- axis represents cumulative survival in %, X-axis represents time in months.
**Figure 17** represents the cumulative survival rate (Kaplan Mayer curve) over 60 months of patients expressing one (B), or two or more (A) or no (C) genes chosen among SEQ ID NO: 8 (gene 266), SEQ ID NO: 9 (gene 442), SEQ ID NO: 10 (gene 160), SEQ ID NO: 11 (gene 356), SEQ ID NO: 12 (gene 440) and SEQ ID NO : 13 (gene 1161). Y- axis represents cumulative survival in %, X-axis represents time in months.

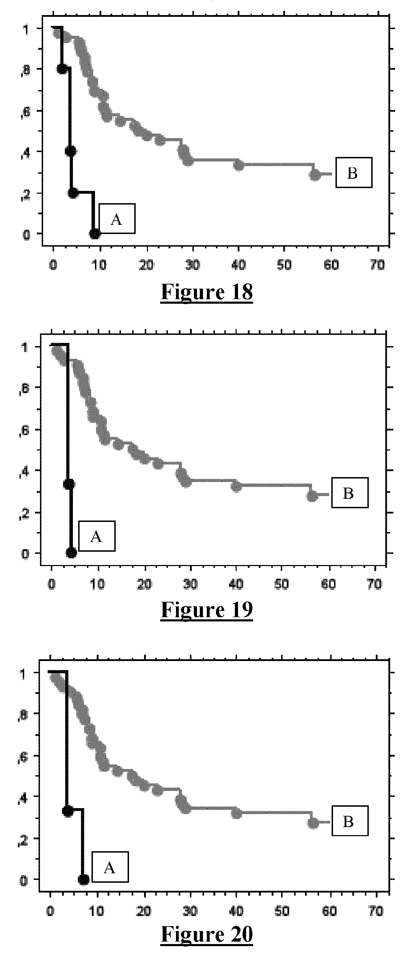

**Figure 18** represents the cumulative survival rate (Kaplan Mayer curve) over 60 months of patients expressing (A) or not (B) the gene SEQ ID NO: 22 (gene 117). Y- axis represents cumulative survival in %, X-axis represents time in months.
**Figure 19** represents the cumulative survival rate (Kaplan Mayer curve) over 60 months of patients expressing (A) or not (B) the gene SEQ ID NO: 21 (gene 118). Y- axis represents cumulative survival in %, X-axis represents time in months.
**Figure 20** represents the cumulative survival rate (Kaplan Mayer curve) over 60 months of patients expressing (A) or not (B) the gene SEQ ID NO: 18 (gene 144). Y- axis represents cumulative survival in %, X-axis represents time in months.

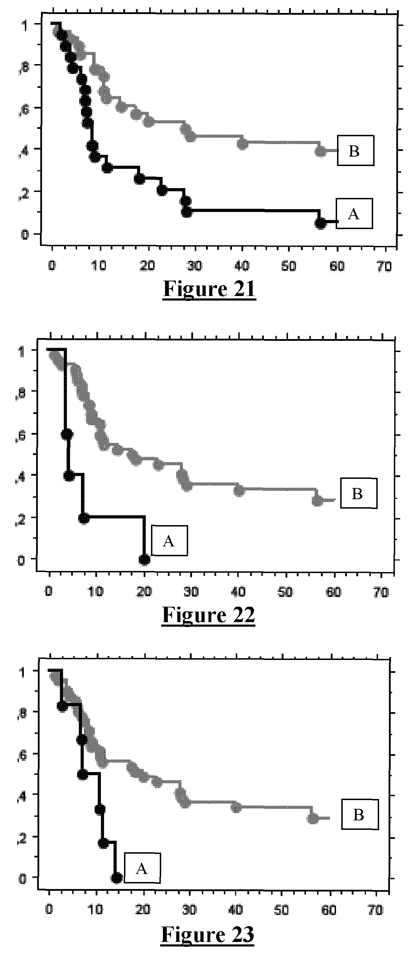

**Figure 21** represents the cumulative survival rate (Kaplan Mayer curve) over 60 months of patients expressing (A) or not (B) the gene SEQ ID NO: 16 (gene 424). Y- axis represents cumulative survival in %, X-axis represents time in months.
**Figure 22** represents the cumulative survival rate (Kaplan Mayer curve) over 60 months of patients expressing (A) or not (B) the gene SEQ ID NO: 19 (gene 437). Y- axis represents cumulative survival in %, X-axis represents time in months.
**Figure 23** represents the cumulative survival rate (Kaplan Mayer curve) over 60 months of patients expressing (A) or not (B) the gene SEQ ID NO: 17 (gene 125). Y- axis represents cumulative survival in %, X-axis represents time in months.

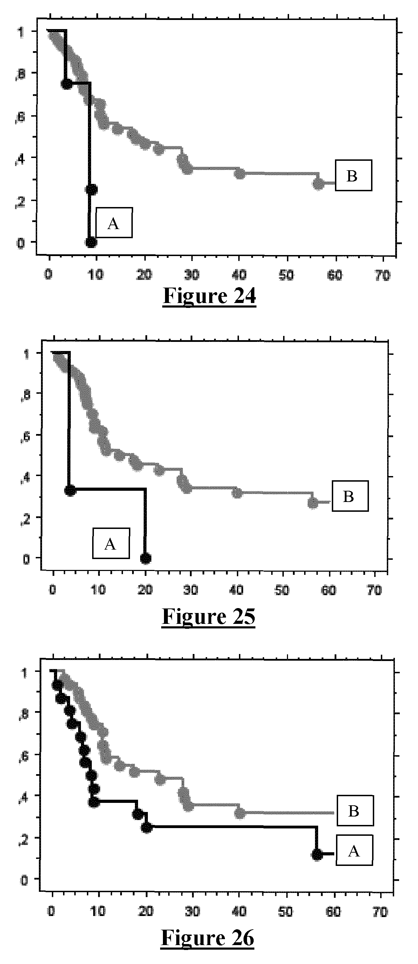

**Figure 24** represents the cumulative survival rate (Kaplan Mayer curve) over 60 months of patients expressing (A) or not (B) the gene SEQ ID NO: 20 (gene 766). Y- axis represents cumulative survival in %, X-axis represents time in months.
**Figure 25** represents the cumulative survival rate (Kaplan Mayer curve) over 60 months of patients expressing (A) or not (B) the gene SEQ ID NO: 14 (gene 71). Y- axis represents cumulative survival in %, X-axis represents time in months.
**Figure 26** represents the cumulative survival rate (Kaplan Mayer curve) over 60 months of patients expressing (A) or not (B) the gene SEQ ID NO: 23 (gene 390). Y- axis represents cumulative survival in %, X-axis represents time in months.

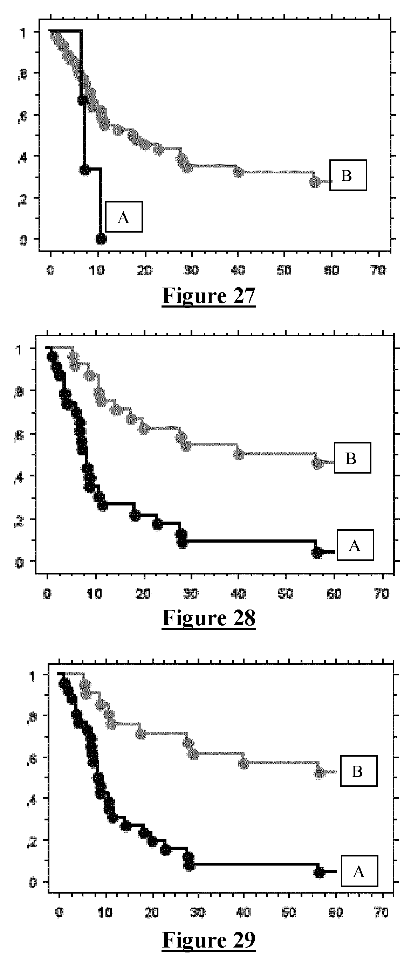

**Figure 27** represents the cumulative survival rate (Kaplan Mayer curve) over 60 months of patients expressing (A) or not (B) the gene SEQ ID NO: 15 (gene 1165). Y- axis represents cumulative survival in %, X-axis represents time in months.
**Figure 28** represents the cumulative survival rate (Kaplan Mayer curve) over 60 months of patients expressing (A) or not (B) at least one of the following genes: SEQ ID NO: 16 (gene 424), SEQ ID NO: 18 (gene 144), SEQ ID NO: 21 (gene 118) and SEQ ID NO: 22 (gene 117). Y- axis represents cumulative survival in %, X-axis represents time in months.
**Figure 29** represents the cumulative survival rate (Kaplan Mayer curve) over 60 months of patients expressing (A) or not (B) at least one of the following genes: SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.

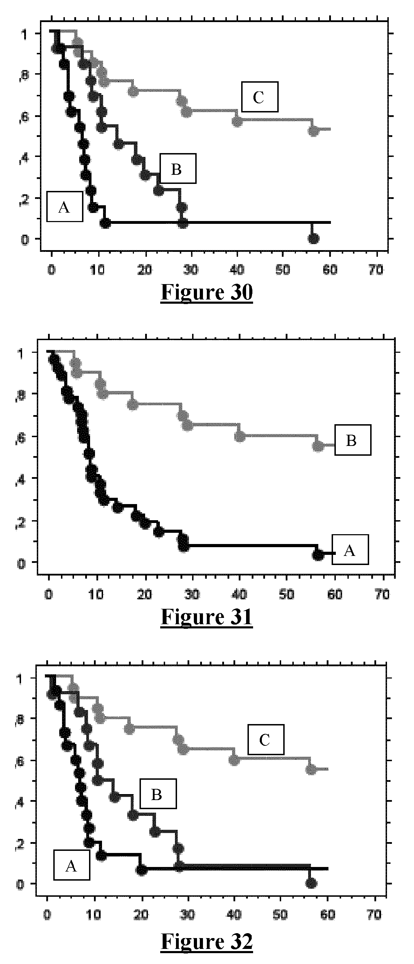

**Figure 30** represents the cumulative survival rate (Kaplan Mayer curve) over 60 months of patients expressing one (B), or two or more (A) or no (C) genes chosen among SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.
**Figure 31** represents the cumulative survival rate (Kaplan Mayer curve) over 60 months of patients expressing (A) or not (B) at least one of the following genes: SEQ ID NO : 14 (gene 71), SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.
**Figure 32** represents the cumulative survival rate (Kaplan Mayer curve) over 60 months of patients expressing one (B), or two or more (A) or no (C) genes chosen among SEQ ID NO : 14 (gene 71), SEQ ID NO: 16 (gene 424), SEQ ID NO: 17 (gene 125), SEQ ID NO: 18 (gene 144), SEQ ID NO: 21 (gene 118) and SEQ ID NO: 22 (gene 117). Y- axis represents cumulative survival in %, X-axis represents time in months.

### EXAMPLE

This work is based on a group of **23 genes,** which can be used to establish the **survival prognosis of lung tumour patients.** All these genes are actively repressed and silent in normal adult somatic cells, since their expression in strictly restricted to placenta or male germinal cells. The inventors have demonstrated that the aberrant expression in malignant cells of at least one of these genes is associated with significantly poorer prognosis for lung cancer patients. Moreover, the detection of the expression of several combinations of these genes allows predicting prognosis in lung tumour patients with higher significance and accuracy than with individual genes.

### I- Methodological approach

The following procedure allowed identifying the genes mentioned above.

### Overview

The expression of the 497 testis- and placenta- specific genes was studied in a series of 271 lung cancer samples by extracting the corresponding expression data from genome-wide transcriptomic data (the latter were obtained by C. and E. Brambilla supported by the Ligue CIT program (Carte d'Identite des Tumeurs)).

For each gene, the patients were divided into two groups,
- those expressing the gene,
- and those not expressing the gene.

For each of the 497 genes of the list, the global and disease free survival probabilities were compared between the patients expressing the gene and those not expressing the gene.

This was done considering the whole period of the study, as well as for 10 years, 5 years and 2.5 years of follow-up.

This was performed considering patients of the main histological subtypes of this population (ADK=adenocarcinoma; BAS= basaloid; LCNE=Large cell neuroendocrine; SQC=squamous cell tumour).

The genes discriminating the patients with good or bad prognosis with a significance corresponding to a p value =<0.05 (Logrank p value, obtained when comparing cumulative global survival and/or disease free survival over 5 years between patients expressing or not expressing the gene) were selected as candidate prognosis markers. For all these genes, the correlation between their expression and prognosis was validated in at least one of the two following lung published cancer transcriptomic studies with survival clinical data using the same Affymetrix technology (website GEO: http://www.ncbi.nlm.nih.gov/geo, respectively GSE4576 and GSE8894).

These studies were selected as external populations of lung cancer patients in order to validate the survival data obtained by analysing the transcriptomic data of the Brambilla study.

### Detailed procedure

### 1- Establishment of a list of placenta and testis restricted genes and analysis of their aberrant expression in lung cancer patients

1a- A list of 497 human genes whose expression was restricted to placenta or male germ cells was established as defined in WO2009/121878. These genes are never expressed in normal adult somatic tissues (adult somatic tissues comprise all tissues except germinal cells, foetal tissues and placenta).
1b- Expression data were extracted from a series of 112 normal adult somatic tissues randomly selected from a genome wide study of normal human tissues (GSE3526 on GEO, this study was chosen because it uses the same probes and measurement technology to detect gene expression as the Brambilla study: Affymetrix Human Genome U133 Plus 2.0 Array). The CEL files (raw data) from the control samples were downloaded from GEO. They were entered in the Genespring software and normalized (RMA algorithm) simultaneously with the CEL files from the Brambilla study.
1c- For each of the 497 genes/probes, the mean hybridization intensity signal value of the 112 control samples and 2 standard deviation was defined as the threshold for expression. This threshold was used to distinguish between the cancer samples expressing the gene and those not expressing the gene.
   The measurement of expression of the genes using Affymetrix microarrays involves the hybridization of fluorescence labeled cDNAs from each tissue sample on microarrays containing gene-specific probes, the fluorescence intensity signal corresponding to each probe of the microarray is measured and changed into a raw value. The absolute value of the fluorescence intensity signal is highly variable and probe-dependent (different probes corresponding to the same gene can give different intensities of fluorescence). Therefore, on the basis of these absolute fluorescence intensity values it is generally not possible to determine whether a gene is expressed or not, and commonly people use this technique to assess variations of expression between samples.
   The definition of a precise threshold for expression was possible because the selected 497 genes are NOT expressed in any normal adult somatic tissue. Therefore the signal values obtained in the 112 normal adult somatic control samples give a high confidence set of values corresponding to the background noise signal, which allow further analyses.
   A threshold signal value for expression could not have been defined for genes, which do not have a restricted expression pattern.
   Indeed in all these types of transcriptomic experiments the background noise signal value is highly dependent on the sequence of the probe. For instance several probes representative of the same gene generally give different signal values. In the case of non-restricted genes (most genes have a pattern of expression, which is not restricted to germinal cells or placenta), it is therefore impossible to use these signal values as "absolute" indicators of the presence or absence of expression. However, one can compare expression levels between two groups of tissues.
   Therefore, in this particular study, since the Inventors have previously demonstrated that all the studied 497 genes are NOT expressed in normal adult somatic tissues, the Inventors were able to define a threshold differentiating expression (ON) and non-expression (OFF). This is a specific key feature of their approach.
1d- Based on this threshold, the expression of each of the 497 genes in each of the samples was defined as negative (OFF) or positive (ON) as follow.
   In each cancer sample:
   - if the normalised signal value was above this threshold, the gene was considered as aberrantly expressed in this sample (gene ON),
   - if it was under this threshold, it was considered as not expressed (gene OFF).
1e- From the Brambilla study (271 cases of lung cancer), the Inventors found that 130 of the 497 genes were aberrantly expressed in at least 1% of these lung cancer cases.

### 2- Correlation between the expression of each individual gene (of the list of 130 genes) and the prognosis for survival in the lung cancer patients, and selection of 23 genes individually associated with the prognosis of a particular histological subtype (these subtypes being either ADK or BAS or LCNE or SQC).

2a- As a first step, using each of the 130 genes individually, we compared the global survival over a period of five years between the groups of patients expressing the gene (yes) versus those not expressing the gene (no). A Logrank Mantel-Cox test was performed and a p value was calculated. This analysis was performed with each one of the following populations: ADK cases (n=91), BAS cases (n=46), LCNE cases (n=47), SQC cases (n=62).
2b- **A total of 23 genes were selected, whose individual expression was significantly associated with a poorer prognosis (as measured by the cumulative global survival and/or disease-free survival over five years; p<0.05) in the Brambilla lung cancer study, as well as in at least one of the external validation populations (see table 1). The following table 1 lists the 23** genes associated with poor prognosis of specific histological subtypes of lung cancer, confirmed by external studies.
Gene ID corresponds to the nomenclature of the Inventors, Type corresponds to the histological subtype, SEQ ID NO gene correspond to the nucleic acid sequence according to the invention, SEQ ID NO prot corresponds to the corresponding amino acid sequence, Gene name corresponds to the name of the genes, ADK_, SQC_ BAS_ and LCNE_ corresponds to the Logrank p value for each histological type : i.e. significance of difference in cumulative global survival probabilities over 5 years between patients expressing the gene and those not expressing the gene, nb of ADK_, SQC_ BAS_ and LCNE_ corresponds to the number of patients expressing the genes.

**Table 1.**

| **Gene ID** | **type** | **SEQ ID NO gene** | **SEQ ID NO prot** | **Gene Name** | **ADK** | **nb ADK+ (/91)** | **BAS** | **nb BAS+ (/46)** | **LCNE** | **nb LCNE+ (/47)** | **SQC** | **nb SQC+ (/62)** |
|---|---|---|---|---|---|---|---|---|---|---|---|---|
| 1292 | **BAS** | **1** | **24** | KRTAP21-1 | | | **0,068** | 7 | | | | |
| 1261 | **BAS** | **2** | **25** | C12orf37 | | | **0,033** | 4 | | | | |
| 417 | **BAS** | **3** | **26** | WBSCR28 | | | **0,021** | 3 | | | | |
| 391 | **BAS** | **4** | **27** | RBM46 | | | **0,017** | 7 | | | | |
| 1371 | **ADK** | **5** | **28** | DKFZp761D1918 | **0,033** | 10 | | | | | | |
| 222 | **ADK** | **6** | **29** | NBPF4 | **0,007** | 3 | | | | | | |
| 59 | **ADK** | **7** | **30** | ADAM12 | **0,007** | 38 | | | | | | |
| 266 | **SQC** | **8** | **31** | CDNA clone IMAGE:5299000 | | | | | | | **0,01** | 9 |
| 442 | **SQC** | **9** | **32** | SOX30 | | | | | | | **0,009** | 9 |
| 160 | **SQC** | **10** | **33** | CDNA clone IMAGE:5296886 | | | | | | | **0,048** | 25 |
| 356 | **SQC** | **11** | **34** | ASZ1 | | | | | | | **0,021** | 4 |
| 440 | **SQC** | **12** | **35** | SLC2A14 | | | | | | | **<0,0001** | 4 |
| 1161 | **SQC** | **13** | **36** | COX8C | | | | | | | **0,017** | 7 |
| 71 | **LCNE** | **14** | **37** | TKTL2 | | | | | **0,052** | 3 | | |
| 1165 | **LCNE** | **15** | **38** | TPTEps 1 | | | | | **0,07** | 3 | | |
| 424 | **LCNE** | **16** | **39** | C9orf11 | | | | | **0,002** | 19 | | |
| 125 | **LCNE** | **17** | **40** | PIWIL1 | | | | | **0,02** | 6 | | |
| 144 | **LCNE** | **18** | **41** | C19orf41 | | | | | **0,0003** | 3 | | |
| 437 | **LCNE** | **19** | **42** | RNF17 | | | | | **0,003** | 5 | | |
| 766 | **LCNE** | **20** | **43** | GALNTL5 | | | | | **0,028** | 4 | | |
| 118 | **LCNE** | **21** | **44** | KISS1 | | | | | **<0,0001** | 3 | | |
| 117 | **LCNE** | 22 | 45 | IGFBP1 | | | | | **<0,0001** | 5 | | |
| 390 | **LCNE** | 23 | 46 | MAGEB6 | | | | | **0,068** | 16 | | |

2c- A detailed **quantitative evaluation of the prognosis** is given in **table 2A-D,** using each of the 23 genes associated with poor prognosis in all lung cancer histological types. The corresponding Kaplan Meyer survival curves obtained using each of these genes are represented in figures.

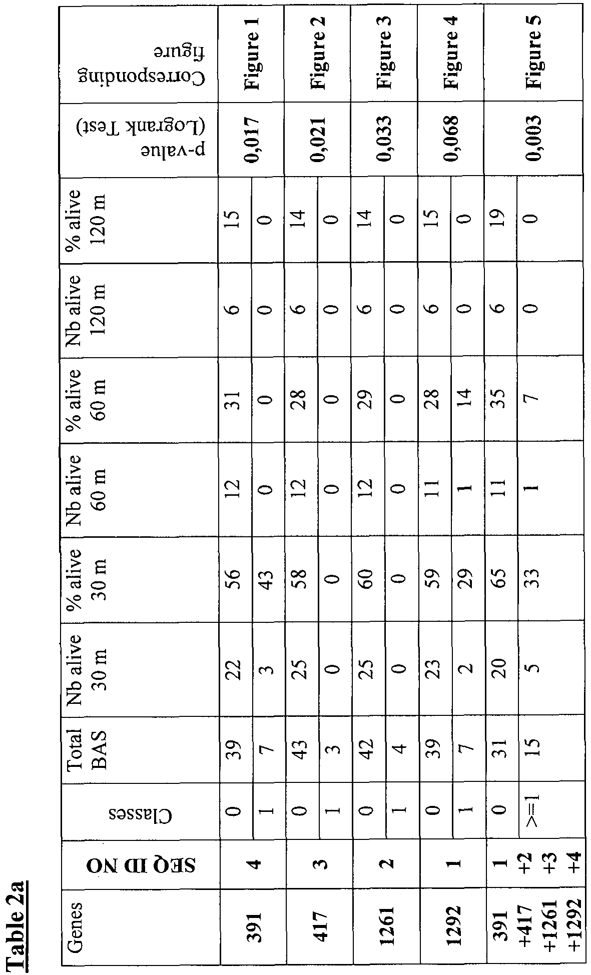

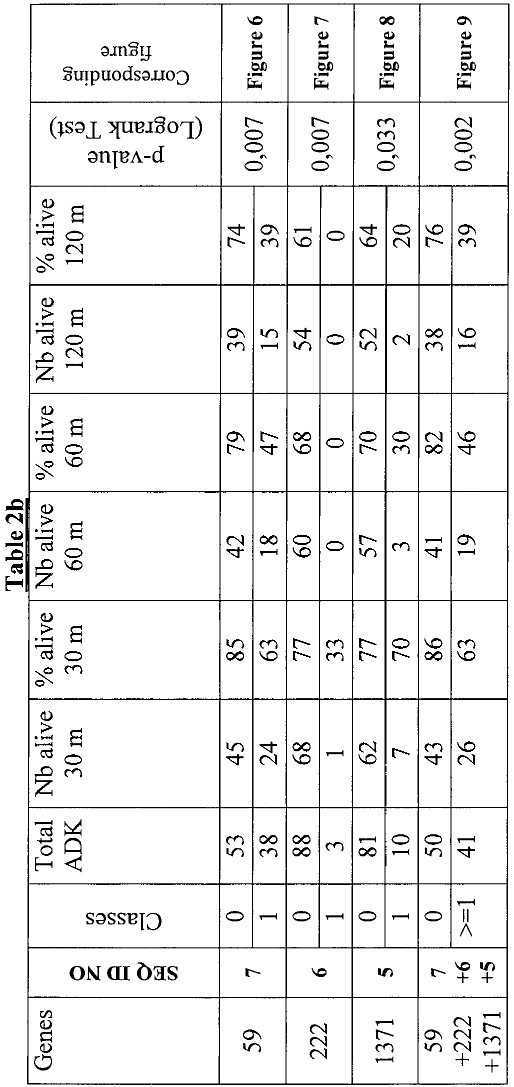

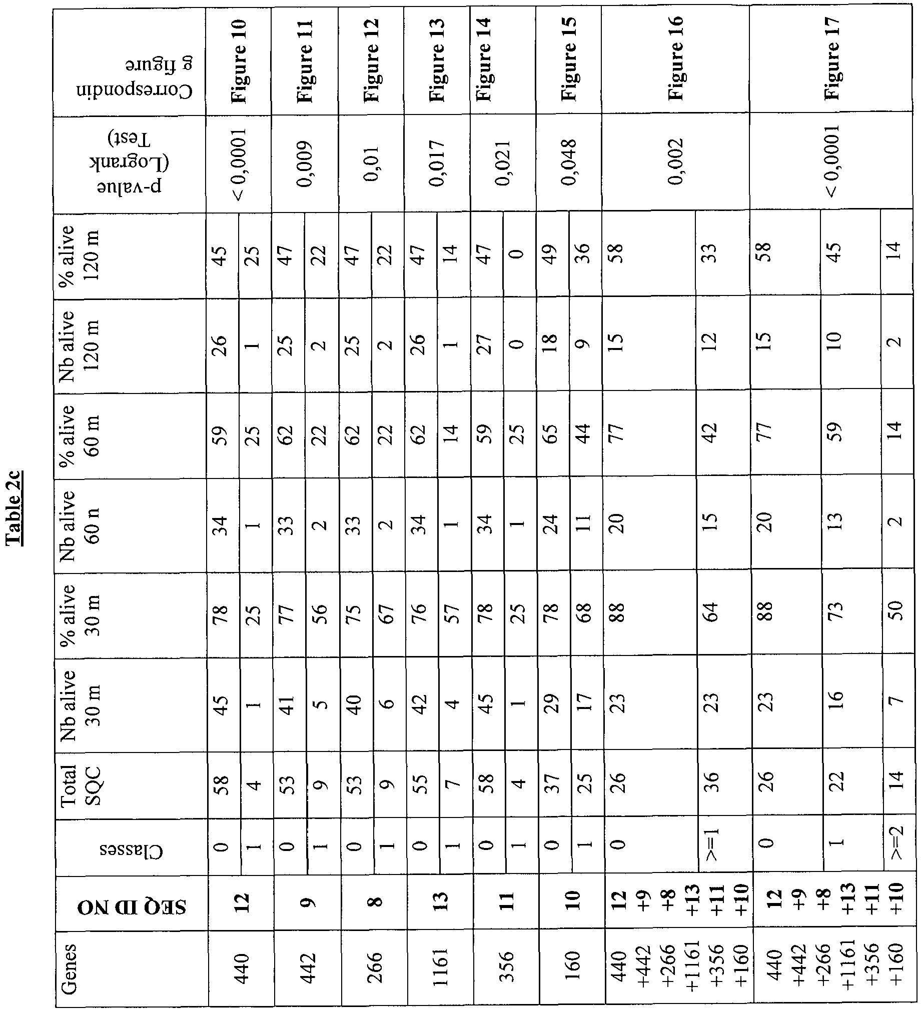

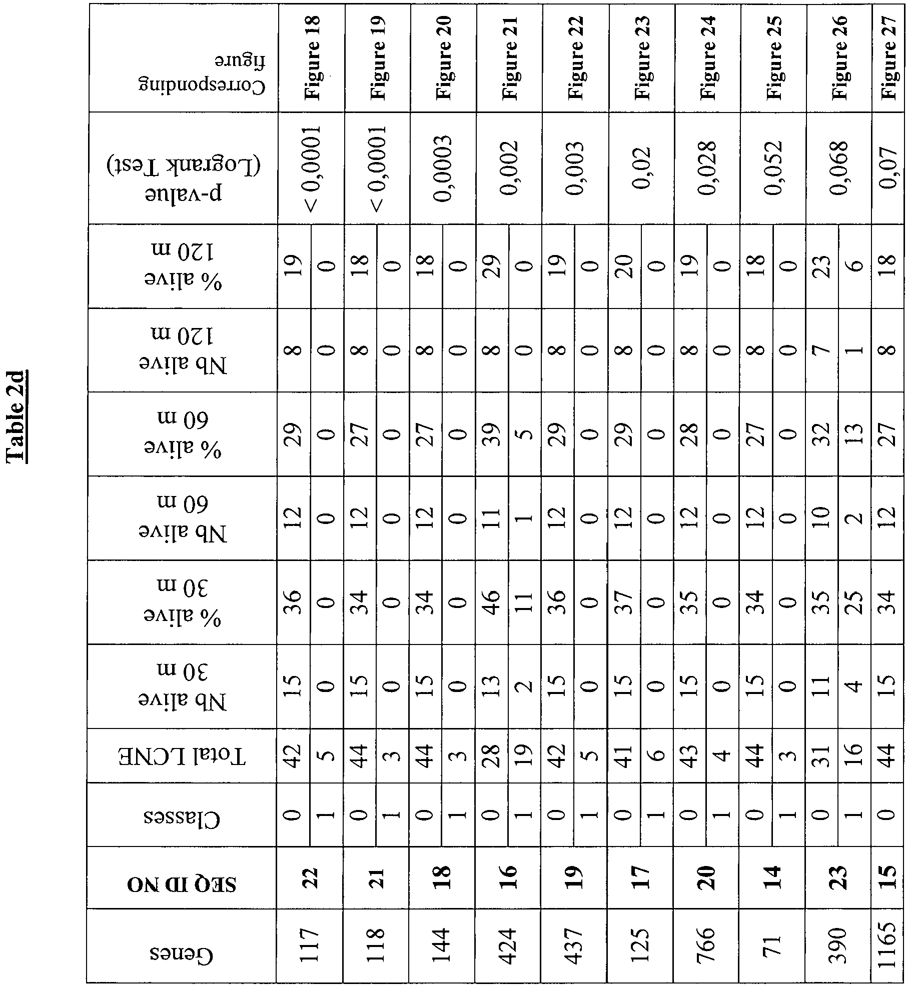

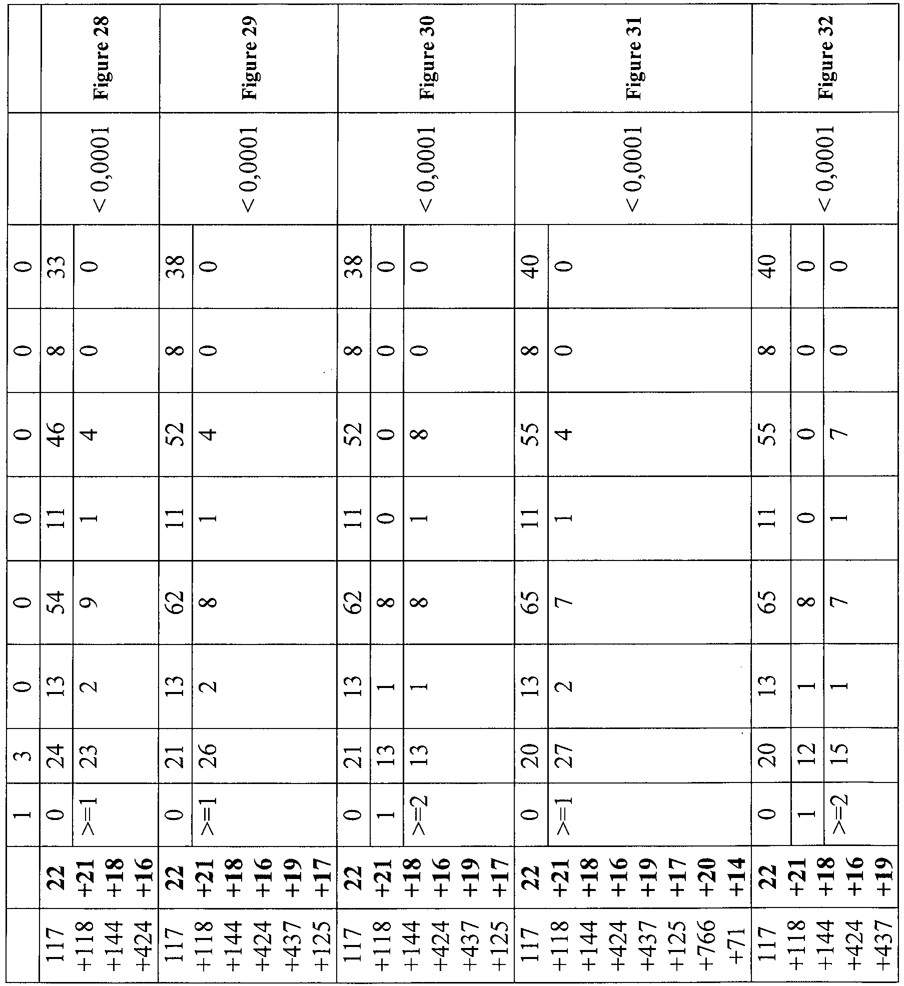

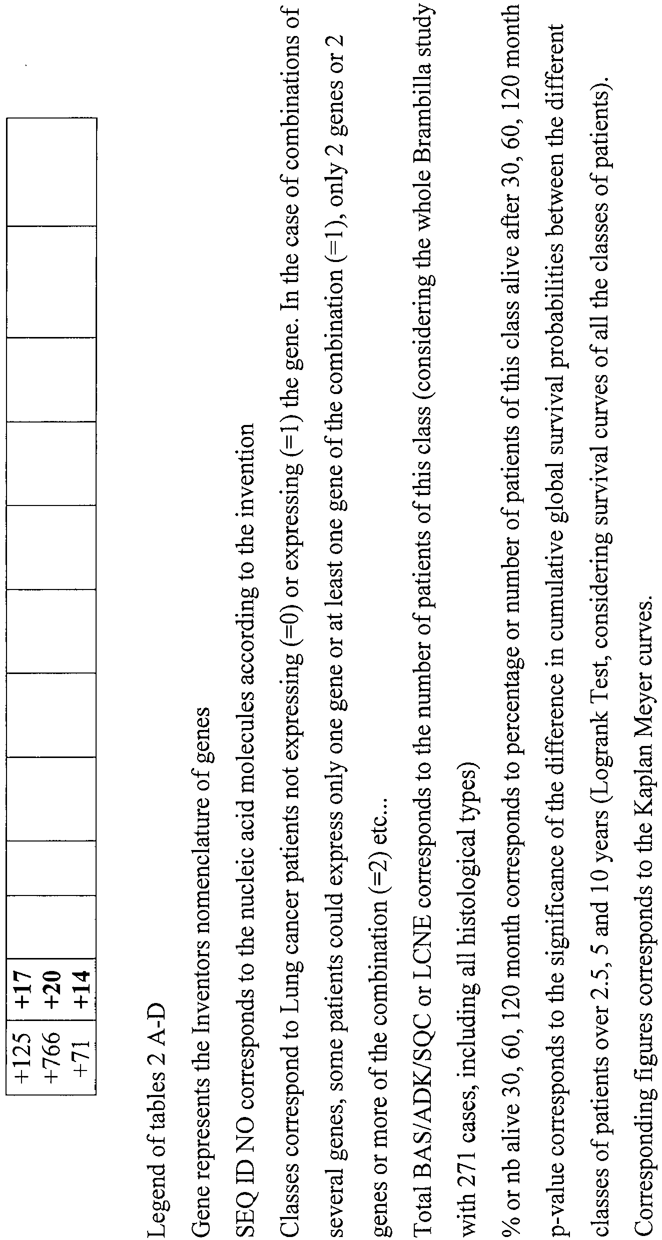

### 4- Twenty three genes (allow an accurate prognosis in lung cancer patients with specific histological subtypes, either individually or in association

4a- Three genes, individually or in association, allow defining groups of different prognosis among ADK patients (table 2B).
4b- Four genes, individually or in association, allow defining groups of different prognosis among BAS patients (table 2A).
4c- Eleven genes, individually or in association, allow defining groups of different prognosis among LCNE patients (table 2C).
4d- Six genes, individually or in association, allow defining groups of different prognosis among SQC patients (table 2D).

### CONCLUSIONS

Using 23 genes aberrantly expressed in lung cancer, the Inventors are able to assess prognosis four lung cancer patients diagnosed with one of the four main histological subtypes of non-small cell lung cancer, including adenocarcinoma, squamous cell carcinoma, large cell neuro endocrine and basaloid tumours. These 23 genes therefore provide a basis for developing easy to use diagnosis tests for histologically classified lung tumours, in order to assess the survival probability of lung cancer patients independently from other prognosis parameters (histology, tumour size and stage...) and adapt therapeutic strategies accordingly.

## Claims

1. Use of at least one element chosen among:
- at least 4 genes chosen among a set of 23 genes comprising or consisting of the nucleic acid sequences SEQ ID NO 1 to 23,
- or complementary sequences of said at least 4 genes chosen among a set of 23 genes comprising or consisting of the nucleic acid sequences SEQ ID NO 1 to 23,
- or sequences having at least 80% homology with said genes,
- or proteins coded by said at least 4 genes chosen among a set of 23 genes comprising or consisting of the nucleic acid sequences SEQ ID NO 1 to 23, said proteins comprising or consisting in amino acid sequences SEQ ID NO 24 to 46,
said
◆ at least 4 genes being such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13, and
d. at least one gene belongs to a fourth set D of 9 genes comprising or consisting of the nucleic acid sequences SEQ ID NO: 14-23,
◆ at least 4 proteins being such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30, and
c. at least one protein belongs to a third set CP of 6 proteins comprising or consisting of the amino acid sequences SEQ ID NO: 31-36,
d. at least one protein belongs to a fourth set DP of 9 proteins comprising or consisting of the amino acid sequences SEQ ID NO: 37-46,
for the implementation of an *in vitro* prognosis method of the survival rate of a patient afflicted by a lung cancer, said lung cancer being previously classified as a lung tumour belonging from the group consisting of squamous cell carcinoma, adenocarcinoma, large cell neuro-endocrine tumours and basaloid tumours,
wherein
- the nucleic acid sequences SEQ ID NO: 1 to 4 and/or the amino acid sequences SEQ ID NO: 24 to 27 are expressed in basaloid tumours,
- the nucleic acid sequences SEQ ID NO: 5 to 7 and/or the amino acid sequences SEQ ID NO: 28 to 30 are expressed in adenocarcinoma,
- the nucleic acid sequences SEQ ID NO: 8 to 13 and/or the amino acid sequences SEQ ID NO: 31 to 36 are expressed in squamous cell carcinoma,
- the nucleic acid sequences SEQ ID NO: 14 to 23 and/or the amino acid sequences SEQ ID NO: 37 to 46 are expressed in large cell neuro-endocrine tumours,
said prognosis being such that:
either
+ during a period of time of 30 months after the diagnosis of said lung cancer
▪ when said tumor is adenocarcinoma:
o if none of the 23 genes, or of the 23 proteins of said set is expressed, the patient survival rate is about 86%,
o if at least one of the genes, or of the proteins of the set B is expressed, the patient survival rate is about 63%,
▪ when said tumor is basaloid tumours:
o if none of the 23 genes, or of the 23 proteins of said set is expressed, the patient survival rate is about 65% ,
o if at least one of the genes, or of the proteins of the set A is expressed, the patient survival rate is about 33%,
▪ when said tumor is squamous cell carcinoma :
o if none of the 23 genes, or of the 23 proteins of said set is expressed, the patient survival rate is about 88% ,
o if at least one of the genes, or of the proteins of the set C is expressed, the patient survival rate is about 64%
▪ when said tumor is large cell neuro-endocrine tumours:
o if none of the 23 genes, or of the 23 proteins of said set is expressed, the patient survival rate is about 65%,
o if at least one of the genes, or of the proteins of the set D is expressed, the patient survival rate is about 7%,
or,
+ during a period of time of 60 months after the diagnosis of said lung cancer
▪ when said tumor is adenocarcinoma :
o if none of the 23 genes, or of the 23 proteins of said set is expressed, the patient survival rate is about 82%,
o if at least one of the genes, or of the proteins of the set B is expressed, the patient survival rate is about 46%,
▪ when said tumor is basaloid tumours:
o if none of the 23 genes, or of the 23 proteins of said set is expressed, the patient survival rate is about 35%,
o if at least one of the genes, or of the proteins of the set A is expressed, the patient survival rate is about 7%,
▪ when said tumor is squamous cell carcinoma :
o if none of the 23 genes, or of the 23 proteins of said set is expressed, the patient survival rate is about 77%,
o if at least one of the genes, or of the proteins of the set C is expressed, the patient survival rate is about 42%
▪ when said tumor is large cell neuro-endocrine tumours:
o if none of the 23 genes, or of the 23 proteins of said set is expressed, the patient survival rate is about 55%,
o if at least one of the genes, or of the proteins of the set D is expressed, the patient survival rate is about 4%,
or,
+ during a period of time of 120 months after the diagnosis of said lung cancer
▪ when said tumor is adenocarcinoma:
o if none of the 23 genes, or of the 23 proteins of said set is expressed, the patient survival rate is about 76%,
o if at least one of the genes, or of the proteins of the set B is expressed, the patient survival rate is about 39%,
▪ when said tumor is basaloid tumours:
o if none of the 23 genes, or of the 23 proteins of said set is expressed, the patient survival rate is about 19%,
o if at least one of the genes, or of the proteins of the set A is expressed, the patient survival rate is about 0%,
▪ when said tumor is squamous cell carcinoma:
o if none of the 23 genes, or of the 23 proteins of said set is expressed, the patient survival rate is about 58%,
o if at least one of the genes, or of the proteins of the set C is expressed, the patient survival rate is about 33%,
▪ when said tumor is large cell neuro-endocrine tumours:
o if none of the 23 genes, or of the 23 proteins of said set is expressed, the patient survival rate is about 40%,
o if at least one of the genes, or of the proteins of the set D is expressed, the patient survival rate is about 0%,
wherein said gene or protein is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
said gene or protein is determined as being not expressed when:
- it is neither expressed in a sample of a patient afflicted by a lung cancer nor in a control sample of an healthy individual,
- or it is expressed in a sample of a patient afflicted by a lung cancer at a level substancially equal or inferior to the level in a control sample of an healthy individual.

2. Use according to claim 1, wherein
◆ said at least 4 genes are such that
a. at least one gene belongs to a first set A of 4 genes comprising or consisting of the nucleic acid sequences SEQ ID NO: 1 to 4,
b. at least one gene belongs to a second set B of 3 genes comprising or consisting of the nucleic acid sequences SEQ ID NO: 5 to 7,
c. at least one gene belongs to a third set C of 6 genes comprising or consisting of the nucleic acid sequences SEQ ID NO: 8 to 13,
d. at least one gene belongs to a fourth set D of 9 genes comprising or consisting of the nucleic acid sequences SEQ ID NO: 14-23, is chosen among the nucleic acid sequences SEQ ID NO 14 or SEQ ID NO: 16 to 22,
◆ said at least 4 proteins are such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30, and
c. at least one protein belongs to a third set CP of 6 proteins comprising or consisting of the amino acid sequences SEQ ID NO: 31-36,
d. at least one protein belongs to a fourth set DP of 9 proteins comprising or consisting of the amino acid sequences SEQ ID NO: 37-46, is chosen among the amino acid sequences SEQ ID NO 37 or SEQ ID NO 39-45.

3. Use according to claim 1 or 2, wherein
a. said at least one gene belongs to a first set A of 4 genes comprising or consisting of the nucleic acid sequences SEQ ID NO: 1 to 4, is chosen among the nucleic acid sequence SEQ ID NO: 1 to 3,
b. at least one gene belongs to a second set B of 3 genes comprising or consisting of the nucleic acid sequences SEQ ID NO: 5 to 7, is chosen among the nucleic acid sequence SEQ ID NO: 5 or 6,
c. at least one gene belongs to a third set C of 6 genes comprising or consisting of the nucleic acid sequences SEQ ID NO: 8 to 13, is chosen among the nucleic acid sequence SEQ ID NO: 8 to 11, and
d. at least one gene belongs to a fourth set D of 9 genes comprising or consisting of the nucleic acid sequences SEQ ID NO: 14-23, is chosen among the nucleic acid sequence SEQ ID NO: 16 to 20.

4. Use according to anyone of claims 1 to 3, wherein
+ during a period of time of 30 months after the diagnosis of said lung cancer
▪ when said tumor is squamous cell carcinoma:
o if none of the 23 genes of said set is expressed, the patient survival rate is about 88%
o if one of the genes of the set C is expressed, the patient survival rate is about 73%,
o if at least two of the genes of the set C are expressed, the patient survival rate is about 50%.
+ during a period of time of 60 months after the diagnosis of said lung cancer
▪ when said tumor is squamous cell carcinoma:
o if none of the 23 genes of said set is expressed, the patient survival rate is about 77%
o if one of the genes of the set C is expressed, the patient survival rate is about 59%,
o if at least two of the genes of the set C are expressed, the patient survival rate is about 14%,
+ during a period of time of 120 months after the diagnosis of said lung cancer
▪ when said tumor is squamous cell carcinoma:
o if none of the 23 genes of said set is expressed, the patient survival rate is about 58%
o if one of the genes of the set C is expressed, the patient survival rate is about 45%,
o if at least two of the genes of the set C are expressed, the patient survival rate is about 14%.

5. *In vitro* prognosis method of the survival rate of a patient afflicted by a lung tumour, from a biological sample containing said lung tumor belonging from the group consisting of adenocarcinoma, squamous cell carcinoma, basaloid tumours and large cell neuro-endocrine tumours, at a time from 30 to 120 months after the diagnosis of said lung cancer,
said method comprising a step of measuring, in said biological sample, the expression of
- at least 4 genes chosen among a group of 23 genes comprising or consisting of the nucleic acid sequences SEQ ID NO 1 to 23,
- or complementary sequences of said genes
said at least 4 genes being such that
a. at least one gene belongs to a first set A of 4 genes comprising or consisting of the nucleic acid sequences SEQ ID NO: 1 to 4
b. at least one gene belongs to a second set B of 3 genes comprising or consisting of the nucleic acid sequences SEQ ID NO: 5 to 7
c. at least one gene belongs to a third set C of 6 genes comprising or consisting of the nucleic acid sequences SEQ ID NO: 8 to 13, and
d. at least one gene belongs to a fourth set D of 9 genes comprising or consisting of the nucleic acid sequences SEQ ID NO: 14-23,
wherein
- the nucleic acid sequences SEQ ID NO: 1 to 4 are expressed in basaloid tumours,
- the nucleic acid sequences SEQ ID NO: 5 to 7 are expressed in adenocarcinoma,
- the nucleic acid sequences SEQ ID NO: 8 to 13 are expressed in squamous cell carcinoma,
- the nucleic acid sequences SEQ ID NO: 14 to 23 are expressed in large cell neuro-endocrine tumours,
said prognosis being such that:
+ during a period of time of 30 months after the diagnosis of said lung cancer
▪ when said tumor is adenocarcinoma:
o if none of the 23 genes of said set is expressed, the patient survival rate is about 86%,
∘ if at least one of the genes of the set B is expressed, the patient survival rate is about 63%,
▪ when said tumor is basaloid tumours:
o if none of the 23 genes of said set is expressed, the patient survival rate is about 65% ,
o if at least one of the genes of the set A is expressed, the patient survival rate is about 33%,
▪ when said tumor is squamous cell carcinoma:
o if none of the 23 genes of said set is expressed, the patient survival rate is about 88% ,
o if at least one of the genes of the set C is expressed, the patient survival rate is about 64%
▪ when said tumor is large cell neuro-endocrine tumours:
o if none of the 23 genes of said set is expressed, the patient survival rate is about 65% ,
o if at least one of the genes of the set D is expressed, the patient survival rate is about 7%,
or,
+ during a period of time of 60 months after the diagnosis of said lung cancer
▪ when said tumor is adenocarcinoma:
o if none of the 23 genes of said set is expressed, the patient survival rate is about 82%,
o if at least one of the genes of the set B is expressed, the patient survival rate is about 46%,
▪ when said tumor is basaloid tumours:
o if none of the 23 genes of said set is expressed, the patient survival rate is about 35%,
o if at least one of the genes of the set A is expressed, the patient survival rate is about 7%,
▪ when said tumor is squamous cell carcinoma:
o if none of the 23 genes of said set is expressed, the patient survival rate is about 77%,
o if at least one of the genes of the set C is expressed, the patient survival rate is about 42%
▪ when said tumor is large cell neuro-endocrine tumours:
o if none of the 23 genes of said set is expressed, the patient survival rate is about 55%,
o if at least one of the genes of the set D is expressed, the patient survival rate is about 4%,
or,
+ during a period of time of 120 months after the diagnosis of said lung cancer
▪ when said tumor is adenocarcinoma:
o if none of the 23 genes of said set is expressed, the patient survival rate is about 76%,
o if at least one of the genes of the set B is expressed, the patient survival rate is about 39%,
▪ when said tumor is basaloid tumours:
o if none of the 23 genes of said set is expressed, the patient survival rate is about 19%,
o if at least one of the genes of the set A is expressed, the patient survival rate is about 0%,
▪ when said tumor is squamous cell carcinoma:
o if none of the 23 genes of said set is expressed, the patient survival rate is about 58%,
o if at least one of the genes of the set C is expressed, the patient survival rate is about 33%,
▪ when said tumor is large cell neuro-endocrine tumours:
o if none of the 23 genes of said set is expressed, the patient survival rate is about 40%,
o if at least one of the genes of the set D is expressed, the patient survival rate is about 0%.

6. Prognosis method, according to claim 5,wherein
a. said at least one gene belongs to a first set A of 4 genes comprising or consisting of the nucleic acid sequences SEQ ID NO: 1 to 4, is chosen among the nucleic acid sequence SEQ ID NO: 1 to 3,
b. at least one gene belongs to a second set B of 3 genes comprising or consisting of the nucleic acid sequences SEQ ID NO: 5 to 7, is chosen among the nucleic acid sequence SEQ ID NO: 5 or 6,
c. at least one gene belongs to a third set C of 6 genes comprising or consisting of the nucleic acid sequences SEQ ID NO: 8 to 13, is chosen among the nucleic acid sequence SEQ ID NO: 8 to 11, and
d. at least one gene belongs to a fourth set D of 9 genes comprising or consisting of the nucleic acid sequences SEQ ID NO: 14-23, is chosen among the nucleic acid sequence SEQ ID NO: 16 to 20.

7. Prognosis method, according to claim 5 or 6, wherein
+ during a period of time of 30 months after the diagnosis of said lung cancer
▪ when said tumor is squamous cell carcinoma:
o if none of the 23 genes of said set is expressed, the patient survival rate is about 88%
o if one of the genes of the set C is expressed, the patient survival rate is about 73%,
o if at least two of the genes of the set C are expressed, the patient survival rate is about 50%.
+ during a period of time of 60 months after the diagnosis of said lung cancer
o when said tumor is squamous cell carcinoma:
o if none of the 23 genes of said set is expressed, the patient survival rate is about 77%
o if one of the genes of the set C is expressed, the patient survival rate is about 59%,
o if at least two of the genes of the set C are expressed, the patient survival rate is about 14%,
+ during a period of time of 120 months after the diagnosis of said lung cancer
▪ when said tumor is squamous cell carcinoma:
o if none of the 23 genes of said set is expressed, the patient survival rate is about 58%
o if one of the genes of the set C is expressed, the patient survival rate is about 45%,
o if at least two of the genes of the set C are expressed, the patient survival rate is about 14%.

8. Prognosis method, according to anyone of claims 5 to 7, wherein the step of measuring is carried out by using a technique chosen among the set consisting of:
a. Quantitative PCR,
b. DNA CHIP, and
c. Northern blot.

9. Prognosis method, according to anyone of claims 5 to 8, wherein the step of measuring is carried out by using nucleic acid molecules consisting of from 15 to 100 nucleotides molecules being complementary to said at least 4 genes.

10. *In vitro* prognosis method of the survival rate of a patient afflicted by a lung tumour, from a biological sample containing said lung tumor, at a time from 30 to 120 months after the diagnosis of said lung cancer,
said method comprising a step of measuring, in said biological sample, the expression of
- at least 4 proteins chosen among a set of 23 proteins coded by 23 genes comprising or consisting of the nucleic acid sequences SEQ ID NO 1 to 23,
said at least 4 proteins being such that
a. at least one protein belongs to a first set AP of 4 proteins comprising or consisting of the amino acid sequences SEQ ID NO: 24-27,
b. at least one protein belongs to a second set BP of 3 proteins comprising or consisting of the amino acid sequences SEQ ID NO: 28-30,
c. at least one protein belongs to a third set CP of 6 proteins comprising or consisting of the amino acid sequences SEQ ID NO: 31-36,
d. at least one protein belongs to a fourth set DP of 9 proteins comprising or consisting of the amino acid sequences SEQ ID NO: 37-46,
wherein
- the amino acid sequences SEQ ID NO: 24 to 27 are expressed in basaloid tumours,
- the amino acid sequences SEQ ID NO: 28 to 30 are expressed in adenocarcinoma,
- the amino acid sequences SEQ ID NO: 31 to 36 are expressed in squamous cell carcinoma,
- the amino acid sequences SEQ ID NO: 37 to 46 are expressed in large cell neuro-endocrine tumours,
said prognosis method being such that
+ during a period of time of 30 months after the diagnosis of said lung cancer
▪ when said tumor is adenocarcinoma:
o if none of the 23 proteins of said set is expressed, the patient survival rate is about 86%,
o if at least one of the proteins of the set BP is expressed, the patient survival rate is about 63%,
▪ when said tumor is basaloid tumours:
o if none of the 23 proteins of said set is expressed, the patient survival rate is about 65%,
o if at least one of the proteins of the set AP is expressed, the patient survival rate is about 33%,
▪ when said tumor is squamous cell carcinoma:
o if none of the 23 proteins of said set is expressed, the patient survival rate is about 88%,
o if at least one of the proteins of the set CP is expressed, the patient survival rate is about 64%
▪ when said tumor is large cell neuro-endocrine tumours:
o if none of the 23 proteins of said set is expressed, the patient survival rate is about 65%,
o if at least one of the proteins of the set DP is expressed, the patient survival rate is about 7%,
or,
+ during a period of time of 60 months after the diagnosis of said lung cancer
▪ when said tumor is adenocarcinoma:
o if none of the 23 proteins of said set is expressed, the patient survival rate is about 82%,
o if at least one of the proteins of the set BP is expressed, the patient survival rate is about 46%,
▪ when said tumor is basaloid tumours:
o if none of the 23 proteins of said set is expressed, the patient survival rate is about 35%,
o if at least one of the proteins of the set AP is expressed, the patient survival rate is about 7%,
▪ when said tumor is squamous cell carcinoma:
o if none of the 23 proteins of said set is expressed, the patient survival rate is about 77%,
o if at least one of the proteins of the set CP is expressed, the patient survival rate is about 42%
▪ when said tumor is large cell neuro-endocrine tumours:
o if none of the 23 proteins of said set is expressed, the patient survival rate is about 55%,
o if at least one of the proteins of the set DP is expressed, the patient survival rate is about 4%,
or,
+ during a period of time of 120 months after the diagnosis of said lung cancer
▪ when said tumor is adenocarcinoma:
o if none of the 23 proteins of said set is expressed, the patient survival rate is about 76%,
o if at least one of the proteins of the set BP is expressed, the patient survival rate is about 39%,
▪ when said tumor is basaloid tumours:
o if none of the 23 proteins of said set is expressed, the patient survival rate is about 19%,
o if at least one of the proteins of the set AP is expressed, the patient survival rate is about 0%,
▪ when said tumor is squamous cell carcinoma:
o if none of the 23 proteins of said set is expressed, the patient survival rate is about 58%,
o if at least one of the proteins of the set CP is expressed, the patient survival rate is about 33%,
▪ when said tumor is large cell neuro-endocrine tumours:
o if none of the 23 proteins of said set is expressed, the patient survival rate is about 40%,
o if at least one of the proteins of the set DP is expressed, the patient survival rate is about 0%.

11. Prognosis method, according to claim 10, wherein
e. said at least one protein belongs to a first set AP of 4 proteins comprising or consisting of the amino acid sequences SEQ ID NO: 24 to 27, is chosen among the amino acid sequence SEQ ID NO: 24 to 27,
f. at least one protein belongs to a second set BP of 3 proteins comprising or consisting of the amino acid sequences SEQ ID NO: 28 to 30, is chosen among the amino acid sequence SEQ ID NO: 28 or 29,
g. at least one protein belongs to a third set CP of 6 proteins comprising or consisting of the amino acid sequences SEQ ID NO: 31 to 36, is chosen among the amino acid sequence SEQ ID NO: 31 to 34, and
h. at least one protein belongs to a fourth set DP of 9 proteins comprising or consisting of the amino acid sequences SEQ ID NO: 37 to 46, is chosen among the amino acid sequence SEQ ID NO: 37 to 43.

12. Prognosis method, according to claim 10 or 11, wherein
+ during a period of time of 30 months after the diagnosis of said lung cancer
▪ when said tumor is squamous cell carcinoma:
o if none of the 23 proteins of said set is expressed, the patient survival rate is about 88%
o if one of the proteins of the set CP is expressed, the patient survival rate is about 73%,
o if at least two of the proteins of the set CP are expressed, the patient survival rate is about 50%.
+ during a period of time of 60 months after the diagnosis of said lung cancer
o when said tumor is squamous cell carcinoma:
o if none of the 23 proteins of said set is expressed, the patient survival rate is about 77%
o if one of the proteins of the set CP is expressed, the patient survival rate is about 59%,
o if at least two of the proteins of the set CP are expressed, the patient survival rate is about 14%,
+ during a period of time of 120 months after the diagnosis of said lung cancer
▪ when said tumor is squamous cell carcinoma:
o if none of the 23 proteins of said set is expressed, the patient survival rate is about 58%
o if one of the proteins of the set CP is expressed, the patient survival rate is about 45%,
o if at least two of the proteins of the set CP are expressed, the patient survival rate is about 14%.

13. Prognosis method, according to anyone of claims 10 to 12, wherein the step of measuring is carried out by using a technique chosen among the set consisting of:
a. western Blot,
b. ELISA,
c. Immunofluorescence, and
d. Immunohistochemistry.

14. Prognosis method, according to anyone of claims 10 to 13, wherein the step of measuring is carried out by using antibodies directed against said at least 2 proteins coded by said at least two genes.

15. Prognosis method, according to anyone of claims 10 to 14, further comprising a step of comparison of said measured expression to the expression in at least one control sample.

## Patentansprüche

1. Verwendung von mindestens einem Element, das aus Folgenden ausgewählt ist:
- mindestens 4 Genen, die aus einem Satz von 23 Genen ausgewählt sind, welche die Nukleinsäuresequenzen SEQ ID NR. 1 bis 23 umfassen bzw. aus den Nukleinsäuresequenzen SEQ ID NR. 1 bis 23 bestehen,
- oder komplementären Sequenzen der mindestens 4 Gene, die aus einem Satz von 23 Genen ausgewählt sind, welche die Nukleinsäuresequenzen SEQ ID NR. 1 bis 23 umfassen bzw. aus den Nukleinsäuresequenzen SEQ ID NR. 1 bis 23 bestehen,
- oder Sequenzen, die eine Homologie von mindestens 80 % zu den Genen aufweisen,
- oder Proteinen, die von den mindestens 4 Genen kodiert werden, die aus einem Satz von 23 Genen ausgewählt sind, welche die Nukleinsäuresequenzen SEQ ID NR. 1 bis 23 umfassen bzw. aus den Nukleinsäuresequenzen SEQ ID NR. 1 bis 23 bestehen, wobei die Proteine die Aminosäuresequenzen SEQ ID NR. 24 bis 46 umfassen bzw. aus den Aminosäuresequenzen SEQ ID NR. 24 bis 46 bestehen,
wobei
• mindestens 4 Gene derart sind, dass
a. mindestens ein Gen zu einem ersten Satz A von 4 Genen gehört, welche die Nukleinsäuresequenzen SEQ ID NR. 1 bis 4 umfassen bzw. aus den Nukleinsäuresequenzen SEQ ID NR. 1 bis 4 bestehen,
b. mindestens ein Gen zu einem zweiten Satz B von 3 Genen gehört, welche die Nukleinsäuresequenzen SEQ ID NR. 5 bis 7 umfassen bzw. aus den Nukleinsäuresequenzen SEQ ID NR. 5 bis 7 bestehen,
c. mindestens ein Gen zu einem dritten Satz C von 6 Genen gehört, welche die Nukleinsäuresequenzen SEQ ID NR. 8 bis 13 umfassen bzw. aus den Nukleinsäuresequenzen SEQ ID NR. 8 bis 13 bestehen, und
d. mindestens ein Gen zu einem vierten Satz D von 9 Genen gehört, welche die Nukleinsäuresequenzen SEQ ID NR. 14 bis 23 umfassen bzw. aus den Nukleinsäuresequenzen SEQ ID NR. 14 bis 23 bestehen,
• mindestens 4 Proteine derart sind, dass
a. mindestens ein Protein zu einem ersten Satz AP von 4 Proteinen gehört, welche die Aminosäuresequenzen SEQ ID NR. 24 bis 27 umfassen bzw. aus den Aminosäuresequenzen SEQ ID NR. 24 bis 27 bestehen,
b. mindestens ein Protein zu einem zweiten Satz BP von 3 Proteinen gehört, welche die Aminosäuresequenzen SEQ ID NR. 28 bis 30 umfassen bzw. aus den Aminosäuresequenzen SEQ ID NR. 28 bis 30 bestehen,
c. mindestens ein Protein zu einem dritten Satz CP von 6 Proteinen gehört, welche die Aminosäuresequenzen SEQ ID NR. 31 bis 36 umfassen bzw. aus den Aminosäuresequenzen SEQ ID NR. 31 bis 36 bestehen,
d. mindestens ein Protein zu einem vierten Satz DP von 9 Proteinen gehört, welche die Aminosäuresequenzen SEQ ID NR. 37 bis 46 umfassen bzw. aus den Aminosäuresequenzen SEQ ID NR. 37 bis 46 bestehen,
für die Implementierung eines *In-vitro-*Prognoseverfahrens der Überlebensrate eines Patienten, der an Lungenkrebs erkrankt ist, wobei der Lungenkrebs vorzugsweise als Lungentumor klassifiziert ist, der zu der Gruppe gehört, die aus Plattenepithelzellkarzinom, Adenokarzinom, großzelligen neuroendokrinen Tumoren und basaloiden Tumoren besteht,
wobei
- die Nukleinsäuresequenzen SEQ ID NR. 1 bis 4 und/oder die Aminosäuresequenzen SEQ ID NR. 24 bis 27 in basaloiden Tumoren exprimiert werden,
- die Nukleinsäuresequenzen SEQ ID NR. 5 bis 7 und/oder die Aminosäuresequenzen SEQ ID NR. 28 bis 30 in einem Adenokarzinom exprimiert werden,
- die Nukleinsäuresequenzen SEQ ID NR. 8 bis 13 und/oder die Aminosäuresequenzen SEQ ID NR. 31 bis 36 in einem Plattenepithelzellkarzinom exprimiert werden,
- die Nukleinsäuresequenzen SEQ ID NR. 14 bis 23 und/oder die Aminosäuresequenzen SEQ ID NR. 37 bis 46 in großzelligen neuroendokrinen Tumoren exprimiert werden,
wobei die Prognose derart ist, dass:
entweder
+ während eines Zeitraums von 30 Monaten nach der Diagnose des Lungenkrebses
• wenn es sich bei dem Tumor um ein Adenokarzinom handelt:
∘ wenn keines der 23 Gene bzw. der 23 Proteine des Satzes exprimiert wird, die Überlebensrate des Patienten ungefähr 86 % beträgt,
∘ wenn mindestens eines der Gene bzw. der Proteine des Satzes B exprimiert wird, die Überlebensrate des Patienten ungefähr 63 % beträgt,
• wenn es sich bei dem Tumor um einen basaloiden Tumor handelt:
∘ wenn keines der 23 Gene bzw. der 23 Proteine des Satzes exprimiert wird, die Überlebensrate des Patienten ungefähr 65 % beträgt,
∘ wenn mindestens eines der Gene bzw. der Proteine des Satzes A exprimiert wird, die Überlebensrate des Patienten ungefähr 33 % beträgt,
• wenn es sich bei dem Tumor um ein Plattenepithelzellkarzinom handelt:
∘ wenn keines der 23 Gene bzw. der 23 Proteine des Satzes exprimiert wird, die Überlebensrate des Patienten ungefähr 88 % beträgt,
o wenn mindestens eines der Gene bzw. der Proteine des Satzes C exprimiert wird, die Überlebensrate des Patienten ungefähr 64 % beträgt,
• wenn es sich bei dem Tumor um einen großzelligen neuroendokrinen Tumor handelt:
o wenn keines der 23 Gene bzw. der 23 Proteine des Satzes exprimiert wird, die Überlebensrate des Patienten ungefähr 65 % beträgt,
∘ wenn mindestens eines der Gene bzw. der Proteine des Satzes D exprimiert wird, die Überlebensrate des Patienten ungefähr 7 % beträgt,
oder
+ während eines Zeitraums von 60 Monaten nach der Diagnose des Lungenkrebses
• wenn es sich bei dem Tumor um ein Adenokarzinom handelt:
∘ wenn keines der 23 Gene bzw. der 23 Proteine des Satzes exprimiert wird, die Überlebensrate des Patienten ungefähr 82 % beträgt,
∘ wenn mindestens eines der Gene bzw. der Proteine des Satzes B exprimiert wird, die Überlebensrate des Patienten ungefähr 46 % beträgt,
• wenn es sich bei dem Tumor um einen basaloiden Tumor handelt:
∘ wenn keines der 23 Gene bzw. der 23 Proteine des Satzes exprimiert wird, die Überlebensrate des Patienten ungefähr 35 % beträgt,
∘ wenn mindestens eines der Gene bzw. der Proteine des Satzes A exprimiert wird, die Überlebensrate des Patienten ungefähr 7 % beträgt,
• wenn es sich bei dem Tumor um ein Plattenepithelzellkarzinom handelt:
∘ wenn keines der 23 Gene bzw. der 23 Proteine des Satzes exprimiert wird, die Überlebensrate des Patienten ungefähr 77 % beträgt,
∘ wenn mindestens eines der Gene bzw. der Proteine des Satzes C exprimiert wird, die Überlebensrate des Patienten ungefähr 42 % beträgt,
• wenn es sich bei dem Tumor um einen großzelligen neuroendokrinen Tumor handelt:
o wenn keines der 23 Gene bzw. der 23 Proteine des Satzes exprimiert wird, die Überlebensrate des Patienten ungefähr 55 % beträgt,
∘ wenn mindestens eines der Gene bzw. der Proteine des Satzes D exprimiert wird, die Überlebensrate des Patienten ungefähr 4 % beträgt,
oder
+ während eines Zeitraums von 120 Monaten nach der Diagnose des Lungenkrebses
• wenn es sich bei dem Tumor um ein Adenokarzinom handelt:
∘ wenn keines der 23 Gene bzw. der 23 Proteine des Satzes exprimiert wird, die Überlebensrate des Patienten ungefähr 76 % beträgt,
∘ wenn mindestens eines der Gene bzw. der Proteine des Satzes B exprimiert wird, die Überlebensrate des Patienten ungefähr 39 % beträgt,
• wenn es sich bei dem Tumor um einen basaloiden Tumor handelt:
∘ wenn keines der 23 Gene bzw. der 23 Proteine des Satzes exprimiert wird, die Überlebensrate des Patienten ungefähr 19 % beträgt,
∘ wenn mindestens eines der Gene bzw. der Proteine des Satzes A exprimiert wird, die Überlebensrate des Patienten ungefähr 0 % beträgt,
• wenn es sich bei dem Tumor um ein Plattenepithelzellkarzinom handelt:
∘ wenn keines der 23 Gene bzw. der 23 Proteine des Satzes exprimiert wird, die Überlebensrate des Patienten ungefähr 58 % beträgt,
∘ wenn mindestens eines der Gene bzw. der Proteine des Satzes C exprimiert wird, die Überlebensrate des Patienten ungefähr 33 % beträgt,
• wenn es sich bei dem Tumor um einen großzelligen neuroendokrinen Tumor handelt:
∘ wenn keines der 23 Gene bzw. der 23 Proteine des Satzes exprimiert wird, die Überlebensrate des Patienten ungefähr 40 % beträgt,
∘ wenn mindestens eines der Gene bzw. der Proteine des Satzes D exprimiert wird, die Überlebensrate des Patienten ungefähr 0 % beträgt,
wobei das Gen bzw. Protein als exprimiert werdend bestimmt wird, wenn:
- es entweder in einer Probe eines Patienten exprimiert wird, der an einem Lungenkrebs erkrankt ist, aber nicht in einer Kontrollprobe einer gesunden Person,
- oder wenn es über einem Schwellenwert exprimiert ist, der einem Hintergrundsignal entspricht, das in einer Reihe von gesunden Vergleichsgeweben für jedes Detektionsverfahren beobachtet wird, und
wobei das Gen bzw. Protein als nicht exprimiert werdend bestimmt wird, wenn:
- es weder in einer Probe eines Patienten exprimiert wird, der an einem Lungenkrebs erkrankt ist, noch in einer Kontrollprobe einer gesunden Person,
- oder wenn es in einer Probe eines Patienten, der an einem Lungenkrebs erkrankt ist, auf einem Niveau exprimiert wird, das im Wesentlichen gleich oder niedriger ist als das Niveau in einer Kontrollprobe einer gesunden Person.

2. Verwendung nach Anspruch 1, wobei
• die mindestens 4 Gene derart sind, dass
a. mindestens ein Gen zu einem ersten Satz A von 4 Genen gehört, welche die Nukleinsäuresequenzen SEQ ID NR. 1 bis 4 umfassen bzw. aus den Nukleinsäuresequenzen SEQ ID NR. 1 bis 4 bestehen,
b. mindestens ein Gen zu einem zweiten Satz B von 3 Genen gehört, welche die Nukleinsäuresequenzen SEQ ID NR. 5 bis 7 umfassen bzw. aus den Nukleinsäuresequenzen SEQ ID NR. 5 bis 7 bestehen,
c. mindestens ein Gen zu einem dritten Satz C von 6 Genen gehört, welche die Nukleinsäuresequenzen SEQ ID NR. 8 bis 13 umfassen bzw. aus den Nukleinsäuresequenzen SEQ ID NR. 8 bis 13 bestehen,
d. mindestens ein Gen zu einem vierten Satz D von 9 Genen gehört, welche die Nukleinsäuresequenzen SEQ ID NR. 14 bis 23 umfassen bzw. aus den Nukleinsäuresequenzen SEQ ID NR. 14 bis 23 bestehen, aus den Nukleinsäuren SEQ ID NR. 14 oder SEQ ID NR. 16 bis 22 ausgewählt ist,
• die mindestens 4 Proteine derart sind, dass
a. mindestens ein Protein zu einem ersten Satz AP von 4 Proteinen gehört, welche die Aminosäuresequenzen SEQ ID NR. 24 bis 27 umfassen bzw. aus den Aminosäuresequenzen SEQ ID NR. 24 bis 27 bestehen,
b. mindestens ein Protein zu einem zweiten Satz BP von 3 Proteinen gehört, welche die Aminosäuresequenzen SEQ ID NR. 28 bis 30 umfassen bzw. aus den Aminosäuresequenzen SEQ ID NR. 28 bis 30 bestehen,
c. mindestens ein Protein zu einem dritten Satz CP von 6 Proteinen gehört, welche die Aminosäuresequenzen SEQ ID NR. 31 bis 36 umfassen bzw. aus den Aminosäuresequenzen SEQ ID NR. 31 bis 36 bestehen,
d. mindestens ein Protein zu einem vierten Satz DP von 9 Proteinen gehört, welche die Aminosäuresequenzen SEQ ID NR. 37 bis 46 umfassen bzw. aus den Aminosäuresequenzen SEQ ID NR. 37 bis 46 bestehen, aus den Aminosäuresequenzen SEQ ID NR. 37 oder SEQ ID NR. 39 bis 45 ausgewählt ist.

3. Verwendung nach Anspruch 1 oder 2, wobei
a. das mindestens eine Gen zu einem ersten Satz A von 4 Genen gehört, welche die Nukleinsäuresequenzen SEQ ID NR. 1 bis 4 umfassen bzw. aus den Nukleinsäuresequenzen SEQ ID NR. 1 bis 4 bestehen, aus der Nukleinsäuresequenz SEQ ID NR. 1 bis 3 ausgewählt ist,
b. mindestens ein Gen zu einem zweiten Satz B von 3 Genen gehört, welche die Nukleinsäuresequenzen SEQ ID NR. 5 bis 7 umfassen bzw. aus den Nukleinsäuresequenzen SEQ ID NR. 5 bis 7 bestehen, aus der Nukleinsäuresequenz SEQ ID NR. 5 oder 6 ausgewählt ist,
c. mindestens ein Gen zu einem dritten Satz C von 6 Genen gehört, welche die Nukleinsäuresequenzen SEQ ID NR. 8 bis 13 umfassen bzw. aus den Nukleinsäuresequenzen SEQ ID NR. 8 bis 13 bestehen, aus der Nukleinsäuresequenz SEQ ID NR. 8 bis 11 ausgewählt ist und
d. mindestens ein Gen zu einem vierten Satz D von 9 Genen gehört, welche die Nukleinsäuresequenzen SEQ ID NR. 14 bis 23 umfassen bzw. aus den Nukleinsäuresequenzen SEQ ID NR. 14 bis 23 bestehen, aus der Nukleinsäuresequenz SEQ ID NR. 16 bis 20 ausgewählt ist.

4. Verwendung nach einem der Ansprüche 1 bis 3, wobei
+ während eines Zeitraums von 30 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 88 % beträgt,
∘ wenn eines der Gene des Satzes C exprimiert wird, die Überlebensrate des Patienten ungefähr 73 % beträgt,
∘ wenn mindestens zwei der Gene des Satzes C exprimiert werden, die Überlebensrate des Patienten ungefähr 50 % beträgt,
+ während eines Zeitraums von 60 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 77 % beträgt,
∘ wenn eines der Gene des Satzes C exprimiert wird, die Überlebensrate des Patienten ungefähr 59 % beträgt,
∘ wenn mindestens zwei der Gene des Satzes C exprimiert werden, die Überlebensrate des Patienten ungefähr 14 % beträgt,
+ während eines Zeitraums von 120 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 58 % beträgt,
∘ wenn eines der Gene des Satzes C exprimiert wird, die Überlebensrate des Patienten ungefähr 45 % beträgt,
∘ wenn mindestens zwei der Gene des Satzes C exprimiert werden, die Überlebensrate des Patienten ungefähr 14 % beträgt.

5. In-vitro-Prognoseverfahren der Überlebensrate eines Patienten, der an einem Lungentumor erkrankt ist, ausgehend von einer biologischen Probe, welche den Lungentumor enthält, der zu der Gruppe gehört, die aus Adenokarzinom, Plattenepithelzellkarzinom, basaloiden Tumoren und großzelligen neuroendokrinen Tumoren besteht, zu einem Zeitpunkt von 30 bis 120 Monaten nach der Diagnose des Lungenkrebses,
wobei das Verfahren einen Schritt des Messens in der biologischen Probe der Expression von
- mindestens 4 Genen, ausgewählt aus einer Gruppe von 23 Genen, welche die Nukleinsäuresequenzen SEQ ID NR. 1 bis 23 umfassen bzw. aus den Nukleinsäuresequenzen SEQ ID NR. 1 bis 23 bestehen,
- oder komplementären Sequenzen der Gene,
umfasst, wobei die mindestens 4 Gene derart sind, dass
a. mindestens ein Gen zu einem ersten Satz A von 4 Genen gehört, welche die Nukleinsäuresequenzen SEQ ID NR. 1 bis 4 umfassen bzw. aus den Nukleinsäuresequenzen SEQ ID NR. 1 bis 4 bestehen,
b. mindestens ein Gen zu einem zweiten Satz B von 3 Genen gehört, welche die Nukleinsäuresequenzen SEQ ID NR. 5 bis 7 umfassen bzw. aus den Nukleinsäuresequenzen SEQ ID NR. 5 bis 7 bestehen,
c. mindestens ein Gen zu einem dritten Satz C von 6 Genen gehört, welche die Nukleinsäuresequenzen SEQ ID NR. 8 bis 13 umfassen bzw. aus den Nukleinsäuresequenzen SEQ ID NR. 8 bis 13 bestehen, und
d. mindestens ein Gen zu einem vierten Satz D von 9 Genen gehört, welche die Nukleinsäuresequenzen SEQ ID NR. 14 bis 23 umfassen bzw. aus den Nukleinsäuresequenzen SEQ ID NR. 14 bis 23 bestehen,
wobei
- die Nukleinsäuresequenzen SEQ ID NR. 1 bis 4 in basaloiden Tumoren exprimiert werden,
- die Nukleinsäuresequenzen SEQ ID NR. 5 bis 7 in einem Adenokarzinom exprimiert werden,
- die Nukleinsäuresequenzen SEQ ID NR. 8 bis 13 in einem Plattenepithelzellkarzinom exprimiert werden,
- die Nukleinsäuresequenzen SEQ ID NR. 14 bis 23 in großzelligen neuroendokrinen Tumoren exprimiert werden,
wobei die Prognose derart ist, dass:
+ während eines Zeitraums von 30 Monaten nach der Diagnose des Lungenkrebses
• wenn es sich bei dem Tumor um ein Adenokarzinom handelt:
∘ wenn keines der 23 Gene des Satzes exprimiert wird, die Überlebensrate des Patienten ungefähr 86 % beträgt,
∘ wenn mindestens eines der Gene des Satzes B exprimiert wird, die Überlebensrate des Patienten ungefähr 63 % beträgt,
• wenn es sich bei dem Tumor um einen basaloiden Tumor handelt:
∘ wenn keines der 23 Gene des Satzes exprimiert wird, die Überlebensrate des Patienten ungefähr 65 % beträgt,
∘ wenn mindestens eines der Gene des Satzes A exprimiert wird, die Überlebensrate des Patienten ungefähr 33 % beträgt,
• wenn es sich bei dem Tumor um ein Plattenepithelzellkarzinom handelt:
∘ wenn keines der 23 Gene des Satzes exprimiert wird, die Überlebensrate des Patienten ungefähr 88 % beträgt,
∘ wenn mindestens eines der Gene des Satzes C exprimiert wird, die Überlebensrate des Patienten ungefähr 64 % beträgt,
• wenn es sich bei dem Tumor um einen großzelligen neuroendokrinen Tumor handelt:
∘ wenn keines der 23 Gene des Satzes exprimiert wird, die Überlebensrate des Patienten ungefähr 65 % beträgt,
∘ wenn mindestens eines der Gene des Satzes D exprimiert wird, die Überlebensrate des Patienten ungefähr 7 % beträgt,
oder
+ während eines Zeitraums von 60 Monaten nach der Diagnose des Lungenkrebses
• wenn es sich bei dem Tumor um ein Adenokarzinom handelt:
o wenn keines der 23 Gene des Satzes exprimiert wird, die Überlebensrate des Patienten ungefähr 82 % beträgt,
∘ wenn mindestens eines der Gene des Satzes B exprimiert wird, die Überlebensrate des Patienten ungefähr 46 % beträgt,
• wenn es sich bei dem Tumor um einen basaloiden Tumor handelt:
∘ wenn keines der 23 Gene des Satzes exprimiert wird, die Überlebensrate des Patienten ungefähr 35 % beträgt,
∘ wenn mindestens eines der Gene des Satzes A exprimiert wird, die Überlebensrate des Patienten ungefähr-7 % beträgt,
• wenn es sich bei dem Tumor um ein Plattenepithelzellkarzinom handelt:
∘ wenn keines der 23 Gene des Satzes exprimiert wird, die Überlebensrate des Patienten ungefähr 77 % beträgt,
∘ wenn mindestens eines der Gene des Satzes C exprimiert wird, die Überlebensrate des Patienten ungefähr 42 % beträgt,
• wenn es sich bei dem Tumor um einen großzelligen neuroendokrinen Tumor handelt:
∘ wenn keines der 23 Gene des Satzes exprimiert wird, die Überlebensrate des Patienten ungefähr 55 % beträgt,
o wenn mindestens eines der Gene des Satzes D exprimiert wird, die Überlebensrate des Patienten ungefähr 4 % beträgt,
oder
+ während eines Zeitraums von 120 Monaten nach der Diagnose des Lungenkrebses
• wenn es sich bei dem Tumor um ein Adenokarzinom handelt:
∘ wenn keines der 23 Gene des Satzes exprimiert wird, die Überlebensrate des Patienten ungefähr 76 % beträgt,
∘ wenn mindestens eines der Gene des Satzes B exprimiert wird, die Überlebensrate des Patienten ungefähr 39 % beträgt,
• wenn es sich bei dem Tumor um einen basaloiden Tumor handelt:
o wenn keines der 23 Gene des Satzes exprimiert wird, die Überlebensrate des Patienten ungefähr 19 % beträgt,
∘ wenn mindestens eines der Gene des Satzes A exprimiert wird, die Überlebensrate des Patienten ungefähr 0 % beträgt,
• wenn es sich bei dem Tumor um ein Plattenepithelzellkarzinom handelt:
∘ wenn keines der 23 Gene des Satzes exprimiert wird, die Überlebensrate des Patienten ungefähr 58 % beträgt,
∘ wenn mindestens eines der Gene des Satzes C exprimiert wird, die Überlebensrate des Patienten ungefähr 33 % beträgt,
• wenn es sich bei dem Tumor um einen großzelligen neuroendokrinen Tumor handelt:
∘ wenn keines der 23 Gene des Satzes exprimiert wird, die Überlebensrate des Patienten ungefähr 40 % beträgt,
∘ wenn mindestens eines der Gene des Satzes D exprimiert wird, die Überlebensrate des Patienten ungefähr 0 % beträgt.

6. Prognoseverfahren nach Anspruch 5, wobei
a. das mindestens eine Gen zu einem ersten Satz A von 4 Genen gehört, welche die Nukleinsäuresequenzen SEQ ID NR. 1 bis 4 umfassen bzw. aus den Nukleinsäuresequenzen SEQ ID NR. 1 bis 4 bestehen, aus der Nukleinsäuresequenz SEQ ID NR. 1 bis 3 ausgewählt ist,
b. mindestens ein Gen zu einem zweiten Satz B von 3 Genen gehört, welche die Nukleinsäuresequenzen SEQ ID NR. 5 bis 7 umfassen bzw. aus den Nukleinsäuresequenzen SEQ ID NR. 5 bis 7 bestehen, aus der Nukleinsäuresequenz SEQ ID NR. 5 oder 6 ausgewählt ist,
c. mindestens ein Gen zu einem dritten Satz C von 6 Genen gehört, welche die Nukleinsäuresequenzen SEQ ID NR. 8 bis 13 umfassen bzw. aus den Nukleinsäuresequenzen SEQ ID NR. 8 bis 13 bestehen, aus der Nukleinsäuresequenz SEQ ID NR. 8 bis 11 ausgewählt ist und
d. mindestens ein Gen zu einem vierten Satz D von 9 Genen gehört, welche die Nukleinsäuresequenzen SEQ ID NR. 14 bis 23 umfassen bzw. aus den Nukleinsäuresequenzen SEQ ID NR. 14 bis 23 bestehen, aus der Nukleinsäuresequenz SEQ ID NR. 16 bis 20 ausgewählt ist.

7. Prognoseverfahren nach Anspruch 5 oder 6, wobei
+ während eines Zeitraums von 30 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 88 % beträgt,
∘ wenn eines der Gene des Satzes C exprimiert wird, die Überlebensrate des Patienten ungefähr 73 % beträgt,
∘ wenn mindestens zwei der Gene des Satzes C exprimiert werden, die Überlebensrate des Patienten ungefähr 50 % beträgt,
+ während eines Zeitraums von 60 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 77 % beträgt,
∘ wenn eines der Gene des Satzes C exprimiert wird, die Überlebensrate des Patienten ungefähr 59 % beträgt,
∘ wenn mindestens zwei der Gene des Satzes C exprimiert werden, die Überlebensrate des Patienten ungefähr 14 % beträgt,
+ während eines Zeitraums von 120 Monaten nach der Diagnose des Lungenkrebses
• wenn es sich bei dem Tumor um ein Plattenepithelzellkarzinom handelt:
∘ wenn keines der 23 Gene des Satzes exprimiert wird, die Überlebensrate des Patienten ungefähr 58 % beträgt,
∘ wenn eines der Gene des Satzes C exprimiert wird, die Überlebensrate des Patienten ungefähr 45 % beträgt,
∘ wenn mindestens zwei der Gene des Satzes C exprimiert werden, die Überlebensrate des Patienten ungefähr 14 % beträgt.

8. Prognoseverfahren nach einem der Ansprüche 5 bis 7, wobei der Schritt des Messens unter Zuhilfenahme einer Technik ausgeführt wird, die aus dem Satz ausgewählt ist, der besteht aus:
a. Quantitativer PCR
b. DNA-CHIP und
c. Northern-Blot.

9. Prognoseverfahren nach einem der Ansprüche 5 bis 8, wobei der Schritt des Messens durch Verwendung von Nukleinsäuremolekülen ausgeführt wird, die aus 15 bis 100 Nukleotidmolekülen bestehen, die zu den mindestens 4 Genen komplementär sind.

10. *In*-*vitro*-Prognoseverfahren der Überlebensrate eines Patienten, der an einem Lungentumor erkrankt ist, ausgehend von einer biologischen Probe, welche den Lungentumor enthält, zu einem Zeitpunkt von 30 bis 120 Monaten nach der Diagnose des Lungenkrebses,
wobei das Verfahren einen Schritt des Messens in der biologischen Probe der Expression von
- mindestens 4 Proteinen, ausgewählt aus einem Satz von 23 Proteinen, die von 23 Genen kodiert werden, welche die Nukleinsäuresequenzen SEQ ID NR. 1 bis 23 umfassen bzw. aus den Nukleinsäuresequenzen SEQ ID NR. 1 bis 23 bestehen,
umfasst, wobei die mindestens 4 Proteine derart sind, dass
a. mindestens ein Protein zu einem ersten Satz AP von 4 Proteinen gehört, welche die Aminosäuresequenzen SEQ ID NR. 24 bis 27 umfassen bzw. aus den Aminosäuresequenzen SEQ ID NR. 24 bis 27 bestehen,
b. mindestens ein Protein zu einem zweiten Satz BP von 3 Proteinen gehört, welche die Aminosäuresequenzen SEQ ID NR. 28 bis 30 umfassen bzw. aus den Aminosäuresequenzen SEQ ID NR. 28 bis 30 bestehen,
c. mindestens ein Protein zu einem dritten Satz CP von 6 Proteinen gehört, welche die Aminosäuresequenzen SEQ ID NR. 31 bis 36 umfassen bzw. aus den Aminosäuresequenzen SEQ ID NR. 31 bis 36 bestehen,
d. mindestens ein Protein zu einem vierten Satz DP von 9 Proteinen gehört, welche die Aminosäuresequenzen SEQ ID NR. 37 bis 46 umfassen bzw. aus den Aminosäuresequenzen SEQ ID NR. 37 bis 46 bestehen,
wobei
- die Aminosäuresequenzen SEQ ID NR. 24 bis 27 in basaloiden Tumoren exprimiert werden,
- die Aminosäuresequenzen SEQ ID NR. 28 bis 30 in einem Adenokarzinom exprimiert werden,
- die Aminosäuresequenzen SEQ ID NR. 31 bis 36 in einem Plattenepithelzellkarzinom exprimiert werden,
- die Aminosäuresequenzen SEQ ID NR. 37 bis 46 in großzelligen neuroendokrinen Tumoren exprimiert werden,
wobei das Prognoseverfahren derart ist, dass
+ während eines Zeitraums von 30 Monaten nach der Diagnose des Lungenkrebses
• wenn es sich bei dem Tumor um ein Adenokarzinom handelt:
∘ wenn keines der 23 Proteine des Satzes exprimiert wird, die Überlebensrate des Patienten ungefähr 86 % beträgt,
∘ wenn mindestens eines der Proteine des Satzes BP exprimiert wird, die Überlebensrate des Patienten ungefähr 63 % beträgt,
• wenn es sich bei dem Tumor um einen basaloiden Tumor handelt:
∘ wenn keines der 23 Proteine des Satzes exprimiert wird, die Überlebensrate des Patienten ungefähr 65 % beträgt,
∘ wenn mindestens eines der Proteine des Satzes AP exprimiert wird, die Überlebensrate des Patienten ungefähr 33 % beträgt,
• wenn es sich bei dem Tumor um ein Plattenepithelzellkarzinom handelt:
∘ wenn keines der 23 Proteine des Satzes exprimiert wird, die Überlebensrate des Patienten ungefähr 88 % beträgt,
∘ wenn mindestens eines der Proteine des Satzes CP exprimiert wird, die Überlebensrate des Patienten ungefähr 64 % beträgt,
• wenn es sich bei dem Tumor um einen großzelligen neuroendokrinen Tumor handelt:
∘ wenn keines der 23 Proteine des Satzes exprimiert wird, die Überlebensrate des Patienten ungefähr 65 % beträgt,
∘ wenn mindestens eines der Proteine des Satzes DP exprimiert wird, die Überlebensrate des Patienten ungefähr 7 % beträgt,
oder
+ während eines Zeitraums von 60 Monaten nach der Diagnose des Lungenkrebses
• wenn es sich bei dem Tumor um ein Adenokarzinom handelt:
∘ wenn keines der 23 Proteine des Satzes exprimiert wird, die Überlebensrate des Patienten ungefähr 82 % beträgt,
∘ wenn mindestens eines der Proteine des Satzes BP exprimiert wird, die Überlebensrate des Patienten ungefähr 46 % beträgt,
• wenn es sich bei dem Tumor um einen basaloiden Tumor handelt:
∘ wenn keines der 23 Proteine des Satzes exprimiert wird, die Überlebensrate des Patienten ungefähr 35 % beträgt,
o wenn mindestens eines der Proteine des Satzes AP exprimiert wird, die Überlebensrate des Patienten ungefähr 7 % beträgt,
• wenn es sich bei dem Tumor um ein Plattenepithelzellkarzinom handelt:
∘ wenn keines der 23 Proteine des Satzes exprimiert wird, die Überlebensrate des Patienten ungefähr 77 % beträgt,
∘ wenn mindestens eines der Proteine des Satzes CP exprimiert wird, die Überlebensrate des Patienten ungefähr 42 % beträgt,
• wenn es sich bei dem Tumor um einen großzelligen neuroendokrinen Tumor handelt:
∘ wenn keines der 23 Proteine des Satzes exprimiert wird, die Überlebensrate des Patienten ungefähr 55 % beträgt,
∘ wenn mindestens eines der Proteine des Satzes DP exprimiert wird, die Überlebensrate des Patienten ungefähr 4 % beträgt,
oder
+ während eines Zeitraums von 120 Monaten nach der Diagnose des Lungenkrebses
• wenn es sich bei dem Tumor um ein Adenokarzinom handelt:
∘ wenn keines der 23 Proteine des Satzes exprimiert wird, die Überlebensrate des Patienten ungefähr 76 % beträgt,
∘ wenn mindestens eines der Proteine des Satzes BP exprimiert wird, die Überlebensrate des Patienten ungefähr 39 % beträgt,
• wenn es sich bei dem Tumor um einen basaloiden Tumor handelt:
∘ wenn keines der 23 Proteine des Satzes exprimiert wird, die Überlebensrate des Patienten ungefähr 19 % beträgt,
∘ wenn mindestens eines der Proteine des Satzes AP exprimiert wird, die Überlebensrate des Patienten ungefähr 0 % beträgt,
• wenn es sich bei dem Tumor um ein Plattenepithelzellkarzinom handelt:
∘ wenn keines der 23 Proteine des Satzes exprimiert wird, die Überlebensrate des Patienten ungefähr 58 % beträgt,
∘ wenn mindestens eines der Proteine des Satzes CP exprimiert wird, die Überlebensrate des Patienten ungefähr 33 % beträgt,
• wenn es sich bei dem Tumor um einen großzelligen neuroendokrinen Tumor handelt:
∘ wenn keines der 23 Proteine des Satzes exprimiert wird, die Überlebensrate des Patienten ungefähr 40 % beträgt,
∘ wenn mindestens eines der Proteine des Satzes DP exprimiert wird, die Überlebensrate des Patienten ungefähr 0 % beträgt.

11. Prognoseverfahren nach Anspruch 10, wobei
e. das mindestens eine Protein zu einem ersten Satz AP von 4 Proteinen gehört, welche die Aminosäuresequenzen SEQ ID NR. 24 bis 27 umfassen bzw. aus den Aminosäuresequenzen SEQ ID NR. 24 bis 27 bestehen, aus der Aminosäuresequenz SEQ ID NR. 24 bis 27 ausgewählt ist,
f. mindestens ein Protein zu einem zweiten Satz BP von 3 Proteinen gehört, welche die Aminosäuresequenzen SEQ ID NR. 28 bis 30 umfassen bzw. aus den Aminosäuresequenzen SEQ ID NR. 28 bis 30 bestehen, aus der Aminosäuresequenz SEQ ID NR. 28 oder 29 ausgewählt ist,
g. mindestens ein Protein zu einem dritten Satz CP von 6 Proteinen gehört, welche die Aminosäuresequenzen SEQ ID NR. 31 bis 36 umfassen bzw. aus den Aminosäuresequenzen SEQ ID NR. 31 bis 36 bestehen, aus der Aminosäuresequenz SEQ ID NR. 31 bis 34 ausgewählt ist und
h. mindestens ein Protein zu einem vierten Satz DP von 9 Proteinen gehört, welche die Aminosäuresequenzen SEQ ID NR. 37 bis 46 umfassen bzw. aus den Aminosäuresequenzen SEQ ID NR. 37 bis 46 bestehen, aus der Aminosäuresequenz SEQ ID NR. 37 bis 43 ausgewählt ist.

12. Prognoseverfahren nach Anspruch 10 oder 11, wobei
+ während eines Zeitraums von 30 Monaten nach der Diagnose des Lungenkrebses
• wenn es sich bei dem Tumor um ein Plattenepithelzellkarzinom handelt:
∘ wenn keines der 23 Proteine des Satzes exprimiert wird, die Überlebensrate des Patienten ungefähr 88 % beträgt,
∘ wenn eines der Proteine des Satzes CP exprimiert wird, die Überlebensrate des Patienten ungefähr 73 % beträgt,
∘ wenn mindestens zwei der Proteine des Satzes CP exprimiert werden, die Überlebensrate des Patienten ungefähr 50 % beträgt,
+ während eines Zeitraums von 60 Monaten nach der Diagnose des Lungenkrebses
• wenn es sich bei dem Tumor um ein Plattenepithelzellkarzinom handelt:
o wenn keines der 23 Proteine des Satzes exprimiert wird, die Überlebensrate des Patienten ungefähr 77 % beträgt,
∘ wenn eines der Proteine des Satzes CP exprimiert wird, die Überlebensrate des Patienten ungefähr 59 % beträgt,
∘ wenn mindestens zwei der Proteine des Satzes CP exprimiert werden, die Überlebensrate des Patienten ungefähr 14 % beträgt,
+ während eines Zeitraums von 120 Monaten nach der Diagnose des Lungenkrebses
• wenn es sich bei dem Tumor um ein Plattenepithelzellkarzinom handelt:
∘ wenn keines der 23 Proteine des Satzes exprimiert wird, die Überlebensrate des Patienten ungefähr 58 % beträgt,
∘ wenn eines der Proteine des Satzes CP exprimiert wird, die Überlebensrate des Patienten ungefähr 45 % beträgt,
∘ wenn mindestens zwei der Proteine des Satzes CP exprimiert werden, die Überlebensrate des Patienten ungefähr 14 % beträgt.

13. Prognoseverfahren nach einem der Ansprüche 10 bis 12, wobei der Schritt des Messens unter Zuhilfenahme einer Technik ausgeführt wird, die aus dem Satz ausgewählt ist, der aus Folgenden besteht:
a. Western-Blot
b. ELISA
c. Immunfluoreszenz und
d. Immunhistochemie.

14. Prognoseverfahren nach einem der Ansprüche 10 bis 13, wobei der Schritt des Messens unter Zuhilfenahme von Antikörpern ausgeführt wird, die gegen die mindestens 2 Proteine gerichtet sind, die von den mindestens zwei Genen kodiert werden.

15. Prognoseverfahren nach einem der Ansprüche 10 bis 14, ferner umfassend einen Schritt des Vergleichens der gemessenen Expression mit der Expression in mindestens einer Kontrollprobe.

## Revendications

1. Utilisation d'au moins un élément choisi parmi :
- au moins 4 gènes choisis parmi un ensemble de 23 gènes comprenant ou consistant en les séquences d'acide nucléique SEQ ID NO 1 à 23,
- ou séquences complémentaires desdits au moins 4 gènes choisis parmi un ensemble de 23 gènes comprenant ou consistant en les séquences d'acide nucléique SEQ ID NO 1 à 23,
- ou séquences ayant au moins 80% d'homologie avec lesdits gènes,
- ou protéines codées par lesdits au moins 4 gènes choisis parmi un ensemble de 23 gènes comprenant ou consistant en les séquences d'acide nucléique SEQ ID NO 1 à 23, lesdites protéines comprenant ou consistant en des séquences d'acide aminé SEQ ID NO 24 à 46,
lesdits
❖ au moins 4 gènes étant tels que
a. au moins un gène appartient à un premier ensemble A de 4 gènes comprenant ou consistant en les séquences d'acide nucléique SEQ ID NO : 1 à 4,
b. au moins un gène appartient à un second ensemble B de 3 gènes comprenant ou consistant en les séquences d'acide nucléique SEQ ID NO : 5 à 7,
c. au moins un gène appartient à un troisième ensemble C de 6 gènes comprenant ou consistant en les séquences d'acide nucléique SEQ ID NO : 8 à 13, et
d. au moins un gène appartient à un quatrième ensemble D de 9 gènes comprenant ou consistant en les séquences d'acide nucléique SEQ ID NO : 14 à 23,
❖ au moins 4 protéines étant telles que
a. au moins une protéine appartient à un premier ensemble AP de 4 protéines comprenant ou consistant en les séquences d'acide aminé SEQ ID NO : 24 -27,
b. au moins une protéine appartient à un second ensemble BP de 3 protéines comprenant ou consistant en les séquences d'acide aminé SEQ ID NO : 28 -30, et
c. au moins une protéine appartient à un troisième ensemble CP de 6 protéines comprenant ou consistant en les séquences d'acide aminé SEQ ID NO : 31 -36,
d. au moins une protéine appartient à un quatrième ensemble DP de 9 protéines comprenant ou consistant en les séquences d'acide aminé SEQ ID NO : 37 -46,
pour l'implémentation d'une méthode de pronostic *in vitro* du taux de survie d'un patient atteint d'un cancer du poumon, ledit cancer du poumon étant auparavant classé comme une tumeur du poumon appartenant au groupe consistant en le carcinome épidermoïde, l'adénocarcinome, les tumeurs neuroendocrines à grandes cellules et les tumeurs basaloïdes, dans laquelle
- les séquences d'acide nucléique SEQ ID NO : 1 à 4 et/ou les séquences d'acide aminé SEQ ID NO : 24 à 27 sont exprimées dans les tumeurs basaloïdes,
- les séquences d'acide nucléique SEQ ID NO : 5 à 7 et/ou les séquences d'acide aminé SEQ ID NO : 28 à 30 sont exprimées dans l'adénocarcinome,
- les séquences d'acide nucléique SEQ ID NO : 8 à 13 et/ou les séquences d'acide aminé SEQ ID NO : 31 à 36 sont exprimées dans le carcinome épidermoïde,
- les séquences d'acide nucléique SEQ ID NO : 14 à 23 et/ou les séquences d'acide aminé SEQ ID NO : 37 à 46 sont exprimées dans les tumeurs neuroendocrines à grandes cellules,
ledit pronostic étant tel que :
soit
+ pendant une période de temps de 30 mois après le diagnostic dudit cancer du poumon
▪quand ladite tumeur est l'adénocarcinome :
o si aucun des 23 gènes, ou des 23 protéines dudit ensemble n'est exprimé, le taux de survie du patient est d'environ 86%,
o si au moins un des gènes, ou des protéines de l'ensemble B est exprimé, le taux de survie du patient est d'environ 63%,
▪ quand ladite tumeur est les tumeurs basaloïdes :
o si aucun des 23 gènes, ou des 23 protéines dudit ensemble n'est exprimé, le taux de survie du patient est d'environ 65%,
o si au moins un des gènes, ou des protéines de l'ensemble A est exprimé, le taux de survie du patient est d'environ 33%,
▪ quand ladite tumeur est le carcinome épidermoïde :
o si aucun des 23 gènes, ou des 23 protéines dudit ensemble n'est exprimé, le taux de survie du patient est d'environ 88%,
o si au moins un des gènes, ou des protéines de l'ensemble C est exprimé, le taux de survie du patient est d'environ 64%,
▪ quand ladite tumeur est les tumeurs neuroendocrines à grandes cellules:
o si aucun des 23 gènes, ou des 23 protéines dudit ensemble n'est exprimé, le taux de survie du patient est d'environ 65%,
o si au moins un des gènes, ou des protéines de l'ensemble D est exprimé, le taux de survie du patient est d'environ 7%,
ou,
+ pendant une période de temps de 60 mois après le diagnostic dudit cancer du poumon
▪ quand ladite tumeur est l'adénocarcinome :
o si aucun des 23 gènes, ou des 23 protéines dudit ensemble n'est exprimé, le taux de survie du patient est d'environ 82%,
o si au moins un des gènes, ou des protéines de l'ensemble B est exprimé, le taux de survie du patient est d'environ 46%,
▪ quand ladite tumeur est les tumeurs basaloïdes :
o si aucun des 23 gènes, ou des 23 protéines dudit ensemble n'est exprimé, le taux de survie du patient est d'environ 35%,
o si au moins un des gènes, ou des protéines de l'ensemble A est exprimé, le taux de survie du patient est d'environ 7%,
▪ quand ladite tumeur est le carcinome épidermoïde :
o si aucun des 23 gènes, ou des 23 protéines dudit ensemble n'est exprimé, le taux de survie du patient est d'environ 77%,
o si au moins un des gènes, ou des protéines de l'ensemble C est exprimé, le taux de survie du patient est d'environ 42%,
▪ quand ladite tumeur est les tumeurs neuroendocrines à grandes cellules:
o si aucun des 23 gènes, ou des 23 protéines dudit ensemble n'est exprimé, le taux de survie du patient est d'environ 55%,
o si au moins un des gènes, ou des protéines de l'ensemble D est exprimé, le taux de survie du patient est d'environ 4%,
ou,
+ pendant une période de temps de 120 mois après le diagnostic dudit cancer du poumon
▪ quand ladite tumeur est l'adénocarcinome :
o si aucun des 23 gènes, ou des 23 protéines dudit ensemble n'est exprimé, le taux de survie du patient est d'environ 76%,
o si au moins un des gènes, ou des protéines de l'ensemble B est exprimé, le taux de survie du patient est d'environ 39%,
▪ quand ladite tumeur est les tumeurs basaloïdes :
o si aucun des 23 gènes, ou des 23 protéines dudit ensemble n'est exprimé, le taux de survie du patient est d'environ 19%,
o si au moins un des gènes, ou des protéines de l'ensemble A est exprimé, le taux de survie du patient est d'environ 0%,
▪ quand ladite tumeur est le carcinome épidermoïde :
o si aucun des 23 gènes, ou des 23 protéines dudit ensemble n'est exprimé, le taux de survie du patient est d'environ 58%,
o si au moins un des gènes, ou des protéines de l'ensemble C est exprimé, le taux de survie du patient est d'environ 33%,
▪ quand ladite tumeur est les tumeurs neuroendocrines à grandes cellules:
o si aucun des 23 gènes, ou des 23 protéines dudit ensemble n'est exprimé, le taux de survie du patient est d'environ 40%,
o si au moins un des gènes, ou des protéines de l'ensemble D est exprimé, le taux de survie du patient est d'environ 0%,
dans laquelle ledit gène ou ladite protéine est déterminé comme étant exprimé quand :
- soit il est exprimé dans un échantillon d'un patient atteint d'un cancer du poumon mais pas dans un échantillon contrôle d'un individu sain,
- soit il est exprimé au-dessus d'un seuil correspondant à un signal de fond observé chez une série de tissus sains de référence pour chaque méthode de détection, et
ledit gène ou ladite protéine est déterminé comme n'étant pas exprimé quand :
- il n'est ni exprimé dans un échantillon d'un patient atteint d'un cancer du poumon ni dans un échantillon contrôle d'un individu sain,
- ou il est exprimé dans un échantillon d'un patient atteint d'un cancer du poumon à un niveau substantiellement égal ou inférieur au niveau dans un échantillon contrôle d'un individu sain.

2. Utilisation selon la revendication 1, dans laquelle
❖ lesdits au moins 4 gènes sont tels que :
a. au moins un gène appartient à un premier ensemble A de 4 gènes comprenant ou consistant en les séquences d'acide nucléique SEQ ID NO : 1 à 4,
b. au moins un gène appartient à un second ensemble B de 3 gènes comprenant ou consistant en les séquences d'acide nucléique SEQ ID NO : 5 à 7,
c. au moins un gène appartient à un troisième ensemble C de 6 gènes comprenant ou consistant en les séquences d'acide nucléique SEQ ID NO : 8 à 13,
d. au moins un gène appartient à un quatrième ensemble D de 9 gènes comprenant ou consistant en les séquences d'acide nucléique SEQ ID NO : 14-23, est choisi parmi les séquences d'acide nucléique SEQ ID NO 14 ou SEQ ID NO : 16 à 22,
❖ lesdites au moins 4 protéines sont telles que
a. au moins une protéine appartient à un premier ensemble AP de 4 protéines comprenant ou consistant en les séquences d'acide aminé SEQ ID NO : 24 -27,
b. au moins une protéine appartient à un second ensemble BP de 3 protéines comprenant ou consistant en les séquences d'acide aminé SEQ ID NO : 28 -30, et
c. au moins une protéine appartient à un troisième ensemble CP de 6 protéines comprenant ou consistant en les séquences d'acide aminé SEQ ID NO : 31 -36,
d. au moins une protéine appartient à un quatrième ensemble DP de 9 protéines comprenant ou consistant en les séquences d'acide aminé SEQ ID NO : 37 -46, est choisie parmi les séquences d'acide aminé SEQ ID NO 37 ou SED ID NO 39-45.

3. Utilisation selon la revendication 1 ou 2, dans laquelle
a. ledit au moins un gène appartient à un premier ensemble A de 4 gènes comprenant ou consistant en les séquences d'acide nucléique SEQ ID NO : 1 à 4, est choisi parmi les séquences d'acide nucléique SEQ ID NO : 1 à 3,
b. au moins un gène appartient à un second ensemble B de 3 gènes comprenant ou consistant en les séquences d'acide nucléique SEQ ID NO : 5 à 7, est choisi parmi les séquences d'acide nucléique SEQ ID NO : 5 ou 6,
c. au moins un gène appartient à un troisième ensemble C de 6 gènes comprenant ou consistant en les séquences d'acide nucléique SEQ ID NO : 8 à 13, est choisi parmi les séquences d'acide nucléique SEQ ID NO : 8 à 11, et
d. au moins un gène appartient à un quatrième ensemble D de 9 gènes comprenant ou consistant en les séquences d'acide nucléique SEQ ID NO : 14-23, est choisi parmi les séquences d'acide nucléique SEQ ID NO : 16 à 20.

4. Utilisation selon l'une des revendications 1 à 3, dans laquelle
+ pendant une période de temps de 30 mois après le diagnostic dudit cancer du poumon
▪ quand ladite tumeur est le carcinome épidermoïde :
o si aucun des 23 gènes dudit ensemble n'est exprimé, le taux de survie du patient est d'environ 88%
o si un des gènes de l'ensemble C est exprimé, le taux de survie du patient est d'environ 73%,
o si au moins deux des gènes de l'ensemble C sont exprimés, le taux de survie du patient est d'environ 50%.
+ pendant une période de temps de 60 mois après le diagnostic dudit cancer du poumon
▪ quand ladite tumeur est le carcinome épidermoïde :
o si aucun des 23 gènes dudit ensemble n'est exprimé, le taux de survie du patient est d'environ 77%
o si un des gènes de l'ensemble C est exprimé, le taux de survie du patient est d'environ 59%,
o si au moins deux des gènes de l'ensemble C sont exprimés, le taux de survie du patient est d'environ 14%,
+ pendant une période de temps de 120 mois après le diagnostic dudit cancer du poumon
▪ quand ladite tumeur est le carcinome épidermoïde :
o si aucun des 23 gènes dudit ensemble n'est exprimé, le taux de survie du patient est d'environ 58%
o si un des gènes de l'ensemble C est exprimé, le taux de survie du patient est d'environ 45%,
o si au moins deux des gènes de l'ensemble C sont exprimés, le taux de survie du patient est d'environ 14%.

5. Méthode de pronostic *in vitro* du taux de survie d'un patient atteint d'une tumeur du poumon, à partir d'un échantillon biologique contenant ladite tumeur du poumon appartenant au groupe consistant en l'adénocarcinome, le carcinome épidermoïde, les tumeurs basaloïdes et les tumeurs neuroendocrines à grandes cellules, à un temps de 30 à 120 mois après le diagnostic dudit cancer du poumon,
ladite méthode comprenant une étape de mesure, dans ledit échantillon biologique, de l'expression de
- au moins 4 gènes choisis parmi un groupe de 23 gènes comprenant ou consistant en les séquences d'acide nucléique SEQ ID NO 1 à 23,
- ou séquences complémentaires desdits gènes
lesdits au moins 4 gènes étant tels que
a. au moins un gène appartient à un premier ensemble A de 4 gènes comprenant ou consistant en les séquences d'acide nucléique SEQ ID NO : 1 à 4
b. au moins un gène appartient à un second ensemble B de 3 gènes comprenant ou consistant en les séquences d'acide nucléique SEQ ID NO : 5 à 7,
c. au moins un gène appartient à un troisième ensemble C de 6 gènes comprenant ou consistant en les séquences d'acide nucléique SEQ ID NO : 8 à 13, et
d. au moins un gène appartient à un quatrième ensemble D de 9 gènes comprenant ou consistant en les séquences d'acide nucléique SEQ ID NO : 14-23,
dans laquelle
- les séquences d'acide nucléique SEQ ID NO : 1 à 4 sont exprimées dans les tumeurs basaloïdes,
- les séquences d'acide nucléique SEQ ID NO: 5 à 7 sont exprimées dans l'adénocarcinome,
- les séquences d'acide nucléique SEQ ID NO : 8 à 13 sont exprimées dans le carcinome épidermoïde,
- les séquences d'acide nucléique SEQ ID NO : 14 à 23 sont exprimées dans les tumeurs neuroendocrines à grandes cellules,
ledit pronostic étant tel que :
+ pendant une période de temps de 30 mois après le diagnostic dudit cancer du poumon
▪ quand ladite tumeur est l'adénocarcinome :
o si aucun des 23 gènes dudit ensemble n'est exprimé, le taux de survie du patient est d'environ 86%,
o si au moins un des gènes de l'ensemble B est exprimé, le taux de survie du patient est d'environ 63%,
▪ quand ladite tumeur est les tumeurs basaloïdes :
o si aucun des 23 gènes dudit ensemble n'est exprimé, le taux de survie du patient est d'environ 65%,
o si au moins un des gènes de l'ensemble A est exprimé, le taux de survie du patient est d'environ 33%,
▪ quand ladite tumeur est le carcinome épidermoïde :
o si aucun des 23 gènes dudit ensemble n'est exprimé, le taux de survie du patient est d'environ 88%,
o si au moins un des gènes de l'ensemble C est exprimé, le taux de survie du patient est d'environ 64%,
▪ quand ladite tumeur est les tumeurs neuroendocrines à grandes cellules:
o si aucun des 23 gènes dudit ensemble n'est exprimé, le taux de survie du patient est d'environ 65%,
o si au moins un des gènes de l'ensemble D est exprimé, le taux de survie du patient est d'environ 7%,
ou,
+ pendant une période de temps de 60 mois après le diagnostic dudit cancer du poumon
▪ quand ladite tumeur est l'adénocarcinome :
o si aucun des 23 gènes dudit ensemble n'est exprimé, le taux de survie du patient est d'environ 82%,
o si au moins un des gènes de l'ensemble B est exprimé, le taux de survie du patient est d'environ 46%,
▪ quand ladite tumeur est les tumeurs basaloïdes :
o si aucun des 23 gènes dudit ensemble n'est exprimé, le taux de survie du patient est d'environ 35%,
o si au moins un des gènes de l'ensemble A est exprimé, le taux de survie du patient est d'environ 7%,
▪ quand ladite tumeur est le carcinome épidermoïde :
o si aucun des 23 gènes dudit ensemble n'est exprimé, le taux de survie du patient est d'environ 77%,
o si au moins un des gènes de l'ensemble C est exprimé, le taux de survie du patient est d'environ 42%
▪ quand ladite tumeur est les tumeurs neuroendocrines à grandes cellules:
o si aucun des 23 gènes dudit ensemble n'est exprimé, le taux de survie du patient est d'environ 55%,
o si au moins un des gènes de l'ensemble D est exprimé, le taux de survie du patient est d'environ 4%,
ou,
+ pendant une période de temps de 120 mois après le diagnostic dudit cancer du poumon
▪ quand ladite tumeur est l'adénocarcinome :
o si aucun des 23 gènes dudit ensemble n'est exprimé, le taux de survie du patient est d'environ 76%,
o si au moins un des gènes de l'ensemble B est exprimé, le taux de survie du patient est d'environ 39%,
▪ quand ladite tumeur est les tumeurs basaloïdes :
o si aucun des 23 gènes dudit ensemble n'est exprimé, le taux de survie du patient est d'environ 19%,
o si au moins un des gènes de l'ensemble A est exprimé, le taux de survie du patient est d'environ 0%,
▪ quand ladite tumeur est le carcinome épidermoïde :
o si aucun des 23 gènes dudit ensemble n'est exprimé, le taux de survie du patient est d'environ 58%,
o si au moins un des gènes de l'ensemble C est exprimé, le taux de survie du patient est d'environ 33%,
▪ quand ladite tumeur est les tumeurs neuroendocrines à grandes cellules:
o si aucun des 23 gènes dudit ensemble n'est exprimé, le taux de survie du patient est d'environ 40%,
o si au moins un des gènes de l'ensemble D est exprimé, le taux de survie du patient est d'environ 0%.

6. Méthode de pronostic, selon la revendication 5, dans laquelle
a. ledit au moins un gène appartient à un premier ensemble A de 4 gènes comprenant ou consistant en les séquences d'acide nucléique SEQ ID NO : 1 à 4, est choisi parmi les séquences d'acide nucléique SEQ ID NO : 1 à 3,
b. au moins un gène appartient à un second ensemble B de 3 gènes comprenant ou consistant en les séquences d'acide nucléique SEQ ID NO : 5 à 7, est choisi parmi les séquences d'acide nucléique SEQ ID NO : 5 ou 6,
c. au moins un gène appartient à un troisième ensemble C de 6 gènes comprenant ou consistant en les séquences d'acide nucléique SEQ ID NO : 8 à 13, est choisi parmi les séquences d'acide nucléique SEQ ID NO : 8 à 11, et
d. au moins un gène appartient à un quatrième ensemble D de 9 gènes comprenant ou consistant en les séquences d'acide nucléique SEQ ID NO : 14-23, est choisi parmi les séquences d'acide nucléique SEQ ID NO : 16 à 20.

7. Méthode de pronostic, selon la revendication 5 ou 6, dans laquelle
+ pendant une période de temps de 30 mois après le diagnostic dudit cancer du poumon
▪ quand ladite tumeur est le carcinome épidermoïde :
o si aucun des 23 gènes dudit ensemble n'est exprimé, le taux de survie du patient est d'environ 88%
o si un des gènes de l'ensemble C est exprimé, le taux de survie du patient est d'environ 73%,
o si au moins deux des gènes de l'ensemble C sont exprimés, le taux de survie du patient est d'environ 50%.
+ pendant une période de temps de 60 mois après le diagnostic dudit cancer du poumon
▪ quand ladite tumeur est le carcinome épidermoïde :
o si aucun des 23 gènes dudit ensemble n'est exprimé, le taux de survie du patient est d'environ 77%
o si un des gènes de l'ensemble C est exprimé, le taux de survie du patient est d'environ 59%,
o si au moins deux des gènes de l'ensemble C sont exprimés, le taux de survie du patient est d'environ 14%,
+ pendant une période de temps de 120 mois après le diagnostic dudit cancer du poumon
▪ quand ladite tumeur est le carcinome épidermoïde :
o si aucun des 23 gènes dudit ensemble n'est exprimé, le taux de survie du patient est d'environ 58%
o si un des gènes de l'ensemble C est exprimé, le taux de survie du patient est d'environ 45%,
o si au moins deux des gènes de l'ensemble C sont exprimés, le taux de survie du patient est d'environ 14%.

8. Méthode de pronostic, selon l'une des revendications 5 à 7, dans laquelle l'étape de mesure est réalisée en utilisant une technique choisie parmi l'ensemble consistant en :
a. PCR quantitative,
b. Puce à ADN, et
c. Northern blot.

9. Méthode de pronostic, selon l'une des revendications 5 à 8, dans laquelle l'étape de mesure est réalisée en utilisant des molécules d'acide nucléique consistant en des molécules de 15 à 100 nucléotides complémentaires desdits au moins 4 gènes.

10. Méthode de pronostic *in vitro* du taux de survie d'un patient atteint d'une tumeur du poumon, à partir d'un échantillon biologique contenant ladite tumeur du poumon, à un temps de 30 à 120 mois après le diagnostic dudit cancer du poumon,
ladite méthode comprenant une étape de mesure, dans ledit échantillon biologique, de l'expression de
- au moins 4 protéines choisies parmi un ensemble de 23 protéines codées par 23 gènes comprenant ou consistant en les séquences d'acide nucléique SEQ ID NO 1 à 23,
lesdites au moins 4 protéines étant telles que
a. au moins une protéine appartient à un premier ensemble AP de 4 protéines comprenant ou consistant en les séquences d'acide aminé SEQ ID NO : 24 -27,
b. au moins une protéine appartient à un second ensemble BP de 3 protéines comprenant ou consistant en les séquences d'acide aminé SEQ ID NO : 28 -30,
c. au moins une protéine appartient à un troisième ensemble CP de 6 protéines comprenant ou consistant en les séquences d'acide aminé SEQ ID NO : 31 -36,
d. au moins une protéine appartient à un quatrième ensemble DP de 9 protéines comprenant ou consistant en les séquences d'acide aminé SEQ ID NO : 37 -46,
dans laquelle
- les séquences d'acide aminé SEQ ID NO : 24 à 27 sont exprimées dans les tumeurs basaloïdes,
- les séquences d'acide aminé SEQ ID NO: 28 à 30 sont exprimées dans l'adénocarcinome,
- les séquences d'acide aminé SEQ ID NO : 31 à 36 sont exprimées dans le carcinome épidermoïde,
- les séquences d'acide aminé SEQ ID NO : 37 à 46 sont exprimées dans les tumeurs neuroendocrines à grandes cellules,
ladite méthode de pronostic étant telle que
+ pendant une période de temps de 30 mois après le diagnostic dudit cancer du poumon
▪ quand ladite tumeur est l'adénocarcinome :
o si aucune des 23 protéines dudit ensemble n'est exprimée, le taux de survie du patient est d'environ 86%,
o si au moins une des protéines de l'ensemble BP est exprimée, le taux de survie du patient est d'environ 63%,
▪ quand ladite tumeur est les tumeurs basaloïdes :
o si aucune des 23 protéines dudit ensemble n'est exprimée, le taux de survie du patient est d'environ 65%,
o si au moins une des protéines de l'ensemble AP est exprimée, le taux de survie du patient est d'environ 33%,
▪ quand ladite tumeur est le carcinome épidermoïde :
o si aucune des 23 protéines dudit ensemble n'est exprimée, le taux de survie du patient est d'environ 88%,
o si au moins une des protéines de l'ensemble CP est exprimée, le taux de survie du patient est d'environ 64%,
▪ quand ladite tumeur est les tumeurs neuroendocrines à grandes cellules:
o si aucune des 23 protéines dudit ensemble n'est exprimée, le taux de survie du patient est d'environ 65%,
o si au moins une des protéines de l'ensemble DP est exprimée, le taux de survie du patient est d'environ 7%,
ou,
+ pendant une période de temps de 60 mois après le diagnostic dudit cancer du poumon
▪ quand ladite tumeur est l'adénocarcinome :
o si aucune des 23 protéines dudit ensemble n'est exprimée, le taux de survie du patient est d'environ 82%,
o si au moins une des protéines de l'ensemble BP est exprimée, le taux de survie du patient est d'environ 46%,
▪ quand ladite tumeur est les tumeurs basaloïdes :
o si aucune des 23 protéines dudit ensemble n'est exprimée, le taux de survie du patient est d'environ 35%,
o si au moins une des protéines de l'ensemble AP est exprimée, le taux de survie du patient est d'environ 7%,
▪ quand ladite tumeur est le carcinome épidermoïde :
o si aucune des 23 protéines dudit ensemble n'est exprimée, le taux de survie du patient est d'environ 77%,
o si au moins une des protéines de l'ensemble CP est exprimée, le taux de survie du patient est d'environ 42%
▪ quand ladite tumeur est les tumeurs neuroendocrines à grandes cellules:
o si aucune des 23 protéines dudit ensemble n'est exprimée, le taux de survie du patient est d'environ 55%,
o si au moins une des protéines de l'ensemble DP est exprimée, le taux de survie du patient est d'environ 4%,
ou,
+ pendant une période de temps de 120 mois après le diagnostic dudit cancer du poumon
▪ quand ladite tumeur est l'adénocarcinome :
o si aucune des 23 protéines dudit ensemble n'est exprimée, le taux de survie du patient est d'environ 76%,
o si au moins une des protéines de l'ensemble BP est exprimée, le taux de survie du patient est d'environ 39%,
▪ quand ladite tumeur est les tumeurs basaloïdes :
o si aucune des 23 protéines dudit ensemble n'est exprimée, le taux de survie du patient est d'environ 19%,
o si au moins une des protéines de l'ensemble AP est exprimée, le taux de survie du patient est d'environ 0%,
▪ quand ladite tumeur est le carcinome épidermoïde :
o si aucune des 23 protéines dudit ensemble n'est exprimée, le taux de survie du patient est d'environ 58%,
o si au moins une des protéines de l'ensemble CP est exprimée, le taux de survie du patient est d'environ 33%,
▪ quand ladite tumeur est les tumeurs neuroendocrines à grandes cellules:
o si aucune des 23 protéines dudit ensemble n'est exprimée, le taux de survie du patient est d'environ 40%,
o si au moins une des protéines de l'ensemble DP est exprimée, le taux de survie du patient est d'environ 0%.

11. Méthode de pronostic, selon la revendication 10, dans laquelle
e. la dite au moins une protéine appartient à un premier ensemble AP de 4 protéines comprenant ou consistant en les séquences d'acide aminé SEQ ID NO : 24 à 27, est choisie parmi les séquences d'acide aminé SEQ ID NO : 24 à 27,
f. au moins une protéine appartient à un second ensemble BP de 3 protéines comprenant ou consistant en les séquences d'acide aminé SEQ ID NO : 28 à 30, est choisie parmi les séquences d'acide aminé SEQ ID NO : 28 ou 29,
g. au moins une protéine appartient à un troisième ensemble CP de 6 protéines comprenant ou consistant en les séquences d'acide aminé SEQ ID NO : 31 à 36, est choisie parmi les séquences d'acide aminé SEQ ID NO : 31 à 34, et
h. au moins une protéine appartient à un quatrième ensemble DP de 9 protéines comprenant ou consistant en les séquences d'acide aminé SEQ ID NO : 37 à 46, est choisie parmi les séquences d'acide aminé SEQ ID NO : 37 à 43.

12. Méthode de pronostic, selon la revendication 10 ou 11, dans laquelle
+ pendant une période de temps de 30 mois après le diagnostic dudit cancer du poumon
▪ quand ladite tumeur est le carcinome épidermoïde :
o si aucune des 23 protéines dudit ensemble n'est exprimée, le taux de survie du patient est d'environ 88%
o si une des protéines de l'ensemble CP est exprimée, le taux de survie du patient est d'environ 73%,
o si au moins deux des protéines de l'ensemble CP sont exprimées, le taux de survie du patient est d'environ 50%.
+ pendant une période de temps de 60 mois après le diagnostic dudit cancer du poumon
quand ladite tumeur est le carcinome épidermoïde :
o si aucune des 23 protéines dudit ensemble n'est exprimée, le taux de survie du patient est d'environ 77%
o si une des protéines de l'ensemble CP est exprimée, le taux de survie du patient est d'environ 59%,
o si au moins deux des protéines de l'ensemble CP sont exprimées, le taux de survie du patient est d'environ 14%,
+ pendant une période de temps de 120 mois après le diagnostic dudit cancer du poumon
▪ quand ladite tumeur est le carcinome épidermoïde :
o si aucune des 23 protéines dudit ensemble n'est exprimée, le taux de survie dù patient est d'environ 58%,
o si une des protéines de l'ensemble CP est exprimée, le taux de survie du patient est d'environ 45%,
o si au moins deux des protéines de l'ensemble CP sont exprimées, le taux de survie du patient est d'environ 14%.

13. Méthode de pronostic, selon l'une des revendications 10 à 12, dans laquelle l'étape de mesure est réalisée en utilisant une technique choisie parmi l'ensemble consistant en :
a. werstern Blot,
b. ELISA,
c. Immunofluorescence, et
d. Immunohistochimie.

14. Méthode de pronostic, selon l'une des revendications 10 à 13, dans laquelle l'étape de mesure est réalisée en utilisant des anticorps dirigés contre lesdites au moins 2 protéines codées par lesdits au moins deux gènes.

15. Méthode de pronostic, selon l'une des revendications 10 à 14, comprenant de plus une étape de comparaison de ladite expression mesurée à l'expression dans au moins un échantillon contrôle.
